# EUROPEAN PATENT SPECIFICATION

(11) **EP 1 481 062 B1**
(45) Date of publication and mention of the grant of the patent: **28.04.2010**
(21) Application number: 03724923.2
(22) Date of filing: 06.03.2003
(51) Int. Cl.: C12N 15/31, C12N 15/63, C07K 14/245, C07K 16/12, A61K 39/108, G01N 33/53

(54) **EXPEC-SPECIFIC PROTEINS, GENES ENCODING THEM AND USES THEREOF**
EXPEC-SPEZIFISCHE PROTEINE, ENTSPRECHENDE GENE UND IHRE VERWENDUNGEN
PROTEINES DE SOUCHES PATHOGENES EXTRAINTESTINALES DE E. COLI, GENES LES-CODANT ET LEUR UTILISATION

(30) Priority: 06.03.2002 EP 02290556
(43) Date of publication of application: 01.12.2004
(73) Proprietor: Mutabilis S.A., 75015 Paris (FR)
(72) Inventor: ESCAICH, Sonia, F-75012 Paris (FR)
(74) Representative: Peaucelle, Chantal
(86) International application number: PCT/EP2003/002925
(87) International publication number: WO 2003/074553

(56) References cited:
- WO-A-01/66572
- WO-A-01/66572
- DATABASE UniProt EBI; 1 March 2005 (2005-03-01), "Putative dihydrolipoamide dehydrogenase CglE of E. coli" XP002330780 Database accession no. Q8VP34
- DATABASE UniProt EBI; 1 November 1996 (1996-11-01), "FyuA precursor of E. coli" XP002330781 Database accession no. Q47230
- DATABASE UniProt EBI; 1 March 2002 (2002-03-01), "Putative macrophage toxin of E. coli." XP002330782 Database accession no. Q8X7W9
- JOHNSON JAMES R ET AL: "Phylogenetic and pathotypic similarities between Escherichia coli isolates from urinary tract infections in dogs and extraintestinal infections in humans." JOURNAL OF INFECTIOUS DISEASES, vol. 183, no. 6, 2001, pages 897-906, XP002211433 ISSN: 0022-1899
- MUEHLDORFER I ET AL: "Characterization of Escherichia coli strains isolated from environmental water habitats and from stool samples of healthy volunteers." RESEARCH IN MICROBIOLOGY, vol. 147, no. 8, 1996, pages 625-635, XP002211434 ISSN: 0923-2508
- JOHNSON JAMES R ET AL: "Phylogenetic and pathotypic similarities between Escherichia coli isolates from urinary tract infections in dogs and extraintestinal infections in humans." JOURNAL OF INFECTIOUS DISEASES, vol. 183, no. 6, 2001, pages 897-906, XP002211433 ISSN: 0022-1899
- MUEHLDORFER I ET AL: "Characterization of Escherichia coli strains isolated from environmental water habitats and from stool samples of healthy volunteers." RESEARCH IN MICROBIOLOGY, vol. 147, no. 8, 1996, pages 625-635, XP002211434 ISSN: 0923-2508

## Description

The invention relates to new products specific to pathogenic strains, particularly to extra-intestinal *E. coli* strains.

It more particularly relates as products to antigenic polypeptides and antibodies directed against said polypeptides and to their use as vaccines and in immunotherapy, respectively.

Although *Escherichia coli* is probably the best known bacterial species and is one of the most common isolated in clinical microbiology laboratories, misconceptions abound regarding the various types of *E. coli* and the infections they cause.

*E. coli* strains of biological significance to humans can be broadly classified in 3 major groups:
1. Commensal strains, which are part of the normal flora.
2. Intestinal pathogenic strains, which are not part of the normal flora. This group contains various pathotypes (EPEC, EHEC, ETEC, EIEC) not including *Shigella.*
3. Extra-intestinal strains (ExPEC) which are responsible for infections outside the gastro-intestinal (GI) tract, but can also be part of the normal flora. All hosts, either immunocompromised or not are susceptible to these infections.

ExPEC strains are responsible for the majority of the urinary tract infections (UTI) particularly cystitis, pyelonephritis, and cathether associated infections.

They are also responsible for abdominal infections, nosocomial pneumoniae, neonatal meningitidis, soft tissue infections, and bone infections. Each one of these localizations can lead to bacteremia with a risk of sepsis in case of organ failure. ExPEC strains are indeed the most common Gram negative bacilli isolated from blood cultures.

750 000 cases of bacterial sepsis occur each year in the US, and are responsible for 225 000 deaths. In a recent study on 1690 cases of sepsis, it was shown that the main bacteria species identified is ExPEC (16% of the cases) and then *S.aureus* (14% of the cases).

These numbers demonstrate the importance of ExPEC strains in both hospital and community acquired infections.

ExPEC strains correspond to a homogenous subset of *E. coli* strains. Analysis of phylogenetic relationships among *E. coli* strains by MLEE has revealed that *E. coli* belong to 4 main phylogenetic groups designated A, B1, B2 and D.

The pathogenesis of ExPEC strains is that of extra-cellular microorganisms, i.e., they are well adapted to growth in the extra-cellular fluids and efficiently resist phagocytosis by polymorphonuclear. Initial studies have shown that virulence factors known to be important for the extra-cellular growth are mainly found in B2/D *E. coli*., thus suggesting that B2/D subgroups contain most of the ExPEC strains. This was reinforced by experiments performed on animals showing that B2/D strains are more virulent than A and B1 strains. Subsequent epidemiological studies have indeed confirmed these hypotheses. B2/D isolates are those predominantly responsible for neonatal meningitidis (87%) and community or nosocomial acquired urosepsis, (93 % and 85%, respectively).

Similar results have been reported for cystitis (70% are due to the sole B2 *E. coli*), thus demonstrating that the importance of ExPEC strains.

These recent findings demonstrate that the B2/D subgroup of strains is the *E. coli* core genome the best adapted to growth in extra-cellular fluids.

In addition to this core genome, ExPEC strains have various pathogenicity islands which encode virulence factors associated with the different pathogenesis of extra-intestinal *E. coli* infections (UTI, urosepsis, neonatal meningotidis...). Among the main virulence factors are the capsule, which is well-known to be important for extra-cellular growth, and the iron chelation systems (aerobactin and enterochelin, for example). In addition, depending on the pathogenesis, these strains can produce toxins (CNF, hemolysin...), adhesins (pap, sfa...) and other iron chelation systems.

The notion that B2/D *E. coli* correspond to a distinct subset of pathogenic *E. coli* strains is reinforced by the fact that B2/D *E. coli* are not broadly isolated from the stools of humans. They were recovered from only 11% of individuals, whereas A and B1 subgroups are present in the stools of 74% of the individuals of a human population.

As mentioned above the pathogenesis of ExPEC strains relies on their ability to multiply in the extra-cellular fluids and to resist bactericidal activity of the complement and phagocytosis by polymorphonuclear. Therefore, as for other extra-cellular pathogens (*Haemophilus influenzae, Streptococcus* pneumonieae and Neisseria meningitidis) a protective antigen against ExPEC has to induce antibodies that promote opsonisation and/or the bactericidal activity of serum.

Considering the above statements, an efficient antigen has to be largely represented among the population of B2/D *E. coli.*

Similarly to other extra-cellular pathogens, the capsular polysaccharide would be an ideal antigen, however most pathogenic B2 strains express the K1 polysaccharide. The latter has a structure identical to that of group B meningococcus, which is non-immunogenic and shares common antigens with the brain. Another possible target may be the lipopolysaccharide (LPS). However there are a large number of different LPS serotypes that are shared by various subgroups.

WO 01/66572 relates to polypeptides which are specific for the *E. coli* subgroups B2/D and genes encoding such polypeptides.

The inventors have now found that some specific components coded by the B2/D genome, but absent from A and B1 *E. coli* strains, are particularly useful as antigens and can specifically prevent the pathologies due to ExPEC strains. Homologs of these antigenic components can be found in other pathogenic bacterial species and therefore are useful to prevent the pathologies caused by these bacteria. Accordingly, any reference to products specific to ExPEC strains and to their uses will encompass components in these species.

For example homologuous antigens could be present in the following species and be as such used for prevention of disease due to the bacteria:
*Pseudomonas aeruginosa, Escherichia coli* O157:H7, *Yersinia pestis, Vibrio cholerae, Legionella pneumophila, Salmonella enterica, Salmonella typhimurium, Haemophilus influenzae, Neisseria meningitidis, Neisseria gonorrhoeae, Bacillus anthracis, Burkholderia cepacia, Campylobacter jejuni, Chlamydia pneumoniae, Chlamydia trachomatis, Clostridium botulinum, Clostridium difficile, Cryptococcus neoformans, Enterobacter cloacae, Enterococcus faecalis, Helicobacter pylori, Klebsiella pneumoniae, Mycobacterium leprae, Mycobacterium tuberculosis, Pseudomonas aeruginosa, Salmonella paratyphi, Salmonella typhi, Staphylococcus aureus, Klebsiella pneumoniae, Listeria monocytogenes, Moxarella catarrhalis, Shigella dysenteriae, Shigella flexneri, Shigella sonnei, Staphylococcus epidermidis, Streptococcus pneumoniae,* and any species falling within the genera of any of the above species.

It is then an object of the invention to provide new isolated antigenic polypeptides, and polynucleotides belonging to the core B2/D genome and not present in commensal *E. coli.*

Another object of the invention is to provide antibodies raised against such antigenic polypeptides, or peptidic fragments.

It is still another object of the invention to provide vectors and host cells containing said polynucleotides.

Another object of the invention is to provide vaccine compositions specific to extra intestinal infections caused by ExPEC and pathologies caused by other pathogenic strains expressing antigenic polypeptides homologous to the ExPEC antigenic polypeptides.

The invention also relates to means for detecting and treating a development of *E. coli* in a human or animal compartment which is extra-intestinal (systemic and non-diarrhoeal infections, such as septicaemia, pyelonephritis, or meningitis in the newborn).

The isolated antigenic polypeptides used according to the invention are selected among polypeptides specific to B2/D *E. coli* strains and not present in A and B1 isolates of *E. coli*. They are encoded by genes belonging to the core B2/D genome and are not present in commensal *E. coli.*

They have a sequence selected in the group comprising the sequences of SEQ ID N°11 to N°66 or 133-145 or homologous sequences with a minimum of 25% of identity with the whole sequences SEQ ID N°11 to N°66, or 133-145, respectively.

The isolated polypeptides having SEQ ID N° 14, 15, 17, 21, 22, 23, 28, 29, 30, 32, 36, 38, 39, 41-44, 46, 49, 50, 52 to 55, 58, 60, 63, 133-138 are new polypeptides and therefore are part of the invention.

The invention also relates to homologous isolated antigenic peptides, comprising polypeptides having at least 25% identity to a polypeptide having a sequence SEQ ID N° as above defined, more particularly having SEQ ID N°14, 15, 17, 21, 22, 23, 28, 29, 30, 32, 36, 38, 39, 41-44, 46, 49, 50, 52 to 55, 58, 60, 63, 133-138, or at least 25% identity to a fragment comprising at least 5, at least 10, at least 20, at least 30, at least 40, at least 50, at least 60 or more than 60 consecutive amino acids of a polypeptide having a sequence corresponding to said SEQ ID N°s, as determined using BLASTP or BLASTX with the default parameters.

Said polypeptides are obtainable by a process comprising the steps of :
a- selecting on the basis of sequence analysis those of the polypeptides which are either located in the outermembrane or secreted by the bacteria,
b- identifying the genes coding for said polypeptides which are conserved in B2/D clinical isolates,
c- purifying the polypeptides identified in step a, which are found in step 2 to be conserved in the B2/D isolates,
d- testing the polypeptides for immunogenicity using animals models.

By the term "conserved", it is meant, according to the invention, that the genes coding for the polypeptides are present with a frequency of at least 50% in B2/D isolates, preferably greater than 60%, more preferably greater than 80% and even more preferably greater than 85%, and in less than 40% in A/B isolates, preferably in less than 20%, more preferably in less than 15%.

The animal models used in step c are infected adult animals, eventually immunodepressed.

The adult animals particularly mice, are infected intraperitoneally, the endpoint being the animal death and/or bacteremia measurement.

The animals can be immunodepressed by injection, for example, of cyclophosphamide which induces a neutropenia. Such a model will validate the use of the antigen for prevention of *E. coli* sepsis in immunodepressed patients. Another animal model could be for example 2 to 3 day old infant mice.

The variants or fractionnal sequences conserving the B2/D properties and which are antigenic as defined in step 4 of the above process are also part of the invention. The term "variant" is herein intended to mean any sequence having insertions and/or deletions and/or substitutions with respect to the parent sequence. The term "fractional" is herein intended to mean any fragment of the parent sequence.

The invention also relates to the use of isolated polynucleotides coding for a polypeptide such as above defined according to the universal genetic code and taking into account the degeneracy of this code. The term "polynucleotide" encompasses any nucleotidic sequence such as DNA, including cDNA, RNA, including mRNA.

Said polynucleotides have preferably sequences corresponding to SEQ ID N°77 to SEQ ID N°132 or 146 to 158.

More preferably, said polynucleotides have sequences corresponding to SEQ ID N° 80, 81, 83, 87, 88, 89, 94, 95, 96, 98, 102, 104, 105, 107-110, 112, 115, 116, 118, 119, 126, 127, 130, 132, 135, 146-151.

The invention also relates to the homologs to said polynucleotides . Said homologs may have at least 25% identity to a polynucleotide having said sequences, or at least 25% identity to a fragment comprising at least 15, at least 30, at least 60, at least 90, at least 120, at least 150, at least 180 or more than 180 consecutive nucleotide of a polynucleotide having one of said SEQ ID N°s, as determined using BLASTN with the default parameters, and are encompassed by the invention inasmuch as they are capable of coding for a polypeptide having the antigenic properties of those according to the invention.

The present application is also aimed towards any vector comprising at least one of said polynucleotides and also any cell transformed by genetic engineering, **characterized in that** it comprises, by transfection, at least one of said polynucleotides and/or at least one vector according to the invention, and/or in that said transformation induces the production by this cell of at least one polypeptide corresponding to a polynucleotide such as above-defined.

The invention also relates to a process for isolating and identifying antigenic polypeptides, therefore useful as vaccine for *E. coli*.

Such a process comprises the steps of
a- selecting on the basis of sequence analysis those of the polypeptides which are either located in the outermembrane or secreted by the bacteria,
b- identifying the genes coding for said polypeptides which are conserved in B2/D clinical isolates,
c- purifying the polypeptides identified in step a, which are found in step 2 to be conserved in B2/D isolates,
d- testing the polypeptides for immunogenicity using animals models.

The selected antigenic polypeptides, alone or in combination, are capable of inducing an antibody response for prevention of infections due to ExPEC strains regardless of the pathogenesis and of the infection site (UTI, pyelonephritis, sepsis, bacteremia, neonatal meningitis).

Such polypeptides particularly have sequences SEQ ID N°1 to SEQ ID N°66, or 133-145 or correspond to homologous sequences.

The invention thus relates to vaccine compositions specific to *E. coli* extra-intestinal infections, comprising an effective amount of at least one antigenic polypeptide or fragment thereof as above defined, with a carrier, particularly at least one polypeptide of SEQ ID N°1 to SEQ ID N°66. except SEQ ID N°8, and 133-145 and the homologous polypeptides.

Such vaccine compositions are particularly useful for preventing urinary system infections, pyelonephritis, sepsis, bacteremia, neonatal meningitis.

The vaccine compositions of the invention are indicated for :
- immunodepressed patients, ideally before the start of the immunosuppressive therapy : patients suffering from cancer, diabetes, leukaemia, transplant patients, patients receiving long-term steroids therapy.
- Patients before surgery where there is a high risk of *E. coli* infections (abdominal surgery).
- In all these cases, the *E. coli* vaccine of the invention could be administered in association with a *Staphylococcus aureus* vaccine,
- Patients with recurrent UTI, especially after one episode of pyelonephritis.
- The prevention of neonatal infections will require vaccination of the mother, implying vaccination long before pregnancy to avoid potential problem. Ideally such a vaccine should be associated with a Group B *Streptococcus* polysaccharide vaccine in order to also prevent late onset neonatal infections. It should be pointed out that the induction of a level of antibodies against B2/D *E. coli* in pregnant women would also prevent UTI, which are always a risk in the context of a pregnancy.

The formulation and the dose of said vaccine compositions can be developped and adjusted by those skilled in the art as a function of the indication targeted, of the method of administration desired, and of the patient under consideration (age, weight).

These compositions comprise one or more physiologically inert vehicles, and in particular any excipient suitable for the formulation and/or for the method of administration desired.

For example the vaccine could be a suspension of the purified polypeptide in sterile water with aluminium based miniral salt as adjuvant and be administred subcutanously with a first and boosting injection.

The antibodies raised against the above-identified polypeptides are also part of the invention.

They are capable of binding to said polypeptides in physiological-type conditions (in vivo or mimicking in vivo) when administered to a human or animal organism, and ELISA-type conditions when said binding product is intended to be used in assays and methods *in vitro.* Such antibodies advantageously inhibit the extra-intestinal growth of ExPEX strains in human or animal.

They are particularly useful for immunotherapy applications with antibodies specific to polypeptidic antigens, for treatment and prevention of severe infections in at risk populations such as neonates or patients undergoing surgical procedures. For these applications specific human monoclonal antibody (Mab) will be derived from the peptides or polypeptides.

The methods for manufacturing such antibodies using the polypeptides according to the invention are available to those skilled in the art. They are conventional methods which comprise, in particular, the immunization of animals such as rabbits and the harvesting of the serum produced, followed optionally by the purification of the serum obtained. A technique suitable for the production of monoclonal antibodies is that of Köhler and Milstein (Nature 1975, 256:495-497).

Said antibodies do not recognize the cells of the human or animal to which it is intended.

In particular for immunotherapy applications with monoclonal antibodies specific to polypeptidic antigens, for treatment and prevention of severe infections in at risk populations such as neonates or patients undergoing surgical procedures. For these applications specific human monoclonal antibody will be derived from the peptides or polypeptides.

The antibodies or fragments thereof are advantageoulsy humanized when intended for a human administration.

Alternatively, humanized Mab could be derived from murine or rat Mab specific of the antigen. These fully humanized Mab are constructed using conventional molecular techniques to graft complementarity-determining regions from the parent murine or rat antibacterial antibody into human IgG1 kappa heavy and light-chain frameworks.

The present invention is also aimed towards the use, in an effective amount, of at least one of polypeptides having SEQ ID N°14, 15, 17, 21, 22, 23, 28, 29, 30, 32, 36, 38, 39, 41-44, 46, 49, 50, 52 to 55, 58, 60, 63, 133-138, antibodies or polynucleotides for the diagnosis of the presence or absence of undesirable extra-intestinal *E. coli*, and/or for the diagnosis of an extra-intestinal *E. coli* infection.

The detection of the presence or absence of such compounds can in particular be carried out by nucleotide hybridization, by PCR amplification or by detection of their polypeptide products. Detection of the presence of such compounds makes it possible to conclude that a B2/D *E. coli* strain is present.

The invention also relates to pharmaceutical compositions for alleviating and/or preventing and/or treating an undesirable growth of *E. coli* comprising an effective amount of at least one polypeptide as above defined, particularly having SEQ ID N°1-66 to 133-145, in combination with a pharmaceutically acceptable carrier.

Preferred pharmaceutical compositions comprise at least one polypeptide having SEQ ID N°14, 15, 17, 21, 22, 23, 28, 29, 30, 32, 36, 38, 39, 41-44, 46, 49, 50, 52 to 55, 58, 60, 63, 133-138,

The present application is also aimed towards any use of a polypeptide such as above defined for the manufacture of a composition, in particular of a pharmaceutical composition, intended to alleviate and/or to prevent and/or to treat an undesirable growth of *E. coli,* such as an *E. coli* infection, (for example systemic and non-diarrhoeal infections), the presence of extra-intestinal *E. coli* or a sanitary contamination.

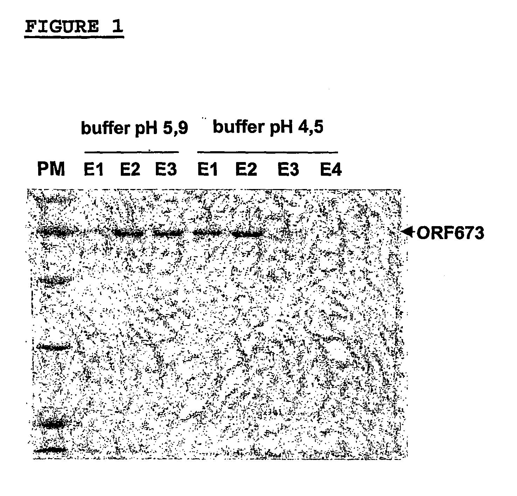

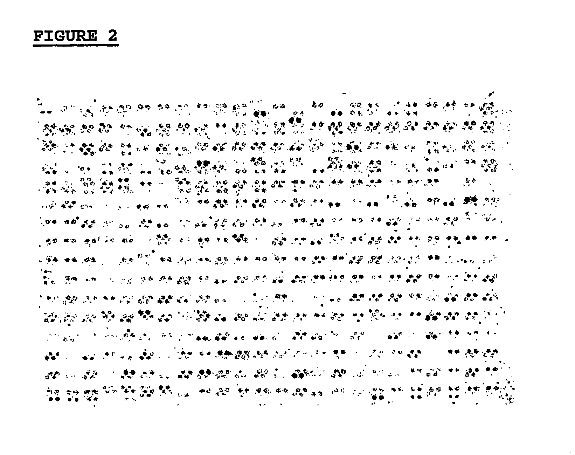

The present invention is illustrated by the examples which follow and which are given and with reference to figures 1 and 2, wherein
- Figure 1 represents a protein purification result after cloning and expression, and
- Figure 2 is a picture of the DNA array after hybridization with the genomic DNA from a B2/D reference strain.

### Example 1: Assay for the immunogenicity of a selected polypeptide from sequences 1-66 and 133-145 (except SEQ ID N°8)

### . Cloning expression and purification of the selected polypeptide.

The nucleic acid having SEQ ID N°95 encoding the polypeptide corresponding to SEQ ID N°28 was cloned without the signal sequence (coding the 16 first amino acids) in a prokaryotic expression vector according to classical methods for cloning. The recombinant plasmid was used to transform the *E. coli* strain BL21. Transformed cells containing the recombinant plasmid were selected in LB medium with 100µg/ml ampicillin. Individual clones are picked and grown in presence of IPTG 1mM to induce recombinant protein expression. Total protein content of the culture cells was extracted by cell lysis. Recombinant protein was purified by affinity columns.

### Protein purification after cloning and expression

Total cell lysat of IPTG-induced bacteria were mixed with Ni-NTA matrix (Qiagen^{®}) for 60 min et 4°C and loaded into a column. After washing the column to remove non specific binding, the recombinant protein was eluate 3 times with 1 ml elution buffer pH 5.9. The protein was then eluate 4 times with 1 ml elution buffer pH 4.5.

Figure 1 represents a Coomassie blue stained SDS gel of recombinant protein after affinity column purification: PM: markers **E1-4:** sample collected from each purification fraction. Arrow indicate the band corresponding to the recombinant protein.

### . Test for immunogenicity in an animal model

Polypeptide preparation from SEQ ID N°28 was injected to Swiss mice to induce an antibody response as follows :

At d0 a first immunisation was done by injecting 20µg of the protein at in 100µg solution of PBS and complet Freund adjuvant (1:1). Control animals were injected with 100µl solution of PBS and complet Freund adjuvant (1:1).

Boosting injection at d21 with 10µg of protein in 100µl PBS and complet Freund adjuvant (1:1).

Sera from vaccinated animals was prepared from blood drawn by puncture in the tail of the mice.

Detection of specific antibodies in animal sera, at d20 before the boosting injection, was performed by western blot according to standart protocol. Purified polypeptide was subjected to electrophoresis (10µg per lane) and transfert to nitrocellulose membrane.

The membranes were then saturated by incubation 35 min with PBS/Tween20 0.1%/powder milk 5%.

Diluted sera was incubated with the membrane for 45 min. Membranes were washed three time 5 min with PBS/tween. Bound antibodies were then recognized by an anti-mouse IgG coupled to horseradish peroxidase enzyme. After washing 3 times with PBS/Tween and 3 time with PBS, enzymatic activity was revealed by addition of chromogenic substrate DAB and hydrogen peroxyde.

Results : Sera from vaccinated animal, diluted at 1/100 revealed a unique band corresponding to the injected polypeptide. No antibody to the polypeptide could be detected in sera from control animals.

At d42, 300 µl of cyclophosphamide and 200µl at d45 were injected IP in the mice to induce neutropenia in order to increase the susceptibility to the challenge infection. At d46 vaccinated and control mice were challenged by intraperitoneal injection of the wt B2/D strain C5 of *E. coli* at a dose equal to 10 time the LD50 (letal dose).

Immunogenicity of the selected polypeptide and protection conferred by vaccination with the seleted polypeptide was assessed by the survival of vaccinated animals three days post challenge.

Another example of vaccination to demonstrate immunogenicity of polypeptides:
- 24 Balb/c mice, female, 6 weeks old were immunized by subcutaneous Injection of the protein in Complete Freund's adjuvant, and 14 control mice were injected with CFA and PBS
- 3 weeks later a boost injection of 10 µg of protein in with incomplete Freund's adjuvant.

### VACCINATION PROTOCOL

Before challenge at d62, sera was collected at d61 to analyze the antibody response in the vaccinated animals.
• WB analysis of sera from immunized mice were performed to detect the antibody response to the recombinant proteine used for immunization as described above.
• An ELISA assay was set up to measure antibody titer in vaccinated animals:
- Each recombinant protein was coated on a 96-well plates with (200 ng/100 µl coating buffer), plates are saturat with 3%BSA in PBS.
- Twofold serial dilution of sera were made in PBS 1X/1% BSA from 1:500, to 1: 1024000 and incubated on the plate, antiboby binding was reavealed using a rabbit Anti-mouse IgG conjugated with Peroxydase and OPD(o-phenylenediamine) chromogen substrate.
- Read the OD₄₉₅ with Sanofi Diagnostics Pasteur PR2100^{®}

• Results:

**Table 1. Sera titer against recombinant protein by ELISA**

| **SEQ ID** | **serum1** | **serum 2** | **serum 3** | **serum 4** | **Control** | **recombinant protein** |
|---|---|---|---|---|---|---|
| **2** | **128** | **64** | **128** | **512** | **<0,5** | |
| **140** | **>64** | **>64** | **>64** | **>64** | **<0,5** | **+** |
| **31** | **>64** | **>64** | **>64** | **>64** | **<0,5** | **+** |
| **49** | **>64** | **>64** | **>64** | **>64** | **<0,5** | **+** |
| **51** | **>64** | **>64** | **>64** | **>64** | **<0,5** | **+** |
| **25** | **>64** | **>64** | **>64** | **>64** | **<0,5** | **+** |
| **7** | **16** | **8** | **>64** | **<0,5** | **<0,5** | **+** |
| **19** | **>64** | **>64** | **>64** | **>64** | **0,5** | **+** |
| **3** | **>64** | **>64** | **>64** | **>64** | **<0,5** | **+** |
| **26** | **512** | **128** | **64** | **256** | **<0,5** | **+** |
| **18** | **>64** | **>64** | **>64** | **>64** | **0,5** | **+** |
| **32** | **>64** | **>64** | **>64** | **>64** | **0,5** | **+** |
| **53** | **>64** | **>64** | **>64** | **>64** | **0,5** | **+** |
| **587** | **>64** | **16** | **32** | **32** | **<0,5** | **+** |
| **11** | **>64** | **32** | **32** | **64** | **<0,5** | **+** |
| **36** | **512** | **256** | **512** | **256** | **<0,5** | **+** |
| **10** | **32** | **128** | **256** | **128** | **0,5** | **+** |
| **47** | **512** | **512** | **512** | **512** | **0,5** | **+** |
| **20** | **1024** | **256** | **256** | **512** | **<0,5** | **+** |
| **17** | **1024** | **512** | **128** | **512** | **<0,5** | **+** |

• To assess the' ability of sera to recognize the native antigen expressed by the bacteria, western blot was also performed on whole bacteria lysat.
To this end, bacteria were grown in LB medium supplemented or not with iron chelator until OD600=0.5-0.6 and pelleted by centrifugation 5 minutes at 10000 rpm. The pellet was lysed by resuspension in 1X loading buffer containing SDS and heated 5 min at 95°C before migration on the gel.Western blot assay was then performed with sera from controls and vaccinated animals. Results in table 2 shows the results obtained with Sera from vaccinated mice against recombinant protein and against *E.coli* lysat.

**Table 2: reactivity in Western Blot of sera from mice vaccinated with polypeptides encoded by the different ORFs**

| **SEQ ID N°** | **whole cell lysate** | **recombinant protein** |
|---|---|---|
| **2** | **+** | **+** |
| **140** | **+** | **+** |
| **26** | **+** | **+** |

• Protection assay, end point: mortality
At d62, 20 vaccinated and 10 control mice were challenged with an *E.coli* virulent strain belonging to B2 group at a dose equal to the LD 50 (5.10⁵ cfu/mice) by intraperitoneal injection. Mortality is recorded at 48h, results in Table 3 are expressed as a percentage of protection representing the difference of survival in vaccinated versus control mice goups.

**Table 3 : Protection obtained in mice challenged after immunization with proteines encoded by the corresponding ORFs.**

| **SEQ ID N°** | **% protection** |
|---|---|
| **2** | **52** |
| **26** | **66** |
| **36** | **46** |
| **10** | **30** |
| **47** | **60** |
| **20** | **25** |

• Protection assay, end point: bacteremia
At d62, 10 vaccinated and 5 control mice were challenged with an Ecoli virulent strain belonging to B2 group at a dose equal to the 1/5 of the LD 50 (1.10⁵ cfu/mice) by intraperitoneal injection.With this infectious dose the mice survived the infection at d48. At 48h blood was collected for each mice in presence of heparin .To assess bacteremia , the blood was plated on LB media and colonies count measured after overnight culture.

### Example 2: Distribution of the DNA sequence of ORFs specific for B2/D group of E.coli in clinical isolates.

To make a DNA arrays membrane specific for B2/D group of *E.coli*, DNA corresponding to ORFs that were identified as specific for B2/D core genome of *E.coli* was amplified by PCR and spotted on nylon membrane using standard methods to those skilled in the art.

Chromosomal DNA from 30 *E.coli* clinical isolate strains ( of which 23 were from pathological conditions and 6 isolated from human normal flora), was prepared and radiolabelled with 33P.

DNA from these clinical isolates was then hybridized to the B2/D specific DNA array, the results were read by a phosphoimager and spots reactivity was analyzed with an image analysis software. If hybridization gave a positive signal for a particular ORF, this ORf is considered to be present in the genome of the isolate. Quality control of the array is the hybridization of a probe DNA from a reference strain of *E.coli* as shown in figure 2, which represents a picture of the DNA array after hybridization with the genomic DNA from a B2/D reference strain.

The details of the method used for these experiments has been described previously in Tinsley et al. Methods Enzymol. 2002, 358; 188-207.

Results presented in Table 4 are expressed as the frequency of each ORF detection in the three different group A, B and D of *E.coli* strains.

**Table 4 :Presence of Orfs encoding antigens in E.coli clinical isolate genomes**

| | Clinical isolate groups | | |
|---|---|---|---|
| Frequency of positive signal % | A | D | B2 |
| SEQ ID N° | N=6 | n=5 | n=18 |
| 86 | 17 | 20 | 100 |
| 119 | 0 | 80 | 100 |
| 137 | 0 | 0 | 100 |
| 77 | 0 | 100 | 94 |
| 78 | 0 | 100 | 100 |
| 79 | 0 | 100 | 100 |
| 80 | 0 | 0 | 100 |
| 84 | 0 | 0 | 100 |
| 82 | 0 | 0 | 100 |
| 88 | 0 | 0 | 100 |
| 83 | 0 | 0 | 94 |
| 85 | 0 | 0 | 78 |
| 88 | 0 | 0 | 56 |
| 81 | 33 | 20 | 100 |
| 89 | 33 | 60 | 67 |
| 90 | 0 | 0 | 61 |
| 91 | 17 | 80 | 67 |
| 94 | 0 | 0 | 100 |
| 92 | 0 | 0 | 100 |
| 93 | 0 | 0 | 100 |
| 96 | 0 | 100 | 100 |
| 85 | 17 | 100 | 100 |
| 97 | 0 | 70 | 100 |
| 98 | 0 | 0 | 100 |
| 99 | 0 | 0 | 78 |
| 101 | 0 | 80 | 100 |
| 102 | 0 | 0 | 100 |
| 104 | 0 | 0 | 11 |
| 103 | 0 | 0 | 100 |
| 105 | 0 | 0 | 67 |
| 100 | 0 | 100 | 100 |
| 111 | 17 | 0 | 61 |
| 115 | 0 | 0 | 78 |
| 114 | 0 | 0 | 83 |
| 113 | 0 | 0 | 94 |
| 120 | 33 | 80 | 16 |
| 125 | 17 | 0 | 89 |
| 109 | 0 | 0 | 33 |
| 110 | 0 | 0 | 17 |
| 124 | 0 | 0 | 72 |
| 126 | 0 | 80 | 78 |
| 116 | 0 | 0 | 33 |
| 112 | 0 | 0 | 22 |
| 106 | 17 | 0 | 33 |
| 117 | 33 | 80 | 11 |
| 132 | 33 | 80 | 22 |
| 122 | 0 | 0 | 22 |
| 74 | 0 | 0 | 89 |
| 70 | 0 | 0 | 33 |
| 73 | 0 | 0 | 89 |
| 71 | 50 | 100 | 22 |
| 75 | 0 | 0 | 0 |
| 76 | 0 | 0 | 56 |
| 67 | 0 | 0 | 50 |
| 69 | 100 | 100 | 100 |
| 68 | 67 | 100 | 100 |
| 152 | 0 | 0 | 94 |
| 153 | 0 | 100 | 100 |
| 150 | 0 | 0 | 11 |
| 142 | 83 | 100 | 78 |
| 157 | 67 | 0 | 94 |
| 156 | 17 | 100 | 100 |
| 100 | 0 | 100 | 100 |
| 154 | 0 | 80 | 67 |
| 147 | 0 | 0 | 100 |
| 146 | 17 | 100 | 100 |
| 158 | 17 | 100 | 89 |
| 107 | 17 | 0 | 78 |
| 72 | 50 | 100 | 44 |
| 151 | 0 | 0 | 11 |
| 149 | 0 | 0 | 28 |
| 148 | 0 | 0 | 6 |

### Example 3 : Vaccines compositions intended for prevention of any form of infection by ExPEC.

The polypeptide coded by a sequence comprising SEQ ID N°28 is conjugated with a toxin and added to a physiologically inert vehicle.

This conjugated peptide is optionnally added to a childhood vaccine.

The composition is sterilized and can be injected parenterally, subcutaneously or intramuscularly.

Said composition can also be sprayed onto mucosa with the aid of a spray.

### SEQUENCE LISTING

<110> MUTABILIS
<120> New products specific to pathogenic strains and their use as vaccines and in immunotherapy
<130> 1494
<160> 132
<170> PatentIn version 3.1
<210> 1
   <211> 163
   <212> PRT
   <213> Escherichia coli
<400> 1
   Met Lys Leu Lys Ala Ile Ile Leu Ala Thr Gly Leu Ile Asn Cys Ile
   1               5                   10                  15
   Val Phe Ser Ala Gln Ala Val Asp Thr Thr Ile Thr Val Thr Gly Asn
               20                  25                  30
   Val Leu Gln Arg Thr Cys Asn Val Pro Gly Asn Val Asp Val Ser Leu
           35                  40                  45
   Gly Asn Leu Tyr Val Ser Asp Phe Pro Asn Ala Gly Ser Gly Ser Pro
       50                  55                  60
   Trp Val Asn Phe Asp Leu Ser Leu Thr Gly Cys Gln Asn Met Asn Thr
   65                  70                  75                  80
   Val Arg Ala Thr Phe Ser Gly Thr Ala Asp Gly Gln Thr Tyr Tyr Ala
                   85                  90                  95
   Asn Thr Gly Asn Ala Gly Gly Ile Lys Ile Glu Ile Gln Asp Arg Asp
               100                 105                 110
   Gly Ser Asn Ala Ser Tyr His Asn Gly Met Phe Lys Thr Leu Asn Val
           115                 120                 125
   Gln Asn Asn Asn Ala Thr Phe Asn Leu Lys Ala Arg Ala Val Ser Lys
       130                 135                 140
   Gly Gln Val Thr Pro Gly Asn Ile Ser Ser Val Ile Thr Val Thr Tyr
   145                 150                 155                 160
   Thr Tyr Ala
<210> 2
   <211> 673
   <212> PRT
   <213> Escherichia coli
<400> 2
   Met Lys Met Thr Arg Leu Tyr Pro Leu Ala Leu Gly Gly Leu Leu Leu
   1 5 10 15
   Pro Ala Ile Ala Asn Ala Gln Thr Ser Gln Gln Asp Glu Ser Thr Leu
   20 25 30
   Val Val Thr Ala Ser Lys Gln Ser Ser Arg Ser Ala Ser Ala Asn Asn
   35 40 45
   Val Ser Ser Thr Val Val Ser Ala Pro Glu Leu Ser Asp Ala Gly Val
   50 55 60
   Thr Ala Ser Asp Lys Leu Pro Arg Val Leu Pro Gly Leu Asn Ile Glu
   65 70 75 80
   Asn Ser Gly Asn Met Leu Phe Ser Thr Ile Ser Leu Arg Gly Val Ser
   85 90 95
   Ser Ala Gln Asp Phe Tyr Asn Pro Ala Val Thr Leu Tyr Val Asp Gly
   100 105 110
   Val Pro Gln Leu Ser Thr Asn Thr Ile Gln Ala Leu Thr Asp Val Gln
   115 120 125
   Ser Val Glu Leu Leu Arg Gly Pro Gln Gly Thr Leu Tyr Gly Lys Ser
   130 135 140
   Ala Gln Gly Gly Ile Ile Asn Ile Val Thr Gln Gln Pro Asp Ser Thr
   145 150 155 160
   Pro Arg Gly Tyr Ile Glu Gly Gly Val Ser Ser Arg Asp Ser Tyr Arg
   165 170 175
   Ser Lys Phe Asn Leu Ser Gly Pro Ile Gln Asp Gly Leu Leu Tyr Gly
   180 185 190
   Ser Val Thr Leu Leu Arg Gln Val Asp Asp Gly Asp Met Ile Asn Pro
   195 200 205
   Ala Thr Gly Ser Asp Asp Leu Gly Gly Thr Arg Ala Ser Ile Gly Asn
       210                 215                 220
   Val Lys Leu Arg Leu Ala Pro Asp Asp Gln Pro Trp Glu Met Gly Phe
   225                 230                 235                 240
   Ala Ala Ser Arg Glu Cys Thr Arg Ala Thr Gln Asp Ala Tyr Val Gly
                   245                 250                 255
   Trp Asn Asp Ile Lys Gly Arg Lys Leu Ser Ile Ser Asp Gly Ser Pro
               260                 265                 270
   Asp Pro Tyr Met Arg Arg Cys Thr Asp Ser Gln Thr Leu Ser Gly Lys
           275                 280                 285
   Tyr Thr Thr Asp Asp Trp Val Phe Asn Leu Ile Ser Ala Trp Gln Gln
       290                 295                 300
   Gln His Tyr Ser Arg Thr Phe Pro Ser Gly Ser Leu Ile Val Asn Met
   305                 310                 315                 320
   Ser Gln Arg Trp Asn Gln Asp Val Gln Glu Leu Arg Ala Ala Thr Leu
                   325                 330                 335
   Gly Asp Ala Arg Thr Val Asp Met Val Phe Gly Leu Tyr Arg Gln Asn
               340                 345                 350
   Thr Arg Glu Lys Leu Asn Ser Ala Tyr Asp Met Pro Thr Met Pro Tyr
           355                 360                 365
   Leu Ser Ser Thr Gly Tyr Thr Thr Ala Glu Thr Leu Ala Ala Tyr Ser
       370                 375                 380
   Asp Leu Thr Trp His Leu Thr Asp Arg Phe Asp Ile Gly Gly Gly Val
   385                 390                 395                 400
   Arg Phe Ser His Asp Lys Ser Ser Thr Gln Tyr His Gly Ser Met Leu
                   405                 410                 415
   Gly Asn Pro Phe Gly Asp Gln Gly Lys Ser Asn Asp Asp Gln Val Leu
               420                 425                 430
   Gly Gln Leu Ser Ala Gly Tyr Met Leu Thr Asp Asp Trp Arg Val Tyr
   435 440 445
   Thr Arg Val Ala Gln Gly Tyr Lys Pro Ser Gly Tyr Asn Ile Val Pro
   450 455 460
   Thr Ala Gly Leu Asp Ala Lys Pro Phe Val Ala Glu Lys Ser Ile Asn
   465 470 475 480
   Tyr Glu Leu Gly Thr Arg Tyr Glu Thr Ala Asp Val Thr Leu Gln Ala
   485 490 495
   Ala Thr Phe Tyr Thr His Thr Lys Asp Met Gln Leu Tyr Ser Gly Pro
   500 505 510
   Val Gly Met Gln Thr Leu Ser Asn Ala Gly Lys Ala Asp Ala Thr Gly
   515 520 525
   Val Glu Leu Glu Ala Lys Trp Arg Phe Ala Pro Gly Trp Ser Trp Asp
   530 535 540
   Ile Asn Gly Asn Val Ile Arg Ser Glu Phe Thr Asn Asp Ser Glu Leu
   545 550 555 560
   Tyr His Gly Asn Arg Val Pro Phe Val Pro Arg Tyr Gly Ala Gly Ser
   565 570 575
   Ser Val Asn Gly Val Ile Asp Thr Arg Tyr Gly Ala Leu Met Pro Arg
   580 585 590
   Leu Ala Val Asn Leu Val Gly Pro His Tyr Phe Asp Gly Asp Asn Gln
   595 600 605
   Leu Arg Gln Gly Thr Tyr Ala Thr Leu Asp Ser Ser Leu Gly Trp Gln
   610 615 620
   Ala Thr Glu Arg Met Asn Ile Ser Val Tyr Val Asp Asn Leu Phe Asp
   625 630 635 640
   Arg Arg Tyr Arg Thr Tyr Gly Tyr Met Asn Gly Ser Ser Ala Val Ala
   645 650 655
   Gln Val Asn Met Gly Arg Thr Val Gly Ile Asn Thr Arg Ile Asp Phe
   660 665 670
   Phe
<210> 3
   <211> 246
   <212> PRT
   <213> Escherichia coli
<400> 3
   Met Asn Lys Val Phe Val Val Ser Val Val Ala Ala Ala Cys Val Phe
   1 5 10 15
   Ala Val Asn Ala Gly Ala Lys Glu Gly Lys Ser Gly Phe Tyr Leu Thr
   20 25 30
   Gly Lys Ala Gly Ala Ser Val Met Ser Leu Ser Asp Gln Arg Phe Leu
   35 40 45
   Ser Gly Asp Glu Glu Glu Thr Ser Lys Tyr Lys Gly Gly Asp Asp His
   50 55 60
   Asp Thr Val Phe Ser Gly Gly Ile Ala Val Gly Tyr Asp Phe Tyr Pro
   65 70 75 80
   Gln Phe Ser Ile Pro Val Arg Thr Glu Leu Glu Phe Tyr Ala Arg Gly
   85 90 95
   Lys Ala Asp Ser Lys Tyr Asn Val Asp Lys Asp Ser Trp Ser Gly Gly
   100 105 110
   Tyr Trp Arg Asp Asp Leu Lys Asn Glu Val Ser Val Asn Thr Leu Met
   115 120 125
   Leu Asn Ala Tyr Tyr Asp Phe Arg Asn Asp Ser Ala Phe Thr Pro Trp
   130 135 140
   Val Ser Ala Gly Ile Gly Tyr Ala Arg Ile His Gln Lys Thr Thr Gly
   145 150 155 160
   Ile Ser Thr Trp Asp Tyr Glu Tyr Gly Ser Ser Gly Arg Glu Ser Leu
   165 170 175
   Ser Arg Ser Gly Ser Ala Asp Asn Phe Ala Trp Ser Leu Gly Ala Gly
   180 185 190
   Val Arg Tyr Asp Val Thr Pro Asp Ile Ala Leu Asp Leu Ser Tyr Arg
   195 200 205
   Tyr Leu Asp Ala Gly Asp Ser Ser Val Ser Tyr Lys Asp Glu Trp Gly
   210 215 220
   Asp Lys Tyr Lys Ser Glu Val Asp Val Lys Ser His Asp Ile Met Leu
   225 230 235 240
   Gly Met Thr Tyr Asn Phe
   245
<210> 4
   <211> 166
   <212> PRT
   <213> Escherichia coli
<400> 4
   Met Lys Leu Lys Ala Ile Ile Leu Ala Thr Gly Leu Ile Asn Cys Ile
   1 5 10 15
   Ala Phe Ser Ala Gln Ala Val Asp Thr Thr Ile Thr Val Thr Gly Arg
   20 25 30
   Val Leu Pro Arg Thr Cys Thr Ile Gly Asn Gly Gly Asn Pro Asn Ala
   35 40 45
   Thr Val Val Leu Asp Asn Ala Tyr Thr Ser Asp Leu Ile Ala Ala Asn
   50 55 60
   Ser Thr Ser Gln Trp Lys Asn Phe Ser Leu Thr Leu Thr Asn Cys Gln
   65 70 75 80
   Asn Val Asn Asn Val Thr Ser Phe Gly Gly Thr Ala Glu Asn Thr Asn
   85 90 95
   Tyr Tyr Arg Asn Thr Gly Asp Ala Thr Asn Ile Met Val Glu Leu Gln
   100 105 110
   Glu Gln Gly Asn Gly Asn Thr Pro Leu Lys Val Gly Ser Thr Lys Val
   115 120 125
   Val Thr Val Ser Asn Gly Gln Ala Thr Phe Asn Leu Lys Val Arg Ala
   130 135 140
   Val Ser Lys Gly Asn Ala Gly Ala Gly Ser Ile Asn Ser Gln Ile Thr
   145 150 155 160
   Val Thr Tyr Thr Tyr Ala
   165
<210> 5
   <211> 1295
   <212> PRT
   <213> Escherichia coli
<400> 5
   Met Asn Lys Ile Tyr Ser Leu Lys Tyr Ser Ala Ala Thr Gly Gly Leu
   1 5 10 15
   Ile Ala Val Ser Glu Leu Ala Lys Arg Val Ser Gly Lys Thr Asn Arg
   20 25 30
   Lys Leu Val Ala Thr Met Leu Ser Leu Ala Val Ala Gly Thr Val Asn
   35 40 45
   Ala Ala Asn Ile Asp Ile Ser Asn Val Trp Ala Arg Asp Tyr Leu Asp
   50 55 60
   Leu Ala Gln Asn Lys Gly Ile Phe Gln Pro Gly Ala Thr Asp Val Thr
   65 70 75 80
   Ile Thr Leu Lys Asn Gly Asp Lys Phe Ser Phe His Asn Leu Ser Ile
   85 90 95
   Pro Asp Phe Ser Gly Ala Ala Ala Ser Gly Ala Ala Thr Ala Ile Gly
   100 105 110
   Gly Ser Tyr Ser Val Thr Val Ala His Asn Lys Lys Asn Pro Gln Ala
   115 120 125
   Ala Glu Thr Gln Val Tyr Ala Gln Ser Ser Tyr Arg Val Val Asp Arg
   130 135 140
   Arg Asn Ser Asn Asp Phe Glu Ile Gln Arg Leu Asn Lys Phe Val Val
   145 150 155 160
   Glu Thr Val Gly Ala Thr Pro Ala Glu Thr Asn Pro Thr Thr Tyr Ser
   165 170 175
   Asp Ala Leu Glu Arg Tyr Gly Ile Val Thr Ser Asp Gly Ser Lys Lys
   180 185 190
   Ile Ile Gly Phe Arg Ala Gly Ser Gly Gly Thr Ser Phe Ile Asn Gly
   195 200 205
   Glu Ser Lys Ile Ser Thr Asn Ser Ala Tyr Ser His Asp Leu Leu Ser
   210 215 220
   Ala Ser Leu Phe Glu Val Thr Gln Trp Asp Ser Tyr Gly Met Met Ile
   225 230 235 240
   Tyr Lys Asn Asp Lys Thr Phe Arg Asn Leu Glu Ile Phe Gly Asp Ser
   245 250 255
   Gly Ser Gly Ala Tyr Leu Tyr Asp Asn Lys Leu Glu Lys Trp Val Leu
   260 265 270
   Val Gly Thr Thr His Gly Ile Ala Ser Val Asn Gly Asp Gln Leu Thr
   275 280 285
   Trp Ile Thr Lys Tyr Asn Asp Lys Leu Val Ser Glu Leu Lys Asp Thr
   290 295 300
   Tyr Ser His Lys Ile Asn Leu Asn Gly Asn Asn Val Thr Ile Lys Asn
   305 310 315 320
   Thr Asp Ile Thr Leu His Gln Asn Asn Ala Asp Thr Thr Gly Thr Gln
   325 330 335
   Glu Lys Ile Thr Lys Asp Lys Asp Ile Val Phe Thr Asn Gly Gly Asp
   340 345 350
   Val Leu Phe Lys Asp Asn Leu Asp Phe Gly Ser Gly Gly Ile Ile Phe
   355 360 365
   Asp Glu Gly His Glu Tyr Asn Ile Asn Gly Gln Gly Phe Thr Phe Lys
   370 375 380
   Gly Ala Gly Ile Asp Ile Gly Lys Glu Ser Ile Val Asn Trp Asn Ala
   385 390 395 400
   Leu Tyr Ser Ser Asp Asp Val Leu His Lys Ile Gly Pro Gly Thr Leu
   405 410 415
   Asn Val Gln Lys Lys Gln Gly Ala Asn Ile Lys Ile Gly Glu Gly Asn
   420 425 430
   Val Ile Leu Asn Glu Glu Gly Thr Phe Asn Asn Ile Tyr Leu Ala Ser
   435 440 445
   Gly Asn Gly Lys Val Ile Leu Asn Lys Asp Asn Ser Leu Gly Asn Asp
   450 455 460
   Gln Tyr Ala Gly Ile Phe Phe Thr Lys Arg Gly Gly Thr Leu Asp Leu
   465 470 475 480
   Asn Gly His Asn Gln Thr Phe Thr Arg Ile Ala Ala Thr Asp Asp Gly
   485 490 495
   Thr Thr Ile Thr Asn Ser Asp Thr Thr Lys Glu Ala Val Leu Ala Ile
   500 505 510
   Asn Asn Glu Asp Ser Tyr Ile Tyr His Gly Asn Ile Asn Gly Asn Ile
   515 520 525
   Lys Leu Thr His Asn Ile Asn Ser Gln Asp Lys Lys Thr Asn Ala Lys
   530 535 540
   Leu Ile Leu Asp Gly Ser Val Asn Thr Lys Asn Asp Val Glu Val Ser
   545 550 555 560
   Asn Ala Ser Leu Thr Met Gln Gly His Ala Thr Glu His Ala Ile Phe
   565 570 575
   Arg Ser Ser Ala Asn His Cys Ser Leu Val Phe Leu Cys Gly Thr Asp
   580 585 590
   Trp Val Thr Val Leu Lys Glu Thr Glu Ser Ser Tyr Asn Lys Lys Phe
   595 600 605
   Asn Ser Asp Tyr Lys Ser Asn Asn Gln Gln Thr Ser Phe Asp Gln Pro
   610 615 620
   Asp Trp Lys Thr Gly Val Phe Lys Phe Asp Thr Leu His Leu Asn Asn
   625 630 635 640
   Ala Asp Phe Ser Ile Ser Arg Asn Ala Asn Val Glu Gly Asn Ile Ser
   645 650 655
   Ala Asn Lys Ser Ala Ile Thr Ile Gly Asp Lys Asn Val Tyr Ile Asp
   660 665 670
   Asn Leu Ala Gly Lys Asn Ile Thr Asn Asn Gly Phe Asp Phe Lys Gln
   675 680 685
   Thr Ile Ser Thr Asn Leu Ser Ile Gly Glu Thr Lys Phe Thr Gly Gly
   690 695 700
   Ile Thr Ala His Asn Ser Gln Ile Ala Ile Gly Asp Gln Ala Val Val
   705 710 715 720
   Thr Leu Asn Gly Ala Thr Phe Leu Asp Asn Thr Pro Ile Ser Ile Asp
   725 730 735
   Lys Gly Ala Lys Val Ile Ala Gln Asn Ser Met Phe Thr Thr Lys Gly
   740 745 750
   Ile Asp Ile Ser Gly Glu Leu Thr Met Met Gly Ile Pro Glu Gln Asn
   755 760 765
   Ser Lys Thr Val Thr Pro Gly Leu His Tyr Ala Ala Asp Gly Phe Arg
   770 775 780
   Leu Ser Gly Gly Asn Ala Asn Phe Ile Ala Arg Asn Met Ala Ser Val
   785 790 795 800
   Thr Gly Asn Ile Tyr Ala Asp Asp Ala Ala Thr Ile Thr Leu Gly Gln
   805 810 815
   Pro Glu Thr Glu Thr Pro Thr Ile Ser Ser Ala Tyr Gln Ala Trp Ala
   820 825 830
   Glu Thr Leu Leu Tyr Gly Phe Asp Thr Ala Tyr Arg Gly Ala Ile Thr
   835 840 845
   Ala Pro Lys Ala Thr Val Ser Met Asn Asn Ala Ile Trp His Leu Asn
   850 855 860
   Ser Gln Ser Ser Ile Asn Arg Leu Glu Thr Lys Asp Ser Met Val Arg
   865 870 875 880
   Phe Thr Gly Asp Asn Gly Lys Phe Thr Thr Leu Thr Val Asn Asn Leu
   885 890 895
   Thr Ile Asp Asp Ser Ala Phe Val Leu Arg Ala Asn Leu Ala Gln Ala
   900 905 910
   Asp Gln Leu Val Val Asn Lys Ser Leu Ser Gly Lys Asn Asn Leu Leu
   915 920 925
   Leu Val Asp Phe Ile Glu Lys Asn Gly Asn Ser Asn Gly Leu Asn Ile
   930 935 940
   Asp Leu Val Ser Ala Pro Lys Gly Thr Ala Val Asp Val Phe Lys Ala
   945 950 955 960
   Thr Thr Arg Ser Ile Gly Phe Ser Asp Val Thr Pro Val Ile Glu Gln
   965 970 975
   Lys Asn Asp Thr Asp Lys Ala Thr Trp Thr Leu Ile Gly Tyr Lys Ser
   980 985 990
   Val Ala Asn Ala Asp Ala Ala Lys Lys Ala Thr Leu Leu Met Ser Gly
   995 1000 1005
   Gly Tyr Lys Ala Phe Leu Ala Glu Val Asn Asn Leu Asn Lys Arg
   1010 1015 1020
   Met Gly Asp Leu Arg Asp Ile Asn Gly Glu Ser Gly Ala Trp Ala
   1025 1030 1035
   Arg Ile Ile Ser Gly Thr Gly Ser Ala Gly Gly Gly Phe Ser Asp
   1040 1045 1050
   Asn Tyr Thr His Val Gln Val Gly Ala Asp Asn Lys His Glu Leu
   1055 1060 1065
   Asp Gly Leu Asp Leu Phe Thr Gly Val Thr Met Thr Tyr Thr Asp
   1070 1075 1080
   Ser His Ala Gly Ser Asp Ala Phe Ser Gly Glu Thr Lys Ser Val
   1085 1090 1095
   Gly Ala Gly Leu Tyr Ala Ser Ala Met Phe Glu Ser Gly Ala Tyr
   1100 1105 1110
   Ile Asp Leu Ile Gly Lys Tyr Val His His Asp Asn Glu Tyr Thr
   1115 1120 1125
   Ala Thr Phe Ala Gly Leu Gly Thr Arg Asp Tyr Ser Ser His Ser
   1130 1135 1140
   Trp Tyr Ala Gly Ala Glu Val Gly Tyr Arg Tyr His Val Thr Asp
   1145 1150 1155
   Ser Ala Trp Ile Glu Pro Gln Ala Glu Leu Val Tyr Gly Ala Val
   1160 1165 1170
   Ser Gly Lys Gln Phe Ser Trp Lys Asp Gln Gly Met Asn Leu Thr
   1175 1180 1185
   Met Lys Asp Lys Asp Phe Asn Pro Leu Ile Gly Arg Thr Gly Val
   1190 1195 1200
   Asp Val Gly Lys Ser Phe Ser Gly Lys Asp Trp Lys Val Thr Ala
   1205 1210 1215
   Arg Ala Gly Leu Gly Tyr Gln Phe Asp Leu Phe Ala Asn Gly Glu
   1220 1225 1230
   Thr Val Leu Arg Asp Ala Ser Gly Glu Lys Arg Ile Lys Gly Glu
   1235 1240 1245
   Lys Asp Gly Arg Met Leu Met Asn Val Gly Leu Asn Ala Glu Ile
   1250 1255 1260
   Arg Asp Asn Leu Arg Phe Gly Leu Glu Phe Glu Lys Ser Ala Phe
   1265 1270 1275
   Gly Lys Tyr Asn Val Asp Asn Ala Ile Asn Ala Asn Phe Arg Tyr
   1280 1285 1290
   Ser Phe
   1295
<210> 6
   <211> 142
   <212> PRT
   <213> Escherichia coli
<400> 6
   Met Ile Asn Ile Pro Ser Pro Thr Ala Val Val Met Ala Leu Val Ala
   1 5 10 15
   Ile Ser Thr Leu Pro Ser Pro Ser Arg Val Lys Leu Met Pro Tyr Pro
   20 25 30
   Pro Arg Ala His Asn Thr Thr Gly Leu Leu Pro Val Arg Glu Ile Cys
   35 40 45
   Phe Pro His His Gly Asp Asp Gly Arg Asn Ser Ile Glu Pro Ser Ile
   50 55 60
   Ser Arg Ala Ala His Thr Asp Arg Leu Arg Phe Val Cys Met Thr Arg
   65 70 75 80
   Thr Gly Ser Thr Thr Ser Arg Pro Phe Cys Pro Ile Pro Arg Ser Pro
   85 90 95
   Ala Leu Asn Ala Ser Gly Gln Gln Asp Ser Gly Phe Trp Gly Val Ser
   100 105 110
   Ser Ile Pro Gly Asp Ile Leu Met Phe Gln Leu His Val Leu Ile Val
   115 120 125
   Phe Ile Cys Lys Ile Asn Leu Ser Asp Asn Asn Ile Ser Tyr
   130 135 140
<210> 7
   <211> 318
   <212> PRT
   <213> Escherichia coli
<400> 7
   Met Tyr Ala Arg Glu Tyr Arg Ser Thr Arg Pro His Lys Ala Ile Phe
   1 5 10 15
   Phe His Leu Ser Cys Leu Thr Leu Ile Cys Ser Ala Gln Val Tyr Ala
   20 25 30
   Lys Pro Asp Met Arg Pro Leu Gly Pro Asn Ile Ala Asp Lys Gly Ser
   35 40 45
   Val Phe Tyr His Phe Ser Ala Thr Ser Phe Asp Ser Val Asp Gly Thr
   50 55 60
   Arg His Tyr Arg Val Trp Thr Ala Val Pro Asn Thr Thr Ala Pro Ala
   65 70 75 80
   Ser Gly Tyr Pro Ile Leu Tyr Met Leu Asp Gly Asn Ala Val Met Asp
   85 90 95
   Arg Leu Asp Asp Glu Leu Leu Lys Gln Leu Ser Glu Lys Thr Pro Pro
   100 105 110
   Val Ile Val Ala Val Gly Tyr Gln Thr Asn Leu Pro Phe Asp Leu Asn
   115 120 125
   Ser Arg Ala Tyr Asp Tyr Thr Pro Ala Ala Glu Ser Arg Lys Thr Asp
   130 135 140
   Leu His Ser Gly Arg Phe Ser Arg Lys Ser Gly Gly Ser Asn Asn Phe
   145 150 155 160
   Arg Gln Leu Leu Glu Thr Arg Ile Ala Pro Lys Val Glu Gln Gly Leu
   165 170 175
   Asn Ile Asp Arg Gln Arg Arg Gly Leu Trp Gly His Ser Tyr Gly Gly
   180 185 190
   Leu Phe Val Leu Asp Ser Trp Leu Ser Ser Ser Tyr Phe Arg Ser Tyr
   195 200 205
   Tyr Ser Ala Ser Pro Ser Leu Gly Arg Gly Tyr Asp Ala Leu Leu Ser
   210 215 220
   Arg Val Thr Ala Val Glu Pro Leu Gln Phe Cys Thr Lys His Leu Ala
   225 230 235 240
   Ile Met Glu Gly Ser Ala Thr Gln Gly Asp Asn Arg Glu Thr His Ala
   245 250 255
   Val Gly Val Leu Ser Lys Ile His Thr Thr Leu Thr Ile Leu Lys Asp
   260 265 270
   Lys Gly Val Asn Ala Val Phe Trp Asp Phe Pro Asn Leu Gly His Gly
   275 280 285
   Pro Met Phe Asn Ala Ser Phe Arg Gln Ala Leu Leu Asp Ile Ser Gly
   290 295 300
   Glu Asn Ala Asn Tyr Thr Ala Gly Cys His Glu Leu Ser His
   305 310 315
<210> 8
   <211> 725
   <212> PRT
   <213> Escherichia coli
<400> 8
   Met Arg Ile Asn Lys Ile Leu Trp Ser Leu Thr Val Leu Leu Val Gly
   1 5 10 15
   Leu Asn Ser Gln Val Ser Val Ala Lys Tyr Ser Asp Asp Asp Asn Asp
   20 25 30
   Glu Thr Leu Val Val Glu Ala Thr Ala Glu Gln Val Leu Lys Gln Gln
   35 40 45
   Pro Gly Val Ser Val Ile Thr Ser Glu Asp Ile Lys Lys Thr Pro Pro
   50 55 60
   Val Asn Asp Leu Ser Asp Ile Ile Arg Lys Met Pro Gly Val Asn Leu
   65 70 75 80
   Thr Gly Asn Ser Ala Ser Gly Thr Arg Gly Asn Asn Arg Gln Ile Asp
   85 90 95
   Ile Arg Gly Met Gly Pro Glu Asn Thr Leu Ile Leu Ile Asp Gly Val
   100 105 110
   Pro Val Thr Ser Arg Asn Ser Val Arg Tyr Ser Trp Arg Gly Glu Arg
           115                 120                 125
   Asp Thr Arg Gly Asp Thr Asn Trp Val Pro Pro Glu Gln Val Glu Arg
       130                 135                 140
   Ile Glu Val Ile Arg Gly Pro Ala Ala Ala Arg Tyr Gly Ser Gly Ala
   145                 150                 155                 160
   Ala Gly Gly Val Val Asn Ile Ile Thr Lys Arg Pro Thr Asn Asp Trp
                   165                 170                 175
   His Gly Ser Leu Ser Leu Tyr Thr Asn Gln Pro Glu Ser Ser Glu Glu
               180                 185                 190
   Gly Ala Thr Arg Arg Ala Asn Phe Ser Leu Ser Gly Pro Leu Ala Gly
           195                 200                 205
   Asp Ala Leu Thr Thr Arg Leu Tyr Gly Asn Leu Asn Lys Thr Asp Ala
       210                 215                 220
   Asp Ser Trp Asp Ile Asn Ser Pro Val Gly Thr Lys Asn Ala Ala Gly
   225                 230                 235                 240
   His Glu Gly Val Arg Asn Lys Asp Ile Asn Gly Val Val Ser Trp Lys
                   245                 250                 255
   Leu Asn Pro Gln Gln Ile Leu Asp Phe Glu Val Gly Tyr Ser Arg Gln
               260                 265                 270
   Gly Asn Ile Tyr Ala Gly Asp Thr Gln Asn Ser Ser Ser Ser Ala Val
           275                 280                 285
   Thr Glu Ser Leu Ala Lys Ser Gly Lys Glu Thr Asn Arg Leu Tyr Arg
       290                 295                 300
   Gln Asn Tyr Gly Ile Thr His Asn Gly Ile Trp Asp Trp Gly Gln Ser
   305                 310                 315                 320
   Arg Phe Gly Val Tyr Tyr Glu Lys Thr Asn Asn Thr Arg Met Asn Glu
                   325                 330                 335
   Gly Leu Ser Gly Gly Gly Glu Gly Arg Ile Leu Ala Gly Glu Lys Phe
   340 345 350
   Thr Thr Asn Arg Leu Ser Ser Trp Arg Thr Ser Gly Glu Leu Asn Ile
   355 360 365
   Pro Leu Asn Val Met Val Asp Gln Thr Leu Thr Val Gly Ala Glu Trp
   370 375 380
   Asn Arg Asp Lys Leu Asp Asp Pro Ser Ser Thr Ser Leu Thr Val Asn
   385 390 395 400
   Asp Arg Asp Ile Ser Gly Ile Ser Gly Ser Ala Ala Asp Arg Ser Ser
   405 410 415
   Lys Asn His Ser Gln Ile Ser Ala Leu Tyr Ile Glu Asp Asn Ile Glu
   420 425 430
   Pro Val Pro Gly Thr Asn Ile Ile Pro Gly Leu Arg Phe Asp Tyr Leu
   435 440 445
   Ser Asp Ser Gly Gly Asn Phe Ser Pro Ser Leu Asn Leu Ser Gln Glu
   450 455 460
   Leu Gly Asp Tyr Phe Lys Val Lys Ala Gly Val Ala Arg Thr Phe Lys
   465 470 475 480
   Ala Pro Asn Leu Tyr Gln Ser Ser Glu Gly Tyr Leu Leu Tyr Ser Lys
   485 490 495
   Gly Asn Gly Cys Pro Lys Asp Ile Thr Ser Gly Gly Cys Tyr Leu Ile
   500 505 510
   Gly Asn Lys Asp Leu Asp Pro Glu Ile Ser Val Asn Lys Glu Ile Gly
   515 520 525
   Leu Glu Phe Thr Trp Glu Asp Tyr His Ala Ser Val Thr Tyr Phe Arg
   530 535 540
   Asn Asp Tyr Gln Asn Lys Ile Val Ala Gly Asp Asn Val Ile Gly Gln
   545 550 555 560
   Thr Ala Ser Gly Ala Tyr Ile Leu Lys Trp Gln Asn Gly Gly Lys Ala
   565 570 575
   Leu Val Asp Gly Ile Glu Ala Ser Met Ser Phe Pro Leu Val Lys Glu
   580 585 590
   Arg Leu Asn Trp Asn Thr Asn Ala Thr Trp Met Ile Thr Ser Glu Gln
   595 600 605
   Lys Asp Thr Gly Asn Pro Leu Ser Val Ile Pro Lys Tyr Thr Ile Asn
   610 615 620
   Asn Ser Leu Asn Trp Thr Ile Thr Gln Ala Phe Ser Ala Ser Phe Asn
   625 630 635 640
   Trp Thr Leu Tyr Gly Arg Gln Lys Pro Arg Thr His Ala Glu Thr Arg
   645 650 655
   Ser Glu Asp Thr Gly Gly Leu Ser Gly Lys Glu Leu Gly Ala Tyr Ser
   660 665 670
   Leu Val Gly Thr Asn Phe Asn Tyr Asp Ile Asn Lys Asn Leu Arg Leu
   675 680 685
   Asn Val Gly Val Ser Asn Ile Leu Asn Lys Gln Ile Phe Arg Ser Ser
   690 695 700
   Glu Gly Ala Asn Thr Tyr Asn Glu Pro Gly Arg Ala Tyr Tyr Ala Gly
   705 710 715 720
   Val Thr Ala Ser Phe
   725
<210> 9
   <211> 1014
   <212> PRT
   <213> Escherichia coli
<400> 9
   Met Gly Asn Gln Trp Gln Gln Lys Tyr Leu Leu Glu Tyr Asn Glu Leu
   1 5 10 15
   Val Ser Asn Phe Pro Ser Pro Glu Arg Val Val Ser Asp Tyr Ile Lys
   20 25 30
   Asn Cys Phe Lys Thr Asp Leu Pro Trp Phe Ser Arg Ile Asp Pro Asp
   35 40 45
   Asn Ala Tyr Phe Ile Cys Phe Ser Gln Asn Arg Ser Asn Ser Arg Ser
   50 55 60
   Tyr Thr Gly Trp Asp His Leu Gly Lys Tyr Lys Thr Glu Val Leu Thr
   65 70 75 80
   Leu Thr Gln Ala Ala Leu Ile Asn Ile Gly Tyr Arg Phe Asp Val Phe
   85 90 95
   Asp Asp Ala Asn Ser Ser Thr Gly Ile Tyr Lys Thr Lys Ser Ala Asp
   100 105 110
   Val Phe Asn Glu Glu Asn Glu Glu Lys Met Leu Pro Ser Glu Tyr Leu
   115 120 125
   His Phe Leu Gln Lys Cys Asp Phe Ala Gly Val Tyr Gly Lys Thr Leu
   130 135 140
   Ser Asp Tyr Trp Ser Lys Tyr Tyr Asp Lys Phe Lys Leu Leu Leu Lys
   145 150 155 160
   Asn Tyr Tyr Ile Ser Ser Ala Leu Tyr Leu Tyr Lys Asn Gly Glu Leu
   165 170 175
   Asp Glu Arg Glu Tyr Asn Phe Ser Met Asn Ala Leu Asn Arg Ser Asp
   180 185 190
   Asn Ile Ser Leu Leu Phe Phe Asp Ile Tyr Gly Tyr Tyr Ala Ser Asp
   195 200 205
   Ile Phe Val Ala Lys Asn Asn Asp Lys Val Met Leu Phe Ile Pro Gly
   210 215 220
   Ala Lys Lys Pro Phe Leu Phe Lys Lys Asn Ile Ala Asp Leu Arg Leu
   225 230 235 240
   Thr Leu Lys Glu Leu Ile Lys Asp Ser Asp Asn Lys Gln Leu Leu Ser
   245 250 255
   Gln His Phe Ser Leu Tyr Ser Arg Gln Asp Gly Val Ser Tyr Ala Gly
   260 265 270
   Val Asn Ser Val Leu His Ala Ile Glu Asn Asp Gly Asn Phe Asn Glu
   275 280 285
   Ser Tyr Phe Leu Tyr Ser Asn Lys Thr Leu Ser Asn Lys Asp Val Phe
   290 295 300
   Asp Ala Ile Ala Ile Ser Val Lys Lys Arg Ser Phe Ser Asp Gly Asp
   305 310 315 320
   Ile Val Ile Lys Ser Asn Ser Glu Ala Gln Arg Asp Tyr Ala Leu Thr
   325 330 335
   Ile Leu Gln Thr Ile Leu Ser Met Thr Pro Ile Phe Asp Ile Val Val
   340 345 350
   Pro Glu Val Ser Val Pro Leu Gly Leu Gly Ile Ile Thr Ser Ser Met
   355 360 365
   Gly Ile Ser Phe Asp Gln Leu Ile Asn Gly Asp Thr Tyr Glu Glu Arg
   370 375 380
   Arg Ser Ala Ile Pro Gly Leu Ala Thr Asn Ala Val Leu Leu Gly Leu
   385 390 395 400
   Ser Phe Ala Ile Pro Leu Leu Ile Ser Lys Ala Gly Ile Asn Gln Glu
   405 410 415
   Val Leu Ser Ser Val Ile Asn Asn Glu Gly Arg Thr Leu Asn Glu Thr
   420 425 430
   Asn Ile Asp Ile Phe Leu Lys Glu Tyr Gly Ile Ala Glu Asp Ser Ile
   435 440 445
   Ser Ser Thr Asn Leu Leu Asp Val Lys Leu Lys Ser Ser Gly Gln His
   450 455 460
   Val Asn Ile Val Lys Leu Ser Asp Glu Asp Asn Gln Ile Val Ala Val
   465 470 475 480
   Lys Gly Ser Ser Leu Ser Gly Ile Tyr Tyr Glu Val Asp Ile Glu Thr
   485 490 495
   Gly Tyr Glu Ile Leu Ser Arg Arg Ile Tyr Arg Thr Glu Tyr Asn Asn
               500                 505                 510
   Glu Ile Leu Trp Thr Arg Gly Gly Gly Leu Lys Gly Gly Gln Pro Phe
           515                 520                 525
   Asp Phe Glu Ser Leu Asn Ile Pro Val Phe Phe Lys Asp Glu Pro Tyr
       530                 535                 540
   Ser Ala Val Thr Gly Ser Pro Leu Ser Phe Ile Asn Asp Asp Ser Ser
   545                 550                 555                 560
   Leu Leu Tyr Pro Asp Thr Asn Pro Lys Leu Pro Gln Pro Thr Ser Glu
                   565                 570                 575
   Met Asp Ile Val Asn Tyr Val Lys Gly Ser Gly Ser Phe Gly Asp Arg
               580                 585                 590
   Phe Val Thr Leu Met Arg Gly Ala Thr Glu Glu Glu Ala Trp Asn Ile
           595                 600                 605
   Ala Ser Tyr His Thr Ala Gly Gly Ser Thr Glu Glu Leu His Glu Ile
       610                 615                 620
   Leu Leu Gly Gln Gly Pro Gln Ser Ser Leu Gly Phe Thr Glu Tyr Thr
   625                 630                 635                 640
   Ser Asn Val Asn Ser Ala Asp Ala Ala Ser Arg Arg His Phe Leu Val
                   645                 650                 655
   Val Ile Lys Val His Val Lys Tyr Ile Thr Asn Asn Asn Val Ser Tyr
               660                 665                 670
   Val Asn His Trp Ala Ile Pro Asp Glu Ala Pro Val Glu Val Leu Ala
           675                 680                 685
   Val Val Asp Arg Arg Phe Asn Phe Pro Glu Pro Ser Thr Pro Pro Asp
       690                 695                 700
   Ile Ser Thr Ile Arg Lys Leu Leu Ser Leu Arg Tyr Phe Lys Glu Ser
   705                 710                 715                 720
   Ile Glu Ser Thr Ser Lys Ser Asn Phe Gln Lys Leu Ser Arg Gly Asn
   725 730 735
   Ile Asp Val Leu Lys Gly Arg Gly Ser Ile Ser Ser Thr Arg Gln Arg
   740 745 750
   Ala Ile Tyr Pro Tyr Phe Glu Ala Ala Asn Ala Asp Glu Gln Gln Pro
   755 760 765
   Leu Phe Phe Tyr Ile Lys Lys Asp Arg Phe Asp Asn His Gly Tyr Asp
   770 775 780
   Gln Tyr Phe Tyr Asp Asn Thr Val Gly Leu Asn Gly Ile Pro Thr Leu
   785 790 795 800
   Asn Thr Tyr Thr Gly Glu Ile Pro Ser Asp Ser Ser Ser Leu Gly Ser
   805 810 815
   Thr Tyr Trp Lys Lys Tyr Asn Leu Thr Asn Glu Thr Ser Ile Ile Arg
   820 825 830
   Val Ser Asn Ser Ala Arg Gly Ala Asn Gly Ile Lys Ile Ala Leu Glu
   835 840 845
   Glu Val Gln Glu Gly Lys Pro Val Ile Ile Thr Ser Gly Asn Leu Ser
   850 855 860
   Gly Cys Thr Thr Ile Val Ala Arg Lys Glu Gly Tyr Ile Tyr Lys Val
   865 870 875 880
   His Thr Gly Thr Thr Lys Ser Leu Ala Gly Phe Thr Ser Thr Thr Gly
   885 890 895
   Val Lys Lys Ala Val Glu Val Leu Glu Leu Leu Thr Lys Glu Pro Ile
   900 905 910
   Pro Arg Val Glu Gly Ile Met Ser Asn Asp Phe Leu Val Asp Tyr Leu
   915 920 925
   Ser Glu Asn Phe Glu Asp Ser Leu Ile Thr Tyr Ser Ser Ser Glu Lys
   930 935 940
   Lys Pro Asp Ser Gln Ile Thr Ile Ile Arg Asp Asn Val Ser Val Phe
   945 950 955 960
   Pro Tyr Phe Leu Asp Asn Ile Pro Glu His Gly Phe Gly Thr Ser Ala
   965 970 975
   Thr Val Leu Val Arg Val Asp Gly Asn Val Val Val Arg Ser Leu Ser
   980 985 990
   Glu Ser Tyr Ser Leu Asn Ala Asp Ala Ser Glu Ile Ser Val Leu Lys
   995 1000 1005
   Val Phe Ser Lys Lys Phe
   1010
<210> 10
   <211> 454
   <212> PRT
   <213> Escherichia coli
<400> 10
   Met Val Asp Met Ile Asn Glu Ser Ala Arg Gln Thr Pro Val Ile Ala
   1 5 10 15
   Gln Thr Asp Val Leu Val Ile Gly Gly Gly Pro Ala Gly Leu Ser Ala
   20 25 30
   Ala Ile Ala Ala Gly Arg Leu Gly Ala Arg Thr Met Ile Val Glu Arg
   35 40 45
   Tyr Gly Ser Leu Gly Gly Val Leu Thr Gln Val Gly Val Glu Ser Phe
   50 55 60
   Ala Trp Tyr Arg His Pro Gly Thr Glu Asp Cys Glu Gly Ile Cys Arg
   65 70 75 80
   Glu Tyr Glu Gly Arg Ala Arg Ala Leu Gly Phe Thr Arg Pro Glu Pro
   85 90 95
   Gln Ser Ile Ser Glu Val Ile Asp Thr Glu Gly Phe Lys Val Val Ala
   100 105 110
   Asp Gln Met Ile Thr Glu Ser Gly Val Glu Pro Leu Tyr His Ser Trp
   115 120 125
   Val Val Asp Val Ile Lys Asp Gly Asp Thr Leu Cys Gly Val Ile Val
   130 135 140
   Glu Asn Lys Ser Gly Arg Gly Ala Ile Leu Ala Lys Arg Ile Val Asp
   145 150 155 160
   Cys Thr Gly Asp Ala Asp Ile Ala Ala Arg Ala Gly Ala Pro Trp Thr
   165 170 175
   Lys Arg Ser Lys Asp Gln Leu Met Gly Val Thr Val Met Phe Ser Cys
   180 185 190
   Ala Gly Val Asp Val Ala Arg Phe Asn Arg Phe Val Ala Glu Glu Leu
   195 200 205
   Lys Pro Thr Tyr Ala Asp Trp Gly Lys Asn Trp Thr Ile Gln Thr Thr
   210 215 220
   Gly Lys Glu Asp Pro Met Phe Ser Pro Tyr Met Glu Asp Ile Phe Thr
   225 230 235 240
   Arg Ala Gln Gln Asp Gly Val Ile Pro Gly Asp Ala Gln Ala Ile Ala
   245 250 255
   Gly Thr Trp Ser Thr Phe Ser Glu Ser Gly Glu Ala Phe Gln Met Asn
   260 265 270
   Met Val Tyr Ala Phe Gly Phe Asp Cys Thr Asp Val Phe Asp Leu Thr
   275 280 285
   Lys Ala Glu Ile Ala Gly Arg Gln Gln Ala Leu Trp Ala Ile Asp Ala
   290 295 300
   Leu Arg His Tyr Val Pro Gly Phe Glu Asn Val Arg Leu Arg Asn Phe
   305 310 315 320
   Gly Ala Thr Leu Gly Thr Arg Glu Ser Arg Leu Ile Glu Gly Glu Ile
   325 330 335
   Arg Ile Ala Asp Asp Tyr Val Leu Asn Gln Gly Arg Cys Ser Asp Ser
   340 345 350
   Val Gly Ile Phe Pro Glu Phe Ile Asp Gly Ser Gly Tyr Leu Ile Leu
   355 360 365
   Pro Thr Thr Gly Arg Phe Phe Gln Ile Pro Tyr Gly Cys Leu Val Pro
   370 375 380
   Gln Lys Val Glu Asn Leu Leu Val Ala Gly Arg Cys Ile Ser Ala Gly
   385 390 395 400
   Val Val Ala His Thr Ser Met Arg Asn Met Met Cys Cys Ala Val Thr
   405 410 415
   Gly Glu Ala Ala Gly Thr Ala Ala Val Val Ser Leu Gln Gln Asn Cys
   420 425 430
   Thr Val Arg Gln Val Ala Ile Pro Asp Leu Gln Asn Thr Leu Gln Gln
   435 440 445
   Gln Gly Val Arg Leu Ala
   450
<210> 11
   <211> 253
   <212> PRT
   <213> Escherichia coli
<400> 11
   Met Ser Ala Lys Arg Arg Leu Leu Ile Ala Cys Thr Leu Ile Thr Ala
   1 5 10 15
   Ile Tyr His Phe Pro Ala Tyr Ser Ser Leu Glu Tyr Lys Gly Thr Phe
   20 25 30
   Gly Ser Ile Asn Ala Gly Tyr Ala Asp Trp Asn Ser Gly Phe Val Asn
   35 40 45
   Thr His Arg Gly Glu Val Trp Lys Val Thr Ala Asp Phe Gly Val Asn
   50 55 60
   Phe Lys Glu Ala Glu Phe Tyr Ser Phe Tyr Glu Ser Asn Val Leu Asn
   65 70 75 80
   His Ala Val Ala Gly Arg Asn His Thr Val Ser Ala Met Thr His Val
   85 90 95
   Arg Leu Phe Asp Ser Asp Met Thr Phe Phe Gly Lys Ile Tyr Gly Gln
   100 105 110
   Trp Asp Asn Ser Trp Gly Asp Asp Leu Asp Met Phe Tyr Gly Phe Gly
   115 120 125
   Tyr Leu Gly Trp Asn Gly Glu Trp Gly Phe Phe Lys Pro Tyr Ile Gly
   130 135 140
   Leu His Asn Gln Ser Gly Asp Tyr Val Ser Ala Lys Tyr Gly Gln Thr
   145 150 155 160
   Asn Gly Trp Asn Gly Tyr Val Val Gly Trp Thr Ala Val Leu Pro Phe
   165 170 175
   Thr Leu Phe Asp Glu Lys Phe Val Leu Ser Asn Trp Asn Glu Ile Glu
   180 185 190
   Leu Asp Arg Asn Asp Ala Tyr Thr Glu Gln Gln Phe Gly Arg Asn Gly
   195 200 205
   Leu Asn Gly Gly Leu Thr Ile Ala Trp Lys Phe Tyr Pro Arg Trp Lys
   210 215 220
   Ala Ser Val Thr Trp Arg Tyr Phe Asp Asn Lys Leu Gly Tyr Asp Gly
   225 230 235 240
   Phe Gly Asp Gln Met Ile Tyr Met Leu Gly Tyr Asp Phe
   245 250
<210> 12
   <211> 492
   <212> PRT
   <213> Escherichia coli
<400> 12
   Met Ala Ser Leu Ile Gly Leu Ala Val Cys Thr Gly Asn Ala Phe Ser
   1 5 10 15
   Pro Ala Leu Ala Ala Glu Ala Lys Gln Pro Asn Leu Val Ile Ile Met
   20 25 30
   Ala Asp Asp Leu Gly Tyr Gly Asp Leu Ala Thr Tyr Gly His Gln Ile
   35 40 45
   Val Lys Thr Pro Asn Ile Asp Arg Leu Ala Gln Glu Gly Val Lys Phe
   50 55 60
   Thr Asp Tyr Tyr Ala Pro Ala Pro Leu Ser Ser Pro Ser Arg Ala Gly
   65 70 75 80
   Leu Leu Thr Gly Arg Met Pro Phe Arg Thr Gly Ile Arg Ser Trp Ile
   85 90 95
   Pro Ser Gly Lys Asp Val Ala Leu Gly Arg Asn Glu Leu Thr Ile Ala
   100 105 110
   Asn Leu Leu Lys Ala Gln Gly Tyr Asp Thr Ala Met Met Gly Lys Leu
   115 120 125
   His Leu Asn Ala Gly Gly Asp Arg Thr Asp Gln Pro Gln Ala Gln Asp
   130 135 140
   Met Gly Phe Asp Tyr Ser Leu Ala Asn Thr Ala Gly Phe Val Thr Asp
   145 150 155 160
   Ala Thr Leu Asp Asn Ala Lys Glu Arg Pro Arg Tyr Gly Met Val Tyr
   165 170 175
   Pro Thr Gly Trp Leu Arg Asn Gly Gln Pro Thr Pro Arg Ala Asp Lys
   180 185 190
   Met Ser Gly Glu Tyr Val Ser Ser Glu Val Val Asn Trp Leu Asp Asn
   195 200 205
   Lys Lys Asp Ser Lys Pro Phe Phe Leu Tyr Val Ala Phe Thr Glu Val
   210 215 220
   His Ser Pro Leu Ala Ser Pro Lys Lys Tyr Leu Asp Met Tyr Ser Gln
   225 230 235 240
   Tyr Met Ser Ala Tyr Gln Lys Gln His Pro Asp Leu Phe Tyr Gly Asp
   245 250 255
   Trp Ala Asp Lys Pro Trp Arg Gly Val Gly Glu Tyr Tyr Ala Asn Ile
   260 265 270
   Ser Tyr Leu Asp Ala Gln Val Gly Lys Val Leu Asp Lys Ile Lys Ala
   275 280 285
   Met Gly Glu Glu Asp Asn Thr Ile Val Ile Phe Thr Ser Asp Asn Gly
   290 295 300
   Pro Val Thr Arg Glu Ala Arg Lys Val Tyr Glu Leu Asn Leu Ala Gly
   305 310 315 320
   Glu Thr Asp Gly Leu Arg Gly Arg Lys Asp Asn Leu Trp Glu Gly Gly
   325 330 335
   Ile Arg Val Pro Ala Ile Ile Lys Tyr Gly Lys His Leu Pro Gln Gly
   340 345 350
   Met Val Ser Asp Thr Pro Val Tyr Gly Leu Asp Trp Met Pro Thr Leu
   355 360 365
   Ala Lys Met Met Asn Phe Lys Leu Pro Thr Asp Arg Thr Phe Asp Gly
   370 375 380
   Glu Ser Leu Val Pro Val Leu Glu Gln Lys Ala Leu Lys Arg Glu Lys
   385 390 395 400
   Pro Leu Ile Phe Gly Ile Asp Met Pro Phe Gln Asp Asp Pro Thr Asp
   405 410 415
   Glu Trp Ala Ile Arg Asp Gly Asp Trp Lys Met Ile Ile Asp Arg Asn
   420 425 430
   Asn Lys Pro Lys Tyr Leu Tyr Asn Leu Lys Ser Asp Arg Tyr Glu Thr
   435 440 445
   Leu Asn Leu Ile Gly Lys Lys Pro Asp Ile Glu Lys Gln Met Tyr Gly
   450 455 460
   Lys Phe Leu Lys Tyr Lys Thr Asp Ile Asp Asn Asp Ser Leu Met Lys
   465 470 475 480
   Ala Arg Gly Asp Lys Pro Glu Ala Val Thr Trp Gly
   485 490
<210> 13
   <211> 345
   <212> PRT
   <213> Escherichia coli
<400> 13
   Leu Ile Ser Leu Ser Phe Ile Pro Val Met Ser Ala Leu Pro Gly Pro
   1               5                   10                  15
   Ile Ala Lys Gly Phe Arg Asn Glu Arg Gly Phe Val Thr Thr Thr Ile
               20                  25                  30
   Cys Ala Met Gly Glu Leu Leu Ala Glu Phe Leu Ser Arg Asn Pro His
           35                  40                  45
   Gln Lys Phe Thr Gln Pro Gly Glu Phe Ile Gly Pro Phe Pro Ser Gly
       50                  55                  60
   Ala Pro Ala Ile Phe Ala Ala Gln Val Ala Lys Leu Ser His Arg Ala
   65                  70                  75                  80
   Ile Phe Phe Gly Cys Val Gly Asn Asp Asp Phe Ala Arg Leu Ile Ile
                   85                  90                  95
   Glu Arg Leu Arg His Glu Gly Val Ile Thr Asp Gly Ile His Val Met
               100                 105                 110
   Asn Asn Ala Val Thr Gly Thr Ala Phe Val Ser Tyr Gln Asn Pro Gln
           115                 120                 125
   Gln Arg Asp Phe Val Phe Asn Ile Pro Asn Ser Ala Cys Gly Leu Phe
       130                 135                 140
   Thr Ala Glu His Ile Asp Lys Asp Leu Leu Lys Gln Cys Asn His Leu
   145                 150                 155                 160
   His Ile Val Gly Ser Ser Leu Phe Ser Phe Arg Met Ile Asp Val Met
                   165                 170                 175
   Arg Lys Ala Ile Thr Thr Ile Lys Ser Ala Gly Gly Thr Val Ser Phe
               180                 185                 190
   Asp Pro Asn Ile Arg Lys Glu Met Leu Ser Ile Pro Glu Met Ala Gln
           195                 200                 205
   Ala Leu Asp Tyr Leu Ile Glu Tyr Thr Asp Ile Phe Ile Pro Ser Glu
       210                 215                 220
   Ser Glu Leu Pro Phe Phe Ala Arg His Lys Asn Leu Ser Glu Glu Gln
   225                 230                 235                 240
   Ile Val Ser Asp Leu Leu His Gly Gly Val Lys His Val Ala Ile Lys
                   245                 250                 255
   Arg Ala Gln Arg Gly Ala Ser Tyr Tyr Lys Leu Lys Asn Gly Thr Leu
               260                 265                 270
   His Ala Gln His Val Ala Gly His Asp Ile Glu Ile Ile Asp Pro Thr
           275                 280                 285
   Gly Ala Gly Asp Cys Phe Gly Ala Thr Phe Ile Thr Leu Phe Leu Ser
       290                 295                 300
   Gly Phe Pro Ala His Lys Ala Leu Gln Tyr Ala Asn Ala Ser Gly Ala
   305                 310                 315                 320
   Leu Ala Val Met Arg Gln Gly Pro Met Glu Gly Ile Ser Ser Leu Ala
                   325                 330                 335
   Asp Ile Glu Asp Phe Leu Gln Gln His
               340                 345
<210> 14
   <211> 192
   <212> PRT
   <213> Escherichia coli
<400> 14
   Met Tyr Met Pro Gly Lys Gln Met Leu Cys Cys Ile Leu Ile Ser Ile
   1               5                   10                  15
   Ile Ser Glu Gly Asp Met Lys Ile Phe Ile Ser Leu Phe Leu Phe Ile
               20                  25                  30
   Ile Ser Thr Asn Ser Phe Ala Asp Asp Ile Thr His Ala Gly Val Val
           35                  40                  45
   Arg Ile Glu Gly Leu Ile Thr Glu Lys Thr Cys Ile Ile Ser Asp Glu
       50                  55                  60
   Ser Lys Asn Phe Thr Val Asn Met Pro Asp Val Pro Ser Ser Ser Val
   65 70 75 80
   Arg Ser Ala Gly Asp Val Thr Glu Lys Val Tyr Phe Ser Ile Thr Leu
   85 90 95
   Thr Arg Cys Gly Ser Asp Val Gly Asn Ala Tyr Ile Lys Phe Thr Gly
   100 105 110
   Asn Thr Val Ser Glu Asp Ala Ser Leu Tyr Lys Leu Glu Asp Gly Ser
   115 120 125
   Val Glu Gly Leu Ala Leu Thr Ile Phe Asp Lys Asn Lys Gly Ser Ile
   130 135 140
   Ser Asn Asp Val Lys Ser Met Val Phe Ser Leu Thr Ser Ser Val Asp
   145 150 155 160
   Asn Ile Leu His Phe Phe Ala Ala Tyr Lys Ala Leu Lys Asn Asn Val
   165 170 175
   Gln Pro Gly Asp Ala Asn Ala Ser Val Ser Phe Ile Val Thr Tyr Asp
   180 185 190
<210> 15
   <211> 201
   <212> PRT
   <213> Escherichia coli
<400> 15
   Met Ile Lys Phe Arg Leu Tyr Ile Pro Pro Val Ile Leu Gly Phe Val
   1 5 10 15
   Ile Val Pro Leu Leu Val Trp Pro Thr Val Ile Ala Leu Ala Val Leu
   20 25 30
   Ile Phe Thr Leu Thr Phe Leu Ala Glu Ile Ile Phe Ser Phe Pro Leu
   35 40 45
   Leu Val Val Arg Ile Ser Leu Gln Glu Leu Gln Leu Glu Leu Leu Val
   50 55 60
   Val Tyr Ala Leu Phe Phe Ser Val Met Gly Gly Ile Gly Trp Gln Phe
   65 70 75 80
   Ser Arg Arg Thr Pro Pro Glu Leu Lys Asn Arg Leu His Cys Trp Leu
                   85                  90                  95
   Val Phe Ser Pro Val Tyr Phe Trp Leu Ile Leu Ser Asn Phe Ile Leu
               100                 105                 110
   Tyr Ile Ser Pro Glu Lys Ser Ala Leu Leu Glu Asn Ile Arg Asn Phe
           115                 120                 125
   Phe Leu Thr Phe Val Trp Leu Pro Leu Asn Phe Ser Pro Phe Trp Pro
       130                 135                 140
   Gln Pro Trp Thr Asp Phe Val Gly Pro Ile Ser Ala Gln Leu Gly Phe
   145                 150                 155                 160
   Ala Leu Gly Tyr Tyr Cys Gln Trp Arg Ser Lys Asn Arg Ser His Arg
                   165                 170                 175
   Lys Lys Trp Gly Asp Trp Val Thr Cys Leu Ser Leu Ala Ile Leu Ala
               180                 185                 190
   Leu Gly Pro Leu Phe Asn Tyr Leu Gln
           195                 200
<210> 16
   <211> 234
   <212> PRT
   <213> Escherichia coli
<400> 16
   Met Lys Phe Asn Leu Ser Asn Leu Ser Ala Val Leu Leu Ala Ser Gly
   1               5                   10                  15
   Met Leu Met Ser Thr Ala Val Thr Ala Ala Pro Gly Asp Ala Thr Gln
               20                  25                  30
   Phe Gly Gly Ala Asp Thr Asp Trp Ser Thr Val Asp Tyr Pro Arg Leu
           35                  40                  45
   Thr Asp Met Asp Asp Asn Val Asp Ser Met Gly Gly Lys Ile Arg Phe
       50                  55                  60
   Thr Gly Arg Val Val Lys Ala Thr Cys Lys Val Ala Thr Asp Ser Lys
   65 70 75 80
   Gln Ile Glu Val Val Leu Pro Val Val Pro Ser Asn Leu Phe Thr Gly
   85 90 95
   Ile Asp Val Glu Ala Gln Gly Ala Ser Asn Gln Thr Asp Phe Asn Ile
   100 105 110
   Asn Leu Thr Glu Cys Ser Asn Thr Asp Asp Gln Lys Ile Glu Phe Arg
   115 120 125
   Phe Thr Gly Thr Ala Asp Ser Ala Asn Lys Thr Leu Ala Asn Glu Val
   130 135 140
   Glu Gly Ser Thr Asp Ala Asp Asn Ser Gly Asn Ala Gly Ala Thr Gly
   145 150 155 160
   Val Gly Ile Arg Ile Tyr Ser Lys Gly Thr Thr Asn Asn Gly Leu Ile
   165 170 175
   Asn Leu Asn Thr Thr Ala Ala Glu Gly Ser Ala Ser Thr Ala Ala Tyr
   180 185 190
   Thr Ile Pro Gly Asn Ala Thr Thr His Asp Phe Ser Ala Ala Phe Thr
   195 200 205
   Ala Gly Tyr Ala Gln Asn Gly Ser Thr Val Ala Pro Gly Val Val Lys
   210 215 220
   Ser Thr Ala Ser Phe Val Val Leu Tyr Glu
   225 230
<210> 17
   <211> 336
   <212> PRT
   <213> Escherichia coli
<400> 17
   Met Arg Ile His Thr Tyr Trp Tyr Arg Arg Tyr Phe Ile Leu Leu Ile
   1 5 10 15
   Ile Ile Phe Ser Asn Val Leu Ser Ser Ile Ala Asn Ala Glu Asp Met
   20 25 30
   Gly Arg Glu Arg Ala Tyr Cys Tyr Pro Gly Ser Pro Ser Asn Asn Thr
   35 40 45
   Thr Pro Ala Ser Phe Ser Tyr Asn Phe Gly Thr Ile Val Val Ser Asp
   50 55 60
   Val Asn Lys Asn Ala Pro Gly Thr Val Leu Pro Ser Gln Ile Trp Lys
   65 70 75 80
   Val Gly Thr Tyr Lys Ala Tyr Cys Asn Ser Leu Asp Asp Tyr Glu Ile
   85 90 95
   Tyr Phe Ser Ala Val Ser Gly Ile Asp Pro Ser Gly Ala Ser Gly Asp
   100 105 110
   His Gln Gly Ser Asp Val Phe Ile Pro Leu Thr His Glu Ile Ser Val
   115 120 125
   Ser Thr His Ile Lys Leu Tyr Asn Gln Asn Gly Thr Met Thr Asp Lys
   130 135 140
   Ile Val Pro Phe Glu Asn Tyr Asn Thr Asn Tyr Pro Gly Asp Arg Ser
   145 150 155 160
   Lys Pro Ser Asn Trp Ala Ser Gly Thr Glu Gly Tyr Ile Lys Ile Arg
   165 170 175
   Ile Asp Lys Lys Ile Ile Ser Asp Val Ser Leu Ser Asn Val Leu Leu
   180 185 190
   Val Ser Leu Tyr Val Ser Gln Ile Pro Thr Glu His Gly Pro Ile Pro
   195 200 205
   Val Phe Asn Ala Tyr Ile Gly Asn Leu Asn Ile Gln Val Pro Gln Gly
   210 215 220
   Cys Thr Ile Asn Glu Gly Thr Ser Phe Thr Val Asn Met Pro Asp Val
   225 230 235 240
   Trp Ala Ser Glu Leu Ser Arg Ala Gly Ala Gly Ala Lys Pro Ala Gly
   245 250 255
   Val Thr Pro Val Ala Thr Thr Ile Pro Ile Asn Cys Thr Asn Lys Asp
   260 265 270
   Thr Asp Ala Val Met Thr Leu Val Phe Asp Gly Asn Ile Ser Ala Thr
   275 280 285
   Arg Asp Thr Asn Gly Lys Gln Ser Ile Ile Gln Ala Gln Asp Asn Pro
   290 295 300
   Asp Val Gly Ile Met Ile Met Asp Ser Gln Gln Asn Ser Val Asp Leu
   305 310 315 320
   Asn Ala Leu Ala Thr Ser Val Gly Val Pro Phe Arg Leu Val Glu Asn
   325 330 335
<210> 18
   <211> 864
   <212> PRT
   <213> Escherichia coli
<400> 18
   Met Asn Leu Lys Leu Lys Arg Cys Glu Tyr Trp Met Ala Ala Gln Lys
   1 5 10 15
   Gln Met Lys Arg Val Val Pro Leu Leu Leu Val Ile Met Pro Ala Cys
   20 25 30
   Ser Ile Ala Gly Met Arg Phe Asn Pro Ala Phe Leu Ser Gly Asp Thr
   35 40 45
   Glu Ala Val Ala Asp Leu Ser Arg Phe Glu Lys Gly Met Thr Tyr Leu
   50 55 60
   Pro Gly Ser Tyr Glu Val Glu Val Trp Val Asn Asp Ser Pro Leu Leu
   65 70 75 80
   Ser Arg Thr Val Thr Phe Lys Ala Asp Asp Glu Asn Gln Leu Ile Pro
   85 90 95
   Cys Leu Ser Leu Ala Asp Leu Leu Ser Leu Gly Ile Asn Lys Asn Ala
   100 105 110
   Leu Pro Glu Gln Ala Leu Ala Ser Ser Glu Asn Ser Cys Leu Asp Leu
   115 120 125
   Arg Ile Trp Phe Pro Asp Val His Tyr Met Pro Glu Leu Asp Ala Gln
   130 135 140
   Arg Leu Lys Leu Thr Phe Pro Gln Ala Ile Ile Lys Arg Asp Ala Arg
   145 150 155 160
   Gly Tyr Ile Pro Pro Glu Gln Trp Asp Asn Gly Ile Thr Ala Phe Leu
   165 170 175
   Leu Asn Tyr Asp Phe Ser Gly Asn Asn Asp Arg Gly Asp Tyr Ser Ser
   180 185 190
   Asn Asn Tyr Tyr Leu Asn Leu Arg Ala Gly Ile Asn Ile Gly Ala Trp
   195 200 205
   Arg Phe Arg Asp Tyr Ser Thr Trp Ser Arg Gly Ser Asn Ser Ala Gly
   210 215 220
   Lys Leu Glu His Ile Ser Ser Thr Leu Gln Arg Val Ile Ile Pro Phe
   225 230 235 240
   Arg Ser Glu Leu Thr Leu Gly Asp Thr Trp Ser Ser Ser Asp Val Phe
   245 250 255
   Asp Ser Val Ser Ile Arg Gly Ile Lys Leu Glu Ser Asp Glu Asn Met
   260 265 270
   Leu Pro Asp Ser Gln Ser Gly Phe Ala Pro Thr Val Arg Gly Ile Ala
   275 280 285
   Lys Ser Arg Ala Gln Val Thr Ile Lys Gln Asn Gly Tyr Val Ile Tyr
   290 295 300
   Gln Thr Tyr Met Pro Pro Gly Pro Phe Glu Ile Ser Asp Leu Asn Pro
   305 310 315 320
   Thr Ser Ser Ala Gly Asp Leu Glu Val Thr Ile Lys Glu Ser Asp Asn
   325 330 335
   Ser Glu Thr Val Tyr Thr Val Pro Tyr Ala Ala Val Pro Ile Leu Gln
   340 345 350
   Arg Glu Gly His Leu Lys Tyr Ser Thr Thr Val Gly Gln Tyr Arg Ser
   355 360 365
   Asn Ser Tyr Asn Gln Lys Ser Pro Tyr Val Phe Gln Gly Glu Leu Ile
   370 375 380
   Trp Gly Leu Pro Trp Asp Ile Thr Ala Tyr Gly Gly Ala Gln Phe Ser
   385 390 395 400
   Glu Asp Tyr Arg Ala Leu Ala Leu Gly Leu Gly Leu Asn Leu Gly Val
   405 410 415
   Phe Gly Ala Thr Ser Phe Asp Val Thr Gln Ala Asn Ser Ser Leu Val
   420 425 430
   Asp Gly Ser Lys His Gln Gly Gln Ser Tyr Arg Phe Leu Tyr Ser Lys
   435 440 445
   Ser Leu Val Gln Thr Gly Thr Ala Phe His Ile Ile Gly Tyr Arg Tyr
   450 455 460
   Ser Thr Gln Gly Phe Tyr Thr Leu Ser Asp Thr Thr Tyr Gln Gln Met
   465 470 475 480
   Ser Gly Thr Val Val Asp Pro Lys Thr Leu Asp Asp Lys Asp Tyr Val
   485 490 495
   Tyr Asn Trp Asn Asp Phe Tyr Asn Leu Arg Tyr Ser Lys Arg Gly Lys
   500 505 510
   Phe Gln Ala Ser Val Ser Gln Pro Phe Gly Asn Tyr Gly Ser Met Tyr
   515 520 525
   Leu Ser Ala Ser Gln Gln Thr Tyr Trp Asn Thr Asp Lys Lys Asp Ser
   530 535 540
   Leu Tyr Gln Val Gly Tyr Asn Thr Ser Ile Lys Gly Ile Tyr Leu Asn
   545 550 555 560
   Val Ala Trp Asn Tyr Ser Lys Ser Pro Gly Thr Asn Ala Asp Lys Ile
   565 570 575
   Val Ser Leu Asn Val Ser Leu Pro Ile Ser Asn Trp Leu Ser Ser Thr
   580 585 590
   Asn Asp Gly Arg Ser Ser Ser Asn Ala Met Thr Ala Thr Tyr Gly Tyr
           595                 600                 605
   Ser Gln Asp Asn His Gly Gln Val Asn Gln Tyr Thr Gly Val Ser Gly
       610                 615                 620
   Ser Leu Leu Glu Gln His Asn Leu Ser Tyr Asn Ile Gln His Gly Phe
   625                 630                 635                 640
   Ala Asn Gln Asp Asn Ser Ser Ser Gly Ser Val Gly Val Asn Tyr Arg
                   645                 650                 655
   Gly Ala Tyr Gly Ser Leu Asn Ser Ala Tyr Ser Tyr Asp Asn Glu Gly
               660                 665                 670
   Asn Gln Gln Ile Asn Tyr Gly Ile Ser Gly Ala Leu Val Val His Glu
           675                 680                 685
   Asn Gly Leu Thr Leu Ser Gln Pro Leu Gly Glu Thr Asn Val Leu Ile
       690                 695                 700
   Lys Ala Pro Gly Ala Asn Asn Val Asp Val Gln Arg Gly Thr Gly Ile
   705                 710                 715                 720
   Ser Thr Asp Trp Arg Gly Tyr Ala Val Val Pro Tyr Ala Thr Glu Tyr
                   725                 730                 735
   Arg Arg Asn Asn Ile Ser Leu Asp Pro Met Ser Met Asn Met His Thr
               740                 745                 750
   Glu Leu Asp Ile Thr Ser Thr Glu Val Ile Pro Gly Lys Gly Ala Leu
           755                 760                 765
   Val Arg Ala Glu Phe Ala Ala His Ile Gly Ile Arg Gly Leu Phe Thr
       770                 775                 780
   Val Arg Tyr Arg Asn Lys Ser Val Pro Phe Gly Ala Thr Ala Ser Ala
   785                 790                 795                 800
   Gln Ile Lys Asn Ser Ser Gln Ile Thr Gly Ile Val Gly Asp Asn Gly
                   805                 810                 815
   Gln Leu Tyr Leu Ser Gly Leu Pro Leu Glu Gly Val Ile Asn Ile Gln
   820 825 830
   Trp Gly Asp Gly Val Gln Gln Lys Cys Gln Ala Asn Tyr Lys Leu Pro
   835 840 845
   Glu Thr Glu Leu Asp Asn Pro Val Ser Tyr Ala Thr Leu Glu Cys Arg
   850 855 860
<210> 19
   <211> 169
   <212> PRT
   <213> Escherichia coli
<400> 19
   Met Gly Ala Ile Tyr Val Lys Arg Leu Ile Leu Ser Val Ala Leu Ile
   1 5 10 15
   Ile Pro Ile Ala Ser Asn Ala Ser Asp Ala Leu Asn Gln Pro Ser Ser
   20 25 30
   Ser Leu Asn Asp Gly Val Glu Thr Phe Phe Ile Ser Cys Phe Asp Met
   35 40 45
   Pro Gln Glu Thr Thr Thr Asp Met Asp Ala Cys Gln Arg Val Gln Leu
   50 55 60
   Ala Gln Val Ser Trp Val Lys Asn Lys Tyr Ser Val Ala Ala Leu Asn
   65 70 75 80
   Arg Leu Lys Gln Asp Asn Lys Asp Asp Pro Gln Arg Leu Gln Glu Leu
   85 90 95
   Thr Ala Ser Phe Asn Ala Glu Ser Glu Ala Trp Thr Glu Leu Ile Glu
   100 105 110
   Lys Ala Ser Lys Ser Val Gln Val Asp Tyr Val Gly Gly Thr Ile Ala
   115 120 125
   Gly Thr Ala Val Ala Ser Arg Gln Ile Gly Leu Leu Glu Leu Gln Ser
   130 135 140
   His Asp Ile Trp Glu His Trp Leu Arg Ser Arg Gly Leu Asn Ser Ser
   145 150 155 160
   Ser Phe Ala Arg Thr Lys Val Gln Ile
   165
<210> 20
   <211> 713
   <212> PRT
   <213> Escherichia coli
<400> 20
   Met Ala Met Phe Thr Pro Ser Phe Ser Gly Leu Lys Gly Arg Ala Leu
   1 5 10 15
   Phe Ser Leu Leu Phe Ala Ala Pro Met Ile His Ala Thr Asp Ser Val
   20 25 30
   Thr Thr Lys Asp Gly Glu Thr Ile Thr Val Thr Ala Asp Ala Asn Thr
   35 40 45
   Ala Thr Glu Ala Thr Asp Gly Tyr Gln Pro Leu Ser Thr Ser Thr Ala
   50 55 60
   Thr Leu Thr Asp Met Pro Met Leu Asp Ile Pro Gln Val Val Asn Thr
   65 70 75 80
   Val Ser Asp Gln Val Leu Glu Asn Gln Asn Ala Thr Thr Leu Asp Glu
   85 90 95
   Ala Leu Tyr Asn Val Ser Asn Val Val Gln Thr Asn Thr Leu Gly Gly
   100 105 110
   Thr Gln Asp Ala Phe Val Arg Arg Gly Phe Gly Ala Asn Arg Asp Gly
   115 120 125
   Ser Ile Met Thr Asn Gly Leu Arg Thr Val Leu Pro Arg Ser Phe Asn
   130 135 140
   Ala Ala Thr Glu Arg Val Glu Val Leu Lys Gly Pro Ala Ser Thr Leu
   145 150 155 160
   Tyr Gly Ile Leu Asp Pro Gly Gly Leu Ile Asn Val Val Thr Lys Arg
   165 170 175
   Pro Glu Lys Thr Phe His Gly Ser Val Ser Ala Thr Ser Ser Ser Phe
   180 185 190
   Gly Gly Gly Thr Gly Gln Leu Asp Ile Thr Gly Pro Ile Glu Gly Thr
   195 200 205
   Gln Leu Ala Tyr Arg Leu Thr Gly Glu Val Gln Asp Glu Asp Tyr Trp
   210 215 220
   Arg Asn Phe Gly Lys Glu Arg Ser Thr Phe Ile Ala Pro Ser Leu Thr
   225 230 235 240
   Trp Phe Gly Asp Asn Ala Thr Val Thr Met Leu Tyr Ser His Arg Asp
   245 250 255
   Tyr Lys Thr Pro Phe Asp Arg Gly Thr Ile Phe Asp Leu Thr Thr Lys
   260 265 270
   Gln Pro Val Asn Val Asp Arg Lys Ile Arg Phe Asp Glu Pro Phe Asn
   275 280 285
   Ile Thr Asp Gly Gln Ser Asp Leu Ala Gln Leu Asn Ala Glu Tyr His
   290 295 300
   Leu Asn Ser Gln Trp Thr Ala Arg Phe Asp Tyr Ser Tyr Ser Gln Asp
   305 310 315 320
   Lys Tyr Ser Asp Asn Gln Ala Arg Val Thr Ala Tyr Asp Ala Thr Thr
   325 330 335
   Gly Thr Leu Thr Arg Arg Val Asp Ala Thr Gln Gly Ser Thr Gln Arg
   340 345 350
   Met His Ala Thr Arg Ala Asp Leu Gln Gly Asn Val Asp Ile Ala Gly
   355 360 365
   Phe Tyr Asn Glu Ile Leu Gly Gly Val Ser Tyr Glu Tyr Tyr Asp Leu
   370 375 380
   Leu Arg Thr Asp Met Ile Arg Cys Lys Lys Ala Lys Asp Phe Asn Ile
   385 390 395 400
   Tyr Asn Pro Val Tyr Gly Asn Thr Ser Lys Cys Thr Thr Val Ser Ala
   405 410 415
   Ser Asp Ser Asp Gln Thr Ile Lys Gln Glu Asn Tyr Ser Ala Tyr Ala
   420 425 430
   Gln Asp Ala Leu Tyr Leu Thr Asp Asn Trp Ile Ala Val Ala Gly Ile
   435 440 445
   Arg Tyr Gln Tyr Tyr Thr Gln Tyr Ala Gly Lys Gly Arg Pro Phe Asn
   450 455 460
   Val Asn Thr Asp Ser Arg Asp Glu Gln Trp Thr Pro Lys Leu Gly Leu
   465 470 475 480
   Val Tyr Lys Leu Thr Pro Ser Val Ser Leu Phe Ala Asn Tyr Ser Gln
   485 490 495
   Thr Phe Met Pro Gln Ser Ser Ile Ala Ser Tyr Ile Gly Asp Leu Pro
   500 505 510
   Pro Glu Ser Ser Asn Ala Tyr Glu Val Gly Ala Lys Phe Glu Leu Phe
   515 520 525
   Asp Gly Ile Thr Ala Asp Ile Ala Leu Phe Asp Ile His Lys Arg Asn
   530 535 540
   Val Leu Tyr Thr Glu Ser Ile Gly Asp Glu Thr Ile Ala Lys Thr Ala
   545 550 555 560
   Gly Arg Val Arg Ser Arg Gly Val Glu Val Asp Leu Ala Gly Ala Leu
   565 570 575
   Thr Glu Asn Ile Asn Ile Ile Ala Ser Tyr Gly Tyr Thr Asp Ala Lys
   580 585 590
   Val Leu Glu Asp Pro Asp Tyr Ala Gly Lys Pro Leu Pro Asn Val Pro
   595 600 605
   Arg His Thr Gly Ser Leu Phe Leu Thr Tyr Asp Ile His Asn Met Pro
   610 615 620
   Gly Asn Asn Thr Leu Thr Phe Gly Gly Gly Gly His Gly Val Ser Arg
   625 630 635 640
   Arg Ser Ala Thr Asn Gly Ala Asp Tyr Tyr Leu Pro Gly Tyr Phe Val
   645 650 655
   Ala Asp Ala Phe Ala Ala Tyr Lys Met Lys Leu Gln Tyr Pro Val Thr
   660 665 670
   Leu Gln Leu Asn Val Lys Asn Leu Phe Asp Lys Thr Tyr Tyr Thr Ser
   675 680 685
   Ser Ile Ala Thr Asn Asn Leu Gly Asn Gln Ile Gly Asp Pro Arg Glu
   690 695 700
   Val Gln Phe Thr Val Lys Met Glu Phe
   705 710
<210> 21
   <211> 606
   <212> PRT
   <213> Escherichia coli
<400> 21
   Met Lys Ile Ser Trp Asn Tyr Ile Phe Lys Asn Lys Trp Arg Phe His
   1 5 10 15
   Ile Thr Ser Ile Ser Leu Phe Leu Ile Met Leu Ala Val Ser Ile Ala
   20 25 30
   Phe Leu His Leu Arg Phe Asn Thr Leu Ser Ser Thr Asp Lys Met Arg
   35 40 45
   Leu Glu Met Tyr Lys Ser Thr Leu Tyr Ser Thr Ile Glu Gln Phe Tyr
   50 55 60
   Val Leu Pro Tyr Met Leu Ser Thr Asp His Ile Ile Arg Gln Ala Val
   65 70 75 80
   Ile Thr Pro Asp Asp Met Thr Ser Ser Glu Leu Asn Gln Arg Ile Ala
   85 90 95
   His Phe Asn Thr Gln Leu Lys Thr Ala Ala Ile Phe Ile Leu Asp Thr
   100 105 110
   Gln Gly Lys Ala Ile Ala Ser Ser Asn Trp Gln Asp Pro Gly Ser Tyr
   115 120 125
   Val Gly Gln Asn Tyr Ser Tyr Arg Pro Tyr Tyr Lys His Ala Met Ser
   130 135 140
   Gly Leu Asn Gly Arg Phe Tyr Gly Ile Gly Ser Thr Thr Asn Thr Pro
   145 150 155 160
   Gly Phe Phe Leu Ser Thr Ser Ile Lys Asp Lys Gly Lys Ile Val Gly
   165 170 175
   Val Val Val Val Lys Ile Ser Leu Asn Glu Ile Glu Lys Ala Trp Ala
   180 185 190
   Glu Gly Pro Glu Asn Ile Ile Val Asn Asp Glu His Gly Ile Ile Phe
   195 200 205
   Leu Ser Ser Lys Ser Pro Trp Arg Met Arg Thr Leu Gln Pro Leu Pro
   210 215 220
   Val Gln Ala Lys Gln Lys Leu Gln Ser Thr Arg Gln Tyr Ser Leu Asp
   225 230 235 240
   Asn Leu Leu Pro Ala Asp Tyr Tyr Pro Cys Tyr Thr Val Ser Asn Phe
   245 250 255
   Thr Phe Leu Lys Asp Lys Lys Glu Gln Leu Cys Leu Phe Pro Gln Tyr
   260 265 270
   Tyr Thr Gln Gln Ile Ala Ile Pro Glu Phe Asn Trp Lys Met Thr Ile
   275 280 285
   Met Val Pro Leu Asp Asn Leu Tyr Trp Ser Trp Ala Ile Ser Leu Val
   290 295 300
   Ile Thr Leu Ile Ile Tyr Leu Leu Phe Leu Leu Phe Ile Lys Tyr Trp
   305 310 315 320
   Arg Met Arg Ser His Ala Gln Gln Leu Leu Thr Leu Ala Asn Glu Thr
   325 330 335
   Leu Glu Lys Gln Val Lys Glu Arg Thr Ser Ala Leu Glu Leu Ile Asn
   340 345 350
   Gln Lys Leu Ile Gln Glu Ile Lys Glu Arg Ser Gln Ala Glu Gln Val
   355 360 365
   Leu Gln Ile Thr Arg Ser Glu Leu Ala Glu Ser Ser Lys Leu Ala Ala
   370 375 380
   Leu Gly Gln Met Ala Thr Glu Ile Ala His Glu Gln Asn Gln Pro Leu
   385 390 395 400
   Ala Ala Ile His Ala Leu Thr Asp Asn Ala Arg Thr Met Leu Lys Lys
   405 410 415
   Glu Met Tyr Pro Gln Val Glu Gln Asn Leu Lys His Ile Ile Ser Val
   420 425 430
   Ile Glu Arg Met Thr Gln Leu Ile Ser Glu Leu Lys Ala Phe Ala Ser
   435 440 445
   Arg His Arg Val Pro Lys Gly Ser Ala Asp Val Ile Lys Val Met Tyr
   450 455 460
   Ser Ala Val Ala Leu Leu Asn His Ser Met Glu Lys Asn Asn Ile Glu
   465 470 475 480
   Arg Arg Ile Lys Ala Pro Ser Met Pro Leu Phe Val Asn Cys Asp Glu
   485 490 495
   Leu Gly Leu Glu Gln Ile Phe Ser Asn Leu Ile Ser Asn Ala Leu Asp
   500 505 510
   Ser Met Glu Gly Ser Ser Tyr Lys Arg Leu Asp Ile Ala Ile Arg Gln
   515 520 525
   Ala Asn Asn Lys Val Ile Ile Thr Ile Lys Asp Ser Gly Gly Gly Phe
   530 535 540
   Ala Pro Glu Val Val Asp Arg Ile Phe Glu Pro Phe Phe Thr Thr Lys
   545 550 555 560
   Arg Arg Gly Met Gly Leu Gly Leu Ala Ile Val Ser Glu Ile Val Arg
   565 570 575
   Asn Ser Asn Gly Ala Leu His Ala Ser Asn His Pro Glu Gly Gly Ala
   580 585 590
   Val Met Thr Leu Thr Trp Pro Glu Trp Gly Glu Glu His Glu
   595 600 605
<210> 22
   <211> 101
   <212> PRT
   <213> Escherichia coli
<400> 22
   Val Leu Thr Pro Gln His Leu Arg Cys Val Leu Thr Cys Ser Asp Leu
   1 5 10 15
   Leu Thr Leu Leu Ser Gly Thr Val Met Ser Gln Met Pro Leu Tyr Phe
   20 25 30
   Leu Asn Thr Gln Lys Lys Leu Thr Ala His Tyr Glu Trp Leu Gln Ile
   35 40 45
   Asn Leu Thr Asp Thr Tyr Glu Leu Val Lys Arg Leu Met Pro Ile Pro
   50 55 60
   Ser Leu Asp Val Val Val Lys Val Gly Lys Leu Val Leu Pro Glu Lys
   65 70 75 80
   Gly His His Gly Phe Tyr Pro Glu Ala Gly Val Val Tyr Arg Thr Val
   85 90 95
   Ala Pro Glu Asn Pro
   100
<210> 23
   <211> 263
   <212> PRT
   <213> Escherichia coli
<400> 23
   Met Met Lys Asn Thr Gly Tyr Ile Leu Ala Leu Cys Leu Thr Ala Ser
   1 5 10 15
   Gly His Val Leu Ala His Asp Val Trp Ile Thr Gly Lys Gln Ala Glu
   20 25 30
   Asn Asn Val Thr Ala Glu Ile Gly Tyr Gly His Asn Phe Pro Ser Lys
               35                  40                  45
   Gly Thr Ile Pro Asp Arg Arg Asp Phe Phe Glu Asn Pro Arg Leu Tyr
       50                  55                  60
   Asn Gly Lys Glu Thr Ile Thr Leu Lys Pro Ala Ser Thr Asp Tyr Val
   65                  70                  75                  80
   Tyr Lys Thr Glu Ser Ala Ser Lys Asp Asn Gly Tyr Val Leu Ser Thr
                   85                  90                  95
   Tyr Met Lys Pro Gly Tyr Trp Ser Arg Thr Ser Ser Gly Trp Lys Pro
               100                 105                 110
   Val Ser Arg Glu Gly Arg Asn Asp Val Ala Tyr Cys Glu Phe Val Thr
           115                 120                 125
   Lys Tyr Ala Lys Ser Phe Ile Pro Gly Glu Gln Gln Met Pro Ala Gln
       130                 135                 140
   Leu Tyr Gln Ser Pro Thr Gly His Glu Leu Glu Ile Ile Pro Leu Ser
   145                 150                 155                 160
   Asp Ile Ser Arg Phe Ser Glu Asn Val Lys Leu Lys Val Leu Tyr Lys
                   165                 170                 175
   Thr Ser Pro Leu Ala Gly Ala Ile Met Glu Leu Asp Ser Val Ser Tyr
               180                 185                 190
   Leu Thr Ser Ser Arg His Thr His Ala Val Glu His Lys His Pro Val
           195                 200                 205
   His Lys Ala Glu Leu Thr Phe Val Thr Asn Glu Asp Gly Ile Val Thr
       210                 215                 220
   Val Pro Ser Leu His Ile Gly Gln Trp Leu Ala Lys Val Gln Asn Lys
   225                 230                 235                 240
   Lys Ser Phe Gln Asp Lys Ser Leu Cys Asp Glu Thr Val Asp Val Ala
                   245                 250                 255
   Thr Leu Ser Phe Ser Arg Asn
               260
<210> 24
   <211> 378
   <212> PRT
   <213> Escherichia coli
<400> 24
   Met Gly Lys Ile Lys Tyr Trp Leu Ile Val Gly Phe Ile Ile Leu Phe
   1               5                   10                  15
   Ala Ile Phe Tyr Ile Ala Ile Ser Asp Arg Asp Ser Thr Leu Ser Arg
               20                  25                  30
   Leu Lys Ser Ala Gly Glu Asn Gly Asp Val Glu Ala Gln Tyr Ala Leu
           35                  40                  45
   Gly Leu Met Tyr Leu Tyr Gly Glu Ile Leu Asp Val Asp Tyr Gln Gln
       50                  55                  60
   Ala Lys Ile Trp Tyr Glu Lys Ala Ala Asp Gln Asn Asp Pro Arg Ala
   65                  70                  75                  80
   Gln Ala Lys Leu Gly Val Met Tyr Ala Asn Gly Leu Gly Val Asn Gln
                   85                  90                  95
   Asp Tyr Gln Gln Ser Lys Leu Trp Tyr Glu Lys Ala Ala Ala Gln Asn
               100                 105                 110
   Asp Val Asp Ala Gln Phe Leu Leu Gly Glu Met Tyr Asp Asp Gly Leu
           115                 120                 125
   Gly Val Ser Gln Asp Tyr Gln His Ala Lys Met Trp Tyr Glu Lys Ala
       130                 135                 140
   Ala Ala Gln Asn Asp Glu Arg Ala Gln Val Asn Leu Ala Val Leu Tyr
   145                 150                 155                 160
   Ala Lys Gly Asn Gly Val Glu Gln Asp Tyr Arg Gln Ala Lys Ser Trp
                   165                 170                 175
   Tyr Glu Lys Ala Ala Ala Gln Asn Ser Pro Asp Ala Gln Phe Ala Leu
               180                 185                 190
   Gly Ile Leu Tyr Ala Asn Ala Asn Gly Val Glu Gln Asp Tyr Gln Gln
   195 200 205
   Ala Lys Asp Trp Tyr Glu Lys Ala Ala Glu Gln Asn Phe Ala Asn Ala
   210 215 220
   Gln Phe Asn Leu Gly Met Leu Tyr Tyr Lys Gly Glu Gly Val Lys Gln
   225 230 235 240
   Asn Phe Arg Gln Ala Arg Glu Trp Phe Glu Lys Ala Ala Ser Gln Asn
   245 250 255
   Gln Pro Asn Ala Gln Tyr Asn Leu Gly Gln Ile Tyr Tyr Tyr Gly Gln
   260 265 270
   Gly Val Thr Gln Ser Tyr Arg Gln Ala Lys Asp Trp Phe Glu Lys Ala
   275 280 285
   Ala Glu Lys Gly His Val Asp Ala Gln Tyr Asn Leu Gly Val Ile Tyr
   290 295 300
   Glu Asn Gly Glu Gly Val Ser Gln Asn Tyr Gln Gln Ala Lys Ala Trp
   305 310 315 320
   Tyr Glu Lys Ala Ala Ser Gln Asn Asp Ala Gln Ala Gln Phe Glu Leu
   325 330 335
   Gly Val Met Asn Glu Leu Gly Gln Gly Glu Ser Ile Asp Leu Lys Gln
   340 345 350
   Ala Arg His Tyr Tyr Glu Arg Ser Cys Asn Asn Gly Leu Lys Lys Gly
   355 360 365
   Cys Glu Arg Leu Lys Glu Leu Leu Tyr Lys
   370 375
<210> 25
   <211> 654
   <212> PRT
   <213> Escherichia coli
<400> 25
   Met Asn Val Ile Arg Thr Val Ile Cys Thr Leu Ile Ile Leu Pro Val
   1 5 10 15
   Gly Leu Gln Ala Ala Thr Ser His Ser Ser Met Val Lys Asp Thr Ile
   20 25 30
   Thr Ile Val Ala Thr Gly Asn Gln Asn Thr Val Phe Glu Thr Pro Ser
   35 40 45
   Met Val Ser Val Val Thr Asn Asp Thr Pro Trp Ser Gln Asn Ala Val
   50 55 60
   Thr Ser Ala Gly Met Leu Lys Gly Val Ala Gly Leu Ser Gln Thr Gly
   65 70 75 80
   Ala Gly Arg Thr Asn Gly Gln Thr Phe Asn Leu Arg Gly Tyr Asp Lys
   85 90 95
   Ser Gly Val Leu Val Leu Val Asp Gly Val Arg Gln Leu Ser Asp Met
   100 105 110
   Ala Lys Ser Ser Gly Thr Tyr Leu Asp Pro Ala Leu Val Lys Arg Ile
   115 120 125
   Glu Val Val Arg Gly Pro Asn Ser Ser Leu Tyr Gly Ser Gly Gly Leu
   130 135 140
   Gly Gly Val Val Asp Phe Arg Thr Ala Asp Ala Ala Asp Phe Leu Pro
   145 150 155 160
   Pro Gly Glu Thr Asn Gly Leu Ser Leu Trp Gly Asn Ile Ala Ser Gly
   165 170 175
   Asp His Ser Thr Gly Ser Gly Leu Thr Trp Phe Gly Lys Thr Gly Lys
   180 185 190
   Thr Asp Ala Leu Leu Ser Val Ile Met Arg Lys Arg Gly Asn Ile Tyr
   195 200 205
   Gln Ser Asp Gly Glu His Ala Pro Asn Lys Glu Lys Pro Ala Ala Leu
   210 215 220
   Phe Ala Lys Gly Ser Val Gly Ile Thr Asp Ser Asn Lys Ala Gly Ala
   225 230 235 240
   Ser Leu Arg Leu Tyr Arg Asn Asn Thr Thr Glu Pro Gly Asn Ser Thr
   245 250 255
   Gln Thr His Gly Asp Ser Gly Leu Arg Asp Arg Lys Thr Val Gln Asn
   260 265 270
   Asp Val Gln Phe Trp Tyr Gln Tyr Ala Pro Val Asp Asn Ser Leu Ile
   275 280 285
   Asn Val Lys Ser Thr Leu Tyr Leu Ser Asp Ile Thr Ile Lys Thr Asn
   290 295 300
   Gly His Asn Lys Thr Ala Glu Trp Arg Asn Asn Arg Thr Ser Gly Val
   305 310 315 320
   Asn Val Val Asn Arg Ser His Thr Leu Ile Phe Pro Gly Ala His Gln
   325 330 335
   Leu Ser Tyr Gly Ala Glu Tyr Tyr Arg Gln Gln Gln Lys Pro Glu Gly
   340 345 350
   Ser Ala Thr Leu Tyr Pro Glu Gly Asn Ile Asp Phe Thr Ser Leu Tyr
   355 360 365
   Phe Gln Asp Glu Met Thr Met Lys Ser Tyr Pro Val Asn Ile Ile Val
   370 375 380
   Gly Ser Arg Tyr Asp Arg Tyr Lys Ser Phe Asn Pro Arg Ala Gly Glu
   385 390 395 400
   Leu Lys Ala Glu Arg Leu Ser Pro Arg Ala Ala Ile Ser Val Ser Pro
   405 410 415
   Thr Asp Trp Leu Met Met Tyr Gly Ser Ile Ser Ser Ala Phe Arg Ala
   420 425 430
   Pro Thr Met Ala Glu Met Tyr Arg Asp Asp Val His Phe Tyr Arg Lys
   435 440 445
   Gly Lys Pro Asn Tyr Trp Val Pro Asn Leu Asn Leu Lys Pro Glu Asn
   450 455 460
   Asn Ile Thr Arg Glu Ile Gly Ala Gly Ile Gln Leu Asp Gly Leu Leu
   465 470 475 480
   Thr Asp Asn Asp Arg Leu Gln Leu Lys Gly Gly Tyr Phe Gly Thr Asp
   485 490 495
   Ala Arg Asn Tyr Ile Ala Thr Arg Val Asp Met Lys Arg Met Arg Ser
   500 505 510
   Tyr Ser Tyr Asn Val Ser Arg Ala Arg Ile Trp Gly Trp Asp Met Gln
   515 520 525
   Gly Asn Tyr Gln Ser Asp Tyr Val Asp Trp Met Leu Ser Tyr Asn Arg
   530 535 540
   Thr Glu Ser Met Asp Ala Ser Ser Arg Glu Trp Leu Gly Ser Gly Asn
   545 550 555 560
   Pro Asp Thr Leu Ile Ser Asp Ile Ser Ile Pro Val Gly His Arg Gly
   565 570 575
   Val Tyr Ala Gly Trp Arg Ala Glu Leu Ser Ala Ser Ala Thr His Val
   580 585 590
   Lys Lys Gly Asp Pro His Gln Ala Gly Tyr Thr Ile His Ser Phe Ser
   595 600 605
   Leu Ser Tyr Lys Pro Val Ser Val Lys Gly Phe Glu Ala Ser Val Thr
   610 615 620
   Leu Asp Asn Ala Phe Asn Lys Leu Ala Met Asn Gly Lys Gly Val Pro
   625 630 635 640
   Leu Ser Gly Arg Thr Val Ser Leu Tyr Thr Arg Tyr Gln Trp
   645 650
<210> 26
   <211> 1376
   <212> PRT
   <213> Escherichia coli
<400> 26
<210> 27
   <211> 349
   <212> PRT
   <213> Escherichia coli
<400> 27
   Met Ile Thr Leu Phe Arg Leu Leu Ala Ile Leu Cys Leu Phe Phe Asn
   1               5                   10                  15
   Val Ser Ala Phe Ala Val Asp Cys Tyr Gln Asp Gly Tyr Arg Gly Thr
               20                  25                  30
   Thr Leu Ile Asn Gly Asp Leu Pro Thr Phe Lys Ile Pro Glu Asn Ala
           35                  40                  45
   Gln Pro Gly Gln Lys Ile Trp Glu Ser Gly Asp Ile Asn Ile Thr Val
       50                  55                  60
   Tyr Cys Asp Asn Ala Pro Gly Trp Ser Ser Asn Asn Pro Ser Glu Asn
   65                  70                  75                  80
   Val Tyr Ala Trp Ile Lys Leu Pro Gln Ile Asn Ser Ala Asp Met Leu
                   85                  90                  95
   Asn Asn Pro Tyr Leu Thr Phe Gly Val Thr Tyr Asn Gly Val Asp Tyr
               100                 105                 110
   Glu Gly Thr Asn Glu Lys Ile Asp Thr His Ala Cys Leu Asp Lys Tyr
           115                 120                 125
   Glu Gln Tyr Tyr Asn Gly Tyr Tyr His Asp Pro Val Cys Asn Gly Ser
       130                 135                 140
   Thr Leu Gln Lys Asn Val Thr Phe Asn Ala His Phe Arg Val Tyr Val
   145                 150                 155                 160
   Lys Phe Lys Ser Arg Pro Ala Gly Asp Gln Thr Val Asn Phe Gly Thr
                   165                 170                 175
   Val Asn Val Leu Gln Phe Asp Gly Glu Gly Gly Ala Asn Met Ala Pro
               180                 185                 190
   Asn Ala Lys Asn Leu Arg Tyr Ala Ile Thr Gly Leu Asp Asn Ile Ser
   195 200 205
   Phe Leu Asp Cys Ser Val Asp Val Arg Ile Ser Pro Glu Ser Gln Ile
   210 215 220
   Val Asn Phe Gly Gln Ile Ala Ala Asn Ser Ile Ala Thr Phe Pro Pro
   225 230 235 240
   Lys Ala Ala Phe Ser Val Ser Thr Ile Lys Asp Ile Ala Ser Asp Cys
   245 250 255
   Thr Glu Gln Phe Asp Val Ala Thr Ser Phe Phe Thr Ser Asp Thr Leu
   260 265 270
   Tyr Asp Asn Thr His Leu Glu Ile Gly Asn Gly Leu Leu Met Arg Ile
   275 280 285
   Thr Asp Gln Lys Thr Gln Glu Asp Ile Lys Phe Asn Gln Phe Lys Leu
   290 295 300
   Phe Ser Thr Tyr Ile Pro Gly Gln Ser Ala Ala Met Ala Thr Arg Asp
   305 310 315 320
   Tyr Gln Ala Glu Leu Thr Gln Lys Pro Gly Glu Pro Leu Val Tyr Gly
   325 330 335
   Pro Phe Gln Lys Asp Leu Ile Val Lys Ile Asn Tyr His
   340 345
<210> 28
   <211> 840
   <212> PRT
   <213> Escherichia coli
<400> 28
   Met Asn Asn Lys Asn Thr Phe Ser Arg Asp Lys Leu Ser His Ala Ile
   1 5 10 15
   Lys Asn Ala Leu Ser Gly Val Val Cys Ser Leu Leu Phe Val Leu Pro
   20 25 30
   Val His Ala Val Glu Phe Asn Val Asp Met Ile Asp Ala Glu Asp Arg
   35 40 45
   Glu Asn Ile Asp Ile Ser Arg Phe Glu Lys Lys Gly Tyr Ile Pro Pro
   50 55 60
   Gly Arg Tyr Leu Val Arg Val Gln Ile Asn Lys Asn Met Leu Pro Gln
   65 70 75 80
   Thr Leu Ile Leu Glu Trp Val Lys Ala Asp Asn Glu Ser Gly Ser Leu
   85 90 95
   Leu Cys Leu Thr Lys Glu Asn Leu Thr Asn Phe Gly Leu Asn Thr Glu
   100 105 110
   Phe Ile Glu Ser Leu Gln Asn Ile Ala Gly Ser Glu Cys Leu Asp Leu
   115 120 125
   Ser Gln Arg Gln Glu Leu Thr Thr Arg Leu Asp Lys Ala Thr Met Ile
   130 135 140
   Leu Ser Leu Ser Val Pro Gln Ala Trp Leu Lys Tyr Gln Ala Thr Asn
   145 150 155 160
   Trp Thr Pro Pro Glu Phe Trp Asp Thr Gly Ile Thr Gly Phe Ile Leu
   165 170 175
   Asp Tyr Asn Val Tyr Ala Ser Gln Tyr Ala Pro His His Gly Asp Ser
   180 185 190
   Thr Gln Asn Val Ser Ser Tyr Gly Thr Leu Gly Phe Asn Leu Gly Ala
   195 200 205
   Trp Arg Leu Arg Ser Asp Tyr Gln Tyr Asn Gln Asn Phe Ala Asp Gly
   210 215 220
   Arg Ser Val Asn Arg Asp Ser Glu Phe Ala Arg Thr Tyr Leu Phe Arg
   225 230 235 240
   Pro Ile Pro Ser Trp Ser Ser Lys Phe Thr Met Gly Gln Tyr Asp Leu
   245 250 255
   Ser Ser Asn Leu Tyr Asp Thr Phe His Phe Thr Gly Ala Ser Leu Glu
   260 265 270
   Ser Asp Glu Ser Met Leu Pro Pro Asp Leu Gln Gly Tyr Ala Pro Gln
   275 280 285
   Ile Thr Gly Ile Ala Gln Thr Asn Ala Lys Val Thr Val Ala Gln Asn
   290 295 300
   Gly Arg Val Leu Tyr Gln Thr Thr Val Ala Pro Gly Pro Phe Thr Ile
   305 310 315 320
   Ser Asp Leu Gly Gln Ser Phe Gln Gly Gln Leu Asp Val Thr Val Glu
   325 330 335
   Glu Glu Asp Gly Arg Thr Ser Thr Phe Gln Val Gly Ser Ala Ser Ile
   340 345 350
   Pro Tyr Leu Thr Arg Lys Gly Gln Val Arg Tyr Lys Thr Ser Leu Gly
   355 360 365
   Lys Pro Thr Ser Val Gly His Asn Asp Ile Asn Asn Pro Phe Phe Trp
   370 375 380
   Thr Ala Glu Ala Ser Trp Gly Trp Leu Asn Asn Val Ser Leu Tyr Gly
   385 390 395 400
   Gly Gly Met Phe Thr Ala Asp Asp Tyr Gln Ala Ile Thr Thr Gly Ile
   405 410 415
   Gly Phe Asn Leu Asn Gln Phe Gly Ser Leu Ser Phe Asp Val Thr Gly
   420 425 430
   Ala Asp Ala Ser Leu Gln Gln Gln Asn Ser Gly Asn Leu Arg Gly Tyr
   435 440 445
   Ser Tyr Arg Phe Asn Tyr Ala Lys His Phe Glu Ser Thr Gly Ser Gln
   450 455 460
   Ile Thr Phe Ala Gly Tyr Arg Phe Ser Asp Lys Asp Tyr Val Ser Met
   465 470 475 480
   Ser Glu Tyr Leu Ser Ser Arg Asn Gly Asp Glu Ser Ile Asp Asn Glu
   485 490 495
   Lys Glu Ser Tyr Val Ile Ser Leu Asn Gln Tyr Phe Glu Thr Leu Glu
   500 505 510
   Leu Asn Ser Tyr Leu Asn Val Thr Arg Asn Thr Tyr Trp Asp Ser Ala
   515 520 525
   Ser Asn Thr Asn Tyr Ser Val Ser Val Ser Lys Asn Phe Asp Ile Gly
   530 535 540
   Asp Phe Lys Gly Ile Ser Ala Ser Leu Ala Val Ser Arg Ile Arg Trp
   545 550 555 560
   Asp Asp Asp Glu Glu Asn Gln Tyr Tyr Phe Ser Phe Ser Leu Pro Leu
   565 570 575
   Gln Gln Asn Arg Asn Ile Ser Tyr Ser Met Gln Arg Thr Gly Ser Ser
   580 585 590
   Asn Thr Ser Gln Met Ile Ser Trp Tyr Asp Ser Ser Asp Arg Asn Asn
   595 600 605
   Ile Trp Asn Ile Ser Ala Ser Ala Thr Asp Asp Asn Ile Arg Asp Gly
   610 615 620
   Glu Pro Thr Leu Arg Gly Ser Tyr Gln His Tyr Ser Pro Trp Gly Arg
   625 630 635 640
   Leu Asn Ile Asn Gly Ser Val Gln Pro Asn Gln Tyr Asn Ser Val Thr
   645 650 655
   Ala Gly Trp Tyr Gly Ser Leu Thr Ala Thr Arg His Gly Val Ala Leu
   660 665 670
   His Asp Tyr Ser Tyr Gly Asp Asn Ala Arg Met Met Val Asp Thr Asp
   675 680 685
   Gly Ile Ser Gly Ile Glu Ile Asn Ser Asn Arg Thr Val Thr Asn Gly
   690 695 700
   Leu Gly Ile Ala Val Ile Pro Ser Leu Ser Asn Tyr Thr Thr Ser Met
   705 710 715 720
   Leu Arg Val Asn Asn Asn Asp Leu Pro Glu Gly Val Asp Val Glu Asn
   725 730 735
   Ser Val Ile Arg Thr Thr Leu Thr Gln Gly Ala Ile Gly Tyr Ala Lys
   740 745 750
   Leu Asn Ala Thr Thr Gly Tyr Gln Ile Val Gly Val Ile Arg Gln Glu
   755 760 765
   Asn Gly Arg Phe Pro Pro Leu Gly Val Asn Val Thr Asp Lys Ala Thr
   770 775 780
   Gly Lys Asp Val Gly Leu Val Ala Glu Asp Gly Phe Val Tyr Leu Ser
   785 790 795 800
   Gly Ile Gln Glu Asn Ser Ile Leu His Leu Thr Trp Gly Asp Asn Thr
   805 810 815
   Cys Glu Val Thr Pro Pro Asn Gln Ser Asn Ile Ser Glu Ser Ala Ile
   820 825 830
   Ile Leu Pro Cys Lys Thr Val Lys
   835 840
<210> 29
   <211> 169
   <212> PRT
   <213> Escherichia coli
<400> 29
   Leu Met Asn Thr Lys Gln Ser Val Ala Gln Leu Ala Val Pro His Arg
   1 5 10 15
   Lys Arg Leu Ser Ser Thr Met Val Val Ala Leu Leu Leu Cys Val Val
   20 25 30
   Ala Gly Ala Val Met Ile Asn Ala Ala Asp Phe Pro Ala Thr Ala Ile
   35 40 45
   Glu Thr Asp Pro Gly Ala Ser Ala Phe Pro Thr Phe Tyr Ala Cys Ala
   50 55 60
   Leu Ile Val Leu Ala Val Leu Leu Val Ile Arg Asp Leu Leu Gln Ala
   65 70 75 80
   Lys Pro Ala Ser Cys Ala Asn Ala Gln Glu Lys Pro Ala Phe Arg Lys
   85 90 95
   Thr Ala Thr Gly Ile Ala Ala Thr Ala Phe Tyr Ile Val Ala Met Ser
   100 105 110
   Tyr Cys Gly Tyr Leu Ile Thr Thr Pro Val Phe Leu Ile Val Ile Met
   115 120 125
   Thr Leu Met Gly Tyr Arg Arg Trp Val Leu Thr Pro Gly Ile Ala Leu
   130 135 140
   Leu Leu Thr Ala Ile Leu Trp Leu Leu Phe Val Glu Ala Leu Gln Val
   145 150 155 160
   Pro Leu Pro Val Gly Thr Phe Phe Glu
   165
<210> 30
   <211> 311
   <212> PRT
   <213> Escherichia coli
<400> 30
   Met Val Leu Leu Ala Gly Ala Ala Leu Ser Ile Ala Pro Val Gln Ala
   1 5 10 15
   Ala Ser Tyr Pro Thr Lys Gln Ile Glu Leu Val Val Pro Tyr Ala Ala
   20 25 30
   Gly Gly Gly Thr Asp Leu Val Ala Arg Ala Phe Ala Asp Ala Ala Lys
   35 40 45
   Asn His Leu Pro Val Ser Ile Gly Val Ile Asn Lys Pro Gly Gly Gly
   50 55 60
   Gly Ala Ile Gly Leu Ser Glu Ile Ala Ala Ala Arg Pro Asn Gly Tyr
   65 70 75 80
   Lys Ile Gly Leu Gly Thr Val Glu Leu Thr Thr Leu Pro Ser Leu Gly
   85 90 95
   Met Val Arg Phe Lys Thr Ser Asp Phe Lys Pro Ile Ala Arg Leu Asn
   100 105 110
   Ala Asp Pro Ala Ala Ile Thr Val Arg Ala Asp Ala Pro Trp Asn Ser
   115 120 125
   Tyr Glu Glu Phe Met Ala Tyr Ser Lys Ala Asn Pro Gly Lys Val Arg
   130 135 140
   Ile Gly Asn Ser Gly Thr Gly Ala Ile Trp His Leu Ala Ala Ala Ala
   145 150 155 160
   Leu Glu Asp Lys Thr Gly Thr Lys Phe Ser His Val Pro Tyr Asp Gly
   165 170 175
   Ala Ala Pro Ala Ile Thr Gly Leu Leu Gly Gly His Ile Glu Ala Val
   180 185 190
   Ser Val Ser Pro Gly Glu Val Ile Asn His Val Asn Gly Gly Lys Leu
   195 200 205
   Lys Thr Leu Val Val Met Ala Asp Glu Arg Met Lys Thr Met Pro Asp
   210 215 220
   Val Pro Thr Leu Lys Glu Lys Gly Val Asp Leu Ser Ile Gly Thr Trp
   225 230 235 240
   Arg Gly Leu Ile Val Ser Gln Lys Thr Pro Gln Asp Val Val Asp Val
   245 250 255
   Leu Ala Lys Ala Ala Lys Glu Thr Ala Glu Glu Pro Ala Phe Gln Asp
   260 265 270
   Ala Leu Gln Lys Leu Asn Leu Asn Tyr Ala Trp Leu Asp Ala Ala Ser
   275 280 285
   Phe Gln Thr Gln Ile Ser Glu Gln Glu Lys Tyr Phe Asp Glu Leu Leu
   290 295 300
   Thr Arg Leu Gly Leu Lys Lys
   305 310
<210> 31
   <211> 722
   <212> PRT
   <213> Escherichia coli
<400> 31
   Met Leu Arg Trp Lys Arg Cys Ile Ile Leu Thr Phe Ile Ser Gly Ala
   1               5                   10                  15
   Ala Phe Ala Ala Pro Glu Ile Asn Val Lys Gln Asn Glu Ser Leu Pro
               20                  25                  30
   Asp Leu Gly Ser Gln Ala Ala Gln Gln Asp Glu Gln Thr Asn Lys Gly
           35                  40                  45
   Lys Ser Leu Lys Glu Arg Gly Ala Asp Tyr Val Ile Asn Ser Ala Thr
       50                  55                  60
   Gln Gly Phe Glu Asn Leu Thr Pro Glu Ala Leu Glu Ser Gln Ala Arg
   65                  70                  75                  80
   Ser Tyr Leu Gln Ser Gln Ile Thr Ser Thr Ala Gln Ser Tyr Ile Glu
                   85                  90                  95
   Asp Thr Leu Ser Pro Tyr Gly Lys Val Arg Leu Asn Leu Ser Ile Gly
               100                 105                 110
   Gln Gly Gly Asp Leu Asp Gly Ser Ser Ile Asp Tyr Phe Val Pro Trp
           115                 120                 125
   Tyr Asp Asn Gln Thr Thr Val Tyr Phe Ser Gln Phe Ser Ala Gln Arg
       130                 135                 140
   Lys Glu Asp Arg Thr Ile Gly Asn Ile Gly Leu Gly Val Arg Tyr Asn
   145                 150                 155                 160
   Phe Asp Lys Tyr Leu Leu Gly Gly Asn Ile Phe Tyr Asp Tyr Asp Phe
                   165                 170                 175
   Thr Arg Gly His Arg Arg Leu Gly Leu Gly Ala Glu Ala Trp Thr Asp
               180                 185                 190
   Tyr Leu Lys Phe Ser Gly Asn Tyr Tyr His Pro Leu Ser Asp Trp Lys
           195                 200                 205
   Asp Ser Glu Asp Phe Asp Phe Tyr Glu Glu Arg Pro Ala Arg Gly Trp
       210                 215                 220
   Asp Ile Arg Ala Glu Val Trp Leu Pro Ser Tyr Pro Gln Leu Gly Gly
   225 230 235 240
   Lys Ile Val Phe Glu Gln Tyr Tyr Gly Asp Glu Val Ala Leu Phe Gly
   245 250 255
   Thr Asp Asn Leu Glu Lys Asp Pro Tyr Ala Val Thr Leu Gly Leu Asn
   260 265 270
   Tyr Gln Pro Val Pro Leu Leu Thr Val Gly Thr Asp Tyr Lys Ala Gly
   275 280 285
   Thr Gly Asp Asn Ser Asp Val Ser Ile Asn Ala Thr Leu Asn Tyr Gln
   290 295 300
   Phe Gly Val Pro Leu Lys Asp Gln Leu Asp Ser Asp Lys Val Lys Ala
   305 310 315 320
   Ala His Ser Leu Met Gly Ser Arg Leu Asp Phe Val Glu Arg Asn Asn
   325 330 335
   Phe Ile Val Leu Glu Tyr Lys Glu Lys Asp Pro Leu Asp Val Thr Leu
   340 345 350
   Trp Leu Lys Ala Asp Ala Thr Asn Glu His Pro Glu Cys Val Ile Lys
   355 360 365
   Asp Thr Pro Glu Ala Ala Val Gly Leu Glu Lys Cys Lys Trp Thr Ile
   370 375 380
   Asn Ala Leu Ile Asn His His Tyr Lys Ile Val Ala Ala Ser Trp Gln
   385 390 395 400
   Ala Lys Asn Asn Ala Ala Arg Thr Leu Val Met Pro Val Ile Lys Glu
   405 410 415
   Asn Thr Leu Thr Glu Gly Asn Asn Asn His Trp Asn Leu Val Leu Pro
   420 425 430
   Ala Trp Gln Tyr Ser Ser Asp Gln Ala Glu Gln Glu Lys Leu Asn Thr
   435 440 445
   Trp Arg Val Arg Leu Ala Leu Glu Asp Glu Lys Gly Asn Arg Gln Asn
   450 455 460
   Ser Gly Val Val Glu Ile Thr Val Gln Gln Asp Arg Lys Ile Glu Leu
   465 470 475 480
   Ile Val Asn Asn Ile Ala Asn Pro Glu Glu Asn Asn His Ser His Glu
   485 490 495
   Ala Ser Ala Gln Ala Asp Gly Val Asp Gly Val Val Met Asp Leu Asp
   500 505 510
   Val Thr Asp Ser Phe Gly Asp Asn Thr Asp Arg Asn Gly Asp Ala Leu
   515 520 525
   Pro Glu Asp Asn Leu Thr Pro Gln Leu Tyr Asp Ala Gln Asp Lys Arg
   530 535 540
   Val Thr Leu Thr Asn Lys Pro Cys Ser Thr Asp Asn Pro Cys Val Phe
   545 550 555 560
   Ile Ala Lys Gln Asp Lys Glu Lys Gly Thr Val Thr Leu Ser Ser Thr
   565 570 575
   Leu Pro Gly Thr Tyr Arg Trp Lys Ala Lys Ala Ala Pro Tyr Asp Asp
   580 585 590
   Ser Asn Tyr Val Asp Val Thr Phe Leu Gly Ala Glu Ile Gly Gly Leu
   595 600 605
   Asn Ala Phe Ile Tyr Arg Val Gly Ala Ala Lys Pro Ser Asn Leu Ile
   610 615 620
   Gly Lys Asp Lys Glu Pro Leu Pro Ser Thr Thr Phe Ile Asp Leu Phe
   625 630 635 640
   Tyr Gly Ala Thr Thr Ile Lys Thr Val Ser Ser Ser Arg Ser Lys Asn
   645 650 655
   Leu Thr Lys Arg Trp Cys Ser Thr Thr Thr Ser Gly Asn Leu Pro Ala
   660 665 670
   Arg Ala Ser Met Val Ser Gly Cys Thr Gly Glu His Ser Asn Glu Asp
   675 680 685
   Ile Val Ile Pro Ala Thr Asn Arg Glu Ala Ala Gln Thr Tyr Gly Ala
   690 695 700
   Gln Ala Gly Asp Gly Leu Gln Gly Tyr Gly Leu Arg Val Leu Tyr Thr
   705 710 715 720
   Lys Lys
<210> 32
   <211> 319
   <212> PRT
   <213> Escherichia coli
<400> 32
   Met Lys Gln Asp Lys Arg Arg Gly Leu Thr Arg Ile Ala Leu Ala Leu
   1 5 10 15
   Ala Leu Ala Gly Tyr Cys Val Ala Pro Val Ala Leu Ala Glu Asp Ser
   20 25 30
   Ala Trp Val Asp Ser Gly Glu Thr Asn Ile Phe Gln Gly Thr Ile Pro
   35 40 45
   Trp Leu Tyr Ser Glu Gly Gly Ser Ala Thr Thr Asp Ala Asp Arg Val
   50 55 60
   Thr Leu Thr Ser Asp Leu Lys Gly Ala Arg Pro Gln Gly Met Lys Arg
   65 70 75 80
   Thr Ser Val Phe Thr Arg Val Ile Asn Ile Gly Asp Thr Glu Gly Asp
   85 90 95
   Val Asp Leu Gly Gly Leu Gly Asp Asn Ala Lys Thr Ile Asp Thr Ile
   100 105 110
   Arg Trp Met Ser Tyr Lys Asp Ala Gln Gly Gly Asp Pro Lys Glu Leu
   115 120 125
   Ala Thr Lys Val Thr Ser Tyr Thr Leu Thr Asp Ala Asp Arg Gly Arg
   130 135 140
   Tyr Ile Gly Ile Glu Ile Thr Pro Thr Thr Gln Thr Gly Thr Pro Asn
   145 150 155 160
   Val Gly Thr Ala Leu His Leu Tyr Asp Val Ser Thr Ala Ser Gly Gly
   165 170 175
   Gly Ser Asp Ser Asp Asn Val Ala Pro Gly Pro Val Val Asn Gln Asn
   180 185 190
   Leu Lys Val Ala Ile Phe Val Asp Gly Thr Ser Ile Asn Leu Ile Asn
   195 200 205
   Gly Ser Thr Pro Ile Glu Leu Gly Lys Thr Tyr Val Ala Lys Leu Tyr
   210 215 220
   Ser Asp Glu Asn Lys Asn Gly Lys Phe Asp Ala Gly Thr Asp Ala Asp
   225 230 235 240
   Val Thr Ala Asn Tyr Asp Phe Arg Trp Val Leu Ser Gly Ser Ser Gln
   245 250 255
   Gln Leu Gly Thr Ser Gly Gly Ile Val Asn Ser Ser Phe Asp Asn Asn
   260 265 270
   Asn Leu Val Ile Pro Ala Thr Asn Asp Glu Ala Arg Thr Asn Leu Asn
   275 280 285
   Gly Pro Ala Arg Asp Gly Lys Glu Ala Leu Ser Ile Pro Thr Asn Gly
   290 295 300
   Asp Gly Val Gln Gly Tyr Lys Leu His Ile Ile Tyr Lys His Lys
   305 310 315
<210> 33
   <211> 629
   <212> PRT
   <213> Escherichia coli
<400> 33
   Met Lys Lys Val Leu Thr Leu Ser Leu Leu Ala Leu Cys Val Ser His
   1 5 10 15
   Ser Ala Val Ala Ala Asn Tyr Thr Phe Asn Asn Asp Asn Ile Ala Leu
   20 25 30
   Ser Phe Asp Asp Thr Asn Ser Thr Ile Val Leu Lys Asp Arg Arg Thr
   35 40 45
   Asn His Pro Ile Thr Pro Gln Glu Leu Phe Phe Leu Thr Leu Pro Asp
   50 55 60
   Glu Thr Lys Ile His Thr Ala Asp Phe Lys Ile Lys His Ile Lys Lys
   65 70 75 80
   Gln Asp Asn Ala Ile Val Ile Asp Phe Thr Arg Pro Asp Phe Asn Val
   85 90 95
   Thr Val Gln Leu Asn Leu Val Lys Gly Lys Tyr Ala Ser Ile Asp Tyr
   100 105 110
   Thr Ile Ala Ala Val Gly Gln Pro Arg Asp Val Ala Lys Ile Thr Phe
   115 120 125
   Phe Pro Thr Lys Lys Gln Phe Gln Ala Pro Tyr Val Asp Gly Ala Ile
   130 135 140
   Thr Ser Ser Pro Ile Ile Ala Asp Ser Phe Phe Ile Leu Pro Asn Lys
   145 150 155 160
   Pro Ile Val Asn Thr Tyr Ala Tyr Glu Ala Thr Thr Asn Leu Asn Val
   165 170 175
   Glu Leu Lys Thr Pro Ile Gln Pro Glu Thr Pro Val Ser Phe Thr Thr
   180 185 190
   Trp Phe Gly Thr Phe Pro Glu Thr Ser Gln Leu Arg Arg Ser Val Asn
   195 200 205
   Gln Phe Ile Asn Ala Val Arg Pro Arg Pro Tyr Lys Pro Tyr Leu His
   210 215 220
   Tyr Asn Ser Trp Met Asp Ile Gly Phe Phe Thr Pro Tyr Thr Glu Gln
   225 230 235 240
   Asp Val Leu Gly Arg Met Asp Glu Trp Asn Lys Glu Phe Ile Ser Gly
   245 250 255
   Arg Gly Val Ala Leu Asp Ala Phe Leu Leu Asp Asp Gly Trp Asp Asp
   260 265 270
   Leu Thr Gly Arg Trp Leu Phe Gly Pro Ala Phe Ser Asn Gly Phe Ser
   275 280 285
   Lys Val Arg Glu Lys Ala Asp Ser Leu His Ser Ser Val Gly Leu Trp
   290 295 300
   Leu Ser Pro Trp Gly Gly Tyr Asn Lys Pro Gln Arg Arg Ser Arg Phe
   305 310 315 320
   Ala Cys Lys Arg Val Trp Val Arg Asn Arg Gly Arg Gln Ala Gly Ala
   325 330 335
   Phe Gly Ser Glu Leu Leu Lys Asn Phe Asn Glu Gln Ile Ile Asn Leu
   340 345 350
   Ile Lys Asn Glu His Ile Thr Ser Phe Lys Leu Asp Gly Met Gly Asn
   355 360 365
   Ala Ser Ser His Ile Lys Gly Ser Pro Phe Ala Ser Asp Phe Asp Ala
   370 375 380
   Ser Ile Ala Leu Leu His Asn Met Arg Arg Ala Asn Pro Asn Leu Phe
   385 390 395 400
   Ile Asn Leu Thr Thr Gly Thr Asn Ala Ser Pro Ser Trp Leu Phe Tyr
   405 410 415
   Ala Asp Ser Ile Trp Arg Gln Gly Asp Asp Ile Asn Leu Tyr Gly Pro
   420 425 430
   Gly Thr Pro Val Gln Gln Trp Ile Thr Tyr Arg Asp Ala Glu Thr Tyr
   435 440 445
   Arg Ser Ile Val Arg Lys Gly Pro Leu Phe Pro Leu Asn Ser Leu Met
   450 455 460
   Tyr His Gly Ile Val Ser Ala Glu Asn Ala Tyr Tyr Gly Leu Glu Lys
   465 470 475 480
   Val Gln Thr Asp Ser Asp Phe Ala Asp Gln Val Trp Ser Tyr Phe Ala
   485 490 495
   Thr Gly Thr Gln Leu Gln Glu Leu Tyr Ile Thr Pro Ser Met Leu Asn
   500 505 510
   Lys Val Lys Trp Asp Thr Leu Ala Lys Ala Ala Lys Trp Ser Lys Glu
   515 520 525
   Asn Ala Ser Val Leu Val Asp Thr His Trp Ile Gly Gly Asp Pro Thr
   530 535 540
   Ala Leu Ala Val Tyr Gly Trp Ala Ser Trp Ser Lys Asp Lys Ala Ile
   545 550 555 560
   Leu Gly Leu Arg Asn Pro Ser Asp Lys Pro Gln Thr Tyr Tyr Leu Asp
   565 570 575
   Leu Ala Lys Asp Phe Glu Ile Pro Ala Gly Asn Ala Ala Gln Phe Ser
   580 585 590
   Leu Lys Ala Val Tyr Gly Ser Asn Lys Thr Val Pro Val Glu Tyr Lys
   595 600 605
   Asn Ala Thr Val Ile Thr Leu Gln Pro Leu Glu Thr Leu Val Phe Glu
   610 615 620
   Ala Val Thr Ile Asn
   625
<210> 34
   <211> 1778
   <212> PRT
   <213> Escherichia coli
<400> 34
   Met Asn Lys Ile Phe Lys Val Ile Trp Asn Pro Ala Thr Gly Ser Tyr
   1 5 10 15
   Thr Val Ala Ser Glu Thr Ala Lys Ser Arg Gly Lys Lys Ser Gly Arg
   20 25 30
   Ser Lys Leu Leu Ile Ser Ala Leu Val Ala Gly Gly Leu Leu Ser Ser
   35 40 45
   Phe Gly Ala Ser Ala Asp Asn Tyr Thr Gly Gln Pro Thr Asp Tyr Gly
       50                  55                  60
   Asp Gly Ser Ala Gly Asp Gly Trp Val Ala Ile Gly Lys Gly Ala Lys
   65                  70                  75                  80
   Ala Asn Thr Phe Met Asn Thr Ser Gly Ala Ser Thr Ala Leu Gly Tyr
                   85                  90                  95
   Asp Ala Ile Ala Glu Gly Glu Tyr Ser Ser Ala Ile Gly Ser Lys Thr
               100                 105                 110
   Leu Ala Thr Gly Gly Ala Ser Met Ala Phe Gly Val Ser Ala Lys Ala
           115                 120                 125
   Met Gly Asp Arg Ser Val Ala Leu Gly Ala Ser Ser Val Ala Asn Gly
       130                 135                 140
   Asp Arg Ser Met Ala Phe Gly Arg Tyr Ala Lys Thr Asn Gly Phe Thr
   145                 150                 155                 160
   Ser Leu Ala Ile Gly Asp Ser Ser Leu Ala Asp Gly Glu Lys Thr Ile
                   165                 170                 175
   Ala Leu Gly Asn Thr Ala Lys Ala Tyr Glu Ile Met Ser Ile Ala Leu
               180                 185                 190
   Gly Asp Asn Ala Asn Ala Ser Lys Glu Tyr Ala Met Ala Leu Gly Ala
           195                 200                 205
   Ser Ser Lys Ala Gly Gly Ala Asp Ser Leu Ala Phe Gly Arg Lys Ser
       210                 215                 220
   Thr Ala Asn Ser Thr Gly Ser Leu Ala Ile Gly Ala Asp Ser Ser Ser
   225                 230                 235                 240
   Ser Asn Asp Asn Ala Ile Ala Ile Gly Asn Lys Thr Gln Ala Leu Gly
                   245                 250                 255
   Val Asn Ser Met Ala Leu Gly Asn Ala Ser Gln Ala Ser Gly Glu Ser
               260                 265                 270
   Ser Ile Ala Leu Gly Asn Thr Ser Glu Ala Ser Glu Gln Asn Ala Ile
   275 280 285
   Ala Leu Gly Gln Gly Ser Ile Ala Ser Lys Val Asn Ser Ile Ala Leu
   290 295 300
   Gly Ser Asn Ser Leu Ser Ser Gly Glu Asn Ala Ile Ala Leu Gly Glu
   305 310 315 320
   Gly Ser Ala Ala Gly Gly Ser Asn Ser Leu Ala Phe Gly Ser Gln Ser
   325 330 335
   Arg Ala Asn Gly Asn Asp Ser Val Ala Ile Gly Val Gly Ala Ala Ala
   340 345 350
   Ala Thr Asp Asn Ser Val Ala Ile Gly Ala Gly Ser Thr Thr Asp Ala
   355 360 365
   Ser Asn Thr Val Ser Val Gly Asn Ser Ala Thr Lys Arg Lys Ile Val
   370 375 380
   Asn Met Ala Ala Gly Ala Ile Ser Asn Thr Ser Thr Asp Ala Ile Asn
   385 390 395 400
   Gly Ser Gln Leu Tyr Thr Ile Ser Asp Ser Val Ala Lys Arg Leu Gly
   405 410 415
   Gly Gly Ala Thr Val Gly Ser Asp Gly Thr Val Thr Ala Val Ser Tyr
   420 425 430
   Ala Leu Arg Ser Gly Thr Tyr Asn Asn Val Gly Asp Ala Leu Ser Gly
   435 440 445
   Ile Asp Asn Asn Thr Leu Gln Trp Asn Lys Thr Ala Gly Ala Phe Ser
   450 455 460
   Ala Asn His Gly Ala Asn Ala Thr Asn Lys Ile Thr Asn Val Ala Lys
   465 470 475 480
   Gly Thr Val Ser Ala Thr Ser Thr Asp Val Val Asn Gly Ser Gln Leu
   485 490 495
   Tyr Asp Leu Gln Gln Asp Ala Leu Leu Trp Asn Gly Thr Ala Phe Ser
   500 505 510
   Ala Ala His Gly Thr Glu Ala Thr Ser Lys Ile Thr Asn Val Thr Ala
   515 520 525
   Gly Asn Leu Thr Ala Gly Ser Thr Asp Ala Val Asn Gly Ser Gln Leu
   530 535 540
   Lys Thr Thr Asn Asp Asn Val Thr Thr Asn Thr Thr Asn Ile Ala Thr
   545 550 555 560
   Asn Thr Thr Asn Ile Thr Asn Leu Thr Asp Ala Val Asn Gly Leu Gly
   565 570 575
   Asp Asp Ser Leu Leu Trp Asn Lys Ala Ala Gly Ala Phe Ser Ala Ala
   580 585 590
   His Gly Thr Glu Ala Thr Ser Lys Ile Thr Asn Val Thr Ala Gly Asn
   595 600 605
   Leu Thr Ala Gly Ser Thr Asp Ala Val Asn Gly Ser Gln Leu Lys Thr
   610 615 620
   Thr Asn Asp Asn Val Thr Thr Asn Thr Thr Asn Ile Ala Thr Asn Thr
   625 630 635 640
   Thr Asn Ile Thr Asn Leu Thr Asp Ala Val Asn Gly Leu Gly Asp Asp
   645 650 655
   Ser Leu Leu Trp Asn Lys Thr Ala Gly Ala Phe Ser Ala Ala His Gly
   660 665 670
   Thr Asp Ala Thr Ser Lys Ile Thr Asn Val Thr Ala Gly Asn Leu Thr
   675 680 685
   Ala Gly Ser Thr Asp Ala Val Asn Gly Ser Gln Leu Lys Thr Thr Asn
   690 695 700
   Asp Asn Val Thr Thr Asn Thr Thr Asn Ile Ala Thr Asn Thr Thr Asn
   705 710 715 720
   Ile Thr Asn Leu Thr Asp Ala Val Asn Gly Leu Gly Asp Asp Ser Leu
   725 730 735
   Leu Trp Asn Lys Thr Ala Gly Ala Phe Ser Ala Ala His Gly Thr Asp
   740 745 750
   Ala Thr Ser Lys Ile Thr Asn Val Lys Ala Gly Asp Leu Thr Ala Gly
   755 760 765
   Ser Thr Asp Ala Val Asn Gly Ser Gln Leu Lys Thr Thr Asn Asp Asn
   770 775 780
   Val Ser Thr Asn Thr Thr Asn Ile Thr Asn Leu Thr Asp Ala Val Asn
   785 790 795 800
   Gly Leu Gly Asp Asp Ser Leu Leu Trp Asn Lys Thr Ala Gly Ala Phe
   805 810 815
   Ser Ala Ala His Gly Thr Asp Ala Thr Ser Lys Ile Thr Asn Val Lys
   820 825 830
   Ala Gly Asp Leu Thr Ala Gly Ser Thr Asp Ala Val Asn Gly Ser Gln
   835 840 845
   Leu Lys Thr Thr Asn Asp Asn Val Ser Thr Asn Thr Thr Asn Ile Thr
   850 855 860
   Asn Leu Thr Asp Ser Val Gly Asp Leu Lys Asp Asp Ser Leu Leu Trp
   865 870 875 880
   Asn Lys Ala Ala Gly Ala Phe Ser Ala Ala His Gly Thr Glu Ala Thr
   885 890 895
   Ser Lys Ile Thr Asn Leu Leu Ala Gly Lys Ile Ser Ser Asn Ser Thr
   900 905 910
   Asp Ala Ile Asn Gly Ser Gln Leu Tyr Gly Val Ala Asp Ser Phe Thr
   915 920 925
   Ser Tyr Leu Gly Gly Gly Ala Asp Ile Ser Asp Thr Gly Val Leu Ser
   930 935 940
   Gly Pro Thr Tyr Thr Ile Gly Gly Thr Asp Tyr Thr Asn Val Gly Asp
   945 950 955 960
   Ala Leu Ala Ala Ile Asn Thr Ser Phe Ser Thr Ser Leu Gly Asp Ala
   965 970 975
   Leu Leu Trp Asp Ala Thr Ala Gly Lys Phe Ser Ala Lys His Gly Ile
   980 985 990
   Asn Asn Ala Pro Ser Val Ile Thr Asp Val Ala Asn Gly Ala Val Ser
   995 1000 1005
   Ser Thr Ser Ser Asp Ala Ile Asn Gly Ser Gln Leu Tyr Gly Val
   1010 1015 1020
   Ser Asp Tyr Ile Ala Asp Ala Leu Gly Gly Asn Ala Val Val Asn
   1025 1030 1035
   Thr Asp Gly Ser Ile Thr Thr Pro Thr Tyr Ala Ile Ala Gly Gly
   1040 1045 1050
   Ser Tyr Asn Asn Val Gly Asp Ala Leu Glu Ala Ile Asp Thr Thr
   1055 1060 1065
   Leu Asp Asp Ala Leu Leu Trp Asp Thr Thr Ala Asn Gly Gly Asn
   1070 1075 1080
   Gly Ala Phe Ser Ala Ala His Gly Lys Asp Lys Thr Ala Ser Val
   1085 1090 1095
   Ile Thr Asn Val Ala Asn Gly Ala Val Ser Ala Thr Ser Asn Asp
   1100 1105 1110
   Ala Ile Asn Gly Ser Gln Leu Tyr Ser Thr Asn Lys Tyr Ile Ala
   1115 1120 1125
   Asp Ala Leu Gly Gly Asp Ala Glu Val Asn Ala Asp Gly Thr Ile
   1130 1135 1140
   Thr Ala Pro Thr Tyr Thr Ile Ala Asn Thr Asp Tyr Asn Asn Val
   1145 1150 1155
   Gly Glu Ala Leu Asp Ala Leu Asp Asn Asn Ala Leu Leu Trp Asp
   1160 1165 1170
   Glu Asp Ala Gly Ala Tyr Asn Ala Ser His Asp Gly Asn Ala Ser
   1175 1180 1185
   Lys Ile Thr Asn Val Ala Ala Gly Asp Leu Ser Thr Thr Ser Thr
   1190 1195 1200
   Asp Ala Val Asn Gly Ser Gln Leu Asn Ala Thr Asn Ile Leu Val
   1205 1210 1215
   Thr Gln Asn Ser Gln Met Ile Asn Gln Leu Ala Gly Asn Thr Ser
   1220 1225 1230
   Glu Thr Tyr Ile Glu Glu Asn Gly Ala Gly Ile Asn Tyr Val Arg
   1235 1240 1245
   Thr Asn Asp Ser Gly Leu Ala Phe Asn Asp Ala Ser Ala Ser Gly
   1250 1255 1260
   Ile Gly Ala Thr Ala Val Gly Tyr Asn Ala Val Ala Ser His Ala
   1265 1270 1275
   Ser Ser Val Ala Ile Gly Gln Asp Ser Ile Ser Glu Val Asp Thr
   1280 1285 1290
   Gly Ile Ala Leu Gly Ser Ser Ser Val Ser Ser Arg Val Ile Val
   1295 1300 1305
   Lys Gly Thr Arg Asn Thr Ser Val Ser Glu Glu Gly Val Val Ile
   1310 1315 1320
   Gly Tyr Asp Thr Thr Asp Gly Glu Leu Leu Gly Ala Leu Ser Ile
   1325 1330 1335
   Gly Asp Asp Gly Lys Tyr Arg Gln Ile Ile Asn Val Ala Asp Gly
   1340 1345 1350
   Ser Glu Ala His Asp Ala Val Thr Val Arg Gln Leu Gln Asn Ala
   1355 1360 1365
   Ile Gly Ala Val Ala Thr Thr Pro Thr Lys Tyr Tyr His Ala Asn
   1370 1375 1380
   Ser Thr Ala Glu Asp Ser Leu Ala Val Gly Glu Asp Ser Leu Ala
   1385 1390 1395
   Met Gly Ala Lys Thr Ile Val Asn Gly Asn Ala Gly Ile Gly Ile
   1400 1405 1410
   Gly Leu Asn Thr Leu Val Leu Ala Asp Ala Ile Asn Gly Ile Ala
   1415 1420 1425
   Ile Gly Ser Asn Ala Arg Ala Asn His Ala Asp Ser Ile Ala Met
   1430 1435 1440
   Gly Asn Gly Ser Gln Thr Thr Arg Gly Ala Gln Thr Asn Tyr Thr
   1445 1450 1455
   Ala Tyr Asn Met Asp Ala Pro Gln Asn Ser Val Gly Glu Phe Ser
   1460 1465 1470
   Val Gly Ser Glu Asp Gly Gln Arg Gln Ile Thr Asn Val Ala Ala
   1475 1480 1485
   Gly Ser Ala Asp Thr Asp Ala Val Asn Val Gly Gln Leu Lys Val
   1490 1495 1500
   Thr Asp Ala Gln Val Ser Gln Asn Thr Gln Ser Ile Thr Asn Leu
   1505 1510 1515
   Asn Thr Gln Val Thr Asn Leu Asp Thr Arg Val Thr Asn Ile Glu
   1520 1525 1530
   Asn Gly Ile Gly Asp Ile Val Thr Thr Gly Ser Thr Lys Tyr Phe
   1535 1540 1545
   Lys Thr Asn Thr Asp Gly Ala Asp Ala Asn Ala Gln Gly Lys Asp
   1550 1555 1560
   Ser Val Ala Ile Gly Ser Gly Ser Ile Ala Ala Ala Asp Asn Ser
   1565 1570 1575
   Val Ala Leu Gly Thr Gly Ser Val Ala Asp Glu Glu Asn Thr Ile
   1580 1585 1590
   Ser Val Gly Ser Ser Thr Asn Gln Arg Arg Ile Thr Asn Val Ala
   1595 1600 1605
   Ala Gly Val Asn Ala Thr Asp Ala Val Asn Val Ser Gln Leu Lys
   1610 1615 1620
   Ser Ser Glu Ala Gly Gly Val Arg Tyr Asp Thr Lys Ala Asp Gly
   1625 1630 1635
   Ser Ile Asp Tyr Ser Asn Ile Thr Leu Gly Gly Gly Asn Ser Gly
   1640 1645 1650
   Thr Thr Arg Ile Ser Asn Val Ser Ala Gly Val Asn Asn Asn Asp
   1655 1660 1665
   Ala Val Asn Tyr Ala Gln Leu Lys Gln Ser Val Gln Glu Thr Lys
   1670 1675 1680
   Gln Tyr Thr Asp Gln Arg Met Val Glu Met Asp Asn Lys Leu Ser
   1685 1690 1695
   Lys Thr Glu Ser Lys Leu Ser Gly Gly Ile Ala Ser Ala Met Ala
   1700 1705 1710
   Met Thr Gly Leu Pro Gln Ala Tyr Thr Pro Gly Ala Ser Met Ala
   1715 1720 1725
   Ser Ile Gly Gly Gly Thr Tyr Asn Gly Glu Ser Ala Val Ala Leu
   1730 1735 1740
   Gly Val Ser Met Val Ser Ala Asn Gly Arg Trp Val Tyr Lys Leu
   1745 1750 1755
   Gln Gly Ser Thr Asn Ser Gln Gly Glu Tyr Ser Ala Ala Leu Gly
   1760 1765 1770
   Ala Gly Ile Gln Trp
   1775
<210> 35
   <211> 227
   <212> PRT
   <213> Escherichia coli
<400> 35
   Met Asn Leu Lys Lys Thr Leu Leu Ser Val Leu Met Ile Leu Gln Leu
   1               5                   10                  15
   Cys Leu Leu Val Gly Cys Asp Tyr Ile Glu Lys Ala Ser Lys Val Asp
               20                  25                  30
   Asp Leu Val Thr Gln Gln Glu Leu Gln Lys Ser Lys Ile Glu Ala Leu
           35                  40                  45
   Glu Lys Gln Gln Glu Leu Asp Lys Arg Lys Ile Glu His Phe Glu Lys
       50                  55                  60
   Gln Gln Thr Thr Ile Ile Asn Ser Thr Lys Thr Leu Ala Gly Val Val
   65                  70                  75                  80
   Lys Ala Val Lys Asn Lys Gln Asp Glu Phe Val Phe Thr Glu Phe Asn
                   85                  90                  95
   Pro Ala Gln Thr Gln Tyr Phe Ile Leu Asn Asn Gly Ser Val Gly Leu
               100                 105                 110
   Ala Gly Lys Ile Leu Ser Ile Asp Ala Val Glu Asn Gly Ser Val Ile
           115                 120                 125
   Arg Ile Ser Leu Val Asn Leu Leu Ser Val Pro Val Ser Asn Met Gly
       130                 135                 140
   Phe Tyr Ala Thr Trp Gly Gly Glu Lys Pro Thr Asp Ile Asn Ala Leu
   145                 150                 155                 160
   Ala Lys Trp Gln Gln Leu Leu Phe Ser Thr Ala Met Asn Ser Ser Leu
                   165                 170                 175
   Lys Leu Leu Pro Gly Gln Trp Gln Asp Ile Asn Leu Thr Leu Lys Gly
               180                 185                 190
   Val Ser Pro Asn Asn Leu Lys Tyr Leu Lys Leu Ala Ile Asn Met Ala
           195                 200                 205
   Asn Ile Gln Phe Asp Arg Leu Gln Pro Ala Glu Ser Pro Gln Arg Lys
       210                 215                 220
   Asn Lys Lys
   225
<210> 36
   <211> 1109
   <212> PRT
   <213> Escherichia coli
<400> 36
   Met Lys Arg Val Val Arg Leu Leu Gly Val Gly Leu Leu Leu Leu Val
   1 5 10 15
   Val Leu Leu Leu Ile Leu Phe Val Leu Ala Gln Thr Thr Pro Leu Ile
   20 25 30
   Ser Ala Gln Asp Glu His Ala Val Trp Leu Arg Leu Leu Ile Thr Ala
   35 40 45
   Ile Val Ile Cys Leu Leu Ser Met Cys Ile Phe Phe Leu Phe Ser Phe
   50 55 60
   Arg Gln Asn Glu Ala Ser Thr Ile Ser Leu Tyr Ala Gln Pro Thr Asp
   65 70 75 80
   Ile Lys Glu Ile Asn Thr Glu Gln Pro Asn Tyr Ala Ser Leu Leu Thr
   85 90 95
   Ile Tyr Leu Arg Asp Arg Tyr Gly Pro Phe Trp Arg Arg Lys Val Arg
   100 105 110
   Leu Leu Leu Val Thr Gly Glu Pro Glu Gln Ala Glu Ala Ile Ala Pro
   115 120 125
   Gly Leu Thr Gly Gln His Trp Leu Glu Gly Asp His Thr Val Leu Ile
   130 135 140
   Tyr Gly Gly Arg Pro Thr Ala Glu Pro Asp Val Thr Leu Leu Thr Ala
   145 150 155 160
   Leu Lys Lys Leu Arg Arg Ser Arg Pro Leu Asp Gly Ile Ile Trp Ala
   165 170 175
   Leu Thr Glu Glu Gln Ser Arg Gln Thr Ala Gln Leu Asp Lys Gly Trp
   180 185 190
   Arg Gly Leu Ile Asn Gly Gly Lys Arg Leu Gly Phe Gln Ala Pro Leu
   195 200 205
   Tyr Leu Trp Gln Val Cys Asp Asp Gly Asp Tyr Gln Thr Gly Arg Pro
   210 215 220
   Leu Gln Ser Val Gly Cys Leu Leu Pro Glu Arg Cys Thr Pro Glu Gln
   225 230 235 240
   Leu Ala Val Met Leu Glu Ala Ala Ala Asp Gly Thr Gly His Val Ala
   245 250 255
   Ala Thr Asp Arg Tyr Arg Met Phe Ser Ala Ala Ser Gly Ser Tyr Pro
   260 265 270
   Cys Arg Ala Gly Tyr Cys Ser Leu Ala Asp Arg Pro Glu Thr Ala Ala
   275 280 285
   Gly Arg Arg Arg Ile Phe Phe Pro Ala Pro Ala Arg Pro Asp Val Gln
   290 295 300
   Pro Ala Ala Cys Arg Arg Ala Gly Gly Gln His Leu Met Gln Trp Leu
   305 310 315 320
   Pro Ser Pro Val Trp Ala Gly Val Thr Val Ile Thr Arg Ala Gly Ala
   325 330 335
   Arg Trp Val Phe Leu Trp Leu Arg Thr Ala Leu Met Ser Ala Val Cys
   340 345 350
   Val Leu Val Ile Trp Gly Ala Gly Met Thr Thr Ser Phe Phe Ala Asn
   355 360 365
   Arg Ala Leu Val Gln Glu Thr Gly Ile Gln Thr Ala Arg Ala Leu Asp
   370 375 380
   Thr Arg Leu Pro Leu Ala Glu Gln Leu Val Ala Leu His Thr Leu Gln
   385 390 395 400
   Gly Glu Leu Glu Arg Leu Gln Tyr Arg Ile Arg Glu Gly Ala Pro Trp
   405 410 415
   Tyr Gln Arg Phe Gly Leu Glu Arg Asn Gln Gln Leu Leu Ala Ala Ala
   420 425 430
   Phe Pro Gly Tyr Ala Gln Ala Ala Asn Arg Leu Val Arg Asp Val Ala
   435 440 445
   Val Asp His Leu Gln Gln Gln Leu Asn Ala Phe Val Ala Leu Pro Pro
   450 455 460
   Asn Ser Pro Gln Arg Thr Ala Thr Gly Glu Gln Arg Tyr Lys Gln Leu
   465 470 475 480
   Lys Ala Leu Leu Met Thr Ser Arg Pro Glu Lys Ala Asp Ala Ala Phe
   485 490 495
   Phe Ser Thr Thr Leu Met Ala Asp Gly Leu Arg Tyr Glu Asn Ile Pro
   500 505 510
   Glu Gly Val Arg Gln Ser Val Leu Pro Ser Leu Leu Thr Phe Trp Thr
   515 520 525
   Ala Asn Leu Pro Glu His Pro Gln Trp Lys Thr Ser Pro Pro Pro Glu
   530 535 540
   Leu Thr Gly Ala Val Arg Lys Ile Leu Leu Arg Gln Ile Gly Val Arg
   545 550 555 560
   Asn Ala Glu Asn Thr Leu Tyr Gln Asn Val Leu Gln Gln Val Ser Arg
   565 570 575
   Asn Tyr Ala Asp Met Thr Leu Ala Asp Met Thr Gly Asp Thr Leu Thr
   580 585 590
   Glu Ser Leu Phe Ser Thr Glu Gln Thr Val Pro Gly Met Phe Thr Arg
   595 600 605
   Gln Ala Trp Glu Gly Gln Val Arg Glu Ala Ile Glu Gln Val Val Thr
   610 615 620
   Ala Arg Arg Glu Glu Ile Asp Trp Val Leu Ser Asp Arg Gln Gln Asp
   625 630 635 640
   Thr Ser Ala Asp Ile Ser Pro Asp Thr Leu Arg Asn Arg Leu Thr Ser
   645 650 655
   Arg Tyr Phe Thr Asp Phe Ala Gly Ser Trp Leu Ala Phe Leu Asn Ser
   660 665 670
   Ile His Trp Lys Lys Glu Asp Ser Leu Ser Gly Ile Leu Asp Gln Leu
   675 680 685
   Thr Leu Met Ala Asp Ala Arg Gln Ser Pro Leu Ile Ala Leu Thr Asp
   690 695 700
   Thr Leu Ala Trp Gln Ala Ala Thr Gly Arg Glu Asn Arg Gly Leu Ser
   705 710 715 720
   Asp Ser Leu Ala Lys Ser Ala Gln Glu Leu Phe Asn Gly Lys Glu Lys
   725 730 735
   Thr Pro Gln Gln Ser Arg Glu Gly Asp Asp Val Pro Val Gly Pro Leu
   740 745 750
   Asp Lys Thr Phe Thr Pro Leu Leu Arg Leu Leu Gly Asp Lys Ala Gly
   755 760 765
   Gly Gly Asp Ser Gln Leu Ser Leu Gln Thr Tyr Leu Thr Arg Val Thr
   770 775 780
   Arg Val Arg Leu Lys Leu Gln Gln Val Thr Asn Ala Pro Asp Pro Gln
   785 790 795 800
   Glu Met Thr Gln Gln Leu Ala Gln Thr Val Leu Gln Gly Lys Thr Val
   805 810 815
   Asp Leu Thr Asp Thr Arg Asp Tyr Gly Arg Leu Ile Ala Ala Ser Leu
   820 825 830
   Gly Glu Glu Trp Ser Gly Phe Gly Gln Ala Leu Phe Val Arg Pro Val
   835 840 845
   Glu Gln Ser Trp Arg Gln Val Leu Thr Pro Ala Ala Asp Ser Leu Asn
   850 855 860
   Arg Gln Trp Gln Arg Ala Ile Val Ser His Trp Asn Gln Asp Phe Ala
   865 870 875 880
   Gly Arg Tyr Pro Phe Lys Ala Ser Gln Asn Asp Ala Ser Leu Pro Leu
   885 890 895
   Leu Ala Gln Tyr Leu Arg Asp Asp Gly Arg Ile Asn Leu Phe Ile Ala
   900 905 910
   Ala Asn Leu Ser Gly Val Leu Lys Arg Glu Gly Arg Tyr Trp Val Ala
   915 920 925
   Asp Ala Met Asn Thr Gln Gly Leu Thr Val Asn Pro Asp Phe Ile Arg
   930 935 940
   Ala Leu Asn Arg Leu Arg Asp Val Ala Asp Thr Ala Phe Ala Ser Gly
   945 950 955 960
   Asp Ala Gly Ile His Phe Glu Leu Arg Ala Lys Pro Ala Arg Asp Val
   965 970 975
   Met Lys Thr His Leu Val Ile Asp Gly Gln Glu Leu Glu Tyr Phe Asn
   980 985 990
   Gln Lys Glu Arg Trp Gln Arg Phe Asn Trp Pro Asp Glu Gln Trp Gln
   995 1000 1005
   Pro Gly Ala Ser Leu Ser Trp Thr Ser Thr Gln Ala Met Glu Arg
   1010 1015 1020
   Ile Leu Ala Asp Tyr Arg Gly Ser Trp Ser Leu Ile Arg Leu Leu
   1025 1030 1035
   Glu Gln Ala Gln Val Thr Pro Val Asp Ser Ser Thr Phe Lys Val
   1040 1045 1050
   Val Trp Lys Ala Gln Asp Gly Leu Pro Leu Asn Tyr Leu Leu Arg
   1055 1060 1065
   Val Glu Gln Gly Lys Gly Pro Leu Ala Leu Leu Glu Leu Lys Asn
   1070 1075 1080
   Phe Arg Leu Pro Gly Gln Val Phe Leu Thr Gly Lys Ser Met Lys
   1085 1090 1095
   Asp Val Glu Glu Tyr Gly Glu Asp Ala Asp Glu
   1100 1105
<210> 37
   <211> 178
   <212> PRT
   <213> Escherichia coli
<400> 37
   Met Phe Pro Ile Arg Phe Lys Arg Pro Ala Leu Leu Cys Met Ala Met
   1 5 10 15
   Leu Thr Val Val Leu Ser Gly Cys Gly Leu Ile Gln Lys Val Val Asp
   20 25 30
   Glu Ser Lys Ser Val Ala Ser Ala Val Phe Tyr Lys Gln Ile Lys Ile
   35 40 45
   Leu His Leu Asp Phe Phe Ser Arg Ser Ala Leu Asn Thr Asp Ala Glu
   50 55 60
   Asp Thr Pro Leu Ser Thr Met Val His Val Trp Gln Leu Lys Thr Arg
   65 70 75 80
   Glu Asp Phe Asp Lys Ala Asp Tyr Asp Thr Leu Phe Met Gln Glu Glu
   85 90 95
   Lys Thr Leu Glu Lys Asp Val Leu Ala Lys His Thr Val Trp Val Lys
   100 105 110
   Pro Glu Gly Thr Ala Ser Leu Asn Val Pro Leu Asp Lys Glu Thr Gln
   115 120 125
   Phe Val Ala Ile Ile Gly Gln Phe Tyr His Pro Asp Glu Lys Ser Asp
   130 135 140
   Ser Trp Arg Leu Val Ile Lys Arg Asp Glu Leu Glu Ala Asp Lys Pro
   145 150 155 160
   Arg Ser Ile Glu Leu Met Arg Ser Asp Leu Arg Leu Leu Pro Leu Lys
   165 170 175
   Asp Lys
<210> 38
   <211> 280
   <212> PRT
   <213> Escherichia coli
<400> 38
   Met Ile Ser Gly Gly Asn Met Leu Lys Glu Trp Met Ile Phe Thr Cys
   1               5                   10                  15
   Ser Leu Leu Thr Leu Ala Gly Ala Ser Leu Pro Leu Ser Gly Cys Ile
               20                  25                  30
   Ser Arg Gly Gln Glu Ser Ile Ser Glu Gly Ala Ala Phe Gly Ala Gly
           35                  40                  45
   Ile Leu Arg Glu Pro Gly Ala Thr Lys Lys Ala Asp Thr Lys Asp Leu
       50                  55                  60
   Asn Val Pro Pro Pro Val Tyr Gly Pro Pro Gln Val Ile Phe Arg Ile
   65                  70                  75                  80
   Asp Asp Asn Arg Tyr Phe Thr Leu Glu Asn Tyr Thr His Cys Glu Asn
                   85                  90                  95
   Gly Gln Thr Phe Tyr Asn Asn Lys Ala Lys Asn Ile His Val Lys Ile
               100                 105                 110
   Leu Asp Ala Ser Gly Tyr Leu Phe Lys Gly Arg Leu Phe Trp Leu Ser
           115                 120                 125
   Thr Arg Asp Asp Phe Leu Ala Phe Pro Ala Thr Leu Asn Thr Arg His
       130                 135                 140
   Ala Ser Cys Met Gly Ser Asn Lys Gly Cys Met Asn Ala Val Ile Val
   145                 150                 155                 160
   Thr Thr Asp Gly Gly Lys Arg Arg Ser Gly Val Pro Tyr Gly Ser Tyr
                   165                 170                 175
   Thr Gln Asn Pro Thr Gly Ala Thr Arg Asp Tyr Asp Met Leu Val Met
               180                 185                 190
   Asn Asp Gly Phe Tyr Leu Leu Arg Tyr Arg Gly Gly Gln Gly Arg Phe
           195                 200                 205
   Ser Pro Val Ile Leu Arg Trp Ile Leu Ser Thr Glu Asp Ser Ser Gly
   210 215 220
   Val Val Arg Ser Glu Asp Ala Tyr Glu Leu Phe Arg Pro Gly Glu Glu
   225 230 235 240
   Val Pro Ser Thr Gly Phe Tyr Lys Ile Asp Leu Ser Arg Phe Tyr Pro
   245 250 255
   Lys Asn Asn Val Met Glu Met Gln Cys Asp Arg Thr Leu Glu Pro Val
   260 265 270
   Gln Pro Ser Glu Ser Lys Ile Gln
   275 280
<210> 39
   <211> 501
   <212> PRT
   <213> Escherichia coli
<400> 39
   Met Glu His Val Ser Ile Lys Thr Leu Tyr His Leu Leu Cys Cys Met
   1 5 10 15
   Leu Leu Phe Ile Ser Ala Met Cys Ala Leu Ala Gln Glu His Glu Pro
   20 25 30
   Ile Gly Ala Gln Asp Glu Arg Leu Ser Thr Leu Ile His Gln Arg Met
   35 40 45
   Gln Glu Ala Lys Val Pro Ala Leu Ser Val Ser Val Thr Ile Lys Gly
   50 55 60
   Val Arg Gln Arg Phe Val Tyr Gly Val Ala Asp Val Ala Ser Gln Lys
   65 70 75 80
   Ala Asn Thr Leu Asp Thr Val Tyr Glu Leu Gly Ser Met Ser Lys Ala
   85 90 95
   Phe Thr Gly Leu Val Val Gln Ile Leu Ile Gln Glu Gly Arg Leu Arg
   100 105 110
   Gln Gly Asp Asp Ile Ile Thr Tyr Leu Pro Glu Met Arg Leu Asn Tyr
   115 120 125
   Gln Gly Lys Pro Ala Ser Leu Thr Val Ala Asp Phe Leu Tyr His Thr
       130                 135                 140
   Ser Gly Leu Pro Phe Ser Thr Leu Ala Arg Leu Glu Asn Pro Met Pro
   145                 150                 155                 160
   Gly Ser Ala Val Ala Gln Gln Leu Arg Asn Glu Asn Leu Leu Phe Ala
                   165                 170                 175
   Pro Gly Ala Lys Phe Ser Tyr Ala Ser Ala Asn Tyr Asp Val Leu Gly
               180                 185                 190
   Ala Val Ile Glu Asn Val Thr Gly Lys Thr Phe Thr Glu Val Ile Ala
           195                 200                 205
   Glu Arg Leu Thr Gln Pro Leu Gly Met Ser Ala Thr Val Ala Val Lys
       210                 215                 220
   Gly Asp Glu Ile Ile Val Asn Lys Ala Ser Gly Tyr Lys Leu Gly Phe
   225                 230                 235                 240
   Gly Lys Pro Val Leu Phe His Ala Pro Leu Ala Arg Asn His Val Pro
                   245                 250                 255
   Ala Ala Tyr Ile His Ser Thr Leu Pro Asp Met Glu Ile Trp Ile Asp
               260                 265                 270
   Ala Trp Leu His Arg Lys Ala Leu Pro Ala Thr Leu Arg Glu Ala Met
           275                 280                 285
   Ser Asn Ser Trp Arg Gly Asn Ser Asp Val Pro Leu Ala Ala Asp Asn
       290                 295                 300
   Arg Ile Leu Tyr Ala Ser Gly Trp Phe Ile Asp Gln Asn Gln Gly Pro
   305                 310                 315                 320
   Tyr Ile Ser His Gly Gly Gln Asn Pro Asn Phe Ser Ser Cys Ile Ala
                   325                 330                 335
   Leu Arg Pro Asp Gln Gln Ile Gly Ile Val Ala Leu Ala Asn Met Asn
               340                 345                 350
   Ser Asn Leu Ile Leu Gln Leu Cys Ala Asp Ile Asp Asn Tyr Leu Arg
   355 360 365
   Ile Gly Lys Tyr Ala Asp Gly Ala Gly Asp Ala Ile Thr Ala Thr Asp
   370 375 380
   Thr Leu Phe Val Tyr Leu Thr Leu Leu Leu Cys Phe Trp Gly Ala Val
   385 390 395 400
   Val Val Val Arg Gly Ala Phe Arg Val Tyr Arg Ala Thr Ala His Gly
   405 410 415
   Pro Gly Lys Gln Gln Arg Leu Arg Leu Arg Val Arg Asp Tyr Ile Ile
   420 425 430
   Ala Leu Ala Val Pro Gly Leu Val Ala Ala Met Leu Tyr Val Ala Pro
   435 440 445
   Gly Ile Leu Ser Pro Gly Leu Asp Trp Arg Phe Ile Leu Val Trp Gly
   450 455 460
   Pro Ser Ser Val Leu Ala Ile Pro Phe Gly Ile Ile Leu Leu Ala Phe
   465 470 475 480
   Val Leu Thr Leu Asn His Gln Ile Lys Arg Ile Leu Leu His Asn Lys
   485 490 495
   Glu Trp Asp Asp Glu
   500
<210> 40
   <211> 682
   <212> PRT
   <213> Escherichia coli
<400> 40
   Met Lys Asn Lys Tyr Ile Ile Ala Pro Gly Ile Ala Val Met Cys Ser
   1 5 10 15
   Ala Val Ile Ser Ser Gly Tyr Ala Ser Ser Asp Lys Lys Glu Asp Thr
   20 25 30
   Leu Val Val Thr Ala Ser Gly Phe Thr Gln Gln Leu Arg Asn Ala Pro
   35 40 45
   Ala Ser Val Ser Val Ile Thr Ser Glu Gln Leu Gln Lys Lys Pro Val
       50                  55                  60
   Ser Asp Leu Val Asp Ala Val Lys Asp Val Glu Gly Ile Ser Ile Thr
   65                  70                  75                  80
   Gly Gly Asn Glu Lys Pro Asp Ile Ser Ile Arg Gly Leu Ser Gly Asp
                   85                  90                  95
   Tyr Thr Leu Ile Leu Val Asp Gly Arg Arg Gln Ser Gly Arg Glu Ser
               100                 105                 110
   Arg Pro Asn Gly Ser Gly Gly Phe Glu Ala Gly Phe Ile Pro Pro Val
           115                 120                 125
   Glu Ala Ile Glu Arg Ile Glu Val Ile Arg Gly Pro Met Ser Ser Leu
       130                 135                 140
   Tyr Gly Ser Asp Ala Ile Gly Gly Val Ile Asn Ile Ile Thr Lys Pro
   145                 150                 155                 160
   Val Asn Asn Gln Thr Trp Asp Gly Val Leu Gly Leu Gly Gly Ile Ile
                   165                 170                 175
   Gln Glu His Gly Lys Phe Gly Asn Ser Thr Thr Asn Asp Phe Tyr Leu
               180                 185                 190
   Ser Gly Pro Leu Ile Lys Asp Lys Leu Gly Leu Gln Leu Tyr Gly Gly
           195                 200                 205
   Met Asn Tyr Arg Lys Glu Asp Ser Ile Ser Gln Gly Thr Pro Ala Lys
       210                 215                 220
   Asp Asn Lys Asn Ile Thr Ala Thr Leu Gln Phe Thr Pro Thr Glu Ser
   225                 230                 235                 240
   Gln Lys Phe Val Phe Glu Tyr Gly Lys Asn Asn Gln Val His Thr Leu
                   245                 250                 255
   Thr Pro Gly Glu Ser Leu Asp Ala Trp Thr Met Arg Gly Asn Leu Lys
               260                 265                 270
   Gln Pro Asn Ser Lys Arg Glu Thr His Asn Ser Arg Ser His Trp Val
   275 280 285
   Ala Ala Trp Asn Ala Gln Gly Glu Ile Leu His Pro Glu Ile Ala Val
   290 295 300
   Tyr Gln Glu Lys Val Ile Arg Glu Val Lys Ser Gly Lys Lys Asp Lys
   305 310 315 320
   Tyr Asn His Trp Asp Leu Asn Tyr Glu Ser Arg Lys Pro Glu Ile Thr
   325 330 335
   Asn Thr Ile Ile Asp Ala Lys Val Thr Ala Phe Leu Pro Glu Asn Val
   340 345 350
   Leu Thr Ile Gly Gly Gln Phe Gln His Ala Glu Leu Arg Asp Asp Ser
   355 360 365
   Ala Thr Gly Lys Lys Thr Thr Glu Thr Gln Ser Val Ser Ile Lys Gln
   370 375 380
   Lys Ala Val Phe Ile Glu Asn Glu Tyr Ala Ala Thr Asp Ser Leu Ala
   385 390 395 400
   Leu Thr Gly Gly Leu Arg Leu Asp Asn His Glu Ile Tyr Gly Ser Tyr
   405 410 415
   Trp Asn Pro Arg Leu Tyr Ala Val Tyr Asn Leu Thr Asp Asn Leu Thr
   420 425 430
   Leu Lys Gly Gly Ile Ala Lys Ala Phe Arg Ala Pro Ser Ile Arg Glu
   435 440 445
   Val Ser Pro Gly Phe Gly Thr Leu Thr Gln Gly Gly Ala Ser Ile Met
   450 455 460
   Tyr Gly Asn Arg Asp Leu Lys Pro Glu Thr Ser Val Thr Glu Glu Ile
   465 470 475 480
   Gly Ile Ile Tyr Ser Asn Asp Ser Gly Phe Ser Ala Ser Ala Thr Leu
   485 490 495
   Phe Asn Thr Asp Phe Lys Asn Lys Leu Thr Ser Tyr Asp Ile Gly Thr
   500 505 510
   Lys Asp Pro Val Thr Gly Leu Asn Thr Phe Ile Tyr Asp Asn Val Gly
   515 520 525
   Glu Ala Asn Ile Arg Gly Val Glu Leu Ala Thr Gln Ile Pro Val Tyr
   530 535 540
   Asp Lys Trp His Val Ser Ala Asn Tyr Thr Phe Thr Asp Ser Arg Arg
   545 550 555 560
   Lys Ser Asp Asp Glu Ser Leu Asn Gly Lys Ser Leu Lys Gly Glu Pro
   565 570 575
   Leu Glu Arg Thr Pro Arg His Ala Ala Asn Ala Lys Leu Glu Trp Asp
   580 585 590
   Tyr Thr Gln Asp Ile Thr Phe Tyr Ser Ser Leu Asn Tyr Thr Gly Lys
   595 600 605
   Gln Ile Trp Ala Ala Gln Arg Asn Gly Ala Lys Val Pro Arg Val Arg
   610 615 620
   Asn Gly Phe Thr Ser Met Asp Ile Gly Leu Asn Tyr Gln Ile Leu Pro
   625 630 635 640
   Asp Thr Leu Ile Asn Phe Ala Val Leu Asn Val Thr Asp Arg Lys Ser
   645 650 655
   Glu Asp Ile Asp Thr Ile Asp Gly Asn Trp Gln Val Asp Glu Gly Arg
   660 665 670
   Arg Tyr Trp Ala Asn Val Arg Val Ser Phe
   675 680
<210> 41
   <211> 164
   <212> PRT
   <213> Escherichia coli
<400> 41
   Met Gly Phe Arg Lys Thr Ile Ile Thr Ser Val Gly Leu Ile Phe Ile
   1 5 10 15
   Ser Phe Ser Phe Val Ala Lys Cys Ser Gln Leu Lys Asn Leu Asn Asn
   20 25 30
   Tyr Ser Val Met Leu Cys Gly Lys Val Ser Asn Asn Ile Leu Asp Asp
   35 40 45
   Ile Gly Gly Tyr Lys Glu Arg Asn Ile Leu Met Leu Arg Ala Ile Lys
   50 55 60
   Lys Ile Ile Ile Met Thr Ile Val Asn Ile Ile Phe Phe Tyr Ser Phe
   65 70 75 80
   Gln Ser Thr Ala Asp Glu Met Val Leu Ile Lys Lys Tyr Gly Phe Gly
   85 90 95
   Leu Glu Arg Asp Ile Lys Gly Arg Pro Leu Ile Tyr Pro Ile Glu Asn
   100 105 110
   Tyr Asp Glu Cys Lys Lys Lys Cys Asn His Met Asn Tyr Ile Ala Asp
   115 120 125
   Val Asn Ala Gln Leu Ala Met Ser Lys Lys Asn Asn Arg Ile Phe Ala
   130 135 140
   Asn Ile Thr Phe Thr Asn Asn Ser Ser Thr Thr Tyr Phe Phe Leu Asn
   145 150 155 160
   Ile Ile Tyr Leu
<210> 42
   <211> 218
   <212> PRT
   <213> Escherichia coli
<400> 42
   Met Asn Gln Ile Lys Asp Asn Lys Val Ile Met Lys Ile Lys Asn Leu
   1 5 10 15
   Ile Ser Val Ile Leu Leu Ser Gly Gly Ile Met Gly Thr Gly Leu Tyr
   20 25 30
   Ser Ser Asp Asn His Gln Lys Ile Arg Ser Arg Phe Asn Ile Gln Glu
   35 40 45
   Ser Tyr Cys Ala Ile Lys Thr Asn Gly Val Leu Gly Phe Ser Asn Arg
   50 55 60
   Lys Asp Val Leu Arg Glu Asn Gly Asp Ser Thr Gly Thr Thr Ser Ser
   65 70 75 80
   Ser Thr Asn Ala Met Met Leu Met Glu Asn Gly Glu Asn Glu Ile Ser
   85 90 95
   Leu Glu Ile Gly Ala Leu Arg Trp Phe Ser Asp Lys Pro Ala Ser Thr
   100 105 110
   Glu Glu Arg Gly His Phe Ser Gln Lys Ala Gly Cys Ser Leu Asp Leu
   115 120 125
   Val Arg Phe Val Lys Gln Glu Glu Thr Ile Leu Ser Ser Ile Lys Val
   130 135 140
   Thr Ile Asn Gln Gln Gly Ile Pro Glu Ala Gln Pro Asp Ser Met His
   145 150 155 160
   Pro Val Ile Arg Lys Glu Ile Leu Ala Glu Gln Ala Glu Pro Gly Phe
   165 170 175
   Ile Asp Pro Asp Tyr Phe Asn Glu Thr Tyr Phe Pro Lys Gly Met Lys
   180 185 190
   Val Tyr Gln Phe Thr Gln Lys Val Ser Val Ala Gly Leu Pro Asp Gly
   195 200 205
   Pro Gly Arg Ser Thr Pro Phe Thr Gly Ala
   210 215
<210> 43
   <211> 2732
   <212> PRT
   <213> Escherichia coli
<400> 43
   Met His Gln Pro Pro Val Arg Phe Thr Tyr Arg Leu Leu Ser Tyr Leu
   1 5 10 15
   Val Ser Ala Ile Ile Ala Gly Gln Pro Leu Leu Pro Ala Val Gly Ala
   20 25 30
   Val Ile Thr Pro Gln Asn Gly Ala Gly Met Asp Lys Ala Ala Asn Gly
   35 40 45
   Val Pro Val Val Asn Ile Ala Thr Pro Asn Gly Ala Gly Ile Ser His
   50 55 60
   Asn Arg Phe Thr Asp Tyr Asn Val Gly Lys Glu Gly Leu Ile Leu Asn
   65 70 75 80
   Asn Ala Thr Gly Lys Leu Asn Pro Thr Gln Leu Gly Gly Leu Ile Gln
   85 90 95
   Asn Asn Pro Asn Leu Lys Ala Gly Gly Glu Ala Lys Gly Ile Ile Asn
   100 105 110
   Glu Val Thr Gly Gly Lys Arg Ser Leu Leu Gln Gly Tyr Thr Glu Val
   115 120 125
   Ala Gly Lys Ala Ala Asn Val Met Val Ala Asn Pro Tyr Gly Ile Thr
   130 135 140
   Cys Asp Gly Cys Gly Phe Ile Asn Thr Pro His Ala Thr Leu Thr Thr
   145 150 155 160
   Gly Lys Pro Val Met Asn Ala Asp Gly Ser Leu Gln Ala Leu Glu Val
   165 170 175
   Thr Glu Gly Ser Ile Thr Ile Asn Gly Ala Gly Leu Asp Gly Thr Arg
   180 185 190
   Ser Asp Ala Val Ser Ile Ile Ala Arg Ala Thr Glu Val Asn Ala Ala
   195 200 205
   Leu His Ala Lys Asp Leu Thr Val Thr Ala Gly Ala Asn Arg Val Thr
   210 215 220
   Ala Asp Gly Arg Val Arg Ala Leu Lys Gly Glu Gly Asp Val Pro Lys
   225 230 235 240
   Val Ala Val Asp Thr Gly Ala Leu Gly Gly Met Tyr Ala Arg Arg Ile
   245 250 255
   His Leu Thr Ser Thr Glu Ser Gly Val Gly Val Asn Leu Gly Asn Leu
   260 265 270
   Tyr Ala Arg Asp Gly Asp Ile Thr Leu Asp Ala Ser Gly Arg Leu Thr
   275 280 285
   Val Asn Asn Ser Leu Ala Thr Gly Ala Val Thr Ala Lys Gly Gln Gly
   290 295 300
   Val Thr Leu Thr Gly Asp His Lys Ala Gly Gly Asn Leu Ser Val Ser
   305 310 315 320
   Ser Arg Arg Asp Ile Val Leu Ser Asn Gly Thr Leu Asn Ser Asp Lys
   325 330 335
   Asp Leu Ser Leu Thr Ala Gly Gly Arg Ile Thr Gln Gln Asn Glu Lys
   340 345 350
   Leu Thr Ala Gly Arg Asp Val Thr Leu Ala Ala Lys Asn Ile Thr Gln
   355 360 365
   Asp Thr Ala Ser Gln Ile Asn Ala Ala Arg Asp Ile Val Thr Val Ala
   370 375 380
   Ser Asp Thr Leu Thr Thr Gln Gly Gln Ile Thr Ala Gly Gln Asn Leu
   385 390 395 400
   Thr Ala Ser Ala Thr Thr Leu Thr Gln Asp Gly Ile Leu Leu Ala Lys
   405 410 415
   Ser His Ala Gly Leu Asn Ala Gly Thr Leu Asn Asn Ser Gly Ala Val
   420 425 430
   Gln Gly Ala Thr Leu Thr Leu Gly Ser Thr Thr Leu Ser Asn Ser Gly
   435 440 445
   Ser Leu Leu Ser Gly Gly Pro Leu Thr Met Asn Thr Arg Asp Phe Thr
   450 455 460
   Gln Ser Gly Arg Thr Gly Ala Lys Gly Lys Val Asp Ile Met Ala Ser
   465 470 475 480
   Gly Lys Leu Thr Ser Thr Gly Leu Leu Val Thr Met His Leu Val Leu
   485 490 495
   Lys Ala Gln Asp Val Thr Gln Asn Gly Val Leu Ser Gly Gly Lys Gly
   500 505 510
   Leu Thr Val Ser Ala Thr Ser Ser Gly Lys Lys Ser Val Thr His Ser
   515 520 525
   Asp Ala Ala Met Thr Leu Asn Val Thr Thr Val Ala Leu Asp Gly Glu
   530 535 540
   Thr Ser Ala Gly Asp Thr Leu Arg Val Gln Ala Asp Lys Leu Ser Thr
   545 550 555 560
   Ala Ala Gly Ala Gln Leu Gln Ser Gly Lys Asn Leu Ser Ile Asn Ala
   565 570 575
   Arg Asp Ala Arg Leu Ala Gly Thr Gln Ala Ala Gln Gln Thr Met Val
   580 585 590
   Val Asn Ala Ser Glu Lys Leu Thr His Ser Gly Lys Ser Ser Ala Pro
   595 600 605
   Ser Leu Ser Leu Ser Ala Pro Glu Leu Thr Ser Ser Gly Val Leu Val
   610 615 620
   Gly Ser Ala Leu Asn Thr Gln Ser Gln Thr Leu Thr Asn Ser Gly Leu
   625 630 635 640
   Leu Gln Gly Glu Ala Ser Leu Thr Val Asn Thr Gln Arg Leu Asp Asn
   645 650 655
   Gln Gln Asn Gly Thr Leu Tyr Ser Ala Ala Asp Leu Thr Leu Asp Ile
   660 665 670
   Pro Asp Ile Arg Asn Ser Gly Leu Ile Thr Gly Asp Asn Gly Leu Met
   675 680 685
   Leu Asn Ala Val Ser Leu Ser Asn Pro Gly Lys Ile Ile Ala Asp Thr
   690 695 700
   Leu Ser Val Arg Ala Thr Thr Leu Asp Gly Asp Gly Leu Leu Gln Gly
   705 710 715 720
   Ala Gly Ala Leu Ala Leu Ala Gly Asp Thr Leu Ser Gln Gly Ser His
   725 730 735
   Gly Arg Trp Leu Thr Ala Asp Asp Leu Ser Leu Arg Gly Lys Thr Leu
   740 745 750
   Asn Thr Ala Gly Thr Thr Gln Gly Gln Asn Ile Thr Val Gln Ala Asp
   755 760 765
   Arg Trp Ala Asn Ser Gly Ser Val Leu Ala Thr Gly Asn Leu Thr Ala
   770 775 780
   Ser Ala Thr Gly Gln Leu Thr Ser Thr Gly Asp Ile Met Ser Gln Gly
   785 790 795 800
   Asp Thr Thr Leu Lys Ala Ala Thr Thr Asp Asn Arg Gly Ser Leu Leu
   805 810 815
   Ser Ala Gly Thr Leu Ser Leu Asp Gly Asn Ser Leu Asp Asn Arg Gly
   820 825 830
   Thr Val Gln Gly Asn His Val Thr Ile Arg Gln Asn Ser Val Thr Asn
   835 840 845
   Ser Gly Thr Leu Thr Gly Ile Ala Ala Leu Thr Leu Ala Ala Arg Met
   850 855 860
   Ala Ser Pro Gln Pro Ala Leu Met Asn Asn Gly Gly Ser Leu Leu Thr
   865 870 875 880
   Ser Gly Asp Leu Thr Ile Thr Ala Gly Ser Ile Thr Ser Ser Gly His
   885 890 895
   Trp Gln Gly Lys Arg Val Leu Ile Thr Ala Asp Ser Leu Ala Asn Ser
   900 905 910
   Gly Ala Ile Gln Ala Ala Asp Ser Leu Thr Ala Arg Leu Thr Gly Glu
   915 920 925
   Leu Val Ser Thr Ala Gly Ser Lys Val Thr Ser Asn Gly Glu Met Ala
   930 935 940
   Leu Ser Ala Leu Asn Leu Ser Asn Ser Gly Gln Trp Ile Ala Lys Asn
   945 950 955 960
   Leu Thr Leu Lys Ala Asn Ser Leu Thr Ser Ala Gly Asp Ile Thr Gly
   965 970 975
   Val Asp Thr Leu Thr Leu Thr Val Asn Gln Thr Leu Asn Asn Gln Ala
   980 985 990
   Asn Gly Lys Leu Leu Ser Ala Gly Val Leu Thr Leu Lys Ala Asp Ser
   995 1000 1005
   Val Thr Asn Asp Gly Gln Leu Gln Gly Asn Val Thr Thr Ile Thr
   1010 1015 1020
   Ala Gly Gln Leu Thr Asn Gly Gly His Leu Gln Gly Glu Thr Leu
   1025 1030 1035
   Thr Leu Thr Ala Ser Gly Gly Val Asn Asn Arg Ser Gly Gly Val
   1040 1045 1050
   Leu Met Ser Arg Asn Ala Leu Asn Val Ser Thr Ala Thr Leu Ser
   1055 1060 1065
   Asn Gln Ser Thr Ile Gln Gly Gly Gly Gly Val Ser Leu Asn Ala
   1070 1075 1080
   Thr Asp Arg Leu Gln Asn Asp Gly Lys Ile Leu Ser Gly Ser Asn
   1085 1090 1095
   Leu Thr Leu Thr Ala Gln Val Leu Ala Asn Thr Gly Ser Gly Leu
   1100 1105 1110
   Val Gln Ala Ala Thr Leu Leu Leu Asp Val Val Asn Thr Val Asn
   1115 1120 1125
   Gly Gly Arg Val Leu Ala Thr Gly Ser Asp Val Lys Gly Thr Thr
   1130 1135 1140
   Leu Asn Asn Thr Gly Thr Leu Gln Gly Ala Thr Leu Val Asn Tyr
   1145 1150 1155
   His Thr Phe Ser Ser Gly Thr Leu Leu Gly Thr Ser Gly Leu Gly
   1160 1165 1170
   Val Lys Gly Ser Ser Leu Leu Gln Asn Gly Thr Gly Arg Leu Tyr
   1175 1180 1185
   Ser Ala Gly Asn Leu Leu Leu Asp Ala Gln Asp Phe Ser Gly Gln
   1190 1195 1200
   Gly Gln Val Val Ala Thr Gly Asp Val Thr Leu Lys Leu Ile Ala
   1205 1210 1215
   Ala Leu Thr Asn His Gly Thr Leu Ala Ala Gly Lys Thr Leu Ser
   1220 1225 1230
   Val Thr Ser Gln Asn Ala Ile Thr Asn Gly Gly Val Met Gln Gly
   1235 1240 1245
   Asp Ala Met Val Leu Gly Ala Gly Glu Ala Phe Thr Asn Asn Gly
   1250 1255 1260
   Leu Thr Ala Gly Lys Gly Asn Ser Val Phe Ser Ala Gln Arg Leu
   1265 1270 1275
   Phe Leu Asn Ala Pro Gly Ser Leu Gln Gly Gly Gly Asp Val Ser
   1280 1285 1290
   Leu Asn Ser Arg Ser Asp Ile Thr Ile Ser Gly Phe Thr Gly Thr
   1295 1300 1305
   Ala Gly Ser Leu Thr Met Asn Val Ala Gly Thr Leu Leu Asn Ser
   1310 1315 1320
   Ala Leu Ile Tyr Ala Gly Asn Asn Leu Lys Leu Phe Thr Asp Arg
   1325 1330 1335
   Leu His Asn Gln His Gly Asp Ile Leu Ala Gly Asn Ser Leu Trp
   1340 1345 1350
   Val Gln Lys Asp Ala Ser Gly Gly Ala Asn Thr Glu Ile Ile Asn
   1355 1360 1365
   Asn Ser Gly Asn Ile Glu Thr His Gln Gly Asp Ile Val Val Arg
   1370 1375 1380
   Thr Gly His Leu Leu Asn Gln Arg Glu Gly Phe Ser Ala Thr Thr
   1385 1390 1395
   Thr Thr Arg Thr Asn Pro Ser Ser Ile Gln Gly Met Gly Asn Ala
   1400 1405 1410
   Leu Val Asp Ile Pro Leu Ser Leu Leu Pro Asp Gly Ser Tyr Gly
   1415 1420 1425
   Tyr Phe Thr Arg Glu Val Glu Asn Gln His Gly Thr Pro Cys Asn
   1430 1435 1440
   Gly His Gly Ala Cys Asn Ile Thr Met Asp Thr Leu Tyr Tyr Tyr
   1445 1450 1455
   Ala Pro Phe Ala Asp Ser Ala Thr Gln Arg Phe Leu Ser Ser Gln
   1460 1465 1470
   Asn Ile Thr Thr Val Thr Gly Ala Asp Asn Pro Ala Gly Arg Ile
   1475 1480 1485
   Ala Ser Gly Arg Asn Leu Ser Ala Glu Ala Glu Arg Leu Glu Asn
   1490 1495 1500
   Arg Ala Ser Phe Ile Leu Ala Asn Gly Asp Ile Ala Leu Ser Gly
   1505 1510 1515
   Arg Glu Leu Ser Asn Gln Ser Trp Gln Thr Gly Thr Glu Asn Glu
   1520 1525 1530
   Tyr Leu Val Tyr Arg Tyr Asp Pro Lys Thr Phe Tyr Gly Ser Tyr
   1535 1540 1545
   Ala Thr Gly Ser Leu Asp Lys Leu Pro Leu Leu Ser Pro Glu Phe
   1550 1555 1560
   Glu Asn Asn Thr Ile Arg Phe Ser Leu Asp Gly Arg Glu Lys Asp
   1565 1570 1575
   Tyr Thr Pro Gly Lys Thr Tyr Tyr Ser Val Ile Gln Ala Gly Gly
   1580 1585 1590
   Asp Val Lys Thr Arg Phe Thr Ser Ser Ile Asn Asn Gly Thr Thr
   1595 1600 1605
   Thr Ala His Ala Gly Ser Val Ser Pro Val Val Ser Ala Pro Val
   1610 1615 1620
   Leu Asn Thr Leu Ser Gln Gln Thr Gly Gly Asp Ser Leu Thr Gln
   1625 1630 1635
   Thr Ala Leu Gln Gln Tyr Glu Pro Val Val Val Gly Ser Pro Gln
   1640 1645 1650
   Trp His Asp Glu Leu Ala Gly Ala Leu Lys Asn Ile Ala Gly Gly
   1655 1660 1665
   Ser Pro Leu Thr Gly Gln Thr Gly Ile Ser Asp Asp Trp Pro Leu
   1670 1675 1680
   Pro Ser Gly Asn Asn Gly Tyr Leu Val Pro Ser Thr Asp Pro Asp
   1685 1690 1695
   Ser Pro Tyr Leu Ile Thr Val Asn Pro Lys Leu Asp Gly Leu Gly
   1700 1705 1710
   Gln Val Asp Ser His Leu Phe Ala Gly Leu Tyr Glu Leu Leu Gly
   1715 1720 1725
   Ala Lys Pro Gly Gln Ala Pro Arg Glu Thr Ala Pro Ser Tyr Thr
   1730 1735 1740
   Asp Glu Lys Gln Phe Leu Gly Ser Ser Tyr Phe Leu Asp Arg Leu
   1745 1750 1755
   Gly Leu Lys Pro Glu Lys Asp Tyr Arg Phe Leu Gly Asp Ala Val
   1760 1765 1770
   Phe Asp Thr Arg Tyr Val Ser Asn Ala Val Leu Ser Arg Thr Gly
   1775 1780 1785
   Ser Arg Tyr Leu Asn Gly Leu Gly Ser Asp Thr Glu Gln Met Arg
   1790 1795 1800
   Tyr Leu Met Asp Asn Ala Ala Arg Gln Gln Lys Gly Leu Gly Leu
   1805 1810 1815
   Glu Phe Gly Val Ala Leu Thr Ala Glu Gln Ile Ala Gln Leu Asp
   1820 1825 1830
   Gly Ser Ile Leu Trp Trp Glu Ser Val Thr Ile Asn Gly Gln Thr
   1835 1840 1845
   Val Met Val Pro Lys Leu Tyr Leu Ser Pro Glu Asp Ile Thr Leu
   1850 1855 1860
   His Asn Gly Ser Val Ile Ser Gly Asn Asn Val Gln Leu Ala Gly
   1865 1870 1875
   Gly Asn Ile Thr Asn Ser Gly Gly Ser Ile Asn Ala Gln Asn Asp
   1880 1885 1890
   Leu Ser Leu Asp Ser Ser Gly Tyr Ile Asp Asn Leu Asn Ala Gly
   1895 1900 1905
   Leu Ile Ser Ala Gly Gly Ser Leu Asp Leu Ser Ala Ile Gly Asp
   1910 1915 1920
   Ile Ser Asn Ile Ser Ser Val Ile Ser Gly Lys Thr Val Gln Leu
   1925 1930 1935
   Glu Ser Val Ser Gly Asn Ile Ser Asn Ile Thr Arg Arg Gln Gln
   1940 1945 1950
   Trp Asn Ala Gly Ser Asp Ser Gln Tyr Gly Gly Val His Leu Ser
   1955 1960 1965
   Gly Thr Asp Thr Gly Pro Val Ala Thr Ile Lys Gly Thr Asp Ser
   1970 1975 1980
   Leu Ser Leu Asp Ala Gly Lys Asn Ile Asp Ile Thr Gly Ala Thr
   1985 1990 1995
   Val Ser Ser Gly Gly Asp Leu Gly Met Ser Ala Gly Asn Asp Ile
   2000 2005 2010
   Asn Ile Ala Ala Asn Leu Ile Ser Gly Ser Lys Ser Gln Ser Gly
   2015 2020 2025
   Phe Trp His Thr Asp Asp Asn Ser Ser Ser Ser Thr Thr Ser Gln
   2030 2035 2040
   Gly Ser Ser Ile Ser Ala Gly Gly Asn Leu Ala Met Ala Ala Gly
   2045 2050 2055
   His Asn Leu Asp Val Thr Ala Ser Ser Val Ser Ala Gly His Ser
   2060 2065 2070
   Ala Leu Leu Ser Cys Arg Ser Arg Pro Ser Leu Glu Cys Ser Gln
   2075 2080 2085
   Gly Lys Ala Lys Thr Ser Arg Asn Gly Arg Ser Glu Ser His Glu
   2090 2095 2100
   Ser His Ala Ala Val Ser Thr Val Thr Ala Gly Asp Asn Phe Leu
   2105 2110 2115
   Leu Val Ala Gly Arg Asp Ile Ala Ser Gln Ala Ala Gly Met Ala
   2120 2125 2130
   Ala Glu Asn Asn Val Val Ile Arg Gly Gly Arg Asp Val Asn Leu
   2135 2140 2145
   Val Ala Glu Ser Ala Gly Ala Gly Asp Ser Tyr Thr Ser Lys Lys
   2150 2155 2160
   Lys Lys Glu Ile Asn Glu Thr Val Arg Gln Gln Gly Thr Glu Ile
   2165 2170 2175
   Ala Ser Gly Gly Asp Thr Thr Val Asn Ala Gly Arg Asp Ile Thr
   2180 2185 2190
   Ala Val Ala Ser Ser Val Thr Ala Thr Gly Asn Ile Ser Val Asn
   2195 2200 2205
   Ala Gly Arg Asp Val Ala Leu Thr Thr Ala Thr Glu Ser Asp Tyr
   2210 2215 2220
   His Tyr Leu Glu Thr Lys Lys Lys Ser Gly Gly Phe Leu Ser Lys
   2225 2230 2235
   Lys Thr Thr Arg Thr Ile Ser Glu Asp Ser Ala Thr Arg Glu Ala
   2240 2245 2250
   Gly Ser Leu Leu Ser Gly Asn Arg Val Thr Val Asn Ala Gly Asp
   2255 2260 2265
   Asn Leu Thr Val Glu Gly Ser Asp Val Val Ala Asp Arg Asp Val
   2270 2275 2280
   Ser Leu Ala Ala Gly Asn His Val Asp Val Leu Ala Ala Thr Ser
   2285 2290 2295
   Thr Asp Thr Ser Trp Arg Phe Lys Glu Thr Lys Lys Ser Gly Leu
   2300 2305 2310
   Met Gly Thr Gly Gly Ile Gly Phe Thr Ile Gly Ser Ser Lys Thr
   2315 2320 2325
   Thr His Asp Arg Arg Glu Ala Gly Thr Thr Gln Ser Gln Ser Ala
   2330 2335 2340
   Ser Thr Ile Gly Ser Thr Ala Gly Asn Val Ser Ile Thr Ala Gly
   2345 2350 2355
   Lys Gln Ala His Ile Ser Gly Ser Asp Val Ile Ala Asn Arg Asp
   2360 2365 2370
   Ile Ser Ile Thr Gly Asp Ser Val Val Val Asp Pro Gly His Asp
   2375 2380 2385
   Arg Arg Thr Val Asp Glu Lys Phe Glu Gln Lys Lys Ser Gly Leu
   2390 2395 2400
   Thr Val Ala Leu Ser Gly Thr Val Gly Ser Ala Ile Asn Asn Ala
   2405 2410 2415
   Val Thr Ser Ala Gln Glu Thr Lys Glu Ser Ser Asp Ser Arg Leu
   2420 2425 2430
   Lys Ala Leu Gln Ala Thr Lys Thr Ala Leu Ser Gly Val Gln Ala
   2435 2440 2445
   Gly Gln Ala Ala Thr Met Ala Ser Ala Thr Gly Asp Pro Asn Ala
   2450 2455 2460
   Gly Val Ser Leu Ser Leu Thr Thr Gln Lys Ser Lys Ser Gln Gln
   2465 2470 2475
   His Ser Glu Ser Asp Thr Val Ser Gly Ser Thr Leu Asn Ala Gly
   2480 2485 2490
   Asn Asn Leu Ser Val Val Ala Thr Gly Lys Asn Arg Gly Asp Asn
   2495 2500 2505
   Arg Gly Asp Ile Val Ile Ala Gly Ser Gln Leu Lys Ala Gly Gly
   2510 2515 2520
   Asn Thr Ser Leu Asp Ala Ala Asn Asp Ile Leu Leu Ser Gly Ala
   2525 2530 2535
   Ala Asn Thr Gln Lys Thr Thr Gly Arg Asn Ser Ser Ser Gly Gly
   2540 2545 2550
   Gly Val Gly Val Ser Ile Gly Ala Gly Lys Gly Ala Gly Ile Ser
   2555 2560 2565
   Ala Phe Ala Ser Val Asn Ala Ala Lys Gly Arg Glu Lys Gly Asn
   2570 2575 2580
   Gly Thr Thr Thr Asp Lys Thr Val Thr Ile Asn Ser Gly Arg Asp
   2585 2590 2595
   Thr Val Leu Asn Gly Ala Gln Val Asn Gly Asn Arg Ile Ile Ala
   2600 2605 2610
   Asp Val Gly His Asp Leu Leu Ile Ser Ser Gln Gln Asp Thr Ser
   2615 2620 2625
   Lys Tyr Asp Ser Lys Gln Thr Ser Val Ala Ala Gly Gly Ser Phe
   2630 2635 2640
   Thr Phe Gly Ser Met Thr Gly Ser Gly Tyr Ile Ala Ala Ser Arg
   2645 2650 2655
   Asp Lys Met Lys Ser Arg Phe Asp Ser Val Ala Glu Gln Thr Gly
   2660 2665 2670
   Met Phe Ala Arg Val Met Val Ala Ser Thr Ser Gln Trp Val Asn
   2675 2680 2685
   Ile Pro Asn Trp Met Val Arg Ser Leu Pro His Cys His Thr Gly
   2690 2695 2700
   Glu Lys Pro Pro Gly Tyr Arg Thr Leu Gly Leu Val Thr Leu Gln
   2705 2710 2715
   Arg Ser Gly Ile Ile Lys Ser Ser His Arg Trp Asn Gln Ser
   2720 2725 2730
<210> 44
   <211> 321
   <212> PRT
   <213> Escherichia coli
<400> 44
   Met Met Leu Lys Lys Thr Ile Phe Ile Leu Thr Leu Phe Ser Gly Asn
   1 5 10 15
   Val Ile Ala Ala Thr Val Glu Leu Gly Phe Glu Asn Glu Gln Tyr Asn
   20 25 30
   Tyr Ala Tyr Arg Ser Ala Asp Val Phe Met Pro Tyr Ile Lys Ser Asn
   35 40 45
   Phe Asn Pro Val Thr Asp Ser Ala Leu Asn Val Ser Leu Thr Tyr Met
   50 55 60
   Tyr Gln Asp Gln Tyr Gly Lys Lys His Lys Lys Thr Ser Glu Asp Arg
   65 70 75 80
   Phe Lys Thr Asn Arg Asp Arg Ile Glu Leu Tyr Leu Lys Gly Tyr Thr
   85 90 95
   Leu Asn Arg Gly Ala Tyr Ser Phe Ser Pro Ser Ala Gly Phe Arg Tyr
   100 105 110
   Glu Ser Trp Asp Val Asn Tyr Asp Asn Pro Lys Lys Gln Asp Lys Trp
   115 120 125
   Lys Leu Glu Leu Arg Phe Tyr Pro Asn Met Thr Tyr Lys Leu Asn Asp
   130 135 140
   Gln Leu Ser Leu Tyr Met Asn Gly Phe Val Ala Pro Val Phe Phe Lys
   145 150 155 160
   Thr Gln Gln Glu Ser Arg Lys Asp Asn Asn Tyr Val Lys Gly Lys Leu
   165 170 175
   Gly Ala Lys Arg Tyr Asn Asn Asp Tyr Tyr Gln Glu Leu Gln Ile Leu
   180 185 190
   Gly Val Arg Tyr Lys Phe Asn Asn Asp Asn Thr Leu Trp Ala Ser Val
   195 200 205
   Tyr Asn Glu Arg Lys Tyr Asn Gln His Ser Ser Lys Tyr Asp Arg Trp
   210 215 220
   Gln Leu Arg Gly Gly Tyr Asp Phe Lys Val Thr Glu Glu Phe Val Leu
   225 230 235 240
   Ser Pro Phe Ile Arg Tyr Asp Leu Ser Tyr Arg Glu Lys Asn Leu Glu
   245 250 255
   Ser Thr Ser Asn Asn Gly Leu Ser Lys Asn Asn Lys Glu Ile Arg Thr
   260 265 270
   Gly Ala Ser Phe Ser Tyr Lys Ile Ile Pro Ser Val Lys Leu Val Gly
   275 280 285
   Glu Ile Tyr Arg Gln Thr Thr Asn Ile Glu Asn Tyr Tyr Gly Glu His
   290 295 300
   Ser Glu Asp Lys Asn Arg Met Phe Tyr Lys Leu Gly Ile Asn Lys Thr
   305 310 315 320
   Phe
<210> 45
   <211> 587
   <212> PRT
   <213> Escherichia coli
<400> 45
   Met Gln His Arg Gln Lys Asn Ile Leu Thr Lys Thr Ser Leu Leu Ser
   1 5 10 15
   Arg Ala Leu Ser Val Pro Cys Cys Asp Met Phe Arg Arg Gly Ser Pro
   20 25 30
   Trp Ile Cys Tyr Leu Ser Leu Ser Val Phe Ser Gly Cys Phe Ile Pro
   35 40 45
   Ala Phe Ser Ser Pro Ala Ala Met Leu Ser Pro Gly Asp Arg Ser Ala
   50 55 60
   Ile Gln Gln Gln Gln Gln Gln Leu Leu Asp Glu Asn Gln Arg Gln Arg
   65 70 75 80
   Asp Ala Leu Glu Arg Pro Leu Thr Ile Thr Pro Ser Pro Glu Thr Ser
   85 90 95
   Ala Gly Thr Glu Gly Pro Cys Phe Thr Val Ser Ser Ile Val Val Ser
   100 105 110
   Gly Ala Thr Arg Leu Thr Ser Ala Glu Thr Asp Arg Leu Val Pro Trp
   115 120 125
   Val Asn Gln Cys Leu Asn Ile Thr Gly Leu Thr Ala Val Thr Asp Ala
   130 135 140
   Val Thr Asp Gly Tyr Ile Arg Arg Gly Tyr Ile Thr Ser Arg Ala Phe
   145 150 155 160
   Leu Thr Glu Gln Asp Leu Ser Gly Gly Val Leu His Ile Thr Val Met
   165 170 175
   Glu Gly Arg Leu Gln Gln Ile Arg Ala Glu Gly Ala Asp Leu Pro Ala
   180 185 190
   Arg Thr Leu Lys Met Val Phe Pro Gly Met Glu Gly Lys Val Leu Asn
   195 200 205
   Leu Arg Asp Ile Glu Gln Gly Met Glu Gln Ile Asn Arg Leu Arg Thr
   210 215 220
   Glu Pro Val Gln Ile Glu Ile Ser Pro Gly Asp Arg Glu Gly Trp Ser
   225 230 235 240
   Val Val Thr Leu Thr Ala Leu Pro Glu Trp Pro Val Thr Gly Ser Val
   245 250 255
   Gly Ile Asp Asn Ser Gly Gln Lys Ser Thr Gly Thr Gly Gln Leu Asn
   260 265 270
   Gly Val Leu Ser Phe Asn Asn Pro Leu Gly Leu Ala Asp Asn Trp Phe
   275 280 285
   Val Ser Gly Gly Arg Ser Ser Asp Phe Ser Val Ser His Asp Ala Arg
   290 295 300
   Asn Phe Ala Ala Gly Val Ser Leu Pro Tyr Gly Tyr Thr Leu Val Asp
   305 310 315 320
   Tyr Thr Tyr Ser Trp Ser Asp Tyr Leu Ser Thr Ile Asp Asn Arg Gly
   325 330 335
   Trp Arg Trp Arg Ser Thr Gly Asp Leu Gln Thr His Arg Leu Gly Leu
   340 345 350
   Ser His Val Leu Phe Arg Asn Gly Asp Met Lys Thr Ala Leu Thr Gly
   355 360 365
   Gly Leu Gln His Arg Ile Ile His Asn Tyr Leu Asp Asp Val Leu Leu
   370 375 380
   Gln Gly Ser Ser Arg Lys Leu Thr Ser Phe Ser Val Gly Leu Asn His
   385 390 395 400
   Thr His Lys Phe Leu Gly Gly Val Gly Thr Leu Asn Pro Val Phe Thr
   405 410 415
   Arg Gly Met Pro Trp Phe Gly Ala Glu Ser Asp His Gly Lys Arg Gly
   420 425 430
   Asp Leu Pro Val Asn Gln Phe Arg Lys Trp Ser Val Ser Ala Ser Phe
   435 440 445
   Gln Arg Pro Val Thr Asp Arg Val Trp Trp Leu Thr Ser Ala Tyr Ala
   450 455 460
   Gln Trp Ser Pro Asp Arg Leu His Gly Val Glu Gln Leu Ser Leu Gly
   465 470 475 480
   Gly Glu Ser Ser Val Arg Gly Phe Lys Asp Gln Tyr Ile Ser Gly Asn
   485 490 495
   Asn Gly Gly Tyr Leu Arg Asn Glu Leu Ser Trp Ser Leu Phe Ser Leu
   500 505 510
   Pro Tyr Val Gly Thr Val Arg Ala Val Ala Ala Leu Asp Gly Gly Trp
   515 520 525
   Leu His Ser Asp Ser Asp Asp Pro Tyr Ser Ser Gly Thr Leu Trp Gly
   530 535 540
   Ala Ala Ala Gly Leu Ser Thr Thr Ser Gly His Val Ser Gly Ser Phe
   545 550 555 560
   Thr Ala Gly Leu Pro Leu Val Tyr Pro Asp Trp Leu Ala Pro Asp His
   565 570 575
   Leu Thr Val Tyr Trp Arg Val Ala Val Ala Phe
   580 585
<210> 46
   <211> 744
   <212> PRT
   <213> Escherichia coli
<400> 46
   Met Asn Lys His Thr Leu Leu Leu Thr Val Leu Phe Leu Asn Leu Ile
   1 5 10 15
   Cys Thr Pro Val Phe Ala Gln Asn Trp Gln Val Ala Thr Phe Gly Gln
   20 25 30
   Ser Thr Asp Leu Asn Phe Ser Ser Leu Ile Asp Ser Ala Lys Ile Gly
   35 40 45
   Arg Asn Asn Ala Trp Leu Ala Gly Asn Asn Asn Phe Leu Glu Ala Gly
       50                  55                  60
   Lys Phe Tyr Thr Leu Pro Thr Asp Phe Phe Ile Glu Ser Arg Gly Gly
   65                  70                  75                  80
   Lys Ile Ala Asn Ser His Asp Gly Met Thr Val Phe Tyr Thr Ile Val
                   85                  90                  95
   Pro Val Thr Gln Thr Phe Arg Leu Glu Ala Asp Leu Thr Leu Glu Gln
               100                 105                 110
   Ile Gly Pro Glu Val Asn Gly Lys Ser Pro Ala Gly Gln Glu Gly Ala
           115                 120                 125
   Gly Leu Phe Val Arg Asp Ile Ile Gly Pro Gln Arg Gln Glu Pro Gln
       130                 135                 140
   Ser Ala Gly Thr Glu Glu Tyr Pro Gln Ala Ser Asn Ile Leu Met Asn
   145                 150                 155                 160
   Ala Phe Ile Thr Gln Asn Lys Lys Asn Asp Asn Leu Val Gln Ile Thr
                   165                 170                 175
   Ser Ile Val Arg Glu Gly Val Ile Lys Thr Trp Gly Asn Glu Gly Ile
               180                 185                 190
   Thr Ile Lys Lys Gln Pro Ile Ile Glu Asn Ile Asn Phe Thr Gln Lys
           195                 200                 205
   Arg Asn Ile His Met Thr Ile Glu Arg Leu Pro Glu Lys Phe Ile Leu
       210                 215                 220
   Thr Ala Phe Asp Thr Asp Arg Lys Glu Asn Gln Ser Trp Gln Phe Ser
   225                 230                 235                 240
   Asp Tyr Ser Gly Phe Met Asn Gln Leu Asp Asn Asn Ser Leu Ala Ile
                   245                 250                 255
   Gly Phe Phe Ala Ala Arg Asn Ala Lys Leu Arg Val Lys Asn Ala Ser
               260                 265                 270
   Phe Lys Pro Gly Lys Pro Leu Val Asp Tyr Lys Gln Leu Thr Ser Arg
   275 280 285
   Gln Phe Ser Arg Val Arg His Lys Ala Pro Glu Leu Phe Leu Ala Ser
   290 295 300
   Pro Gln Ser Val Val Arg Asn Ser Thr Thr Leu Gln Phe Leu Ala Asn
   305 310 315 320
   Gln Ala Gly Ile Val Ser Ile Asp Asn Asp Lys Gln Thr Lys Gln Val
   325 330 335
   Gln Ala Gly Glu Leu Val Gln Phe Pro Val Thr Leu Gln Lys Lys His
   340 345 350
   Asn Asp Phe Thr Val Asn Phe Asn Val Asp Gly Asn Ile Ser Lys Lys
   355 360 365
   Ala Ile Arg Ile Glu Gln Val Lys Ser Asn Leu Thr Asp Pro Tyr Glu
   370 375 380
   Ile Tyr Val Cys Ser Asp Cys Arg Gln Gly Ala Arg Gly Ser Lys Asn
   385 390 395 400
   Asp Pro Val Asp Leu Gln Thr Ala Val Lys Phe Val Ala Pro Gly Gly
   405 410 415
   Asn Ile Tyr Leu Asn Asp Gly Gln Tyr His Gly Ile Thr Leu Asp Arg
   420 425 430
   Glu Leu Ser Gly Ile Pro Gly Lys Tyr Lys Thr Ile Ser Ala Ile Asn
   435 440 445
   Pro His Lys Ala Ile Phe Ile Asn Lys Thr Phe Asn Leu Asp Ala Ser
   450 455 460
   Tyr Trp His Leu Lys Ser Val Val Phe Asp Gly Asn Val Asp Asn Gly
   465 470 475 480
   Asn Asn Lys Pro Ala Tyr Leu Arg Ile Ala Gly Ser Tyr Asn Ile Ile
   485 490 495
   Glu His Val Ile Ala Arg Asn Asn Asp Asp Thr Gly Ile Ser Ile Ser
   500 505 510
   Ala Lys Asp Lys Asn Arg Phe Phe Trp Pro Ala His Asn Leu Val Leu
           515                 520                 525
   Asn Ser Asp Ser Tyr Asn Asn Leu Asp Leu Ser Gly Ile Asn Ala Asp
       530                 535                 540
   Gly Phe Ala Ala Lys Leu Gly Val Gly Pro Gly Asn Ile Phe Arg Gly
   545                 550                 555                 560
   Cys Ile Ala His Asn Asn Ala Asp Asp Gly Trp Asp Leu Phe Asn Lys
                   565                 570                 575
   Ile Glu Asp Gly Pro Asn Ala Ser Val Thr Ile Glu Asn Ser Val Ala
               580                 585                 590
   Tyr Glu Asn Gly Leu Pro Tyr Asn Lys Ala Asp Ile Leu Lys Gly Ser
           595                 600                 605
   Ile Gly Asn Gly Gly Glu Gly Gln Pro Ser Lys Ser Gln Val Ile Asn
       610                 615                 620
   Ser Ile Ala Ile Asn Asn Asn Met Asp Gly Phe Thr Asp Asn Phe Asn
   625                 630                 635                 640
   Thr Gly Ser Leu Ile Val Arg Asn Asn Ile Ala Met Asn Asn Ala Arg
                   645                 650                 655
   Tyr Asn Tyr Ile Leu Arg Thr Asn Pro Tyr Lys Phe Pro Ser Ser Ile
               660                 665                 670
   Leu Phe Asp Asn Asn Tyr Ser Ile Arg Asp Asp Trp Glu Asn Lys Ile
           675                 680                 685
   Lys Asp Phe Leu Gly Asp Thr Val Asn Ser Val Asn Tyr Lys Leu Leu
       690                 695                 700
   Val Ser His Glu Thr Gly Pro Val Gln Lys Asp Leu Phe Phe Thr Arg
   705                 710                 715                 720
   Asp Asp Ser Gly Asn Ile Ile Tyr Pro Asp Phe Phe Leu Asn Ile Ile
                   725                 730                 735
   Asn Lys Phe Asn Glx Thr Met Pro
   740
<210> 47
   <211> 136
   <212> PRT
   <213> Escherichia coli
<400> 47
   Met Lys Thr Phe Ile Lys Thr Leu Leu Val Ala Val Thr Ile Leu Phe
   1 5 10 15
   Ser Val Phe Ala Thr Ala Lys Gln Val Lys Leu Pro Asn Asn Ile Lys
   20 25 30
   Tyr Val Asn Thr Thr Glu Ala Phe Ser Cys Thr Glu Ile Asp Gly Met
   35 40 45
   Asn Cys Gln Thr Lys Asn Pro Phe Asn Tyr Lys Asp Asn Ser Tyr Val
   50 55 60
   Phe Val Leu Glu Arg Gly Gly Ala Trp Cys Tyr Asp Tyr Thr Val Ser
   65 70 75 80
   Val Leu Asn Leu Lys Thr Gly Lys Ala Gln Met Leu Glu Tyr Lys Asp
   85 90 95
   Asn Gln Leu Cys Ser Gly Ser Asn Lys Pro Phe Phe Glu Ile Lys Asn
   100 105 110
   Gly Val Pro Thr Val Gly Val Ile Asp Thr Ser Gly Lys Pro Val Val
   115 120 125
   Val Ala Leu Asp Lys Leu Lys Thr
   130 135
<210> 48
   <211> 225
   <212> PRT
   <213> Escherichia coli
<400> 48
   Met Gln Leu Pro Val Lys Leu Leu Met Ser Leu Ile Ser Leu Val Ser
   1 5 10 15
   Val Ile Ala Arg Ala Gly Lys Tyr Lys Asn Tyr Ile Arg Asp Glu Ile
   20 25 30
   Lys Tyr Trp Arg Tyr Thr Ser Tyr Lys Gly Gly Glu Phe Pro Glu Gly
   35 40 45
   Phe Thr Asp Glu Lys Phe Ser Ser Ala Ile Tyr Asn Gly Arg Ile Phe
   50 55 60
   Thr Met Lys Arg Leu His Thr Leu Met Leu Phe Leu Ala Val Leu Phe
   65 70 75 80
   Thr Gly Phe Asn Val Glu Ala Ala Ser Val Lys Gln Ala Leu Ser Cys
   85 90 95
   Asp Pro Asn Ala Arg Ala Glu Gln Pro Gly Ala Cys Pro Thr Thr Tyr
   100 105 110
   Glu Leu Tyr Glu Gly Asp Ala Ala Tyr Lys Ala Ala Leu Asp Lys Ala
   115 120 125
   Leu Lys Pro Val Gly Leu Ser Gly Met Phe Gly Lys Gly Gly Tyr Met
   130 135 140
   Asp Gly Pro Gly Gly Asn Val Thr Pro Val Thr Ile Asn Gly Thr Val
   145 150 155 160
   Trp Leu Gln Gly Asp Gly Cys Lys Ala Asn Thr Cys Gly Trp Asp Phe
   165 170 175
   Ile Val Thr Leu Tyr Asn Pro Lys Thr His Glu Val Val Gly Tyr Arg
   180 185 190
   Tyr Phe Gly Leu Asp Asp Pro Ala Tyr Leu Val Trp Phe Gly Glu Ile
   195 200 205
   Gly Val His Glu Phe Ala Tyr Leu Val Lys Asn Tyr Val Ala Ala Val
   210 215 220
   Asn
   225
<210> 49
   <211> 721
   <212> PRT
   <213> Escherichia coli
<400> 49
   Met Lys Thr Gln Ile Thr Phe Ala Ala Leu Leu Pro Ala Leu Ala Ser
   1               5                   10                  15
   Phe Ile Pro Leu His Ala His Ala Ser Ser Thr Ser Glu Asp Glu Met
               20                  25                  30
   Ile Val Thr Gly Asn Thr Ala Ala Asp Thr Thr Asp Ser Ala Ala Gly
           35                  40                  45
   Ala Gly Phe Lys Thr Asn Asp Ile Asp Val Gly Pro Leu Gly Thr Lys
       50                  55                  60
   Ser Trp Ile Glu Thr Pro Tyr Ser Ser Thr Thr Val Thr Lys Glu Met
   65                  70                  75                  80
   Ile Glu Asn Gln Gln Ala Gln Ser Val Ser Glu Met Leu Lys Tyr Ser
                   85                  90                  95
   Pro Ser Thr Gln Met Gln Ala Arg Gly Gly Met Asp Val Gly Arg Pro
               100                 105                 110
   Gln Ser Arg Gly Met Gln Gly Ser Val Val Ala Asn Ser Arg Leu Asp
           115                 120                 125
   Gly Leu Asn Ile Val Ser Thr Thr Ala Phe Pro Val Glu Met Leu Glu
       130                 135                 140
   Arg Met Asp Val Leu Asn Ser Leu Thr Gly Ala Leu Tyr Gly Pro Ala
   145                 150                 155                 160
   Ser Pro Ala Gly Gln Phe Asn Phe Val Ala Lys Arg Pro Thr Glu Glu
                   165                 170                 175
   Thr Leu Arg Lys Val Thr Leu Gly Tyr Gln Ser Arg Ser Ala Phe Thr
               180                 185                 190
   Gly His Ala Asp Leu Gly Gly His Phe Asp Glu Asn Lys Arg Phe Gly
           195                 200                 205
   Tyr Arg Val Asn Leu Leu Asp Gln Glu Gly Glu Gly Asn Val Asp Asp
   210 215 220
   Ser Thr Leu Arg Arg Lys Leu Val Ser Val Ala Leu Asp Trp Asn Ile
   225 230 235 240
   Gln Pro Gly Thr Gln Leu Gln Leu Asp Ala Ser His Tyr Glu Phe Ile
   245 250 255
   Gln Lys Gly Tyr Val Gly Ser Phe Asn Tyr Gly Pro Asn Val Lys Leu
   260 265 270
   Pro Ser Ala Pro Asn Pro Lys Asp Lys Asn Leu Ala Leu Ser Thr Ala
   275 280 285
   Gly Asn Asp Leu Thr Thr Asp Thr Ile Ser Thr Arg Leu Ile His Tyr
   290 295 300
   Phe Asn Asp Asp Trp Ser Met Asn Ala Gly Val Gly Trp Gln Gln Ala
   305 310 315 320
   Asp Arg Ala Met Arg Ser Val Ser Ser Lys Ile Leu Asn Asn Gln Gly
   325 330 335
   Asp Ile Ser Arg Ser Met Lys Asp Ser Thr Ala Ala Gly Arg Phe Arg
   340 345 350
   Val Leu Ser Asn Thr Ala Gly Leu Asn Gly His Ile Asp Thr Gly Ser
   355 360 365
   Ile Gly His Asp Leu Ser Leu Ser Thr Thr Gly Tyr Val Trp Ser Leu
   370 375 380
   Tyr Ser Ala Lys Gly Thr Gly Ser Ser Tyr Ser Trp Gly Thr Thr Asn
   385 390 395 400
   Met Tyr His Pro Asp Ala Ile Asp Glu Gln Gly Asp Gly Lys Ile Arg
   405 410 415
   Thr Gly Gly Pro Arg Tyr Arg Ser Ser Val Asn Thr Gln Gln Ser Val
   420 425 430
   Thr Leu Gly Asp Thr Val Thr Phe Thr Pro Gln Trp Ser Ala Met Phe
           435                 440                 445
   Tyr Leu Ser Gln Ser Trp Leu Gln Thr Lys Asn Tyr Asp Lys His Gly
       450                 455                 460
   Asn Gln Thr Asn Gln Val Asp Glu Asn Gly Leu Ser Pro Asn Ala Ala
   465                 470                 475                 480
   Leu Met Tyr Lys Ile Thr Pro Asn Thr Met Ala Tyr Val Ser Tyr Ala
                   485                 490                 495
   Asp Ser Leu Glu Gln Gly Gly Thr Ala Pro Thr Asp Glu Ser Val Lys
               500                 505                 510
   Asn Ala Gly Gln Thr Leu Asn Pro Tyr Arg Ser Lys Gln Tyr Glu Val
           515                 520                 525
   Gly Leu Lys Ser Asp Ile Gly Glu Met Asn Leu Gly Ala Ala Leu Phe
       530                 535                 540
   Arg Leu Glu Arg Pro Phe Ala Tyr Leu Asp Thr Asp Asn Val Tyr Lys
   545                 550                 555                 560
   Glu Gln Gly Asn Gln Val Asn Asn Gly Leu Glu Leu Thr Ala Ala Gly
                   565                 570                 575
   Asn Val Trp Gln Gly Leu Asn Ile Tyr Ser Gly Val Thr Phe Leu Asp
               580                 585                 590
   Pro Lys Leu Lys Asp Thr Ala Asn Ala Ser Thr Ser Asn Lys Gln Val
           595                 600                 605
   Val Gly Val Pro Lys Val Gln Ala Asn Leu Leu Ala Glu Tyr Ser Leu
       610                 615                 620
   Pro Ser Ile Pro Glu Trp Val Tyr Ser Ala Asn Val His Tyr Thr Gly
   625                 630                 635                 640
   Lys Arg Ala Ala Asn Asp Thr Asn Thr Ser Tyr Ala Ser Ser Tyr Thr
                   645                 650                 655
   Thr Trp Asp Leu Gly Thr Arg Tyr Thr Thr Lys Val Ser Asn Val Pro
               660                 665                 670
   Thr Thr Phe Arg Val Val Val Asn Asn Val Phe Asp Lys His Tyr Trp
   675 680 685
   Ala Ser Ile Phe Pro Ser Gly Thr Asp Gly Asp Asn Gly Ser Pro Ser
   690 695 700
   Ala Phe Ile Gly Gly Gly Arg Glu Val Arg Ala Ser Val Thr Phe Asp
   705 710 715 720
   Phe
<210> 50
   <211> 669
   <212> PRT
   <213> Escherichia coli
<400> 50
   Met Lys Asn Ile Thr Leu Trp Gln Arg Leu Arg Gln Val Ser Ile Ser
   1 5 10 15
   Thr Ser Leu Arg Cys Ala Phe Leu Met Gly Ala Leu Leu Thr Leu Ile
   20 25 30
   Val Ser Ser Val Ser Leu Tyr Ser Trp His Glu Gln Ser Ser Gln Ile
   35 40 45
   Arg Tyr Ser Leu Asp Lys Tyr Phe Pro Arg Ile His Ser Ala Phe Leu
   50 55 60
   Ile Glu Gly Asn Leu Asn Leu Val Val Asp Gln Leu Asn Glu Phe Leu
   65 70 75 80
   Gln Ala Pro Asn Thr Thr Val Arg Leu Gln Leu Arg Thr Gln Ile Ile
   85 90 95
   Gln His Leu Asp Thr Ile Glu Arg Leu Ser Arg Gly Leu Ser Ser Arg
   100 105 110
   Glu Arg Gln Gln Leu Thr Val Ile Leu Gln Asp Ser Arg Ser Leu Leu
   115 120 125
   Ser Glu Leu Asp Arg Ala Leu Tyr Asn Met Phe Leu Leu Arg Glu Lys
   130 135 140
   Val Ser Glu Leu Ser Ala Arg Ile Asp Trp Leu His Asp Asp Phe Thr
   145 150 155 160
   Thr Glu Leu Asn Ser Leu Val Gln Asp Phe Thr Trp Gln Gln Gly Thr
   165 170 175
   Leu Leu Asp Gln Ile Ala Ser Arg Gln Gly Asp Thr Ala Gln Tyr Leu
   180 185 190
   Lys Arg Ser Arg Glu Val Gln Asn Glu Gln Gln Gln Val Tyr Thr Leu
   195 200 205
   Ala Arg Ile Glu Asn Gln Ile Val Asp Asp Leu Arg Asp Arg Leu Asn
   210 215 220
   Glu Leu Lys Ser Gly Arg Asp Asp Asp Ile Gln Val Glu Thr His Leu
   225 230 235 240
   Arg Tyr Phe Glu Asn Leu Lys Lys Thr Ala Asp Glu Asn Ile Arg Met
   245 250 255
   Leu Asp Asp Trp Pro Gly Thr Ile Thr Leu Arg Gln Thr Ile Asp Glu
   260 265 270
   Leu Leu Asp Met Gly Ile Val Lys Asn Lys Met Pro Asp Thr Met Arg
   275 280 285
   Glu Tyr Val Ala Ala Gln Lys Ala Leu Glu Asp Ala Ser Arg Thr Arg
   290 295 300
   Glu Ala Thr Gln Gly Arg Phe Arg Thr Leu Leu Glu Ala Gln Leu Gly
   305 310 315 320
   Ser Thr His Gln Gln Met Gln Met Phe Asn Gln Arg Met Glu Gln Ile
   325 330 335
   Val His Val Ser Gly Gly Leu Ile Leu Val Ala Thr Ala Leu Ala Leu
   340 345 350
   Leu Leu Ala Trp Val Phe Asn His Tyr Phe Ile Arg Ser Arg Leu Val
   355 360 365
   Lys Arg Phe Thr Leu Leu Asn Gln Ala Val Val Gln Ile Gly Leu Gly
   370 375 380
   Gly Thr Glu Thr Thr Ile Pro Val Tyr Gly Asn Asp Glu Leu Gly Arg
   385 390 395 400
   Ile Ala Gly Leu Leu Arg His Thr Leu Gly Gln Leu Asn Val Gln Lys
   405 410 415
   Gln Gln Leu Glu Gln Glu Ile Thr Asp Arg Lys Val Ile Glu Ala Asp
   420 425 430
   Leu Arg Ala Thr Gln Asp Glu Leu Ile Gln Thr Ala Lys Leu Ala Val
   435 440 445
   Val Gly Gln Thr Met Thr Thr Leu Ala His Glu Ile Asn Gln Pro Leu
   450 455 460
   Asn Ala Leu Ser Met Tyr Leu Phe Thr Ala Arg Arg Ala Ile Glu Gln
   465 470 475 480
   Thr Gln Lys Glu Gln Ala Ser Met Met Leu Gly Lys Ala Glu Gly Val
   485 490 495
   Ile Ser Arg Ile Asp Ala Ile Ile Arg Ser Leu Arg Gln Phe Thr Arg
   500 505 510
   Arg Ala Glu Leu Glu Thr Ser Leu His Ala Val Asp Leu Ala Gln Met
   515 520 525
   Phe Ser Ala Ala Trp Glu Leu Leu Ala Met Arg His Arg Ser Leu Gln
   530 535 540
   Ala Thr Leu Val Leu Pro Gln Gly Thr Ala Thr Val Ser Gly Asp Glu
   545 550 555 560
   Val Arg Thr Gln Gln Val Leu Val Asn Val Leu Ala Asn Ala Leu Asp
   565 570 575
   Val Cys Gly Gln Gly Ala Val Ile Thr Val Asn Trp Gln Met Gln Gly
   580 585 590
   Lys Thr Leu Asn Val Phe Ile Gly Asp Asn Gly Pro Gly Trp Pro Glu
   595 600 605
   Ala Leu Leu Pro Ser Leu Leu Lys Pro Phe Thr Thr Ser Lys Glu Val
   610 615 620
   Gly Leu Gly Ile Gly Leu Ser Ile Cys Val Ser Leu Met Glu Gln Met
   625 630 635 640
   Lys Gly Glu Leu Arg Leu Ala Ser Thr Met Thr Arg Asn Ala Cys Val
   645 650 655
   Val Leu Gln Phe Arg Leu Thr Asp Val Glu Asp Ala Lys
   660 665
<210> 51
   <211> 753
   <212> PRT
   <213> Escherichia coli
<400> 51
   Met Asn Val Ile Lys Leu Ala Ile Gly Ser Gly Ile Leu Leu Leu Ser
   1 5 10 15
   Cys Gly Ala Tyr Ser Gln Ser Ile Ser Glu Lys Thr Asn Ser Asp Lys
   20 25 30
   Lys Gly Ala Ala Glu Phe Ser Pro Leu Ser Val Ser Val Gly Lys Thr
   35 40 45
   Thr Ser Glu Gln Glu Ala Leu Glu Lys Thr Gly Ala Thr Ser Ser Arg
   50 55 60
   Thr Thr Asp Lys Asn Leu Gln Ser Leu Asp Ala Thr Val Arg Ser Met
   65 70 75 80
   Pro Gly Thr Tyr Thr Gln Ile Asp Pro Gly Gln Gly Ala Ile Ser Val
   85 90 95
   Asn Ile Arg Gly Met Ser Gly Phe Gly Arg Val Asn Thr Met Val Asp
   100 105 110
   Gly Ile Thr Gln Ser Phe Tyr Gly Thr Ser Thr Ser Gly Thr Thr Thr
   115 120 125
   His Gly Ser Thr Asn Asn Met Ala Gly Val Leu Ile Asp Pro Asn Leu
       130                 135                 140
   Leu Val Ala Val Asp Val Thr Arg Gly Asp Ser Ser Gly Ser Glu Gly
   145                 150                 155                 160
   Ile Asn Ala Leu Ala Gly Ser Ala Asn Met Arg Thr Ile Gly Val Asp
                   165                 170                 175
   Asp Val Ile Phe Asn Gly Asn Thr Tyr Gly Leu Arg Ser Arg Phe Ser
               180                 185                 190
   Val Gly Ser Asn Gly Leu Gly Arg Ser Gly Met Ile Ala Leu Gly Gly
           195                 200                 205
   Lys Ser Asp Ala Phe Thr Asp Thr Gly Ser Ile Gly Val Met Ala Ala
       210                 215                 220
   Val Ser Gly Ser Ser Val Tyr Ser Asn Phe Ser Asn Gly Ser Gly Ile
   225                 230                 235                 240
   Asn Ser Lys Glu Phe Gly Tyr Asp Lys Tyr Met Lys Gln Asn Pro Lys
                   245                 250                 255
   Ser Gln Leu Tyr Lys Met Asp Ile Arg Pro Asp Glu Phe Asn Ser Phe
               260                 265                 270
   Glu Leu Ser Ala Arg Thr Tyr Glu Asn Lys Phe Thr Arg Arg Asp Ile
           275                 280                 285
   Thr Ser Asp Asp Tyr Tyr Ile Lys Tyr His Tyr Thr Pro Phe Ser Glu
       290                 295                 300
   Leu Ile Asp Phe Asn Val Thr Ala Ser Thr Ser Arg Gly Asn Gln Lys
   305                 310                 315                 320
   Tyr Arg Asp Gly Ser Leu Tyr Thr Phe Tyr Lys Thr Ser Ala Gln Asn
                   325                 330                 335
   Arg Ser Asp Ala Leu Asp Ile Asn Asn Thr Ser Arg Phe Thr Val Ala
               340                 345                 350
   Asp Asn Asp Leu Glu Phe Met Leu Gly Ser Lys Leu Met Arg Thr Arg
   355 360 365
   Tyr Asp Arg Thr Ile His Ser Ala Ala Gly Asp Pro Lys Ala Asn Gln
   370 375 380
   Glu Ser Ile Glu Asn Asn Pro Phe Ala Pro Ser Gly Gln Gln Asp Ile
   385 390 395 400
   Ser Ala Leu Tyr Thr Gly Leu Lys Val Thr Arg Gly Ile Trp Glu Ala
   405 410 415
   Asp Phe Asn Leu Asn Tyr Thr Arg Asn Arg Ile Thr Gly Tyr Lys Pro
   420 425 430
   Ala Cys Asp Ser Arg Val Ile Cys Val Pro Gln Gly Ser Tyr Asp Ile
   435 440 445
   Asp Asp Lys Glu Gly Gly Phe Asn Pro Ser Val Gln Leu Ser Ala Gln
   450 455 460
   Val Thr Pro Trp Leu Gln Pro Phe Ile Gly Tyr Ser Lys Ser Met Arg
   465 470 475 480
   Ala Pro Asn Ile Gln Glu Met Phe Phe Ser Asn Ser Gly Gly Ala Ser
   485 490 495
   Met Asn Pro Phe Leu Lys Pro Glu Arg Ala Glu Thr Trp Gln Ala Gly
   500 505 510
   Phe Asn Ile Asp Thr Arg Asp Leu Leu Val Glu Gln Asp Ala Leu Arg
   515 520 525
   Phe Lys Ala Leu Ala Tyr Arg Ser Arg Ile Gln Asn Tyr Ile Tyr Ser
   530 535 540
   Glu Ser Tyr Leu Val Cys Ser Gly Gly Arg Lys Cys Ser Leu Pro Glu
   545 550 555 560
   Val Ile Gly Asn Gly Trp Glu Gly Ile Ser Asp Glu Tyr Ser Asp Asn
   565 570 575
   Met Tyr Ile Tyr Val Asn Ser Ala Ser Asp Val Ile Ala Lys Gly Phe
   580 585 590
   Glu Leu Glu Met Asp Tyr Asp Ala Gly Phe Ala Phe Gly Arg Leu Ser
   595 600 605
   Phe Ser Gln Gln Gln Thr Asp Gln Pro Thr Ser Ile Ala Ser Thr His
   610 615 620
   Phe Gly Ala Gly Asp Ile Thr Glu Leu Pro Arg Lys Tyr Met Thr Leu
   625 630 635 640
   Asp Thr Gly Val Arg Phe Phe Asp Asn Ala Leu Thr Leu Gly Thr Ile
   645 650 655
   Ile Lys Tyr Thr Gly Lys Ala Arg Arg Leu Ser Pro Asp Phe Glu Gln
   660 665 670
   Asp Glu His Thr Gly Ala Ile Ile Lys Gln Asp Leu Pro Gln Ile Pro
   675 680 685
   Thr Ile Ile Asp Leu Tyr Gly Thr Tyr Glu Tyr Asn Arg Asn Leu Thr
   690 695 700
   Leu Lys Leu Ser Val Gln Asn Leu Met Asn Arg Asp Tyr Ser Glu Ala
   705 710 715 720
   Leu Asn Lys Leu Asn Met Met Pro Gly Leu Gly Asp Glu Thr His Pro
   725 730 735
   Ala Asn Ser Ala Arg Gly Arg Thr Trp Ile Phe Gly Gly Asp Ile Arg
   740 745 750
   Phe
<210> 52
   <211> 133
   <212> PRT
   <213> Escherichia coli
<400> 52
   Met Ser Ser Lys Thr Lys Cys Trp Leu Trp Met Leu Leu Val Ile Leu
   1 5 10 15
   Ser Glu Thr Ser Ala Thr Ser Thr Leu Lys Met Phe Asp Asn Ser Glu
   20 25 30
   Gly Met Thr Lys Thr Leu Leu Leu Ala Leu Ile Val Val Leu Tyr Cys
   35 40 45
   Ile Cys Tyr Tyr Ser Leu Ser Arg Ala Val Lys Asp Ile Pro Val Gly
   50 55 60
   Leu Ala Tyr Ala Thr Trp Ser Gly Thr Gly Ile Leu Met Val Ser Thr
   65 70 75 80
   Leu Gly Ile Leu Phe Tyr Gly Gln His Pro Asp Thr Ala Ala Ile Ile
   85 90 95
   Gly Met Val Ile Ile Ala Ser Gly Ile Ile Ile Met Asn Leu Phe Ser
   100 105 110
   Lys Met Gly Ser Glu Glu Ala Glu Glu Thr Pro Val Thr Asn Leu Asp
   115 120 125
   Lys Lys Ile Ala Asn
   130
<210> 53
   <211> 286
   <212> PRT
   <213> Escherichia coli
<400> 53
   Met Tyr Ile Lys Lys His Trp Ile Ala Leu Ser Ile Leu Leu Ile Pro
   1 5 10 15
   Cys Ile Gly Asn Ala Gln Glu Ile Lys Ile Asp Glu Ser Trp Leu His
   20 25 30
   Gln Ser Leu Asn Val Ile Gly Arg Thr Asp Ser Arg Phe Gly Pro Arg
   35 40 45
   Leu Thr Asn Asp Leu Tyr Pro Glu Tyr Thr Val Ala Gly Arg Lys Asp
   50 55 60
   Trp Phe Asp Phe Tyr Gly Tyr Val Asp Leu Pro Lys Phe Phe Gly Val
   65 70 75 80
   Gly Ser His Tyr Asp Val Gly Ile Trp Asp Glu Gly Ser Pro Leu Phe
   85 90 95
   Thr Glu Ile Glu Pro Arg Phe Ser Ile Asp Lys Leu Thr Gly Leu Asn
   100 105 110
   Leu Ala Phe Gly Pro Phe Lys Glu Trp Phe Ile Ala Asn Asn Tyr Val
   115 120 125
   Tyr Asp Met Gly Asp Asn Gln Ser Ser Arg Gln Ser Thr Trp Tyr Met
   130 135 140
   Gly Leu Gly Thr Asp Ile Asp Thr Gly Leu Pro Ile Lys Leu Ser Ala
   145 150 155 160
   Asn Ile Tyr Ala Lys Tyr Gln Trp Gln Asn Tyr Gly Ala Ala Asn Glu
   165 170 175
   Asn Glu Trp Asp Gly Tyr Arg Phe Lys Ile Lys Tyr Ser Ile Pro Leu
   180 185 190
   Thr Asn Leu Phe Gly Gly Arg Leu Val Tyr Asn Ser Phe Thr Asn Phe
   195 200 205
   Asp Phe Gly Ser Asp Leu Ala Asp Lys Ser His Asn Asn Lys Arg Thr
   210 215 220
   Ser Asn Ala Ile Ala Ser Ser His Ile Leu Ser Leu Leu Tyr Glu His
   225 230 235 240
   Trp Lys Phe Ala Phe Thr Leu Arg Tyr Phe His Asn Gly Gly Gln Trp
   245 250 255
   Asn Ala Gly Glu Lys Val Asn Phe Gly Asp Gly Pro Phe Glu Leu Lys
   260 265 270
   Asn Thr Gly Trp Gly Thr Tyr Thr Thr Ile Gly Tyr Gln Phe
   275 280 285
<210> 54
   <211> 172
   <212> PRT
   <213> Escherichia coli
<400> 54
   Met Arg Ile Ala Pro Arg Thr Phe Phe Ala Ile Ser Ala Leu Ala Phe
   1 5 10 15
   Ile Val Ala Ser Gly Phe Ser Phe Trp Arg Leu Ser Pro Ala Glu Asn
   20 25 30
   Thr Gly Ile Met Ser Cys Ser Thr Lys Gly Ile Met Arg Phe Glu Asn
   35 40 45
   Met Glu Lys Glu Asn Val Asn Gly Asn Ile His Phe Asn Phe Gly Ser
   50 55 60
   Gln Gly Lys Gly Ser Met Val Leu Glu Gly Tyr Thr Asp Ser Ala Ala
   65 70 75 80
   Gly Trp Leu Tyr Leu Gln Arg Tyr Val Lys Phe Thr Tyr Thr Ser Lys
   85 90 95
   Arg Val Ser Ala Thr Glu Arg His Tyr Arg Ile Ser Gln Trp Glu Ser
   100 105 110
   Ser Ala Ser Ser Ile Asp Glu Ser Pro Asp Val Ile Phe Asp Tyr Phe
   115 120 125
   Met Arg Glu Met Ser Asp Ser His Asp Gly Leu Phe Leu Asn Ala Gln
   130 135 140
   Lys Leu Asn Asp Lys Ala Ile Leu Leu Ser Ser Ile Asn Ser Pro Leu
   145 150 155 160
   Trp Ile Cys Thr Leu Lys Ser Gly Ser Lys Leu Asp
   165 170
<210> 55
   <211> 182
   <212> PRT
   <213> Escherichia coli
<400> 55
   Met Lys Ile Lys Val Ile Ala Leu Ala Thr Phe Val Ser Ala Val Phe
   1 5 10 15
   Ala Gly Ser Ala Met Ala Tyr Asp Gly Thr Ile Thr Phe Thr Gly Lys
   20 25 30
   Val Val Ala Gln Thr Cys Thr Val Asn Thr Ser Asp Lys Asp Leu Ala
   35 40 45
   Val Thr Leu Pro Thr Val Ala Thr Ser Ser Leu Lys Asp Asn Ala Ala
   50 55 60
   Thr Ser Gly Leu Thr Pro Phe Ala Ile Arg Leu Thr Gly Cys Ala Thr
   65 70 75 80
   Gly Met Asn Ser Ala Gln Asn Val Lys Ala Tyr Phe Glu Pro Ser Ser
   85 90 95
   Asn Ile Asp Leu Ala Thr His Asn Leu Lys Asn Thr Ala Thr Pro Thr
   100 105 110
   Lys Ala Asp Asn Val Gln Ile Gln Leu Leu Asn Ser Asn Gly Thr Ser
   115 120 125
   Thr Ile Leu Leu Gly Glu Ala Asp Asn Gly Gln Asp Val Gln Ser Glu
   130 135 140
   Thr Ile Gly Ser Asp Gly Ser Ala Thr Leu Arg Tyr Met Ala Gln Tyr
   145 150 155 160
   Tyr Ala Thr Gly Gln Ser Thr Ala Gly Asp Val Lys Ala Thr Val His
   165 170 175
   Tyr Thr Ile Ala Tyr Glu
   180
<210> 56
   <211> 359
   <212> PRT
   <213> Escherichia coli
<400> 56
   Met Lys Arg Ile Phe Phe Ile Pro Leu Phe Leu Ile Leu Leu Pro Lys
   1 5 10 15
   Leu Ala Val Ala Gly Pro Asp Asp Tyr Val Pro Ser Gln Ile Ala Val
   20 25 30
   Asn Thr Ser Thr Leu Pro Gly Val Val Ile Gly Pro Ala Asp Ala His
           35                  40                  45
   Thr Tyr Pro Arg Val Ile Gly Glu Leu Ala Gly Thr Ser Asn Gln Tyr
       50                  55                  60
   Val Phe Asn Gly Gly Ala Ile Ala Leu Met Arg Gly Lys Phe Thr Pro
   65                  70                  75                  80
   Ala Leu Pro Lys Ile Gly Ser Ile Thr Val Tyr Phe Pro Ser Arg Lys
                   85                  90                  95
   Gln Arg Asp Ser Ser Asp Phe Asp Ile Tyr Asp Ile Gly Val Ser Gly
               100                 105                 110
   Leu Gly Ile Ile Ile Gly Met Ala Gly Tyr Trp Pro Ala Thr Pro Leu
           115                 120                 125
   Val Pro Ile Asn Ser Ser Gly Ile Tyr Ile Asp Pro Val Gly Ala Asn
       130                 135                 140
   Thr Asn Pro Asn Thr Tyr Asn Gly Ala Thr Ala Ser Phe Gly Ala Arg
   145                 150                 155                 160
   Leu Phe Val Ala Phe Val Ala Thr Gly Arg Leu Pro Asn Gly Tyr Ile
                   165                 170                 175
   Thr Ile Pro Thr Arg Gln Leu Gly Thr Ile Leu Leu Glu Ala Lys Arg
               180                 185                 190
   Thr Ser Leu Asn Asn Lys Gly Leu Thr Ala Pro Val Met Leu Asn Gly
           195                 200                 205
   Gly Arg Ile Gln Val Gln Ser Gln Thr Cys Thr Met Gly Gln Lys Asn
       210                 215                 220
   Tyr Val Val Pro Leu Asn Thr Val Tyr Gln Ser Gln Phe Thr Ser Leu
   225                 230                 235                 240
   Tyr Lys Glu Ile Gln Gly Gly Lys Ile Asp Ile His Leu Gln Cys Pro
                   245                 250                 255
   Asp Gly Ile Asp Val Tyr Ala Thr Leu Thr Asp Ala Ser Gln Pro Val
   260 265 270
   Asn Arg Thr Asp Ile Leu Thr Leu Ser Ser Glu Ser Thr Ala Lys Gly
   275 280 285
   Phe Gly Ile Arg Leu Tyr Lys Asp Ser Asp Val Thr Ala Ile Ser Tyr
   290 295 300
   Gly Glu Asp Ser Pro Val Lys Gly Asn Gly Ser Gln Trp His Phe Ser
   305 310 315 320
   Asp Tyr Arg Gly Glu Val Asn Pro His Ile Asn Leu Arg Ala Asn Tyr
   325 330 335
   Ile Lys Ile Ala Asp Ala Thr Thr Pro Gly Ser Val Lys Ala Ile Ala
   340 345 350
   Thr Ile Thr Phe Ser Tyr Gln
   355
<210> 57
   <211> 844
   <212> PRT
   <213> Escherichia coli
<400> 57
   Met Asn Ala Asn Asn Leu Ser Cys Leu Ile Tyr Cys Arg Cys Ser Leu
   1 5 10 15
   Leu Leu Phe Ala Ala Leu Gly Leu Thr Val Thr Asn His Ser Phe Ala
   20 25 30
   Ala Glu Glu Ala Glu Phe Asp Ser Glu Phe Leu His Leu Asp Lys Gly
   35 40 45
   Ile Asn Ala Ile Asp Ile Arg Arg Phe Ser His Gly Asn Pro Val Pro
   50 55 60
   Glu Gly Arg Tyr Tyr Ser Asp Ile Tyr Val Asn Asn Val Trp Lys Gly
   65 70 75 80
   Lys Ala Asp Leu Gln Tyr Leu Arg Thr Ala Asn Thr Gly Ala Pro Thr
   85 90 95
   Leu Cys Leu Thr Pro Glu Leu Leu Ser Leu Ile Asp Leu Val Lys Asp
   100 105 110
   Thr Met Ser Gly Asn Thr Ser Cys Phe Pro Ala Ser Thr Gly Leu Ser
   115 120 125
   Ser Ala Arg Ile Asn Phe Asp Leu Ser Thr Leu Arg Leu Asn Ile Glu
   130 135 140
   Ile Pro Gln Ala Leu Leu Asn Thr Arg Pro Arg Gly Tyr Ile Ser Pro
   145 150 155 160
   Ala Gln Trp Gln Ser Gly Val Pro Ala Ala Phe Ile Asn Tyr Asp Ala
   165 170 175
   Asn Tyr Tyr Gln Tyr Ser Ser Ser Gly Thr Ser Asn Glu Gln Thr Tyr
   180 185 190
   Leu Gly Leu Lys Ala Gly Phe Asn Leu Trp Gly Trp Ala Leu Arg His
   195 200 205
   Arg Gly Ser Glu Ser Trp Asn Asn Ser Tyr Pro Ala Gly Tyr Gln Asn
   210 215 220
   Ile Glu Thr Ser Ile Met His Asp Leu Ala Pro Leu Arg Ala Gln Phe
   225 230 235 240
   Thr Leu Gly Asp Phe Tyr Thr Asn Gly Glu Leu Met Asp Ser Leu Ser
   245 250 255
   Leu Arg Gly Val Arg Leu Ala Ser Asp Glu Arg Met Leu Pro Gly Ser
   260 265 270
   Leu Arg Gly Tyr Ala Pro Ala Val Arg Gly Ile Ala Asn Ser Asn Ala
   275 280 285
   Lys Val Thr Ile Tyr Gln Asn Ala His Ile Leu Tyr Glu Thr Thr Val
   290 295 300
   Pro Ala Gly Pro Phe Val Ile Asn Asp Leu Tyr Pro Ser Gly Tyr Ala
   305 310 315 320
   Gly Asp Leu Leu Val Lys Ile Thr Glu Ser Asn Gly Gln Thr Arg Met
   325 330 335
   Phe Thr Val Pro Phe Ala Ala Val Ala Gln Leu Ile Arg Pro Gly Phe
   340 345 350
   Ser Arg Trp Gln Met Ser Val Gly Lys Tyr Arg Tyr Ala Asn Lys Thr
   355 360 365
   Tyr Asn Asp Leu Ile Ala Gln Gly Thr Tyr Gln Tyr Gly Leu Thr Asn
   370 375 380
   Asp Ile Thr Leu Asn Ser Gly Leu Thr Thr Ala Ser Gly Tyr Thr Ala
   385 390 395 400
   Gly Leu Ala Gly Leu Ala Phe Asn Thr Pro Leu Gly Ala Ile Ala Ser
   405 410 415
   Asp Ile Thr Leu Ser Arg Thr Ala Phe Arg Tyr Ser Gly Val Thr Arg
   420 425 430
   Lys Gly Tyr Ser Leu His Ser Ser Tyr Ser Ile Asn Ile Pro Ala Ser
   435 440 445
   Asn Thr Asn Ile Thr Leu Ala Ala Tyr Arg Tyr Ser Ser Lys Asp Phe
   450 455 460
   Tyr His Leu Lys Asp Ala Leu Ser Ala Asn His Asn Ala Phe Ile Asp
   465 470 475 480
   Asp Val Ser Val Lys Ser Thr Ala Phe Tyr Arg Pro Arg Asn Gln Phe
   485 490 495
   Gln Ile Ser Ile Asn Gln Glu Leu Gly Glu Lys Trp Gly Gly Met Tyr
   500 505 510
   Leu Thr Gly Thr Thr Tyr Asn Tyr Trp Gly His Lys Gly Ser Arg Asn
   515 520 525
   Glu Tyr Gln Ile Gly Tyr Ser Asn Phe Trp Lys Gln Leu Gly Tyr Gln
   530 535 540
   Ile Gly Leu Ser Gln Ser Arg Asp Asn Glu Gln Gln Arg Arg Asp Asp
   545 550 555 560
   Arg Phe Tyr Ile Asn Phe Thr Leu Pro Leu Gly Gly Ser Val Gln Ser
   565 570 575
   Pro Val Phe Ser Thr Val Leu Asn Tyr Ser Lys Glu Glu Lys Asn Ser
   580 585 590
   Ile Gln Thr Ser Ile Ser Gly Thr Gly Gly Glu Asp Asn Gln Phe Ser
   595 600 605
   Tyr Gly Ile Ser Gly Asn Ser Gln Glu Asn Gly Pro Ser Gly Tyr Ala
   610 615 620
   Met Asn Gly Gly Tyr Arg Ser Pro Tyr Val Asn Ile Thr Thr Thr Val
   625 630 635 640
   Gly His Asp Thr Gln Asn Asn Asn Gln Arg Ser Phe Gly Ala Ser Gly
   645 650 655
   Ala Val Val Ala His Pro Tyr Gly Val Thr Leu Ser Asn Asp Leu Ser
   660 665 670
   Asp Thr Phe Ala Ile Ile His Ala Glu Gly Ala Gln Gly Ala Val Ile
   675 680 685
   Asn Asn Ala Ser Gly Ser Arg Leu Asp Phe Trp Gly Asn Gly Val Val
   690 695 700
   Pro Tyr Val Thr Pro Tyr Glu Lys Asn Gln Ile Ser Ile Asp Pro Ser
   705 710 715 720
   Asn Leu Asp Leu Asn Val Glu Leu Ser Ala Thr Glu Gln Glu Ile Ile
   725 730 735
   Pro Arg Ala Asn Ser Ala Thr Leu Val Lys Phe Asp Thr Lys Thr Gly
   740 745 750
   Arg Ser Leu Leu Phe Asp Ile Arg Met Ser Thr Gly Asn Pro Pro Pro
   755 760 765
   Met Ala Ser Glu Val Leu Asp Glu His Gly Gln Leu Ala Gly Tyr Val
   770 775 780
   Ala Gln Ala Gly Lys Val Phe Thr Arg Gly Leu Pro Glu Lys Gly His
   785 790 795 800
   Leu Ser Val Val Trp Gly Pro Asp Asn Lys Asp Arg Cys Ser Phe Val
   805 810 815
   Tyr His Val Ala His Asn Lys Asp Asp Met Gln Ser Gln Leu Val Pro
   820 825 830
   Val Leu Cys Ile Gln His Pro Asn Gln Glu Lys Thr
   835 840
<210> 58
   <211> 277
   <212> PRT
   <213> Escherichia coli
<400> 58
   Met Val Lys Cys His Thr Leu Ile Asn Arg Arg Asn Lys Cys Leu Leu
   1 5 10 15
   Ile Val Phe Ile Val Leu Ile Gly Trp Ile Ile Phe Arg Pro Lys Ala
   20 25 30
   Tyr Thr Tyr Ser Leu Asn Asp Lys Glu Lys Glu Met Leu Ile Met Leu
   35 40 45
   Ser Gln His Pro Glu Thr Arg Tyr Phe Gly Phe Tyr Ser Ile Glu Leu
   50 55 60
   Pro Ala Asp Tyr Lys Pro Thr Gly Met Val Met Phe Ile Gln Gly Ser
   65 70 75 80
   Ala Met Ile Pro Val Glu Thr Lys Leu Gln Tyr Tyr Pro Pro Phe Leu
   85 90 95
   Gln Tyr Met Thr Arg Tyr Glu Ala Glu Leu Lys Asn Thr Ser Ala Leu
   100 105 110
   Asp Pro Leu Asp Thr Pro Tyr Leu Lys Gln Val His Pro Leu Ser Pro
   115 120 125
   Pro Met Asn Gly Val Ile Phe Glu Arg Met Lys Ala Lys Tyr Thr Pro
   130 135 140
   Asp Phe Ala Arg Val Leu Asp Ala Trp Lys Trp Glu Asn Gly Val Thr
   145 150 155 160
   Phe Ser Val Lys Ile Glu Ala Lys Asp Gly Arg Ala Thr Arg Tyr Asp
   165 170 175
   Gly Ile Ser Lys Ile Ala Glu Tyr Ser Tyr Gly Tyr Asn Ile Pro Glu
   180 185 190
   Lys Lys Val Gln Leu Leu Thr Ile Leu Ser Gly Leu Gln Pro Arg Ala
   195 200 205
   Asp Asn Gln Pro Pro Ser Glu Asn Lys Leu Ala Ile Gln Tyr Ala Gln
   210 215 220
   Val Asp Ala Ser Leu Leu Gly Glu Tyr Glu Leu Ser Val Asp Tyr Lys
   225 230 235 240
   Asn Ser Asn Asn Ile Lys Ile Ser Leu Gln Thr Asp Asn Asn Ser Tyr
   245 250 255
   Ile Asp Ser Leu Leu Asp Ile Arg Tyr Pro Ser Asn Gly Asn Arg Ala
   260 265 270
   Trp Tyr Asn Ser Ile
   275
<210> 59
   <211> 366
   <212> PRT
   <213> Escherichia coli
<400> 59
   Met Leu Pro Glu Pro Val Tyr Arg Arg Trp Ile Ile Leu Leu Ile Ser
   1 5 10 15
   Met Leu Thr Val Gly Thr Leu Phe Ile Leu Ser Val Trp Asn Ser Ala
   20 25 30
   Thr Tyr Trp Asp Ile Phe Ile Tyr Gly Val Leu Pro Met Leu Phe Leu
   35 40 45
   Trp Leu Cys Leu Phe Gly Ile Ala Leu Asn Lys Tyr Glu Gln Ser Val
   50 55 60
   Ala Ala Cys Ile Ser Trp Glu Ser Glu Arg Gln Gln Val Lys Gln Leu
   65 70 75 80
   Trp Gln His Trp Ser Gln Lys Gln Leu Ala Ile Val Gly Asn Val Leu
   85 90 95
   Phe Thr Pro Glu Glu Lys Gly Met Ser Val Leu Leu Gly Pro Gln Glu
   100 105 110
   Glu Ile Pro Ala Tyr Pro Lys Lys Ala Arg Pro Leu Phe Ser Ala Ser
   115 120 125
   Arg Tyr Ser Leu Ser Ser Ile Phe His Asp Ile His Gln Gln Leu Thr
   130 135 140
   Gln Gln Phe Pro Asp Tyr Arg His Tyr Leu His Thr Ile Tyr Val Leu
   145 150 155 160
   Gln Pro Glu Lys Trp Arg Gly Glu Thr Val Arg Gln Ala Ile Phe His
   165 170 175
   Gln Trp Asp Leu Val Pro Glu Arg Thr Asn Thr Leu Asn Gln Ile Gln
   180 185 190
   Ser Leu Tyr Asp Glu Arg Phe Asp Gly Leu Ile Leu Val Val Cys Leu
   195 200 205
   Gln Asn Trp Pro Glu Asn Lys Pro Glu Asp Thr Ser Glu Leu Val Ser
   210 215 220
   Ala Gln Leu Ile Ser Ser Ser Ser Phe Val Arg Gln His Gln Ile Pro
   225 230 235 240
   Val Ile Ala Gly Leu Gly Arg Val Met Pro Leu Glu Pro Glu Glu Leu
   245 250 255
   Glu His Asn Leu Asp Val Leu Phe Glu Tyr Asn Gln Leu Asp Asn Lys
   260 265 270
   Gln Leu Gln His Val Trp Val Ser Gly Leu Asp Glu Gly Thr Ile Glu
   275 280 285
   Asn Leu Met Gln Tyr Ala Glu Gln His Gln Trp Ser Leu Pro Lys Lys
   290 295 300
   Arg Pro Leu His Met Ile Asp His Ser Phe Gly Pro Thr Gly Glu Phe
   305 310 315 320
   Ile Phe Pro Val Ser Leu Ala Met Leu Ser Glu Ala Ala Lys Glu Thr
   325 330 335
   Glu Gln Asn His Leu Ile Ile Tyr Gln Ser Ala Gln Tyr Ala Gln Lys
   340 345 350
   Lys Ser Leu Cys Leu Ile Thr Arg Lys Leu Tyr Leu Arg Thr
   355 360 365
<210> 60
   <211> 260
   <212> PRT
   <213> Escherichia coli
<400> 60
   Met Leu Asn Arg Lys Leu Asn Ile Arg Leu Arg His Ser Leu Asn Ser
   1 5 10 15
   His Cys Ile Pro Ser Ile Ile Ile Asn Asn Thr Val Arg Ser Phe Gln
   20 25 30
   Arg Ser Val Met Asn Thr Arg Ala Leu Phe Pro Leu Leu Phe Thr Val
   35 40 45
   Ala Ser Phe Ser Ala Ser Ala Gly Asn Trp Ala Val Lys Asn Gly Trp
   50 55 60
   Cys Gln Thr Met Thr Glu Asp Gly Gln Ala Leu Val Met Leu Lys Asn
   65 70 75 80
   Gly Thr Ile Gly Ile Thr Gly Leu Met Gln Gly Cys Pro Asn Gly Val
   85 90 95
   Gln Thr Leu Leu Gly Ser Arg Ile Ser Ile Asn Gly Asn Leu Ile Pro
   100 105 110
   Thr Ser Gln Met Cys Asn Gln Gln Thr Gly Phe Arg Ala Val Glu Val
   115 120 125
   Glu Ile Gly Gln Ala Pro Glu Met Val Lys Lys Ala Val His Ser Ile
   130 135 140
   Ala Glu Arg Asp Val Ser Val Leu Gln Ala Phe Gly Val Arg Met Glu
   145 150 155 160
   Phe Thr Arg Gly Asp Met Leu Lys Val Cys Pro Lys Phe Val Thr Ser
   165 170 175
   Leu Ala Gly Phe Ser Pro Lys Gln Thr Thr Thr Ile Asn Lys Asp Ser
   180 185 190
   Val Leu Gln Ala Ala Arg Gln Ala Tyr Ala Arg Glu Tyr Asp Glu Glu
   195 200 205
   Thr Thr Glu Thr Ala Asp Phe Gly Ser Tyr Glu Val Lys Gly Asn Lys
   210 215 220
   Val Glu Phe Glu Val Phe Asn Pro Glu Asp Arg Ala Tyr Asp Lys Val
   225 230 235 240
   Thr Val Thr Val Gly Ala Asp Gly Asn Ala Thr Gly Ala Ser Val Glu
   245 250 255
   Phe Ile Gly Lys
   260
<210> 61
   <211> 385
   <212> PRT
   <213> Escherichia coli
<400> 61
   Val Val Ile Ile Asn Ser Thr Ile Leu Ser Gly Ala Gly Ala Ile Pro
   1 5 10 15
   Ser Leu Thr Ser Leu Leu Pro Asp Ile Arg Lys Met Leu Leu Val Thr
   20 25 30
   Asp Arg Asn Ile Ala Gln Leu Asp Gly Val Gln Gln Ile Arg Ala Leu
   35 40 45
   Leu Glu Lys His Cys Pro Gln Val Asn Val Ile Asp Asn Val Pro Ala
   50 55 60
   Glu Pro Thr His His Asp Val Arg Gln Leu Met Asp Ala Pro Gly Asp
   65 70 75 80
   Ala Ser Phe Asp Val Val Val Gly Ile Gly Gly Gly Ser Val Leu Asp
   85 90 95
   Val Ala Lys Leu Leu Ser Val Leu Cys His Pro Gln Ser Pro Gly Leu
   100 105 110
   Asp Ala Leu Leu Ala Gly Glu Lys Pro Thr Gln Arg Val Gln Ser Trp
   115 120 125
   Leu Ile Pro Thr Thr Ala Gly Thr Gly Ser Glu Ala Thr Pro Asn Ala
   130 135 140
   Ile Leu Ala Ile Pro Glu Gln Ser Thr Lys Val Gly Ile Ile Ser Gln
   145 150 155 160
   Val Leu Leu Pro Asp Tyr Val Ala Leu Phe Pro Glu Leu Thr Thr Ser
   165 170 175
   Met Pro Ala His Ile Ala Ala Ser Thr Gly Ile Asp Ala Leu Cys His
   180 185 190
   Leu Leu Glu Cys Phe Thr Ala Thr Val Ala Asn Pro Val Ser Asp Asn
   195 200 205
   Ala Ala Leu Thr Gly Leu Ser Lys Leu Phe Arg His Ile Gln Pro Ala
   210 215 220
   Val Asn Asp Pro Gln Asp Leu Arg Ala Lys Leu Glu Met Leu Trp Ala
   225 230 235 240
   Ser Tyr Tyr Gly Gly Val Ala Ile Thr His Ala Gly Thr His Leu Val
   245 250 255
   His Ala Leu Ser Tyr Pro Leu Gly Gly Lys Tyr His Leu Pro His Gly
   260 265 270
   Val Ala Asn Ala Ile Leu Leu Ala Pro Cys Met Ala Phe Val Arg Pro
   275 280 285
   Trp Ala Val Glu Lys Phe Ala Arg Val Trp Asp Cys Ile Pro Asp Ala
   290 295 300
   Glu Thr Ala Leu Ser Ala Glu Glu Lys Ser His Ala Leu Val Thr Trp
   305 310 315 320
   Leu Gln Ala Leu Val Asn Gln Leu Lys Leu Pro Asn Asn Leu Ala Ala
   325 330 335
   Leu Gly Val Pro Pro Glu Asp Ile Ala Ser Leu Ser Glu Ala Ala Leu
   340 345 350
   Asn Val Lys Arg Leu Met Asn Asn Val Pro Cys Gln Ile Asp Leu Gln
   355 360 365
   Asp Val Gln Ala Ile Tyr Gln Thr Leu Phe Pro Gln His Pro Phe Lys
   370 375 380
   Glu
   385
<210> 62
   <211> 105
   <212> PRT
   <213> Escherichia coli
<400> 62
   Met Asn Ile Arg Lys Leu Phe Cys Pro Gly Asn Thr Pro Arg Ile Leu
   1 5 10 15
   Leu Phe Leu Phe Phe Phe Val Val Ser Ala Ile Thr Thr Ile Ala Cys
   20 25 30
   Gly Tyr Thr Glu Lys Asn Ala Thr Gly Asn Val Leu Leu Leu Phe Leu
   35 40 45
   Leu Leu Leu Leu Ala His Arg Asn Thr Leu Thr Ser Ile Thr Ala Leu
   50 55 60
   Leu Phe Leu Phe Cys Cys Ala Leu Tyr Ala Pro Ala Gly Met Thr Tyr
   65 70 75 80
   Gly Lys Ile Asn Asn Ser Phe Ile Val Ala Leu Leu Gln Thr Thr Thr
   85 90 95
   Asp Glu Ala Ala Glu Phe Thr Gly Met
   100 105
<210> 63
   <211> 147
   <212> PRT
   <213> Escherichia coli
<400> 63
   Met Asn Ile Gln Ala Ile Lys Glu Met Val Asn Leu Ile Cys Ser Phe
   1 5 10 15
   Leu Phe Ile Phe Phe Leu Ser Ser Ala Phe Val Ser Phe Gly Cys Tyr
   20 25 30
   Ala Ile Tyr Glu Leu Phe Leu Trp Asn Asp Ile Ile Val Tyr Ser Trp
   35 40 45
   Gly Tyr Ile Leu Ile Val Phe Leu Pro Phe Thr Leu Tyr Val Met Ser
   50 55 60
   Phe Glu Ile Leu Phe Phe Ala Ile Ser Gly Arg Arg Leu Ser Lys Val
   65 70 75 80
   Thr Met Val Arg Leu Trp Leu Ile Ile Lys Ile Ile Ile Ala Phe Ser
   85 90 95
   Ile Cys Ala Val Leu Ile Phe Ser Ser Ile Tyr Lys Lys Glu Leu Leu
   100 105 110
   Ser Arg Asn Tyr Ile Ala Cys Ser Gly Ile Pro Ser Gly Trp Met Pro
   115 120 125
   Gly Leu Ala Thr Lys Tyr Val Lys Glu Lys Ser Leu Cys Glu Lys Asn
   130 135 140
   Gly Asn Asn
   145
<210> 64
   <211> 178
   <212> PRT
   <213> Escherichia coli
<400> 64
   Met Phe Pro Ile Arg Phe Lys Arg Pro Ala Leu Leu Cys Met Ala Met
   1 5 10 15
   Leu Thr Val Val Leu Ser Gly Cys Gly Leu Ile Gln Lys Val Val Asp
   20 25 30
   Glu Ser Lys Ser Val Ala Ser Ala Val Phe Tyr Lys Gln Ile Lys Ile
   35 40 45
   Leu His Leu Asp Phe Phe Ser Arg Ser Ala Leu Asn Thr Asp Ala Glu
   50 55 60
   Asp Thr Pro Leu Ser Thr Met Val His Val Trp Gln Leu Lys Thr Arg
   65 70 75 80
   Glu Asp Phe Asp Lys Ala Asp Tyr Asp Thr Leu Phe Met Gln Glu Glu
   85 90 95
   Lys Thr Leu Glu Lys Asp Val Leu Ala Lys His Thr Val Trp Val Lys
   100 105 110
   Pro Glu Gly Thr Ala Ser Leu Asn Val Pro Leu Asp Lys Glu Thr Gln
   115 120 125
   Phe Val Ala Ile Ile Gly Gln Phe Tyr His Pro Asp Glu Lys Ser Asp
   130 135 140
   Ser Trp Arg Leu Val Ile Lys Arg Asp Glu Leu Glu Ala Asp Lys Pro
   145 150 155 160
   Arg Ser Ile Glu Leu Met Arg Ser Asp Leu Arg Leu Leu Pro Leu Lys
   165 170 175
   Asp Lys
<210> 65
   <211> 209
   <212> PRT
   <213> Escherichia coli
<400> 65
   Met Phe Leu Lys Arg Lys Trp Tyr Tyr Ala Val Thr Thr Ser Val Val
   1               5                   10                  15
   Ile Thr Leu Cys Gly Gly Gly Tyr Tyr Met Tyr Arg Gln Glu Tyr Gln
               20                  25                  30
   Met Val Val Thr Val Pro Thr Ala Asp Ala Asn Asp Pro Asn Trp Pro
           35                  40                  45
   Asn Lys Arg Ile Gln Phe Asp Thr Ser Glu Trp Leu Gln Gln Leu Gln
       50                  55                  60
   Tyr Ile Lys Ile Asp Asp His Tyr Ile Leu Asn Thr Gln Tyr Thr Pro
   65                  70                  75                  80
   Ile Ala Asn Leu Asp Asp Phe Gly Ile Thr Leu Lys Leu Gln Asn Ala
                   85                  90                  95
   Leu Asn Gly Ser Asp Lys Arg Leu Pro Ala Leu Tyr Gly Leu Ala Glu
               100                 105                 110
   Met Asp Ala Gln Lys Phe Lys Asp Leu Met Arg Gly Lys Ile Lys Cys
           115                 120                 125
   Glu Tyr Leu Arg Thr Thr Phe Asp Ala Glu Thr Leu Lys Pro Val Asn
       130                 135                 140
   Asp Tyr Phe Leu Ile Ser Phe Thr Tyr Lys Asp Lys Trp Tyr Glu Phe
   145                 150                 155                 160
   Glu Thr Glu Arg Lys Ile Ser Lys Thr Ser Asp Asp Gly Tyr Phe Leu
                   165                 170                 175
   Trp Ala Phe Asp Asn Thr Val His Glu Ala Gly Tyr Trp His Asn Thr
               180                 185                 190
   Asp Pro Ala Ala Tyr Ser Tyr Arg Asp Tyr Gln Asn Gly Lys Ala Val
           195                 200                 205
   Lys
<210> 66
   <211> 424
   <212> PRT
   <213> Escherichia coli
<400> 66
   Met Asp Ile Trp Arg Gly His Ser Phe Leu Met Thr Ile Ser Ala Arg
   1               5                   10                  15
   Phe Arg Gln Tyr Val Phe Ser Leu Met Ser Ile Leu Leu Gln Glu Arg
               20                  25                  30
   Lys Met Asn Ile Phe Thr Leu Ser Lys Ala Pro Leu Tyr Leu Leu Ile
           35                  40                  45
   Ser Leu Phe Leu Pro Thr Met Ala Met Ala Ile Asp Pro Pro Glu Arg
       50                  55                  60
   Glu Leu Ser Arg Phe Ala Leu Lys Thr Asn Tyr Leu Gln Ser Pro Asp
   65                  70                  75                  80
   Glu Gly Val Tyr Glu Leu Ala Phe Asp Asn Ala Ser Lys Lys Val Phe
                   85                  90                  95
   Ala Ala Val Thr Asp Arg Val Asn Arg Glu Ala Asn Lys Gly Tyr Leu
               100                 105                 110
   Tyr Ser Phe Asn Ser Asp Ser Leu Lys Val Glu Asn Lys Tyr Thr Met
           115                 120                 125
   Pro Tyr Arg Ala Phe Ser Leu Ala Ile Asn Gln Asp Lys His Gln Leu
       130                 135                 140
   Tyr Ile Gly His Thr Gln Ser Ala Ser Leu Arg Ile Ser Met Phe Asp
   145                 150                 155                 160
   Thr Pro Thr Gly Lys Leu Val Arg Thr Ser Asp Arg Leu Ser Phe Lys
                   165                 170                 175
   Ala Ala Asn Ala Ala Asp Ser Arg Phe Glu His Phe Arg His Met Val
               180                 185                 190
   Tyr Ser Gln Asp Ser Asp Thr Leu Phe Val Ser Tyr Ser Asn Met Leu
           195                 200                 205
   Lys Thr Ala Glu Gly Met Lys Pro Leu His Lys Leu Leu Met Leu Asp
       210                 215                 220
   Gly Thr Thr Leu Ala Leu Lys Gly Glu Val Lys Asp Ala Tyr Lys Gly
   225                 230                 235                 240
   Thr Ala Tyr Gly Leu Thr Met Asp Glu Lys Thr Gln Lys Ile Tyr Val
                   245                 250                 255
   Gly Gly Arg Asp Tyr Ile Asn Glu Ile Asp Ala Lys Asn Gln Thr Leu
               260                 265                 270
   Leu Arg Thr Ile Pro Leu Lys Asp Pro Arg Pro Gln Ile Thr Ser Val
           275                 280                 285
   Gln Asn Leu Ala Val Asp Ser Ala Ser Asp Arg Ala Phe Val Val Val
       290                 295                 300
   Phe Asp His Asp Asp Arg Ser Gly Thr Lys Asp Gly Leu Tyr Ile Phe
   305                 310                 315                 320
   Asp Leu Arg Asp Gly Lys Gln Leu Gly Tyr Val His Thr Gly Ala Gly
                   325                 330                 335
   Ala Asn Ala Val Lys Tyr Asn Pro Lys Tyr Asn Glu Leu Tyr Val Thr
               340                 345                 350
   Asn Phe Thr Ser Gly Thr Ile Ser Val Val Asp Ala Thr Lys Tyr Ser
           355                 360                 365
   Ile Thr Arg Glu Phe Asn Met Pro Val Tyr Pro Asn Gln Met Val Leu
       370                 375                 380
   Ser Asp Asp Met Asp Thr Leu Tyr Ile Gly Ile Lys Glu Gly Phe Asn
   385                 390                 395                 400
   Arg Asp Trp Asp Pro Asp Val Phe Val Glu Gly Ala Lys Glu Arg Ile
                   405                 410                 415
   Leu Ser Ile Asp Leu Lys Lys Ser
               420
<210> 67
   <211> 489
   <212> DNA
   <213> Escherichia coli
<400> 67
   atgaaactga aagctattat attggccacc ggtcttatta actgtattgt attttcagca      60
   caggcagtgg atacgacgat tactgtgacg ggtaatgttt tgcaaagaac atgtaatgta     120
   ccagggaatg tggatgtttc tttgggtaat ctgtatgtat cagactttcc caatgcagga     180
   agtggatctc catgggttaa ttttgatctg tctctcaccg gatgccagaa tatgaatact     240
   gttcgggcaa catttagtgg tactgcggat gggcagacat actatgcgaa tacagggaat     300
   gctggcggta tcaagattga aattcaggac agggatggaa gtaatgcatc atatcacaat     360
   ggtatgttca agacgcttaa tgtacaaaat aataatgcaa cctttaatct taaagcccgt     420
   gcagtgagta aaggccaggt tactcctgga aatatcagtt ctgttataac cgtcacctat     480
   acctatgcg                                                             489
<210> 68
   <211> 2019
   <212> DNA
   <213> Escherichia coli
<400> 68
   atgaaaatga cacggcttta tcctctggcc ttggggggat tattgctccc cgccattgct       60
   aatgcccaga cttcacagca agacgaaagc acgctggtgg ttaccgccag taaacaatct     120
   tcccgctcgg catcagccaa caacgtctcg tctactgttg tcagcgcgcc ggaattaagc     180
   gacgccggcg tcaccgccag cgacaaactc cccagagtct tgcccgggct caatattgaa     240
   aatagcggca acatgctttt ttcgacgatc tcgctacgcg gcgtctcttc agcgcaggac     300
   ttctataacc ccgccgtcac cctgtatgtc gatggcgtcc ctcagctttc caccaacacc     360
   atccaggcgc ttaccgatgt gcaaagcgtg gagttgctgc gaggcccaca gggaacgtta     420
   tatggcaaaa gcgctcaggg cgggatcatc aacatcgtca cccagcagcc ggacagcacg     480
   ccgcgcggct atattgaagg cggcgtcagt agccgcgaca gttatcgaag taagttcaac     540
   ctgagcggcc ccattcagga tggcctgctg tacggcagcg tcaccctgtt acgccaggtt     600
   gatgacggcg acatgattaa ccccgcgacg ggaagcgatg acttaggcgg cacccgcgcc     660
   agcataggga atgtgaaact gcgtctggcg ccggacgatc agccctggga aatgggcttt     720
   gccgcctcac gcgaatgtac ccgcgccacc caggacgcct atgtgggatg gaatgatatt     780
   aagggccgta agctgtcgat cagcgatggt tcaccagacc cgtacatgcg gcgctgcact     840
   gacagccaga ccctgagtgg gaaatacacc accgatgact gggttttcaa cctgatcagc 900
   gcctggcagc agcagcatta ttcgcgcacc ttcccttccg gttcgttaat cgtcaatatg 960
   tctcagcgct ggaatcagga tgtgcaggag ctgcgcgctg caaccctggg cgatgcgcgt 1020
   accgttgata tggtgtttgg gctgtaccgg cagaacaccc gcgagaagtt aaattcagcc 1080
   tacgacatgc cgacaatgcc ttatttaagc agtaccggct ataccaccgc tgaaacgctg 1140
   gccgcataca gtgacctgac ctggcattta accgatcgtt ttgatatcgg cggcggcgtg 1200
   cgcttctcgc atgataaatc cagtacacaa tatcacggca gcatgctcgg caacccgttt 1260
   ggcgaccagg gtaagagcaa tgacgatcag gtgctcgggc agctatccgc aggctatatg 1320
   ctgaccgatg actggagagt gtatacccgt gtagcccagg gatataaacc ttccgggtac 1380
   aacatcgtgc ctactgcggg tcttgatgcc aaaccgttcg tcgccgagaa atccatcaac 1440
   tatgaacttg gcacccgcta cgaaaccgct gacgtcacgc tgcaagccgc gacgttttat 1500
   acccacacca aagacatgca gctttactct ggcccggtcg ggatgcagac attaagcaat 1560
   gcgggtaaag ccgacgccac cggcgttgag cttgaagcga agtggcggtt tgcgccaggc 1620
   tggtcatggg atatcaatgg caacgtgatc cgttccgaat tcaccaatga cagtgagttg 1680
   tatcacggta accgggtgcc gttcgtacca cgttatggcg cgggaagcag cgtgaacggc 1740
   gtgattgata cgcgctatgg cgcactgatg ccccgactgg cggttaatct ggtcgggccg 1800
   cattatttcg atggcgacaa ccagttgcgg caaggcacct atgccaccct ggacagcagc 1860
   ctgggctggc aggcgactga acggatgaac atttccgtct atgtcgataa cctgttcgac 1920
   cgtcgttacc gtacctatgg ctacatgaac ggcagcagcg ccgtcgcgca ggtcaatatg 1980
   ggtcgcaccg tcggtatcaa tacgcgaatt gatttcttc 2019
<210> 69
   <211> 738
   <212> DNA
   <213> Escherichia coli
<400> 69
   atgaataagg tttttgttgt ttcagtggtg gccgcagcct gtgtatttgc agtaaatgca 60
   ggagcaaagg aaggtaaaag cggtttttat ctgaccggta aagccggtgc ctctgtgatg 120
   tcactttcag accagcgttt cctgtcagga gatgaggaag aaacatcaaa gtataaaggc 180
   ggcgatgacc atgatacggt attcagtggc ggtattgcgg tcggttatga tttttatccg 240
   cagttcagta ttccggttcg tacagaactg gagttttacg ctcgtggaaa agctgattcg 300
   aagtataacg tagataaaga cagctggtca ggtggttact ggcgtgatga cctgaagaat 360
   gaggtgtcag tcaacacact aatgctgaat gcgtactatg acttccggaa tgacagcgca 420
   ttcacaccat gggtatccgc agggattggc tacgccagaa ttcaccagaa aacaaccggt 480
   atcagtacct gggattatga gtacggaagc agtggtcgcg aatcgttgtc acgttcaggc 540
   tctgctgaca acttcgcatg gagccttggc gcgggtgtcc gctatgacgt aaccccggat 600
   atcgctctgg acctcagcta tcgctatctt gatgcaggtg acagcagtgt gagttacaag 660
   gacgagtggg gcgataaata taagtcagaa gttgatgtta aaagtcatga catcatgctt 720
   ggtatgactt ataacttc 738
<210> 70
   <211> 498
   <212> DNA
   <213> Escherichia coli
<400> 70
   atgaaactga aagctattat attggccacc ggtcttatta actgtattgc attttcagca 60
   caggcagtgg atacgacgat tactgttaca gggagggtat tgccacgtac ctgtaccatt 120
   ggtaatggag gaaacccaaa cgccaccgtt gttttggata acgcttacac ttctgacctg 180
   atagcagcca acagcacctc tcagtggaaa aatttttcgt tgacattgac gaattgtcag 240
   aatgtaaaca atgttacttc atttggtgga accgcagaaa atacaaatta ttacagaaat 300
   acaggggatg ctactaatat catggttgag ctacaggaac aaggtaatgg taataccccc 360
   ttgaaagttg gttcaacaaa agttgttaca gtgagcaatg ggcaggcgac attcaatctt 420
   aaagtccgtg ccgtaagcaa aggtaatgct ggtgcgggaa gtattaattc acaaattact 480
   gtcacctata cctatgcg 498
<210> 71
   <211> 3885
   <212> DNA
   <213> Escherichia coli
<400> 71
   atgaataaaa tatactccct taaatatagt gctgccactg gcggactcat tgctgtttct 60
   gaattagcga aaagagtttc tggtaaaaca aaccgaaaac ttgtagcaac aatgttgtct 120
   ctggctgttg ccggtacagt aaatgcagca aatattgata tatcaaatgt atgggcgaga 180
   gactatcttg atcttgcaca aaataaaggt attttccagc ccggagcaac agacgtaaca 240
   atcactttaa aaaacggaga taaattctct ttccataatc tctcaattcc ggatttttct 300
   ggtgcagcag cgagtggcgc agctaccgca ataggaggtt cttatagtgt tactgttgca 360
   cataacaaaa agaaccctca ggccgcagaa acccaggttt acgctcagtc ttcttacagg    420
   gttgttgaca gaagaaattc caatgatttt gagattcaga ggttaaataa atttgttgtg    480
   gaaacagtag gtgccacccc ggcagagacc aaccctacaa catattctga tgcattagaa    540
   cgctacggta tagtcacttc tgacggttca aaaaaaatca taggttttcg tgctggctct    600
   ggaggaacat catttattaa tggtgaatcc aaaatctcaa caaattcagc atatagccat    660
   gatctgttaa gtgctagtct atttgaggtc acccaatggg actcatacgg catgatgatt    720
   tataaaaatg ataaaacatt tcgtaatctt gaaatattcg gagacagcgg ctctggagca    780
   tacttatatg ataacaaact agaaaaatgg gtattagtcg gaacaaccca tggtattgcc    840
   agcgttaatg gtgaccaact gacatggata acaaaataca atgataaact ggttagtgag    900
   ttaaaagata cctatagtca taaaataaat ctgaatggca ataatgtaac cattaaaaac    960
   acagatataa cattacacca aaacaatgca gataccactg gtactcaaga aaaaataact   1020
   aaagacaaag atattgtgtt cacaaatggg ggagatgtcc tgtttaagga taatttggat   1080
   tttggtagcg gtggtattat ctttgacgaa ggccatgaat ataacataaa cggtcaggga   1140
   tttacattta aaggagcagg aattgatatc ggaaaagaaa gcattgtaaa ctggaatgca    1200
   ttgtattcca gtgatgatgt tttacacaaa ataggccccg gtactctgaa tgttcaaaaa   1260
   aaacaggggg caaatataaa gataggtgaa ggaaatgtta ttcttaatga agaaggaaca   1320
   tttaacaata tataccttgc aagcggaaat ggtaaggtaa tactaaataa agataattcc   1380
   cttggcaatg atcaatatgc ggggatattt tttactaaac gtggtggtac gctagattta   1440
   aatggacaca atcagacttt tactagaatt gccgccactg acgatggaac aacaataact   1500
   aactcagata caacgaaaga agccgttctg gcaatcaata acgaagactc ctacatatat   1560
   catgggaaca taaatggcaa tataaaacta acgcacaata ttaattctca ggataagaaa   1620
   actaatgcaa aattaattct ggatggtagt gtcaacacaa aaaatgatgt tgaagtcagt   1680
   aatgccagtc ttaccatgca aggccatgca acagagcatg caatattcag aagctcagcg   1740
   aatcattgct ccctggtatt tctttgtgga acggactggg tcaccgtttt gaaagaaaca   1800
   gagagttcat ataataaaaa attcaattct gattacaaaa gtaataatca gcagacctca   1860
   tttgatcagc ctgactggaa aaccggggtg tttaaatttg atacattaca cctgaacaat   1920
   gctgactttt caatatcacg caatgccaat gttgaaggaa atatatcagc aaataaatca   1980
   gctatcacaa tcggcgataa aaatgtttac attgataatc ttgcagggaa aaatattact   2040
   aataatggtt ttgacttcaa acaaactatc agtactaatc tatccatagg agaaactaaa   2100
   tttacaggtg gcatcactgc acataacagc caaatagcca taggtgatca agctgtagtt 2160
   acacttaatg gtgcaacctt tctggataat actcctataa gtatagataa aggagcaaaa 2220
   gttatagcac aaaattccat gttcacaaca aaaggtattg atatctccgg tgaactgact 2280
   atgatgggaa tccctgaaca gaatagtaaa actgtaacgc cgggtctcca ctacgctgct 2340
   gatggattca ggctgagtgg tggaaatgca aatttcattg ccagaaatat ggcatctgtc 2400
   accggaaata tttatgctga tgatgcagca accattactc tgggacagcc tgaaactgaa 2460
   acaccgacta tatcgtctgc ttatcaggca tgggcagaga ctcttttgta tggctttgat 2520
   accgcttatc gaggcgcaat aacagccccc aaagctacag ttagcatgaa taatgcgatc 2580
   tggcatctaa atagccagtc atcaattaat cgtctagaaa caaaagacag tatggtgcgt 2640
   tttactggtg ataatgggaa gtttacaacc cttacagtga acaaccttac tatagatgac 2700
   agtgcatttg tgctgcgtgc aaatctggcc caagcagatc agcttgttgt caataaatcg 2760
   ttgtctggta aaaacaacct tctgttagtc gacttcattg agaaaaatgg aaacagcaac 2820
   ggactgaata tcgatctggt cagcgcacca aaaggaactg cagtagatgt ctttaaagct 2880
   acgactcgga gtattggctt cagtgatgta acaccggtta tcgagcaaaa gaacgataca 2940
   gacaaagcaa catggactct gatcggctat aaatctgtgg ccaacgccga tgcggctaaa 3000
   aaggcaacat tactgatgtc aggcggctat aaagccttcc ttgctgaggt caacaacctt 3060
   aacaaacgta tgggtgatct gcgtgacatt aacggtgagt ccggtgcatg ggcccgaatc 3120
   attagcggaa ccgggtctgc cggcggtgga ttcagtgaca actacaccca cgttcaggtc 3180
   ggtgcggata acaaacatga actcgatggc cttgacctct tcaccggggt gaccatgacc 3240
   tataccgaca gccatgcagg cagtgatgcc ttcagtggtg aaacgaagtc tgtgggtgcc 3300
   ggtctctatg cctctgccat gtttgagtcc ggagcatata tcgacctcat cggtaagtac 3360
   gttcaccatg acaacgagta taccgcaact ttcgccggcc ttggcaccag agactacagc 3420
   tcccactcct ggtatgccgg tgcggaagtc ggttaccgtt accatgtaac tgactctgca 3480
   tggattgagc cgcaggcgga acttgtttac ggtgctgtat ccgggaaaca gttctcctgg 3540
   aaggaccagg gaatgaacct caccatgaag gataaggact ttaatccgct gattgggcgt 3600
   accggtgttg atgtgggtaa atccttctcc ggtaaggact ggaaagtcac agcccgcgcc 3660
   ggccttggct accagtttga cctgtttgcc aacggtgaaa ccgtactgcg tgatgcgtcc 3720
   ggtgagaaac gtatcaaagg tgaaaaagac ggtcgtatgc tcatgaatgt tggtctcaac 3780
   gccgaaattc gcgataatct tcgcttcggt cttgagtttg agaaatcggc atttggtaaa 3840
   tacaacgtgg ataacgcgat caacgccaac ttccgttact ctttc 3885
<210> 72
   <211> 426
   <212> DNA
   <213> Escherichia coli
<400> 72
   atgattaata ttcccagtcc caccgctgtt gttatggcgc tggtagccat cagcacgctt 60
   cccagcccta gcagggtaaa gcttatgcca tatcctccca gagcccacaa caccacaggt 120
   ttactgccag tacgggaaat ttgctttccc caccacgggg acgatggcag aaacagcatt 180
   gagccaagca tcagcagggc agcccataca gacagactca gatttgtctg tatgaccaga 240
   acagggagca caaccagcag accgttctgc ccgataccga gaagcccggc actgaacgca 300
   agtggccagc aggacagtgg tttttggggc gtatcttcga tcccaggtga cattttaatg 360
   tttcaactcc atgtattaat tgtgtttatt tgtaaaatta atttatctga caataacatt 420
   tcttat 426
<210> 73
   <211> 954
   <212> DNA
   <213> Escherichia coli
<400> 73
   atgtatgccc gcgagtatcg ctcaacacgc ccgcataaag cgattttctt tcatctttct 60
   tgcctcaccc ttatctgtag tgcgcaagtt tatgcgaagc cggatatgcg gccactgggg 120
   ccgaatatag ccgataaagg ctccgtgttt taccatttca gcgccacctc tttcgactct 180
   gtcgatggca cacgccatta tcgggtatgg acggccgtgc cgaatacaac cgcaccggca 240
   tcgggttacc cgattttata tatgcttgac ggtaacgcag ttatggaccg cctggatgac 300
   gaactgctca aacaattgtc agaaaaaaca ccgccagtga tcgtggctgt cgggtatcag 360
   accaacctcc ctttcgatct caacagcagg gcttacgact atacgccagc agcagaaagc 420
   agaaaaacag atctccactc agggcgtttt agccgtaaga gtggtggcag caacaacttc 480
   cgccagttac tggaaacgcg tattgcccca aaagtggaac agggactgaa tatcgatcgg 540
   caacgccgcg gcttatgggg gcactcctac ggcggcctct tcgtgctgga ttcctggctg 600
   tcctcctctt acttccggtc gtactacagc gccagcccgt cgttgggcag aggttatgat 660
   gctttgctaa gccgcgttac ggcggttgag cctctgcaat tctgcaccaa acacctggcg 720
   ataatggaag gctcggcgac acagggtgat aaccgggaaa cgcatgctgt cggggtgctg 780
   tcgaaaattc ataccaccct cactatactg aaagataaag gcgtcaatgc cgtattttgg 840
   gatttcccca acctgggaca cgggccgatg ttcaatgcct cctttcgcca ggcactgtta 900
   gatatcagtg gtgaaaacgc aaattacaca gcaggttgtc atgagttaag ccac 954
<210> 74
   <211> 2175
   <212> DNA
   <213> Escherichia coli
<400> 74
   atgagaatta acaaaatcct ctggtcgcta actgtgctcc tagttgggtt gaatagccag 60
   gtatcagtag ccaaatactc cgacgatgat aatgacgaga ctctggtggt ggaagccacc 120
   gctgagcagg tattaaaaca gcagccgggc gtgtcggtta ttaccagcga ggatattaaa 180
   aagacccctc cggtaaacga cctttcagat attattcgta aaatgcctgg tgttaatctt 240
   accggcaata gcgcctcggg cacacgcggt aataaccgcc agatcgatat tcgtggtatg 300
   gggccggaaa acaccttaat tttaattgat ggtgtaccgg tgacgtcacg taactccgtg 360
   cgttatagct ggcgtgggga gcgtgatacc cgcggtgaca ccaactgggt gccaccggaa 420
   caggttgagc gtattgaagt gatccgcggc cctgcggcgg cgcgctacgg ttcgggggcc 480
   gccgggggcg tggtgaacat cattaccaaa cgtcccacca acgactggca cggttcgctg 540
   tcgttataca ccaaccagcc ggaaagtagc gaagagggcg ctacgcgtcg cgccaatttc 600
   agccttagtg ggcctctggc tggtgatgct cttaccacgc gtttgtatgg taacctgaat 660
   aaaacggatg ctgacagttg ggatattaat tctccggtcg gtacgaaaaa cgcagccggg 720
   catgaagggg tacgtaacaa agatattaac ggcgttgtct cgtggaaatt aaatccgcag 780
   cagattctcg atttcgaagt cggatatagc cgccagggga atatctatgc gggcgatacg 840
   cagaacagtt cttccagtgc agttaccgaa agcctggcaa aatccggcaa agagacgaac 900
   cgcctgtacc gacagaatta tggcattacg cataatggta tctgggactg gggacaaagt 960
   cgctttggtg tttattacga gaaaaccaat aatacccgca tgaatgaagg attatccggc 1020
   ggtggtgaag gacgtatttt agcgggtgaa aagtttacga ccaatcgcct gagttcctgg 1080
   cgaaccagcg gtgagcttaa tattcctttg aatgtgatgg ttgatcaaac gctgaccgtt 1140
   ggtgcagagt ggaaccgcga taagctcgat gatccttcct ctaccagcct gacggtgaat 1200
   gacagagata tcagcggtat ttctggctct gctgcggatc gcagcagtaa aaatcattct 1260
   caaatcagtg cgctgtatat tgaagataac attgagccgg ttcctggcac gaatatcatt 1320
   cccggcctgc gctttgatta tctcagcgac tccggcggga acttcagccc cagtctgaat 1380
   ctttcgcagg aattgggcga ttatttcaaa gtcaaagcag gggttgcccg aacctttaaa 1440
   gccccaaacc tgtatcaatc cagtgaaggc tatctgctct actcgaaagg caatggctgt 1500
   ccaaaagata ttacatcagg cgggtgctac ctgatcggta ataaagatct cgatccggaa 1560
   atcagcgtca ataaagaaat tggactggag ttcacctggg aagattacca cgcaagtgtg 1620
   acctacttcc gcaatgatta ccagaataag atcgtggccg gggataacgt tatcgggcaa 1680
   accgcttcag gcgcatatat cctcaagtgg cagaatggcg ggaaagctct ggtggacggt 1740
   atcgaagcca gtatgtcttt cccactggtg aaagagcgtc tgaactggaa taccaatgcc 1800
   acatggatga tcacttcgga gcaaaaagac accggtaatc ctctgtcggt catcccgaaa 1860
   tatactatca ataactcgct taactggacc atcacccagg cgttttctgc cagcttcaac 1920
   tggacgttat atggcagaca aaaaccgcgt actcatgcgg aaacccgcag tgaagatact 1980
   ggcggtctgt caggtaaaga gctgggcgct tattcactgg tggggacgaa cttcaattac 2040
   gatattaata aaaatctgcg tcttaatgtc ggcgtcagta atatcctcaa taaacagatc 2100
   ttccgatctt ctgaaggggc gaatacctat aacgagccag gccgggctta ttatgccgga 2160
   gttaccgcat cattc 2175
<210> 75
   <211> 3042
   <212> DNA
   <213> Escherichia coli
<400> 75
   atgggtaacc aatggcaaca aaaatatctt cttgagtaca atgagttggt atcaaatttc 60
   ccttcacctg aaagagttgt cagcgattac attaagaatt gttttaaaac tgacttgccg 120
   tggtttagtc ggattgatcc tgataatgct tatttcatct gcttttctca aaaccggagt 180
   aatagcagat cttatactgg atgggatcat cttgggaaat ataaaacaga agtactgaca 240
   ctcactcaag ccgctcttat taatattggt tatcgttttg atgtttttga tgatgcaaat 300
   tcaagcacag gaatttataa aacaaagagt gcagatgtgt ttaacgaaga aaatgaagaa 360
   aaaatgctcc cgtcggaata cctgcatttt ttacaaaagt gtgattttgc aggtgtttat 420
   ggaaaaactc tgtcagatta ctggtcgaaa tactatgata aatttaagct tttactaaaa 480
   aattattata tttcttctgc tttgtatctt tataaaaatg gagagcttga tgagcgtgaa 540
   tataatttct ccatgaacgc cttaaatcgc agtgataata tatcactatt attctttgat 600
   atttatggat attacgcatc tgatattttt gtagccaaaa ataatgataa ggtaatgctt 660
   ttcattcctg gtgcaaaaaa acctttttta ttcaagaaga atatcgctga tttgcggctt 720
   acccttaaag aacttattaa ggatagtgac aacaaacaat tactttccca acatttttca 780
   ttatatagtc gtcaagatgg agtttcctat gcaggagtaa attctgttct acatgcaata 840
   gaaaatgatg gtaattttaa tgagtcttac tttctgtatt ccaataagac acttagcaat 900
   aaagatgttt ttgatgctat agctatttct gttaagaaac gcagtttcag tgatggtgat 960
   atcgttataa aatcaaacag tgaagctcaa cgagactatg ctctgactat actccagacg 1020
   attttatcaa tgacccctat atttgatatc gtagtcccgg aggtatctgt tccgcttgga 1080
   ctggggatta ttacttccag tatggggatc agttttgatc aactgattaa tggtgatact 1140
   tatgaagaac gtcgttctgc tatacctggt ttggcgacaa atgcagtatt gcttggtctg 1200
   tcttttgcaa ttccactctt gattagtaag gcaggaataa accaggaggt acttagcagc 1260
   gttataaata atgagggcag gactctgaat gaaacaaata tcgatatatt tttgaaggaa 1320
   tatggaattg ctgaagatag tatatcctca actaatttgt tagacgttaa gcttaaaagt 1380
   tccgggcagc atgtcaatat tgtaaagctt agtgatgaag ataatcaaat tgtcgctgta 1440
   aaagggagtt ctctgagcgg catctactat gaagtggaca ttgaaacagg atatgagatt 1500
   ttatcccgaa gaatttatcg taccgaatat aataatgaaa ttctctggac tcgaggtggt 1560
   ggtctaaaag gggggcagcc atttgatttt gaaagtctca atattcctgt attttttaaa 1620
   gatgaaccct attctgcagt gaccggatct ccgttatcat ttattaatga tgacagctca 1680
   cttttatatc ctgatacaaa cccaaaatta ccgcaaccaa cgtcagaaat ggatattgtt 1740
   aattatgtta agggttctgg aagctttggg gatagatttg taactttgat gagaggagct 1800
   actgaggaag aagcatggaa tattgcctct tatcatacgg ctgggggaag tacagaagaa 1860
   ttacacgaaa ttttgttagg tcagggccca cagtcaagct taggttttac tgaatatacc 1920
   tcaaatgtta acagtgcaga tgcagcaagc agacgacact ttctggtagt tataaaagtg 1980
   cacgtaaaat atatcaccaa taataatgtt tcatatgtta atcattgggc aattcctgat 2040
   gaagccccgg ttgaagtact ggctgtggtt gacaggagat ttaattttcc tgagccatca 2100
   acgcctcctg atatatcaac catacgtaaa ttgttatctc tacgatattt taaagaaagt 2160
   atcgaaagca cctccaaatc taactttcag aaattaagtc gcggtaatat tgatgtgctt 2220
   aaaggacggg gaagtatttc atcgacacgt cagcgtgcaa tctatccgta ttttgaagcc 2280
   gctaatgctg atgagcaaca acctctcttt ttctacatca aaaaagatcg ctttgataac 2340
   catggctatg atcagtattt ctatgataat acagtggggc taaatggtat tccaacattg 2400
   aacacctata ctggggaaat tccatcagac tcatcttcac tcggctcaac ttattggaag 2460
   aagtataatc ttactaatga aacaagcata attcgtgtgt caaattctgc tcgtggggcg 2520
   aatggtatta aaatagcact tgaggaagtc caggagggta aaccagtaat cattacaagc 2580
   ggaaatctaa gtggttgtac gacaattgtt gcccgaaaag aaggatatat ttataaggta 2640
   catactggta caacaaaatc tttggctgga tttaccagta ctaccggggt gaaaaaagca 2700
   gttgaagtac ttgagctact tacaaaagaa ccaatacctc gcgtggaggg aataatgagc 2760
   aatgatttct tagtcgatta tctgtcggaa aattttgaag attcattaat aacttactca 2820
   tcatctgaaa aaaaaccaga tagtcaaatc actattattc gtgataatgt ttctgttttc 2880
   ccttacttcc ttgataatat acctgaacat ggctttggta catcggcgac tgtactggtg 2940
   agagtggacg gcaatgttgt cgtaaggtct ctgtctgaga gttattctct gaatgcagat 3000
   gcctccgaaa tatcggtatt gaaggtattt tcaaaaaaat tt 3042
<210> 76
   <211> 1362
   <212> DNA
   <213> Escherichia coli
<400> 76
   atggtggaca tgattaatga aagtgcacgg caaacgccag tcattgcaca aacggacgtt 60
   ctggttatcg ggggcggtcc ggcaggatta tccgctgcca ttgcggcagg gcggttaggt 120
   gccagaacca tgattgttga gcgctacggg tcgctaggcg gcgtattgac gcaggtcggg 180
   gtagaaagtt ttgcctggta tcgtcatccg gggacggaag attgtgaagg gatctgtcgt 240
   gagtatgaag gccgcgcacg agcgctgggt ttcacacgac cagaacctca gtcaattagc 300
   gaagttatag atactgaagg atttaaagtt gtcgccgatc agatgattac ggaatctggc 360
   gttgagccgt tatatcactc ctgggttgtg gacgtgatca aggacgggga tacgttatgc 420
   ggtgttatcg tcgagaataa atcaggtcga ggggcaattc tggcgaaaag aatcgtcgat 480
   tgcacggggg atgctgatat tgccgctcgt gcaggcgcgc cctggacgaa acggagcaag 540
   gaccaactga tgggcgtcac cgtgatgttc agttgcgcag gtgttgatgt ggcacgcttt 600
   aaccgttttg ttgcggaaga acttaagccg acctacgcgg attggggcaa aaactggacg 660
   attcaaacca cgggtaaaga agacccgatg tttagcccgt atatggagga tatttttacc 720
   cgcgcgcaac aggatggtgt gattccaggt gacgcccagg cgattgccgg aacctggtcg 780
   accttttctg aaagcggtga ggctttccag atgaatatgg tgtacgcctt tggttttgac 840
   tgtaccgatg tcttcgattt aaccaaagct gagattgccg gaaggcagca agcattatgg 900
   gcaattgacg cactacgcca ctatgttccg ggctttgaaa atgtacggtt acgcaatttt 960
   ggtgccacgc tggggacgcg tgaatcacgg cttattgagg gggaaatacg tattgctgat 1020
   gattacgtcc ttaatcaggg gcgttgttcg gacagtgtag ggattttccc ggaatttatt 1080
   gatggttccg gttatctcat tttgccaacg accgggcgtt tctttcagat cccttatggt 1140
   tgtctggtgc cgcaaaaagt ggagaacctt ttggtcgccg gtcgctgtat ttccgcaggc 1200
   gtagttgcac atacttctat gcgtaacatg atgtgttgtg ccgttaccgg tgaggccgca 1260
   ggtactgccg ccgtggtttc gctacagcaa aattgcaccg tgcgtcaggt tgctatccct 1320
   gatttgcaaa acacgctgca acagcagggc gttcgtctgg ca 1362
<210> 77
   <211> 759
   <212> DNA
   <213> Escherichia coli
<400> 77
   atgtctgcca aaagacgact tcttattgcg tgtaccttga taacagctat ctatcatttt 60
   cctgcatatt cttcattaga atataaagga acctttggtt caataaatgc gggttatgca 120
   gactggaaca gtggatttgt aaacactcac cgtggtgaag tatggaaagt gactgcggat 180
   tttggggtaa attttaaaga agcagaattt tactcatttt atgaaagtaa tgtactcaat 240
   catgctgtag cagggagaaa tcatacggtt tcagcaatga cgcatgtcag actctttgac 300
   tctgatatga cattctttgg caaaatttat ggccaatggg ataactcatg gggtgacgat 360
   ctggacatgt tttatggatt cggttacctc ggctggaacg gcgagtgggg cttttttaaa 420
   ccgtatattg gattgcataa tcaatctggt gactacgtat cagctaaata tggtcaaacg 480
   aatggttgga atggttatgt tgttggctgg acagcagtat taccatttac gttatttgac 540
   gaaaaatttg ttttatctaa ctggaatgaa atagaactgg acaggaacga tgcttacacg 600
   gagcagcaat ttggccggaa cgggttaaat ggcggtttaa ctattgcctg gaagttctat 660
   cctcgctgga aagcaagtgt gacgtggcgt tatttcgata ataagctggg ctacgatggc 720
   tttggcgatc aaatgattta tatgcttggt tatgatttc 759
<210> 78
   <211> 1476
   <212> DNA
   <213> Escherichia coli
<400> 78
   atggccagtt tgatcggcct tgcagtttgc acagggaatg cttttagtcc tgccttagcc 60
   gcagaggcta aacaacctaa tttagtcatt attatggcgg atgatttagg ttatggcgat 120
   ttagcaacat atggtcatca gatcgttaaa acacctaata tcgacaggct tgcccaggaa 180
   ggggtcaaat ttactgacta ctatgccccc gctcctttaa gttcaccttc acgcgcaggg 240
   ctattaaccg gccggatgcc atttcgtact ggaattcgct catggattcc ttcaggcaaa 300
   gatgttgcct tagggcgtaa cgaactcacg attgctaatc tactcaaagc gcaagggtac 360
   gacacggcaa tgatgggtaa gctgcatctg aatgcaggcg gcgatcgcac cgatcagcca 420
   caagcacaag atatgggctt tgattactca ctggctaata cggcgggctt tgttaccgac 480
   gccacgctgg ataacgctaa agaacgcccg cgttatggca tggtttaccc gacaggctgg 540
   ctacgtaatg ggcaacccac tccacgagcc gataaaatga gcggtgagta tgtcagttcg 600
   gaagtcgtca actggctgga taacaaaaag gacagcaagc ctttcttcct ctatgttgct 660
   tttaccgaag tgcatagccc cctggcttcg cccaaaaaat acctcgacat gtactcacaa 720
   tatatgagcg cgtatcagaa gcagcatcct gatttatttt atggcgactg ggcagacaaa 780
   ccctggcgtg gtgtggggga atattatgcc aatatcagct atctggatgc acaggttgga 840
   aaagtgctgg ataaaatcaa agcgatgggt gaagaagata acacaatcgt tatttttacc 900
   agtgataacg gtccggtaac gcgtgaagcg cgcaaagtgt atgagctgaa tttggcaggg 960
   gaaacggatg gattacgcgg tcgcaaggat aacctttggg aaggcggaat tcgtgttcca 1020
   gccattatta aatatggtaa acatctacca cagggaatgg tttcagatac acccgtttat 1080
   ggtctggact ggatgcctac tttagcgaaa atgatgaact tcaaattacc tacagaccgt 1140
   actttcgatg gtgaatcgct ggttcctgtt cttgagcaaa aagcattgaa acgcgaaaag 1200
   ccattaattt tcgggattga tatgccattc caggatgatc caaccgatga atgggcgatc 1260
   cgtgatggtg actggaagat gattatcgat cgcaataata aaccgaaata tctctacaat 1320
   ctgaaatctg atcgttatga aacacttaat ctgatcggta aaaaaccaga tattgaaaaa 1380
   cagatgtatg gtaagttttt aaaatataaa actgatattg ataatgattc tctaatgaaa 1440
   gccagaggtg ataaaccaga agcggtgacc tggggc 1476
<210> 79
   <211> 954
   <212> DNA
   <213> Escherichia coli
<400> 79
   gtgacaacaa ctatctgcgc tatgggcgaa ttgctggccg agtttttgtc ccgcaaccca 60
   catcaaaaat tcactcagcc tggggagttt atcgggccat ttcccagcgg tgcgccagca 120
   atttttgctg ctcaggtggc aaaactgtcc catcgggcca tcttctttgg atgtgttggt 180
   aatgatgatt ttgcccgact cattatagag cgtctccgtc atgaaggtgt cattaccgat 240
   gggatccatg ttatgaacaa tgccgtcaca ggtacggcgt tcgtgagtta tcaaaatccc 300
   cagcagcggg atttcgtctt taatatccct aacagcgcct gcggtttgtt tactgccgag 360
   cacattgata aggatctgct taaacagtgt aaccatctgc atattgtggg ctcatcgttg 420
   ttctcatttc gcatgatcga tgtcatgcgt aaagcaataa cgacgatcaa atcggctggc 480
   ggcaccgttt ctttcgatcc caatattcgc aaagagatgc tgagcattcc tgaaatggcg 540
   caggctctcg attatttgat tgaatatacg gatattttta tccccagcga aagcgaactc 600
   cctttcttcg cgcgtcacaa aaatctgtca gaggaacaga ttgttagcga tcttctccac 660
   ggcggcgtaa aacatgtggc gataaaacgc gcccagcgtg gggccagcta ttacaagctt 720
   aaaaacggta cattacacgc ccagcatgtt gcaggtcacg atatcgaaat tatcgatcca 780
   acgggtgcag gcgactgctt tggcgcaacg tttatcactc ttttcttatc cggtttcccg 840
   gcacacaagg cgctgcaata tgcaaatgcc agcggcgcgc tcgccgtaat gcggcaaggt 900
   ccgatggaag ggatatcctc actggcagac attgaagact ttttgcagca gcac 954
<210> 80
   <211> 513
   <212> DNA
   <213> Escherichia coli
<400> 80
   atgaagatat tcattagttt atttttgttt ataatatcaa caaattcttt tgctgatgat 60
   atcactcatg ccggagtggt tcgtattgaa gggttaatta ccgaaaaaac ctgcattatt 120
   tctgatgagt caaaaaattt tacagttaat atgccagacg tacccagtag ttcggtaagg 180
   agtgcagggg atgttactga aaaggtttat ttttccataa cgttaacccg ctgtggtagt 240
   gatgttggca acgcgtatat aaagtttacc ggcaatacag tttctgaaga tgccagttta 300
   tataagctgg aagatggctc ggtagagggg cttgcactta cgatttttga taagaacaaa 360
   ggcagtatta gtaatgatgt taaaagcatg gttttttcac ttacatcatc agttgataat 420
   atattgcatt tttttgcggc ttacaaagca ttaaaaaata atgtccaacc aggggatgca 480
   aatgcgtcag tatcgtttat tgtcacctat gat 513
<210> 81
   <211> 603
   <212> DNA
   <213> Escherichia coli
<400> 81
   atgattaaat tccggcttta tattccccct gtaattctcg gttttgttat cgtaccatta 60
   ttggtatggc cgacggttat tgccttagcc gtacttatat tcacgttaac ttttctggcg 120
   gaaataatat tctcctttcc gctcctggtt gtgcgtattt ctcttcagga attacaactt 180
   gagttattgg ttgtatatgc actttttttc agtgtaatgg gtggcatcgg ttggcaattc 240
   tcccgcagaa cgcctcctga attaaaaaac aggctacatt gctggctggt cttttctccg 300
   gtctatttct ggttaattct ctcgaatttc attctttata tttctccaga gaaatcagcg 360
   ttgctggaaa atatccgaaa tttctttctg acatttgtct ggcttcccct gaatttttcc 420
   cctttttggc cgcagccgtg gactgatttt gtcggcccga ttagtgccca gcttggtttt 480
   gcgttgggat attattgcca gtggcgtagc aaaaatagaa gccataggaa gaagtggggc 540
   gattgggtaa cgtgcttaag tttggcgatt ttagctctgg ggccgttatt caattattta 600
   caa 603
<210> 82
   <211> 702
   <212> DNA
   <213> Escherichia coli
<400> 82
   atgaaattca atttatctaa tttatccgca gtattactgg catcaggtat gctgatgtct 60
   actgcggtaa ccgcagcacc cggcgatgca acacaatttg gtggggcgga tactgactgg 120
   agcaccgttg attatcccag gctcactgat atggatgaca acgttgattc aatggggggg 180
   aaaatccgct ttactggccg tgtagtgaaa gctacctgta aggtcgcaac cgattcaaaa 240
   cagattgaag ttgtcctgcc ggttgtgcct tccaaccttt tcactggtat cgacgtagaa 300
   gcacaggggg cgagcaacca gaccgatttc aatattaatc tgaccgaatg tagcaataca 360
   gatgatcaga aaattgagtt ccgttttacc ggtactgcag atagcgctaa taaaacgctc 420
   gctaacgaag tagaaggatc aacggatgct gacaacagcg gcaatgcggg ggcgactggt 480
   gtagggattc gaatttactc caaaggtacg acgaataatg gtctgattaa cctgaatacc 540
   actgcggcag agggtagcgc ctccaccgcc gcttatacaa ttccaggaaa tgctacgacc 600
   catgatttca gcgcggcctt tactgcaggt tatgctcaaa acggtagcac tgttgcacca 660
   ggtgtagtta agtcaacagc aagttttgtt gtgctgtacg ag 702
<210> 83
   <211> 1008
   <212> DNA
   <213> Escherichia coli
<400> 83
   atgcgtatac atacttattg gtatagaaga tatttcattt tattgattat tatattttca      60
   aatgttcttt cttctattgc taatgctgaa gatatggggc gagaacgtgc atattgttat     120
   ccgggttcac cgagtaataa tactacgcct gcatcctttt cttataattt tggtactata     180
   gtggtttctg atgtcaacaa aaatgcgcct ggcactgtat tgccatcaca aatctggaag     240
   gttggaacct ataaggctta ttgtaattct cttgatgatt atgaaattta cttcagtgct     300
   gtctctggaa tagatccgtc tggtgccagt ggtgatcatc aagggagtga tgtatttatt     360
   ccactcaccc atgaaatatc tgtctctact catataaaac tttataatca aaatggcaca     420
   atgacagata aaattgtgcc attcgaaaat tataatacca attatccggg ggacagaagc     480
   aaaccatcta attgggcatc aggtactgaa ggatatatta aaatcaggat tgataaaaaa     540
   attatatctg atgtttcatt aagtaacgta ttattggtgt cattatatgt cagccagatc     600
   cctaccgaac atggtcctat ccctgtcttt aatgcctaca taggaaactt aaatattcag     660
   gttccgcaag gttgcactat taatgagggt acgagtttta ctgttaatat gccggatgtg     720
   tgggccagtg aattgagccg ggctggtgcc ggagcgaagc ccgctggtgt tactcctgta     780
   gcaacaacta ttccgattaa ttgtacgaat aaagatacag atgcggtaat gacgttggta     840
   ttcgacggta acatttccgc cacacgtgat accaatggga aacaaagtat tattcaggca     900
   caagataatc ctgatgttgg tattatgatt atggatagtc agcaaaactc cgtagattta     960
   aatgccctgg caacatcagt aggcgttccg ttcagattgg tggaaaac                 1008
<210> 84
   <211> 2592
   <212> DNA
   <213> Escherichia coli
<400> 84
   atgaacctaa agctcaaaag atgcgaatat tggatggcgg cacaaaagca gatgaaacgg      60
   gttgtgccgc ttcttctggt tattatgcct gcatgttcaa tcgcgggaat gcgctttaac     120
   cctgcttttc tgtcgggtga tactgaagct gttgctgact tatcccgctt cgagaaaggg     180
   atgacttatc ttcctggtag ctatgaagtc gaagtttggg tcaatgattc ccctttactc     240
   tctcgtactg taacttttaa agcagacgat gagaatcaac tgattccctg cctttcactt     300
   gctgacttat taagccttgg aattaacaaa aatgcgctgc cagagcaggc tttggcttca     360
   tctgaaaata gttgccttga tttgcgtatc tggtttcccg atgtgcatta catgccggag    420
   ctggatgcac agagacttaa actgaccttt ccacaggcga taataaaacg tgacgctcgc    480
   ggatatattc caccagaaca gtgggataac ggtattacag cttttttgct gaattatgac    540
   ttttctggta ataacgatcg tggtgattac tcttcaaata actattattt aaatcttcgc    600
   gctgggatca atattggtgc atggcgtttt cgcgattatt caacctggag tcgtgggagt    660
   aattcagcag gtaaactgga gcatatcagt agtacgttgc agcgcgttat tattcctttc    720
   agaagtgaat taacgctagg agatacatgg tcatcatcag atgttttcga cagtgttagt    780
   attcgtggca taaaactgga atctgacgaa aatatgttgc ccgatagtca aagtggtttc    840
   gctcccacgg tgcgcggaat tgcgaaaagt cgcgctcagg taacaatcaa acagaatggt    900
   tatgtcattt atcaaaccta tatgccgccg ggaccgtttg agattagcga tcttaacccg    960
   acatcatctg cgggagatct ggaagttacc atcaaagagt ctgataattc agaaactgtc   1020
   tataccgtac cttatgccgc tgtccccatc ctgcaacgag aaggtcattt aaaatattct   1080
   actacggttg gccaatatcg aagcaatagc tataaccaga aaagtcctta tgtatttcag   1140
   ggggaattaa tttggggttt accctgggat attacggctt atggtggggc acaattctct   1200
   gaggattacc gggcgttggc gctcggcctt ggcctgaatc tgggtgtatt tggtgcaaca   1260
   tcgtttgatg ttactcaggc taacagttcg cttgtggatg ggagcaaaca tcaagggcaa   1320
   tcttatcgtt ttctttattc caaatcgtta gttcagacag gaacagcatt ccatattatt   1380
   ggctatcgtt attcaaccca gggcttttac actttaagtg atacgacata ccaacaaatg   1440
   tcagggactg ttgttgatcc aaaaacgtta gatgataaag attacgttta taactggaat   1500
   gatttttata acttgcgtta tagcaaacgt ggaaaatttc aggctagtgt atcgcaacct   1560
   ttcggtaact acgggtctat gtatttatcg gctagtcagc aaacatactg gaatactgat   1620
   aaaaaagatt ctttatacca agttggttat aacaccagta ttaagggtat ctatctaaat   1680
   gttgcgtgga attacagtaa atcaccaggg acaaatgcgg ataaaattgt ctcgctaaat   1740
   gtctcattac ctataagtaa ttggttatct tccacgaatg atgggcgctc atcatcgaat   1800
   gccatgactg caacgtatgg ttatagtcag gataaccacg gacaggtaaa ccaatatacg   1860
   ggggtatctg gttctctgtt ggagcagcat aatctcagtt ataacataca acatggtttt   1920
   gctaatcagg ataatagcag tagtggttct gttggtgtta attatcgtgg ggcatatggt   1980
   tccttgaatt ccgcctacag ttacgataat gaaggtaatc aacaaataaa ctatggcatc   2040
   agtggtgctc ttgttgtaca tgaaaatggt cttacgttga gtcaaccatt aggtgaaact   2100
   aatgttttga taaaagcgcc tggagcgaat aatgtggatg ttcagcgggg gacaggaata 2160
   tccactgact ggcgtggata tgcagttgtt ccttatgcaa cagaatatag acgtaataat 2220
   atttcattag atcctatgtc aatgaatatg catactgaac tggatatcac ttccactgaa 2280
   gttattccgg gaaaaggtgc gttagttcgt gcagagtttg ctgctcatat cggtattcgt 2340
   ggtttgttca cagttcgtta tcgtaataaa tcagtcccat tcggtgctac agccagcgct 2400
   cagattaaaa acagtagtca aattaccggg attgtcggcg ataatggaca actttatctc 2460
   tcaggattgc ctttagaagg tgttattaat atccagtggg gagacggtgt tcagcaaaaa 2520
   tgtcaggcta attacaagct ccctgaaaca gaactggata atcctgttag ctatgcaact 2580
   ctggagtgcc gc 2592
<210> 85
   <211> 507
   <212> DNA
   <213> Escherichia coli
<400> 85
   atgggagcga tttatgttaa acgtttgatt ctgtcggtag cactgataat accgatagca 60
   tccaatgctt ctgatgcttt gaaccagccg agcagtagtc taaatgatgg tgttgagact 120
   ttttttattt cctgctttga tatgcctcag gaaacaacta ctgatatgga cgcttgtcag 180
   agagttcagt tagctcaggt tagttgggtt aagaataagt attcggtggc cgccctgaat 240
   cgtttgaaac aagacaacaa ggatgatcca cagcgtctgc aggaattaac tgcttctttt 300
   aacgcggaaa gtgaagcttg gacagaatta attgagaaag cgtcaaagtc cgtccaggtt 360
   gattatgtag gaggaactat agctggcact gcagttgcat cacgtcaaat tggtcttctg 420
   gaattacaat cccacgatat ctgggagcac tggctacgat ctcgaggact caactcctcc 480
   tcttttgcca gaaccaaagt tcaaatc 507
<210> 86
   <211> 2139
   <212> DNA
   <213> Escherichia coli
<400> 86
   atggctatgt tcacaccttc attctcagga ctcaaaggtc gggcgctctt ttcactgctt 60
   tttgcggcac cgatgattca tgcaacagac tctgtaacga ccaaagatgg cgaaacaatc 120
   actgttacag cagatgcaaa taccgcaact gaggcaaccg atggttatca acctctgagc 180
   acctccacgg cgacattaac cgatatgccg atgctggata tcccgcaggt ggtcaatacg 240
   gttagcgatc aggttctgga aaaccagaat gcgacaacgc tggatgaggc gctttataac 300
   gtcagtaacg tggtacagac caatacatta ggcgggactc aggatgcttt tgtacgccgt 360
   gggtttggcg caaaccggga tggctccatc atgaccaacg gtctgcgaac cgtacttcct 420
   cgtagtttca acgccgcaac agagcgtgtg gaagtgctaa aaggcccggc ctccacgctg 480
   tatggcattc tcgatcctgg cggactgatt aacgtcgtga ccaagcgccc ggaaaaaaca 540
   ttccatggtt cggtttcagc cacctcctcc agttttggtg gcggcactgg gcaacttgat 600
   atcacaggtc ccattgaagg cactcagctg gcgtatcgcc ttaccgggga agtgcaggat 660
   gaagattact ggcgaaactt cggtaaagag cgcagtacat ttattgcccc gtcactcacc 720
   tggtttggtg ataatgcaac agtaaccatg ctctattccc atcgggacta taaaactcca 780
   ttcgatcgtg gaacgatttt cgaccttacg acgaaacagc ccgtaaacgt tgatcgaaaa 840
   atacgttttg acgaaccgtt taatattaca gatggtcagt ccgatctggc gcaactcaac 900
   gcagaatatc atctcaatag ccagtggaca gcgcgctttg attacagcta cagccaggat 960
   aaatacagcg ataatcaggc gcgtgttacc gcgtatgatg caacgacagg aacactgaca 1020
   cggcgtgttg atgcaactca gggatctacc cagcgtatgc atgctactcg tgcggatctg 1080
   caagggaatg ttgatattgc cggattctat aatgagattc tgggtggggt gtcatatgaa 1140
   tattatgatc ttctgcgtac agatatgatt cgctgtaaaa aagctaaaga tttcaatata 1200
   tacaaccctg tttatggtaa taccagcaaa tgtacaacgg tttcggcgtc ggacagcgat 1260
   cagacgatca aacaggagaa ctactcagct tatgcacagg acgcgctcta tctgaccgat 1320
   aactggattg ccgtcgccgg gatccgctat cagtattaca cgcaatatgc gggtaaaggc 1380
   cgtcctttta atgtcaatac tgacagccgc gatgaacaat ggacgcccaa actggggtta 1440
   gtctacaaac tgacgccatc ggtatcctta tttgccaatt attcgcaaac atttatgccg 1500
   cagtcgtcaa ttgccagcta cattggcgat cttccaccag aatcatctaa tgcttacgaa 1560
   gtcggggcaa aattcgagct attcgatggt atcaccgcag atattgcgct gtttgatatc 1620
   cataaacgta atgtgttgta taccgaaagt attggtgatg aaaccatcgc caaaacggca 1680
   ggccgcgttc gttcaagagg ggtagaagtc gaccttgcgg gagcattaac tgaaaacatt 1740
   aatatcattg ccagctacgg ctataccgat gcaaaggttc tggaagatcc tgattatgca 1800
   gggaaaccat tgccgaatgt tcctcgtcat accggttcgc tattcctgac ctatgatatt 1860
   cataacatgc caggcaataa cacactgacg tttggcggtg gcggacatgg tgtaagccgt 1920
   cgttcggcaa ccaatggggc tgactattat ctgcctggct atttcgttgc cgatgccttc 1980
   gccgcataca aaatgaaatt gcagtatccg gtcactctgc aattaaacgt caaaaacctg 2040
   tttgataaaa cgtattacac ctcttccatc gccacaaata atctgggcaa ccagattggc 2100
   gatccgcgtg aagtgcaatt cacggtgaaa atggaattt 2139
<210> 87
   <211> 1818
   <212> DNA
   <213> Escherichia coli
<400> 87
   atgaaaatat cgtggaatta tatatttaag aacaaatggc gatttcacat tacaagcatt 60
   tcactttttc ttatcatgct cgcggtttca atcgcttttt tgcacttgcg ttttaatacc 120
   ttgtccagta ccgataaaat gcggcttgaa atgtataagt ccacattata ttccaccatc 180
   gagcaatttt atgttttacc ctatatgctc tcaacagacc atatcatccg tcaggcggta 240
   attacgcctg acgatatgac gtccagcgaa ctcaatcaac gaattgcaca tttcaatact 300
   caactcaaaa ccgcagcaat atttattctg gatacccaag gtaaggccat cgcttctagc 360
   aactggcagg accccggcag ctatgtaggg caaaattata gctatcgccc ctattataaa 420
   cacgccatgt ctggcttaaa tggacgcttt tacggtattg gtagcactac gaatacaccg 480
   ggattcttcc tctctacaag tataaaagat aaaggaaaaa ttgtcggtgt tgtagtagta 540
   aaaataagtc ttaatgaaat tgaaaaagca tgggccgaag gtcctgaaaa tattatcgtg 600
   aatgatgaac atgggattat atttttaagt tcaaaatcgc catggcgaat gcgaacactg 660
   caaccgttac ctgttcaggc aaaacaaaaa ctacaatcta cccgccaata tagtctcgac 720
   aatcttttac cggcggatta ttatccctgt tataccgtga gcaattttac tttcctgaaa 780
   gataaaaaag aacaactctg tttattcccg caatattata cgcaacaaat agccattcca 840
   gaatttaact ggaaaatgac aattatggtc cccttagata acctgtactg gtcatgggct 900
   atttcgttag tcattacact aattatttac ctgctgtttt tgttatttat taaatactgg 960
   agaatgcgat ctcatgcaca acaattatta acacttgcga atgaaacatt agaaaaacag 1020
   gttaaagagc gtacatctgc cctggaattg atcaatcaaa aattaataca ggagataaaa 1080
   gagcgcagtc aagctgaaca agtattacaa attacgcgta gtgaactggc agagtccagc 1140
   aaactggcgg cgcttggaca gatggcaacc gaaattgccc atgaacaaaa tcaaccgtta 1200
   gccgccattc acgcacttac tgataacgcg cgtactatgc taaaaaaaga gatgtatccg 1260
   caggttgaac agaatctgaa acatattatt tcagtgattg agcggatgac gcagctcatt 1320
   tccgaactta aagcatttgc ctcgcgccat cgcgtaccta aaggttctgc cgatgtcatc 1380
   aaagtgatgt atagcgccgt ggcgttactt aatcacagca tggagaaaaa taacattgag 1440
   cgacgaataa aagccccatc catgccgtta tttgtcaatt gcgatgagct cggtcttgaa 1500
   cagatattca gtaatttaat tagcaacgcc ttagattcta tggaaggtag ctcttacaaa 1560
   cgactggata tcgccattcg ccaggcaaat aacaaagtta ttattaccat taaagacagc 1620
   ggtggcggtt ttgcacctga agttgtcgat cgcatatttg aaccattttt taccactaaa 1680
   cgtagaggaa tggggttggg actggcaata gtcagcgaaa ttgtccgaaa ttcgaacggc 1740
   gcactccacg ccagtaatca tcctgaaggc ggcgcagtaa tgacattaac ctggcctgaa 1800
   tggggagaag aacatgaa 1818
<210> 88
   <211> 303
   <212> DNA
   <213> Escherichia coli
<400> 88
   gtgcttacac cacaacattt acgttgtgtg ttaacatgta gcgatttact gactcttttg 60
   agtggtaccg ttatgtctca aatgcccctc tattttctta atacccaaaa gaaactcact 120
   gctcactatg aatggcttca aatcaacctg actgatacct acgaactagt taaaaggtta 180
   atgccgattc cttcactgga cgtggtggtt aaagtaggga aacttgtcct cccggagaaa 240
   gggcatcatg gtttttaccc tgaagctgga gttgtctata gaacagtagc tccagaaaat 300
   cca 303
<210> 89
   <211> 789
   <212> DNA
   <213> Escherichia coli
<400> 89
   atgatgaaaa atacaggcta tatcttagct ctttgtctga cagcatcggg gcatgtccta 60
   gcccatgatg tctggattac aggtaaacag gcagagaaca acgttaccgc agagattggt 120
   tatggtcata atttcccctc aaaggggaca attcctgaca gaagggattt ctttgaaaat 180
   ccccggcttt ataacgggaa agagacaata acactgaagc cagcgtccac ggattatgtc 240
   tataaaactg agtctgcaag caaagataat ggttacgttc tgtcaacgta tatgaaaccg 300
   ggatactggt cgagaacctc gtcaggatgg aaaccggtca gccgggaggg cagaaatgat 360
   gtggcttact gtgaatttgt cactaaatat gcaaaatctt ttattcctgg tgaacagcag 420
   atgccagcac aactctatca gtctccaaca gggcatgagc ttgaaatcat tccgttatcc 480
   gatataagtc gtttcagtga aaatgtgaag ctgaaagttc tgtataaaac gtccccgctc 540
   gccggagcta tcatggagct tgactcggtc agttatctga catcatcccg tcatactcat 600
   gcagttgagc acaaacatcc tgttcataaa gcagaactca cctttgtaac taatgaggat 660
   ggtatcgtca cagtaccttc tcttcatatc ggacagtggc tggcgaaagt ccaaaataag 720
   aaaagttttc aggacaaaag cctgtgtgat gaaactgtcg atgtggcaac cttaagcttc 780
   tcccgaaat 789
<210> 90
   <211> 1134
   <212> DNA
   <213> Escherichia coli
<400> 90
   atgggaaaaa taaaatattg gctaatagta ggatttatta tactttttgc gattttttac     60
   attgctatta gtgacaggga ttctacgctt tctaggttga aatcagcagg tgaaaacgga    120
   gatgtagaag ctcagtatgc tttggggctc atgtatttgt atggagaaat tctggatgtt    180
   gattatcagc aggcaaagat ttggtatgaa aaagccgctg accaaaatga tccgcgtgcg    240
   caggccaaac tcggtgtgat gtatgcaaat ggtctcgggg taaatcagga ttatcagcaa    300
   tcaaaattat ggtatgaaaa ggcggctgcg caaaatgatg ttgatgcgca atttttgctt    360
   ggggagatgt atgacgatgg tctcggggta agccaagact accagcatgc aaagatgtgg    420
   tatgaaaaag cggctgctca aaatgatgag cgtgctcagg tcaatctcgc tgttctatac    480
   gcaaagggta atggtgttga acaggattat cgacaggcca aaagctggta tgaaaaggct    540
   gcagctcaaa atagtcctga tgcgcagttc gctcttggaa ttctgtatgc caatgctaat    600
   ggtgtagagc aggactatca gcaggcaaaa gactggtatg agaaagcagc agaacaaaat    660
   ttcgccaatg ctcagtttaa tcttggtatg ctctattaca aaggtgaggg tgttaaacaa    720
   aactttcggc aagccagaga atggtttgaa aaagccgcat ctcaaaatca gccgaatgcc    780
   caatataatt taggtcagat ttattactac ggtcagggtg tgactcagag ctatcgacag    840
   gcgaaagact ggtttgaaaa agcggcagag aaaggtcatg tcgatgctca atataatctc    900
   ggtgtaatat acgaaaatgg tgaaggtgtg agtcagaact atcaacaggc aaaggcttgg    960
   tatgaaaagg cagcctcaca aaatgatgcg caggcgcagt tcgaacttgg cgttatgaat   1020
   gaactgggtc agggtgaaag catagacctg aaacaagcaa gacattacta tgagcggtca   1080
   tgtaataatg ggcttaagaa aggttgtgaa cggttaaaag agttattata caaa         1134
<210> 91
   <211> 1962
   <212> DNA
   <213> Escherichia coli
<400> 91
   atgaatgtaa tcagaactgt catttgtaca ttaattatac ttccggtggg attacaggca      60
   gcgaccagtc attcttctat ggttaaagat acaatcacca ttgtcgcgac aggaaatcag     120
   aacacggtat ttgaaacgcc gtcgatggtc agtgtcgtca cgaatgacac accgtggagt     180
   cagaatgcgg ttacatcggc cggcatgctg aaaggtgttg ccggtctcag ccagactggt     240
   gcaggacgga ccaatgggca gacctttaat ttacgcggct atgacaaaag cggggtactt     300
   gttcttgttg acggcgttcg ccaactcagt gacatggcaa aaagcagtgg cacttatctg     360
   gatccggcac tcgtcaaacg tatcgaagtt gtccgcgggc caaactccag tctgtacggc     420
   agtggcgggc tgggaggtgt agtggacttc agaactgccg atgcagcaga ttttcttccc     480
   cccggagaga caaacggttt aagtctgtgg ggaaatatcg ccagtggtga ccacagcaca     540
   ggctcggggc tcacctggtt tggtaaaact ggaaaaacag atgcgctcct ttctgtcatt     600
   atgcgtaaaa gaggtaatat ctatcaaagt gatggtgagc acgcacctaa caaggaaaaa     660
   cctgcagccc tgtttgcgaa aggctctgtc ggtataacag acagtaacaa agcaggtgcc     720
   agcttgcgtc tctaccggaa taacaccact gaaccgggca attccactca gacacatggt     780
   gacagcggcc tgcgtgacag aaaaacagta caaaatgacg tacagttctg gtaccagtac     840
   gctcctgtgg ataacagcct catcaatgta aagtcaacgt tatatctcag tgatatcact     900
   atcaagacaa acggtcacaa caaaacggca gaatggagaa acaacagaac ctccggtgtt     960
   aatgttgtca acaggagtca tactctgatt tttccgggag cccatcagtt aagttatggc    1020
   gctgaatatt accgtcagca gcagaagcca gaaggctctg ccacactata tccggaagga    1080
   aacattgact ttacatcgtt gtatttccag gatgaaatga caatgaaaag ctacccggtt    1140
   aacattatcg tcggttcccg ctatgaccgg tacaagagct tcaatccccg tgccggagaa    1200
   ctgaaagccg aacgcctgtc cccaagggcg gcgatttcag tctcaccgac agactggctg    1260
   atgatgtacg gctccatatc ctctgcattc cgagcgccca caatggcaga aatgtacagg    1320
   gatgatgtac atttttaccg caagggtaaa cccaattact gggttcctaa ccttaatctg    1380
   aaaccagaaa ataacatcac ccgtgagatt ggcgcaggta ttcaactgga tggcctgctt    1440
   acagacaatg accggctgca gttaaaaggc ggatatttcg gaacggatgc cagaaactat    1500
   attgccacac gcgtggatat gaaacggatg cgttcttatt cttataatgt atcccgggcc    1560
   cgtatctggg gatgggatat gcagggtaat taccagtctg attatgttga ctggatgctt   1620
   tcttataacc ggacggaaag tatggatgcc agcagcaggg aatggctggg ctccggcaat   1680
   cctgacacac ttatcagtga catcagcata cctgttggtc atagaggcgt ttatgccgga   1740
   tggcgtgctg aactttcagc atcagccacg catgtgaaaa aaggcgatcc ccatcaggct   1800
   ggttatacca tacattcctt ttcactgtct tataagcctg taagtgttaa aggctttgag   1860
   gcgtcagtaa ctctggataa tgccttcaac aagcttgcca tgaatggcaa aggtgtgccg   1920
   ctttcaggca gaactgtcag tctttatacc cgttatcagt gg                      1962
<210> 92
   <211> 4128
   <212> DNA
   <213> Escherichia coli
<400> 92
   atgaataaaa tatacgctct aaaatattgt tatattacta acacagtaaa ggttgtctct     60
   gaactagccc gaagggtatg taaagggagt acccgcagag gaaaaagact ttcagtactt    120
   acctctctgg cactatctgc attactccca accgttgctg gtgcatcaac ggttggtggc    180
   aacaatcctt accagacata ccgcgacttt gcagaaaaca aagggcagtt tcaggctggc    240
   gcaacaaaca ttcctatttt taataataaa ggggaattag taggacatct tgataaagcg    300
   cccatggttg attttagcag tgtgaatgta agctcaaatc ccggcgttgc aacattaatt    360
   aacccgcaat atatagccag tgtaaaacat aataaaggat atcagagcgt cagcttcggt    420
   gatggtcaga acagttacca tattgtggat cgtaatgaac acagttcatc tgatctccac    480
   acaccaagac ttgataagct cgtaactgag gttgctccgg ctaccgtaac cagctcatca    540
   acagctgata tattgaaccc ttcaaaatac tcggcattct acagggctgg ttcgggaagt    600
   cagtatattc aggatagtca gggtaagcga cattgggtaa caggtgggta tggttatctg    660
   acaggaggaa tactcccgac atcattcttt tatcacggct cagacggcat tcagctgtat    720
   atggggggca acatacatga tcatagcatc ctgccctctt ttggagaggc cggcgacagt    780
   ggttctccat tatttggctg gaatacggcc aaagggcagt gggaactggt cggtgtttac    840
   tcgggagtag gaggggggac caatttgata tattctctta ttcctcagag ttttctctca    900
   cagatctatt cagaggataa tgacgctccc gtctttttta atgcctcatc cggcgccccc    960
   ctgcaatgga aatttgacag cagcaccggc actggctctc tgaaacaggg ttccgatgaa   1020
   tatgccatgc acgggcaaaa aggttctgac ctgaacgcag gtaaaaatct gacattcctg   1080
   ggacataatg gtcagattga cctggaaaac tctgtcacgc agggtgccgg ttcactgaca 1140
   tttactgatg actacactgt caccacttca aacggaagta cctggaccgg ggccggtatt 1200
   attgtggaca aggatgcctc cgtaaactgg caggttaatg gtgtgaaagg tgacaacctg 1260
   cataaaatcg gcgaaggaac cctggttgta cagggaaccg gtgttaatga gggcggcctg 1320
   aaagtcgggg atgggaccgt tgtcctcaat cagcaggctg acagttcagg acacgttcag 1380
   gcattcagta gcgtgaatat tgccagcggc cgcccgacag tcgtgctggc agacaaccag 1440
   caggttaatc cggacaatat atcctggggc taccgggggg gggttctgga tgttaacggg 1500
   aatgacctga catttcataa gctgaatgcc gccgattatg gcgcaactct cggtaacagc 1560
   agtgataaaa cggctaatat cactctggat tatcagacgc gtccggcaga cgtaaaagtt 1620
   aatgaatggt catcatcaaa caggggaaca gtaggttcat tatatattta taataatccc 1680
   tatactcata ccgtcgatta ttttatcctg aaaacaagta gttatggctg gttccctacc 1740
   ggtcaggtca gtaacgagca ctgggaatat gtcggacatg accagaacag tgcacaggca 1800
   ctgcttgcaa acagaattaa taataaaggg tatctgtatc atggcaagtt gctgggaaat 1860
   attaatttct caaataaagc aaccccgggt acaaccggcg cattggttat ggacggctca 1920
   gcgaatatgt ccggtacatt tactcaggaa aacggtcgtc tgaccattca gggccacccg 1980
   gttatccatg cttcaacgtc tcagagtatt gcaaatacag tctcgtctct gggcgacaat 2040
   tccgttctga cacagcccac ctcatttaca caggatgact gggagaacag gacgttcagc 2100
   tttggttcgc tcgtgttaaa agatacagac tttggtctgg gccgcaatgc cacactgaac 2160
   acaaccatcc aggcagataa ctccagcgtc acgctgggcg acagtcgggt atttatcgac 2220
   aaaaaagatg gccagggaac agcatttacc cttgaagaag gcacatctgt tgcaactaaa 2280
   gatgcagata aaagcgtctt caacggcacc gtcaacctgg ataatcagtc agtgctgaat 2340
   atcaatgaga tattcaatgg cggaatacag gcgaacaaca gtaccgtgaa tatctcctca 2400
   gacagtgccg ttctggagaa ctcaacgctg accagtaccg ccctgaatct gaacaaggga 2460
   gcaaatgttc tggccagtca gagttttgtt tctgacggtc cggtgaatat ttctgatgcc 2520
   accctgagtc tgaacagccg tcctgatgag gtatctcaca cacttttacc tgtatacgat 2580
   tatgccggtt catggaacct gaagggagac gatgcccgcc tgaacgtggg gccgtacagt 2640
   atgttgtcag gtaatatcaa tgttcaggat aaagggactg tcaccctcgg aggggaaggg 2700
   gaactgagtc ctgacctgac tcttcagaat cagatgttgt acagcctgtt taacgggtac 2760
   cgcaatacct ggagcgggag cctgaatgca ccggatgcca ccgtcagcat gacagacacc 2820
   cagtggtcga tgaacggaaa ctccacggca ggaaatatga aacttaaccg gacaatagtc 2880
   ggttttaacg ggggaacatc atcgttcacg acactgacaa cagataatct ggacgcggtt 2940
   cagtcagcat ttgtcatgcg tacagacctt aacaaggcag acaaactggt gataaacaag 3000
   tcggcaacag gtcatgacaa cagcatctgg gttaacttcc tgaaaaaacc ctctgacaag 3060
   gacacgcttg atattccact ggtcagcgca cctgaagcga cagctgataa tctgttcagg 3120
   gcatcaacac gggttgtggg attcagtgat gtcaccccca cccttagtgt cagaaaagag 3180
   gacgggaaaa aagagtgggt cctcgatggt taccaggttg cacgtaacga cggccagggt 3240
   aaggctgccg ccacattcat gcacatcagc tataacaact tcatcactga agttaacaac 3300
   ctgaacaaac gcatgggcga tttgagggat attaacggcg aagccggtac gtgggtgcgt 3360
   ctgctgaacg gttccggctc tgctgatggc ggtttcactg accactatac cctgctgcag 3420
   atgggggctg accgtaagca cgaactggga agtatggacc tgtttaccgg cgtgatggcc 3480
   acctacactg acacagatgc gtcagcaggc ctgtacagcg gtaaaacaaa atcatggggt 3540
   ggtggtttct atgccagtgg tctgttccgg tccggcgctt actttgattt gattgccaaa 3600
   tatattcaca atgaaaacaa atatgacctg aactttgccg gagctggtaa acagaacttc 3660
   cgcagccatt cactgtatgc aggtgcagaa gtcggatacc gttatcatct gacagatacg 3720
   acgtttgttg aacctcaggc ggaactggtc tggggaagac tgcagggcca aacatttaac 3780
   tggaacgaca gtggaatgga tgtctcaatg cgtcgtaaca gcgttaatcc tctggtaggc 3840
   agaaccggcg ttgtttccgg taaaaccttc agtggtaagg actggagtct gacagcccgt 3900
   gccggcctgc attatgagtt cgatctgacg gacagtgctg acgttcacct gaaggatgca 3960
   gcgggagaac atcagattaa tggcagaaaa gacggtcgta tgctttacgg tgtggggtta 4020
   aatgcccggt ttggcgacaa tacgcgtctg gggctggaag ttgaacgctc tgcattcggt 4080
   aaatacaaca cagatgatgc gataaacgct aatattcgtt attcattc 4128
<210> 93
   <211> 1047
   <212> DNA
   <213> Escherichia coli
<400> 93
   atgattacac tttttcgact actggcgatt ctttgccttt tttttaacgt ttcagctttt 60
   gctgttgatt gctatcagga tgggtacaga ggaacaaccc tcataaatgg agatttacca 120
   acgttcaaaa ttccagagaa tgcgcaacct gggcaaaaaa tttgggagag cggagatatt 180
   aatatcacag tttattgtga caatgcacca ggatggtcaa gtaataaccc atcagaaaat 240
   gtctatgcct ggatcaaatt gccccaaata aatagtgccg atatgttgaa taatccgtat 300
   ttaacatttg gcgtgactta taatggtgta gattatgaag ggacaaatga aaaaattgat 360
   actcatgcgt gcctggataa atatgaacaa tactataatg ggtattatca tgaccctgta 420
   tgcaatggca gcactcttca aaaaaatgta acatttaacg cccattttcg cgtctatgta 480
   aaattcaaaa gccgcccggc aggagatcag acggtaaact ttggcacagt caacgtgctg 540
   caattcgacg gtgaaggcgg ggcgaacatg gcccccaacg cgaaaaattt acgctatgcg 600
   attacggggt tagataatat ttcattcctt gactgtagtg tcgacgtccg catttccccg 660
   gaaagtcaga tagtcaattt tgggcagatc gctgcgaatt ccattgcaac tttcccaccg 720
   aaggcagcat tcagcgtttc taccataaaa gacattgcgt ctgattgtac cgaacagttt 780
   gatgttgcaa ccagtttctt tacttcagat acattatatg acaatacgca tctggaaata 840
   ggtaacggct tgctcatgcg aattactgat caaaaaacgc aagaagatat taaatttaac 900
   cagttcaaat tatttagtac ttatattccc ggtcagagtg cggcaatggc aacccgcgat 960
   taccaggccg aattaaccca aaaacctggt gaaccactcg tctatggccc atttcagaaa 1020
   gacctgatag ttaaaatcaa ctaccac 1047
<210> 94
   <211> 2520
   <212> DNA
   <213> Escherichia coli
<400> 94
   atgaacaata aaaacacgtt ttcccgggat aagttatccc atgcaattaa aaatgccctg 60
   tctggcgttg tgtgttccct actcttcgtt ttgccagtcc acgccgtaga attcaacgtc 120
   gatatgattg acgcagaaga ccgtgagaat atcgacatct ctcgttttga gaaaaaaggc 180
   tatatccccc ctggtagata cctcgttcgt gtgcaaataa ataaaaatat gttgccacaa 240
   acgttaatac tggaatgggt aaaagccgat aatgaaagtg gttcgttact ctgcttaacc 300
   aaagaaaatt tgactaattt cggtcttaat acggaattta ttgaatcatt gcaaaacata 360
   gctggcagcg aatgtctcga tttaagccaa cgtcaggagt taacgacacg acttgataaa 420
   gctacgatga tattatcgct aagtgttccc caggcatggt taaaatacca ggcaacaaac 480
   tggacgccac cagagttttg ggataccggt atcaccgggt ttatccttga ttacaacgtg 540
   tacgccagcc agtatgcccc acatcacgga gacagcaccc aaaacgtcag ctcctatggt 600
   acgttaggct ttaacctcgg cgcatggcgc ttacgtagcg attaccaata taatcagaat 660
   tttgctgatg gacgctcggt aaaccgcgac agcgaatttg cgcgaactta tctgtttcgc 720
   cctatcccct cctggtcgtc aaaattcact atgggccagt acgacctgag ctccaatctt 780
   tacgatacct tccactttac tggcgcatcg ctggaaagtg atgaaagcat gctgccgcca 840
   gatttacagg gttatgcgcc acaaattacc ggcatcgcgc agaccaacgc gaaagtaact 900
   gtggcacaaa atggtcgtgt actttatcaa accactgtcg cgccaggccc ttttactatt 960
   tctgatttgg ggcaatcgtt tcaggggcag ctggatgtca cagtggaaga agaagatggc 1020
   cgcaccagca ccttccaggt tggctccgca tccattccct atttaacccg taaagggcaa 1080
   gtgcgctata aaacgtcact gggaaaaccg acatccgtcg ggcataacga tatcaataat 1140
   ccctttttct ggacggcgga agcctcctgg ggctggctga acaatgtgtc gttgtatggt 1200
   ggtggcatgt tcaccgctga tgattatcag gctatcacta ccggtattgg ctttaacctt 1260
   aaccaattcg gttcgctttc ttttgatgtc actggagcag acgcgtcttt acagcaacaa 1320
   aatagcggca atctgcgtgg ttacagctat cgcttcaact atgcaaagca tttcgaatcg 1380
   acaggcagtc agattacctt cgcgggttat cgcttctcag ataaagatta cgtgtcgatg 1440
   agtgagtacc tcagctcgcg taatggcgat gagtcaatcg ataatgaaaa agagagttat 1500
   gtcatttcct tgaaccagta ctttgaaacg ctggaattaa actcttatct caacgttaca 1560
   cgcaatactt attgggacag cgccagcaat accaactact ccgtatctgt aagcaaaaac 1620
   tttgatattg gcgatttcaa aggtatatct gcatcgctgg cagtaagtcg aatccgctgg 1680
   gatgacgacg aagagaatca atattacttc tctttctctc tacctttaca acaaaaccgc 1740
   aacatctcct acagtatgca gcgaacggga agcagtaata cttcgcagat gatttcctgg 1800
   tacgattcat cagatcgcaa caatatctgg aatatttcag cgtcggcaac ggacgacaat 1860
   atacgtgatg gcgaaccaac actgcgcggc agctaccagc actattcgcc gtggggacgc 1920
   ctgaacatta atggcagtgt acagccgaat cagtacaatt ctgttaccgc aggctggtac 1980
   ggttcactta ccgctacacg tcatggtgtc gcccttcacg attatagcta tggcgataac 2040
   gcccgcatga tggtcgatac cgatggcatc tccggcattg aaatcaactc taaccgtacc 2100
   gttaccaacg ggctgggcat cgccgtgata ccttcgttat cgaactacac cacctccatg 2160
   ttgcgggtga acaataacga tctgccagaa ggtgtcgatg tcgaaaactc ggttattcgt 2220
   actacgctca cccagggtgc catcggctac gcaaaactga atgccaccac cggataccaa 2280
   atcgtcggcg ttattcgtca ggaaaatggc cgcttccctc cactaggtgt gaatgtcacg 2340
   gataaagcga caggtaaaga tgtgggcctg gtagcggaag atggcttcgt ttatctcagc 2400
   ggtattcagg aaaacagtat tctgcattta acctggggtg ataatacctg tgaagtcacg 2460
   ccgccaaacc aaagtaacat tagtgaaagc gcgataattt taccttgtaa aacagtcaaa 2520
<210> 95
   <211> 507
   <212> DNA
   <213> Escherichia coli
<400> 95
   ttgatgaaca caaaacagtc tgttgctcaa ctcgccgtac cgcaccgcaa gcgcctttca 60
   tcaacgatgg tggtggcgct gttactttgt gtggttgctg gcgcggtgat gattaatgcc 120
   gctgattttc cagcaactgc cattgaaacg gatcccggtg caagtgcctt ccctaccttc 180
   tatgcctgtg ccctgattgt gctcgctgtc ttgctggtga tacgcgatct tttgcaggca 240
   aaaccagcct cttgcgccaa cgcacaggaa aaaccggcat tcaggaaaac agcaacagga 300
   attgcggcaa ccgcgtttta tattgtggcg atgagctact gcggttatct cattactact 360
   cctgttttcc tcatcgtcat tatgacgttg atgggctaca ggcgatgggt actcacaccg 420
   ggtattgcgc tgctgttaac ggcaatcctc tggttgctgt ttgtcgaagc gttacaggtg 480
   ccattgcctg tcggcacatt tttcgaa 507
<210> 96
   <211> 933
   <212> DNA
   <213> Escherichia coli
<400> 96
   atggtacttc ttgcaggcgc tgccctcagc attgcgcctg tacaggcagc ctcctaccca 60
   accaaacaga tcgagttagt cgttccctac gctgccggag gcggtacgga tctggttgcc 120
   cgtgcctttg ctgatgccgc caaaaaccat ttacccgtca gcatcggggt tatcaataaa 180
   cctggcggag gcggtgctat cggcctgagt gaaatcgccg ctgcccgccc taacggttac 240
   aaaattggtt taggcacggt tgaactgacc acccttccca gcctcggaat ggtgcgtttt 300
   aaaaccagcg actttaaacc cattgcccgt ctgaatgcgg atccggctgc tatcacagtc 360
   cgtgccgatg cgccgtggaa tagctatgaa gaatttatgg cttactccaa agcgaatccc 420
   ggaaaagtac gcattggtaa ctcaggcacc ggagctatct ggcatctggc ggcagctgca 480
   ctggaagaca aaacgggcac aaagttttct catgtcccgt atgacggcgc agcccctgcc 540
   attacaggcc tgttaggcgg gcatattgaa gcggtttccg taagcccagg agaagttatc 600
   aaccatgtga atggcggcaa gctgaagaca ctggtagtga tggcggatga gcgaatgaaa 660
   accatgcctg acgtcccgac gttaaaagag aaaggcgttg atctctccat cggcacctgg 720
   cgcggcctga ttgtgtcgca aaaaacgccg caggatgtgg tggatgttct ggcaaaggca 780
   gcaaaagaga cggctgaaga gcctgcattc caggatgcac tgcaaaagtt gaatctcaac 840
   tatgcatggc ttgacgctgc cagcttccag acccaaatca gcgaacagga aaagtacttt 900
   gacgagttgc tgactcgcct gggcctgaaa aaa 933
<210> 97
   <211> 2166
   <212> DNA
   <213> Escherichia coli
<400> 97
   atgctgcgat ggaaacgctg tattattcta acatttatct ctggtgctgc tttcgcggcg 60
   ccagagataa atgttaagca aaacgaatcg ttacctgatt taggtagcca ggcagcacaa 120
   caggatgaac aaaccaacaa gggtaaatcg ctgaaagagc gcggagccga ttacgtcatc 180
   aactccgcca cgcaagggtt tgaaaacttg acccctgagg cgctggaatc tcaggccaga 240
   agctatctgc aaagtcaaat cacctcaacc gcacaatctt atattgaaga cacactctct 300
   ccctacggta aggtccgttt gaacctctcc attggtcagg gcggcgatct ggatggcagt 360
   tccatcgatt attttgttcc ctggtacgat aatcaaacca ctgtttattt cagccaattt 420
   tctgcgcaac gaaaagaaga tcgtacgatc gggaatattg gccttggggt aaggtataat 480
   tttgataaat atctattggg tggaaatata ttttatgatt atgactttac ccgtggacat 540
   cgccgtttag gtttaggcgc cgaagcctgg acggattatt taaaattctc aggcaactat 600
   tatcacccac tttctgactg gaaagactct gaagatttcg acttttatga agaacgccct 660
   gcgcgcggtt gggatattcg tgccgaagtc tggttacctt cttatccgca actggggggc 720
   aaaattgtct tcgagcaata ttacggcgat gaagtcgccc tttttggtac ggataatttg 780
   gagaaagatc cctacgcggt aacgcttgga ctgaattatc aaccagtgcc gttactgaca 840
   gttgggacgg actataaagc ggggaccgga gataacagtg atgtcagcat taatgccact 900
   cttaattatc agttcggcgt tccgctaaaa gatcaattgg atagcgataa agtgaaagcg 960
   gcgcactcgc tgatgggcag ccgtcttgat ttcgttgagc gtaataactt tattgttctg 1020
   gaatacaaag aaaaagatcc gcttgatgtc accctgtggt tgaaagcgga tgccaccaac 1080
   gagcaccctg agtgcgtcat taaggacact cccgaagcgg ccgtcggtct ggaaaaatgt 1140
   aagtggacca ttaacgcact cattaatcat cattacaaaa tcgttgcggc ctcctggcag 1200
   gcgaaaaaca atgccgcccg cacgctggtg atgccggtta tcaaagagaa tactctgaca 1260
   gagggtaaca ataaccactg gaacctggtg ctgcctgcct ggcagtacag ttccgatcaa 1320
   gccgaacaag aaaaactcaa tacctggcga gtacgtctgg cgctggaaga tgaaaagggc 1380
   aaccgacaga actctggcgt ggtggaaatc accgttcagc aggaccgtaa aatagagttg 1440
   attgttaata acatcgcgaa cccagaagag aacaaccaca gccacgaagc cagcgcacag 1500
   gcagatggcg ttgatggtgt agtgatggat ctcgatgtaa ccgacagctt tggcgataac 1560
   accgaccgca acggcgatgc gttgccggaa gataacctta cgcctcagct ttacgacgcg 1620
   caggacaaac gagtgacgtt aaccaacaag ccctgctcga ccgataaccc ctgcgttttt 1680
   attgccaaac aagataaaga aaagggcact gtcaccctct ccagtacctt acctggcacc 1740
   tatcgctgga aagcaaaagc cgcgccctac gatgacagta actatgtgga tgtcactttc 1800
   ctcggggcag aaattggtgg gctaaatgct tttatctatc gtgtgggggc ggctaaaccc 1860
   agcaacctga taggtaaaga taaagaaccg ttgccgtcaa caacatttat cgatttgttt 1920
   tatggcgcga caacaataaa gacggtgtct tccagcaggt cgaaaaacct gacgaagaga 1980
   tggtgcagta cgactacaag tgggaattta ccggcaagag catcaatggt aagtgggtgc 2040
   acaggcgaac actccaatga ggacattgtg attccggcca ctaaccgtga agcggcgcaa 2100
   acctatggcg cacaagcggg agatggcttg cagggatacg gtttacgcgt gctgtatacc 2160
   aaaaaa 2166
<210> 98
   <211> 957
   <212> DNA
   <213> Escherichia coli
<400> 98
   atgaagcagg ataaaagacg cggtctgacc cggatcgcat tagcgctggc actggcaggt 60
   tattgtgtgg cacctgtggc gctggctgaa gacagcgcct gggtcgacag cggtgaaacc 120
   aatattttcc aggggaccat tccgtggctc tattcggaag ggggaagtgc tacgacagat 180
   gccgaccgtg taacgttgac ttctgatcta aaaggcgctc gcccgcaagg catgaaacgg 240
   acaagcgttt ttactcgggt gataaatatt ggtgataccg aaggcgacgt ggatcttggt 300
   ggattgggcg ataacgcgaa aactatcgat actatccgct ggatgagcta caaggatgcg 360
   cagggggggg atccaaaaga gctggcaacg aaggtgacca gttacactct taccgatgcc 420
   gaccgtggtc gctatatcgg tattgaaatt acgccaacca cgcagaccgg tacgccaaac 480
   gtcgggactg cgctgcatct ttatgacgtt tctactgcca gcggcggcgg aagcgacagc 540
   gataacgttg caccggggcc ggtggttaac cagaacctga aagtcgccat ctttgttgat 600
   ggtaccagta tcaaccttat caacggtagc acaccaatcg aacttggcaa aacctacgtg 660
   gccaaactgt actcggatga gaacaaaaat ggcaagtttg atgcgggtac cgatgctgac 720
   gtcaccgcca attatgactt ccgttgggta ctttctggca gcagccaaca gcttggcact 780
   tcgggtggca tcgttaactc aagcttcgat aataacaatt tggtcatccc tgcgaccaac 840
   gacgaagcca gaaccaacct taacggccct gcgcgcgatg gaaaagaggc actttccatc 900
   ccgaccaacg gcgacggggt acagggttac aaacttcaca ttatttacaa acacaaa 957
<210> 99
   <211> 1887
   <212> DNA
   <213> Escherichia coli
<400> 99
   atgaagaaag tgctcactct ctcactactg gctctgtgtg tgtctcatag tgcagtagca 60
   gcaaactata cgttcaataa cgataatatt gccctctcgt ttgatgatac aaactcgacg 120
   attgtgctga aggaccgtag aactaaccat ccgatcacac cacaggaatt gttctttctg 180
   acactaccgg atgagacaaa aatccacacc gcagatttca aaatcaagca catcaaaaaa 240
   caggacaatg cgattgtcat cgactttacg cgcccagatt ttaacgtaac agtgcagttg 300
   aaccttgtga agggaaaata tgccagcatc gactacacta ttgccgccgt tgggcaacca 360
   cgagacgtcg ccaagattac cttcttcccg accaaaaaac agtttcaggc tccttacgta 420
   gacggcgcaa tcactagctc accgatcatt gcggactcgt tctttatcct gccgaataaa 480
   ccgatcgtga atacctacgc ctatgaagca acaaccaatc tcaacgtaga actgaaaact 540
   ccaattcagc cagagacgcc ggttagcttt accacctggt tcggtacttt cccggaaacc 600
   agccagttgc gacgcagtgt gaaccagttt attaatgccg tacgtccacg tccgtacaag 660
   ccttatttgc attacaacag ttggatggat atcggctttt tcactccgta caccgaacag 720
   gatgttctgg gacgcatgga cgaatggaac aaggaattca ttagcggccg cggagtggcg 780
   ttagacgctt ttctgctgga cgatggctgg gacgatctta ccggacgctg gttatttggc 840
   ccggcattca gcaacggttt tagcaaagta cgagagaaag ccgatagcct gcacagctcc 900
   gttgggctat ggctttcacc gtgggggggt tacaataagc cgcagcgacg ttcgcgtttc 960
   gcatgcaaaa gagtatgggt tcgaaaccgt ggacggcaag ctggcgcttt cgggagcgaa 1020
   ctacttaaaa acttcaatga gcagatcatt aatcttatca aaaatgaaca cattacctcg 1080
   tttaaactcg acggaatggg gaacgccagt tcacatataa agggtagccc gttcgcctcg 1140
   gattttgatg cgtcaatagc tctgctgcac aatatgcgca gagcaaaccc gaatctattt 1200
   atcaacctga ccaccggcac caacgccagc ccgtcctggt tgttctatgc tgattctatc 1260
   tggcgtcagg gggatgatat aaacctgtat ggccccggca cgccggtgca gcagtggata 1320
   acatatcgtg atgccgagac ataccgctct attgtacgta aaggcccgct attcccgctg 1380
   aactcgctga tgtaccacgg gatagtcagc gccgagaatg cctattacgg gttagagaag 1440
   gtgcaaacgg acagcgactt tgccgatcag gtctggagct acttcgcgac cggcacccag 1500
   ctgcaggagc tgtatattac cccgtccatg ctgaacaagg tgaagtggga tacgctggcg 1560
   aaggctgcaa aatggtcgaa ggaaaatgcc agcgtgctgg ttgataccca ctggattggc 1620
   ggcgacccaa cggcgcttgc cgtgtacggc tgggcatcct ggagcaaaga caaagccatt 1680
   ctcggtttgc gcaacccatc ggataagcca cagacctact atctggattt ggcgaaggat 1740
   ttcgaaatac cggcaggaaa cgcggcgcag tttagtctga aagcggtata cggcagcaat 1800
   aaaacagtgc ccgttgagta taaaaacgcg acggtgatta cgttgcagcc gctggaaacg 1860
   ctggtgtttg aggcggtgac cattaac 1887
<210> 100
   <211> 5334
   <212> DNA
   <213> Escherichia coli
<400> 100
   atgaacaaaa tatttaaagt tatctggaat ccggcaacag gcagttacac cgttgccagc 60
   gaaacggcga agagccgtgg taaaaaaagc gggcgcagta agctgttaat ttctgcactg 120
   gttgcgggtg ggttgttgtc gtcgtttggg gcaagtgcag ataattacac tgggcagcca 180
   actgattatg gcgatggctc agcaggtgac ggctgggttg ctatcggtaa aggggcaaaa 240
   gcaaatacct ttatgaacac tagtggcgcg agtacagctt taggatatga cgcgatagcc 300
   gaaggtgagt acagttctgc catcgggtca aaaacccttg caactggtgg agcatccatg 360
   gcgttcgggg ttagtgcaaa agcaatgggt gacagaagtg tcgcgctagg tgcatcgtca 420
   gtagcaaatg gcgatcgttc gatggctttt ggtcgttacg caaagacgaa tggttttaca 480
   tctcttgcta ttggggactc ctcccttgcc gatggtgaaa aaactattgc gttaggaaat 540
   acggctaaag cttacgaaat tatgagcatc gccctcggtg ataatgccaa tgcgtcaaaa 600
   gagtatgcaa tggcgctggg agcaagtagc aaagctggcg gtgctgatag cctcgcattc 660
   ggcagaaaat ctacagctaa tagcactggc tcactggcaa taggtgctga cagtagcagt 720
   tcgaacgata acgccatcgc gatagggaac aaaacgcaag ccctgggagt gaattcgatg 780
   gccctgggta atgcaagtca ggcatctggc gaatccagta ttgcattagg taacaccagt 840
   gaagccagcg aacaaaatgc gattgcgctg gggcaaggta gcattgcaag caaagtgaac 900
   tcaatcgcgt tgggaagtaa cagtttgtcc tcgggagaga atgccatcgc attgggagag 960
   ggtagtgccg ctggtggcag caacagcctt gctttcggta gccagtccag ggcaaacggc 1020
   aatgattctg tcgccatcgg tgtaggggct gcagcagcga ccgacaattc tgtcgctatc 1080
   ggcgcaggat cgaccacaga tgcaagcaat acggtttcag ttggcaacag cgcaacaaaa 1140
   cgcaaaattg ttaatatggc tgctggtgcc ataagcaaca ccagtaccga tgccatcaac 1200
   ggctcacagc tttatacgat cagtgattca gtcgccaagc gactcggagg aggcgctact 1260
   gtaggcagcg atggcaccgt aaccgcagta agctacgcgt tgagaagcgg aacctataat 1320
   aacgtgggtg atgctctgtc aggaatcgac aataataccc tacaatggaa taaaaccgcg 1380
   ggggcgttca gcgccaatca cggtgcaaat gccaccaaca aaatcactaa tgttgctaaa 1440
   ggtacggttt ctgcaaccag caccgatgta gtaaacggct ctcaattgta cgacctgcag 1500
   caggatgctc tgttgtggaa cggcacagca ttcagtgccg cacacggcac cgaagccacc 1560
   agcaaaatca ctaacgtcac cgctggcaac ctgactgccg gcagcactga cgccgttaac 1620
   ggctctcagc tcaaaaccac caacgacaac gtgacgacca acaccaccaa catcgccact 1680
   aacaccacca atatcaccaa cctgactgac gctgttaacg gtctcggtga cgactccctg 1740
   ctgtggaaca aagcagctgg cgcattcagc gccgcgcacg gcaccgaagc caccagcaaa 1800
   atcaccaacg tcaccgctgg caacctgact gccggtagca ctgacgccgt taacggctcc 1860
   cagctcaaaa ccaccaacga caacgtgacg accaacacca ccaacatcgc cactaacacc 1920
   accaatatca ccaacctgac tgacgctgtt aacggtctcg gtgacgactc cctgctgtgg 1980
   aacaaaacag ctggcgcatt cagcgccgcg cacggcactg acgccaccag caagatcacc 2040
   aacgtcaccg ctggcaacct gactgccggc agcactgacg ccgttaacgg ctcccagctc 2100
   aaaaccacca acgacaacgt gacgaccaac accaccaaca tcgccactaa caccaccaat 2160
   atcaccaacc tgactgacgc tgttaacggt ctcggtgacg actccctgct gtggaacaaa 2220
   acagctggcg cattcagcgc cgcgcacggc actgacgcca ccagcaagat caccaatgtc 2280
   aaagccggtg acctgacagc tggcagcact gacgccgtta acggctctca gctcaaaacc 2340
   accaacgata acgtgtcgac caacaccacc aacatcacca acctgactga cgctgttaac 2400
   ggtctcggtg acgactccct gctgtggaac aaaacagctg gcgcattcag cgccgctcac 2460
   ggcactgacg ccaccagcaa gatcaccaat gtcaaagccg gtgacctgac agctggcagc 2520
   actgacgccg ttaacggctc ccagctcaaa accaccaacg ataacgtgtc gaccaacacc 2580
   accaacatca ctaacctgac ggattccgtt ggcgacctta aggacgattc tctgctgtgg 2640
   aacaaagcgg ctggcgcatt cagcgccgcg cacggtaccg aagctaccag caagatcacc 2700
   aacttactgg ctggcaagat atcttctaac agcactgatg ccattaatgg ctcacaactt 2760
   tatggcgtag cggattcatt tacgtcatat cttggtggtg gtgctgatat cagcgatacg 2820
   ggtgtattaa gtgggccaac ctacactatt ggtggtactg actacactaa cgtcggtgat 2880
   gctctggcag ccattaacac atcatttagc acatcactcg gcgacgccct actttgggat 2940
   gcaaccgcag gcaaattcag cgccaaacac ggcattaata atgctcccag tgtaatcact 3000
   gatgttgcaa acggtgcagt ctcgtccacc agcagcgacg ccattaacgg ttcacaactt 3060
   tatggtgtta gtgactacat tgccgatgct ctgggcggga atgctgtggt gaacactgac 3120
   ggcagtatca ctacaccaac ttatgccatc gctggcggca gttacaacaa cgtcggtgac 3180
   gcgctggaag cgatcgatac cacgctggat gatgctctgc tgtgggatac aacagccaat 3240
   ggcggtaacg gtgcatttag cgccgctcac gggaaagata aaactgccag tgtaatcact 3300
   aacgtcgcta acggtgcagt ctctgccacc agcaacgatg ccattaatgg ctcacagctc 3360
   tatagcacta ataagtacat cgctgatgcg ctgggtggtg atgcagaagt caacgctgac 3420
   ggtactatca ctgcaccgac ttacaccatt gcaaataccg attacaacaa cgtcggtgaa 3480
   gccctggatg cgctcgataa taacgcgctg ctgtgggatg aagacgcagg tgcctacaac 3540
   gccagccatg atggcaatgc cagcaaaatc accaacgttg cggctggtga tctctccaca 3600
   accagtaccg atgctgttaa cggttcccag ttaaacgcaa ccaatattct ggttacgcaa 3660
   aatagccaaa tgattaacca gcttgctggt aacactagcg aaacctacat cgaggaaaac 3720
   ggtgcgggta ttaactatgt acgtaccaac gacagcggct tagcgttcaa cgatgccagc 3780
   gcttcaggta ttggcgctac agctgtaggt tataacgcag ttgcctctca tgccagcagt 3840
   gtagccatcg gtcaggacag catcagcgaa gttgatacgg gtatcgctct gggtagcagt 3900
   tccgtttcca gccgtgtaat agttaaaggg actcgtaaca ccagcgtatc ggaagaaggt 3960
   gttgtgattg gttatgacac cacggatggc gaactgcttg gcgcgttgtc gattggtgat 4020
   gacggtaaat atcgtcaaat catcaacgtc gcggatggtt ctgaagccca tgatgcggtc 4080
   actgttcgcc agttgcaaaa cgccattggt gcagtcgcaa ccacaccaac caaatactat 4140
   cacgccaact caacggctga agactcactg gcagtcggtg aagactcgct ggcaatgggc 4200
   gcgaaaacca tcgttaatgg taatgcgggt attggtatcg gcctgaacac gctggttctg 4260
   gctgatgcga tcaacggtat tgctatcggt tctaacgcac gcgcaaatca tgccgacagc 4320
   attgcaatgg gtaatggttc tcagactacc cgtggtgcgc agaccaacta cactgcctac 4380
   aacatggatg caccgcagaa ctctgtgggt gagttctctg tcggcagtga agacggtcaa 4440
   cgtcagatca ccaacgtcgc agcaggttcg gcggataccg atgcggttaa cgtgggtcag 4500
   ttgaaagtaa cggacgcgca ggtttcccag aatacccaga gcattactaa cctgaacact 4560
   caggtcacta atctggatac tcgcgtgacc aatatcgaaa acggcattgg cgatatcgta 4620
   accaccggta gcactaagta cttcaagacc aacaccgatg gcgcagatgc caacgcgcag 4680
   ggtaaagaca gtgttgcgat tggttctggt tccattgctg ccgctgacaa cagcgtcgca 4740
   ctgggcacgg gttccgtagc agacgaagaa aacaccatct ctgtgggttc ttctaccaac 4800
   cagcgtcgta tcaccaacgt tgctgccggt gttaatgcca ccgatgcggt taacgtttcg 4860
   caactgaagt cttctgaagc aggcggcgtt cgctacgaca ccaaagctga tggctctatc 4920
   gactacagca acatcactct cggtggcggc aatagcggta cgactcgcat cagcaacgtt 4980
   tctgctggcg tgaacaacaa cgacgcagtg aactatgcgc agttgaagca aagtgtgcag 5040
   gaaacgaagc aatacaccga tcagcgcatg gttgagatgg ataacaaact gtccaaaact 5100
   gaaagcaagc tgagtggtgg tatcgcttct gcaatggcaa tgaccggtct gccgcaggct 5160
   tacacgccgg gtgccagcat ggcctctatt ggtggcggta cttacaacgg tgaatcggct 5220
   gttgctttag gtgtgtcgat ggtgagcgcc aatggtcgtt gggtctacaa attacaaggt 5280
   agtaccaata gccagggtga atactccgcc gcactcggtg ccggtattca gtgg 5334
<210> 101
   <211> 681
   <212> DNA
   <213> Escherichia coli
<400> 101
   atgaacctaa agaaaacact gttaagcgtg ttaatgatat tgcaactttg cttattggta 60
   gggtgtgact atattgaaaa agcgagtaag gtcgacgatc tcgttacaca gcaagagttg 120
   caaaaaagca aaattgaggc gcttgaaaaa caacaagaac tcgacaagcg caagatagaa 180
   cactttgaaa aacaacaaac taccatcata aacagtacca aaacgctcgc tggtgtggtg 240
   aaggcagtta aaaacaaaca ggacgaattt gtctttacag aatttaaccc ggcacaaacc 300
   caatacttta ttttaaataa cggctctgtt ggtttggcag ggaaaatact gtctattgac 360
   gcagtagaaa acggcagtgt tattcgtatt tcactggtta acttattaag tgttcctgta 420
   tcaaatatgg gtttctacgc aacatggggg ggagaaaaac ccaccgacat caacgcatta 480
   gcaaaatggc agcaattgct atttagtacc gcaatgaact cctccctgaa attattacca 540
   ggtcaatggc aagacattaa tttgacgcta aaaggtgtct cgcccaacaa cctcaaatat 600
   ctgaaattag ccatcaacat ggcaaatatt cagttcgacc gtcttcaacc tgctgaatct 660
   ccacagcgga aaaacaaaaa a 681
<210> 102
   <211> 3327
   <212> DNA
   <213> Escherichia coli
<400> 102
   atgaaaagag ttgtgcgtct tttgggtgtg gggttactgc tccttgttgt gttgttgctc 60
   attttgtttg ttctggctca gaccacaccg ctgatatcag cacaggatga gcatgctgtc 120
   tggcttcgtc tgttgataac agcgattgtg atctgtttgc taagtatgtg catatttttc 180
   ctcttttctt tccggcagaa cgaagcctcg acgatatcac tatacgctca accgactgat 240
   ataaaggaaa taaatacgga gcagccgaac tatgcatcac tgctgacgat atatttacgc 300
   gaccgctacg gtccgttctg gcggcgtaaa gtccgcctgc tgctggtgac cggcgagcct 360
   gaacaggcag aagccatcgc gccggggctg accgggcaac actggctgga aggcgaccac 420
   acggtgctga tatatggcgg caggccaaca gcggagcctg atgtcacact gctgaccgcc 480
   ttaaaaaaac tgcgccgcag ccgtccgctg gacggcatca tctgggcgct gacagaagaa 540
   cagagccgcc agacagcgca actcgacaaa ggctggcgcg gactgataaa cggcggtaag 600
   cgactcggtt ttcaggctcc actctatttg tggcaggtct gtgacgacgg tgattatcag 660
   accggacgcc ccctgcaaag cgtcggctgc ctgctgccgg aacgctgtac cccggaacaa 720
   ctggctgtaa tgctggaagc agccgctgac ggaacagggc atgtcgcagc tactgaccga    780
   taccgcatgt tttctgctgc gtctggctca tacccttgca gagcggggta ttgctcactg    840
   gcagaccgtc ctgaaaccgc tgctggcagg cggcgcattt tcttccctgc gcctgcgcgg 900
   cctgatgttc agcccgccgc ttgccgccgt gccggaggcc agcacctcat gcagtggctg 960
   ccgtcaccgg tctgggcggg cgtgacggtg ataacgcgcg cgggcgcacg gtgggttttc 1020
   ctgtggctgc gtaccgcact gatgtccgct gtctgcgtgc tggtgatatg gggggccgga 1080
   atgacgacct cgttcttcgc caaccgcgct cttgttcagg aaaccggtat ccagacggca 1140
   cgtgcgcttg atacccgcct gccgctggca gaacaactgg tggcgctgca taccctgcag 1200
   ggcgaactgg aacgcctgca atatcgtatc cgcgaaggtg cgccgtggta tcagcgtttt 1260
   ggccttgaac gtaaccaaca actgctcgcc gccgcttttc ccggctatgc gcaggcggca 1320
   aaccggctgg tgcgcgacgt ggccgttgac catctgcaac agcaactgaa cgcctttgtc 1380
   gccctgccgc ccaacagtcc tcagcgtacc gccaccggtg aacaacgcta taagcagctt 1440
   aaggcattgc tgatgacttc ccgcccggaa aaggccgacg ctgccttttt cagtaccacg 1500
   ctgatggcgg acggtctgcg ctacgagaat atcccggaag gtgtgcggca gagcgtgttg 1560
   ccgtcactgc tgaccttctg gacggcgaac ctgccggaac acccgcagtg gaaaacatcg 1620
   ccgccaccgg aactgaccgg cgcagtgcgt aaaatcctgc tgcgccagat tggtgtgcgt 1680
   aatgccgaaa acaccctcta ccagaacgtg ctgcaacagg tgtcccgcaa ctacgccgat 1740
   atgacgctgg cggacatgac cggggatacc ctcaccgaat ctcttttcag tacggaacag 1800
   acggtgccgg ggatgttcac ccgtcaggcg tgggaaggac aggtcaggga agccatcgag 1860
   caggtggtga cggcgcggcg cgaggaaatc gactgggtac tcagcgaccg ccagcaggat 1920
   acctctgcgg atatctcgcc ggatacgctg cgtaaccgtc tcacctcacg ctactttacc 1980
   gactttgccg gaagctggct ggcgtttctc aacagcattc actggaaaaa ggaagactcg 2040
   ctctccggca ttctcgacca gctgacactg atggccgatg cccgtcagtc gccactgatt 2100
   gcgctgacgg acaccctcgc gtggcaggcg gcgacaggca gggaaaaccg tggtctgtca 2160
   gactcgctgg cgaaatcggc acaggaactg tttaacggca aggagaaaac gccgcagcaa 2220
   tcccgtgaag gtgacgacgt gcctgtcggg ccgctggata aaaccttcac gccgctgctg 2280
   cgtttgctgg gcgataaggc cggaggcggc gacagccagc tgagtctaca gacctacctc 2340
   acccgcgtca cccgcgtgcg cctcaaactg caacaggtga ccaacgcccc cgacccgcag 2400
   gagatgaccc aacaactggc gcagacggtc ttacagggta aaaccgttga cctcaccgac 2460
   acccgcgact acggacggtt aatcgccgcc agtctgggcg aagaatggag tggcttcggt 2520
   caggcgctgt tcgttcgccc ggtagagcag tcgtggcggc aggtgctgac gcctgcggcg 2580
   gacagcctga accgccagtg gcagcgggcg attgtcagcc actggaatca ggacttcgct 2640
   ggccgctatc cgttcaaagc ctcacagaac gatgcctccc tccccctgct ggcgcagtac 2700
   ctgcgcgatg acgggcgcat caacctgttt atcgccgcca acctttccgg cgtgctgaaa 2760
   cgagagggcc gctactgggt ggctgacgcc atgaacacgc aggggctgac ggtcaatccg 2820
   gactttatcc gcgccctgaa ccgcctgcgc gacgtggccg ataccgcctt tgccagcggc 2880
   gatgccggga tacattttga actgcgggca aaaccggcgc gtgacgtgat gaagacgcat 2940
   ctggtgattg acgggcagga gctggaatat ttcaaccaga aagaacgctg gcagcgtttt 3000
   aactggccgg atgaacagtg gcaacccggc gcatcgctaa gctggaccag cacacaggcg 3060
   atggagcgca tactggcgga ttaccgggga agctggagtc ttattcgcct gctggaacag 3120
   gcgcaggtga cgccggtgga cagcagcacc tttaaggtgg tgtggaaagc gcaggacggc 3180
   ctgccgctga attacctgct acgggttgaa cagggtaaag ggccgctggc gctgctggag 3240
   ctgaaaaact tccgcctgcc gggacaggtg tttctgaccg gaaaaagtat gaaggatgtg 3300
   gaagagtatg gggaagacgc cgatgag 3327
<210> 103
   <211> 534
   <212> DNA
   <213> Escherichia coli
<400> 103
   atgtttccta ttcgttttaa acgtccggcg ttgctctgta tggcgatgct gacggttgtt 60
   ctgagtggct gcggcctgat tcagaaagtg gtggatgaat cgaaaagcgt ggcctcagcc 120
   gttttctaca aacaaatcaa aatactgcat ctcgatttct tctcccgcag cgccctgaat 180
   acggatgcgg aagatacgcc gctttccacg atggtgcatg tctggcaact gaaaacccgc 240
   gaagattttg acaaggcgga ttacgacacc ctgtttatgc aggaagagaa gacgctggag 300
   aaggacgtac tggcaaaaca caccgtctgg gtaaaaccgg aaggcacggc atccctgaat 360
   gtgccgctgg ataaagagac gcagtttgtc gccattattg ggcagtttta tcaccctgat 420
   gaaaaaagcg acagctggcg tctggtgatc aaaagggacg aactggaggc cgacaagccg 480
   cgctcgattg aactgatgag aagcgacctg cgactgctgc ctctcaagga taaa 534
<210> 104
   <211> 840
   <212> DNA
   <213> Escherichia coli
<400> 104
   atgatttcag ggggaaatat gttgaaagaa tggatgatat ttacgtgcag tttattgact 60
   ctggctgggg cgtcactgcc cctcagtggc tgtatttcca gaggccagga gtctatatcc 120
   gaaggggcgg catttggggc agggatcctg cgcgaaccgg gagcaacaaa aaaagccgac 180
   acgaaagacc tcaatgtgcc accaccggtt tatggtccgc cgcaggtgat atttcgcatt 240
   gatgacaacc gctatttcac gctagaaaat tatacccact gcgagaacgg gcagacgttt 300
   tataataata aagcaaaaaa cattcatgtt aaaatattag acgcttcagg gtatttattt 360
   aaaggccgct tattctggtt atcaacgcgt gatgattttc tggcctttcc tgccacgtta 420
   aataccagac acgcttcctg tatggggtcg aataaaggct gtatgaatgc ggtcattgtc 480
   actaccgatg gtggaaaaag acgcagtggt gtgccatacg gcagttatac ccagaatccg 540
   accggtgcca cgagggatta tgacatgctg gtgatgaatg acggcttcta cctgcttaga 600
   tatcgggggg gacagggcag atttagtccg gtgatactta gatggattct cagtactgaa 660
   gatagctctg gtgttgtgcg ttcagaagat gcttatgaat tgttccgtcc cggagaagag 720
   gtaccctcca ccggttttta taaaatcgac ctgtcacgtt tttatcccaa aaacaacgtt 780
   atggaaatgc agtgtgacag gacgctggag ccagttcaac cttcagagag taaaattcaa 840
<210> 105
   <211> 1503
   <212> DNA
   <213> Escherichia coli
<400> 105
   atggaacacg ttagcattaa aacattatat catctcctgt gctgtatgct gctctttatt     60
   tccgctatgt gcgctttggc gcaagaacat gagcctatcg gggcgcaaga tgagcgcctg    120
   tcgacattaa ttcaccaacg gatgcaggag gccaaggtcc cagccctttc cgtaagtgtg    180
   accattaagg gggtacgtca gcgatttgtc tacggtgttg ccgatgtggc tagtcagaaa    240
   gcgaatactc tagacacagt ttacgagctg ggatcgatga gtaaggcgtt taccggactt    300
   gtggtgcaaa tactgattca ggaaggcaga ctccggcaag gggatgatat cattacctat    360
   ctgccggaaa tgcgcttgaa ttatcaggga aaacctgctt ccctgaccgt ggctgatttc    420
   ctttatcata catcaggatt gcctttttca acactggctc ggctggaaaa ccctatgcct    480
   gggagcgctg tggcacagca actgcgcaac gagaatctgc tgtttgcgcc gggtgcgaag    540
   tttagctatg cctccgccaa ttatgatgtg ttgggcgcgg tgattgaaaa tgtgacggga    600
   aaaaccttta cagaggtcat tgcggaacga ctcacgcagc cgctgggcat gtcggcgact    660
   gtggcagtta agggggatga gattattgtc aacaaggcaa gcggctataa actgggattc    720
   ggcaaacccg ttctgtttca tgcgcctctg gcccggaacc atgttcctgc cgcctatatc    780
   catagcactc tgcctgatat ggaaatatgg atagacgcct ggttgcacag aaaggctttg    840
   ccggcaacgc tgcgtgaggc gatgagtaac agttggcgtg gtaatagtga tgttccgctt    900
   gccgcagaca atcgtatcct ctatgccagc ggttggttta tcgaccagaa tcaaggccct    960
   tacatcagtc acggtgggca gaatccaaac ttttcttctt gcattgcgtt gcgaccggat   1020
   cagcagattg gcattgttgc gctggcaaat atgaattcga atctgatact acagctttgc   1080
   gcggatatcg ataattatct gcgcattggc aaatatgctg acggcgctgg tgatgcaatt   1140
   acagccaccg ataccctttt cgtctacctc acgttgttgc tgtgtttttg gggggcggtg   1200
   gttgtagtgc gcggtgcttt ccgtgtttat cgcgcaacgg cgcatggccc tggaaaacag   1260
   cagaggttac gtttacgcgt acgtgactat atcatcgcct tggcggttcc tgggctcgtg   1320
   gccgccatgc tctatgtcgc accgggtata ctatctccag gacttgactg gcgttttatc   1380
   ttggtatggg gtccatcgag cgtgttggcg ataccgttcg gaattatcct gttagctttc   1440
   gttctgacat taaatcatca aattaaacga attctattac acaacaagga gtgggacgat   1500
   gag                                                                 1503
<210> 106
   <211> 2046
   <212> DNA
   <213> Escherichia coli
<400> 106
   atgaagaaca aatatatcat tgctccgggc attgccgtga tgtgttctgc agttatatca 60
   tcaggttatg ccagttctga taaaaaagaa gatacgcttg ttgttactgc ctccgggttc 120
   actcagcagc tcagaaatgc cccggccagt gtctcagtca ttacttcaga acaactgcaa 180
   aaaaaaccgg tttcagatct ggtcgatgca gtaaaagatg ttgaagggat tagtatcact 240
   ggtgggaatg aaaaaccgga tatcagtata cgtggtctaa gtggcgatta cacgctgatt 300
   ctggtcgatg gacgacgtca gagcggtcgg gaatccagac caaacggcag cggcggtttt 360
   gaagccggat ttatccctcc tgtggaagca attgaacgca ttgaagtgat ccgtggccct 420
   atgtcttccc tgtatggttc tgatgccatc ggaggggtca ttaatatcat aaccaaacca 480
   gttaataacc aaacatggga tggcgtactt ggacttgggg ggattattca ggaacatggg 540
   aaatttggta actcaaccac aaatgacttc tatctgtcag gcccattgat taaggataaa 600
   cttggtcttc agctatatgg aggaatgaac tatcgcaagg aagatagtat ctctcaggga 660
   acaccggcaa aagataataa gaatataacg gcaacgctcc agtttactcc gactgaaagc 720
   cagaagtttg tttttgaata tggaaaaaat aaccaggtgc atacattaac acctggtgag 780
   tctctcgatg cctggactat gcggggaaat cttaaacaac caaacagtaa aagagaaacg 840
   cataattcac gtagtcactg ggtagcagca tggaatgccc agggcgaaat actgcatcct 900
   gaaattgctg tttatcagga gaaagttatt cgtgaggtta aatcaggtaa aaaagataaa 960
   tataatcatt gggatcttaa ttacgagtca agaaaaccgg aaataaccaa cacaatcata 1020
   gatgcaaaag tgacggcatt tctgccggaa aatgtactga ccatcggagg tcaatttcag 1080
   catgcagagc tccgtgatga ctcagccacg ggtaaaaaaa cgacagaaac acagtctgtt 1140
   tcaattaaac agaaagctgt ttttatagaa aatgaatatg cagcaacgga ttctctcgcc 1200
   ctgactggag gactgcgtct cgataatcat gaaatctatg gcagttactg gaatccaaga 1260
   ttgtacgctg tttataacct gaccgataat ctcacactca aaggggggat cgcaaaagca 1320
   tttcgggctc cttcaattcg tgaggtgagt cctggatttg gaacactgac gcagggtggt 1380
   gcctctatta tgtatggaaa cagggacctg aaaccggaga ccagtgtaac cgaagagatc 1440
   ggtattattt atagtaatga tagtggtttt tcggcgagcg cgacgctgtt taatactgat 1500
   tttaaaaata agttgaccag ttacgatata ggtacaaaag atccagtcac cgggttaaac 1560
   acttttattt atgataatgt aggtgaggca aatatcagag gggtggagct tgcaactcag 1620
   attcctgtgt atgataaatg gcatgtatct gcaaactata catttactga ctctcgtcga 1680
   aaaagtgatg acgaaagtct caatggcaag tcgctgaaag gggaacctct ggaaagaact 1740
   cccagacatg cagccaatgc aaaactggaa tgggattaca ctcaggatat tacattttat 1800
   tcatctctga attatacggg aaaacaaatc tgggcagcac aaagaaatgg tgctaaggtt 1860
   ccccgcgttc gtaatggatt cacatctatg gatattggtc taaattacca gattctgcca 1920
   gacacgctga ttaattttgc cgttcttaac gtcacagaca gaaagagcga ggatatcgat 1980
   accattgatg gtaactggca ggtcgatgaa ggacgccgtt attgggctaa tgtaagagta 2040
   tccttc 2046
<210> 107
   <211> 492
   <212> DNA
   <213> Escherichia coli
<400> 107
   atggggttta gaaaaacaat aatcacttcg gtaggtttga tatttatttc attctctttt 60
   gtggcaaagt gctctcaact caaaaatttg aataattact cagtgatgct ttgtggaaaa 120
   gtgtcaaata atatcctgga tgatattggt ggttataaag aaagaaatat attaatgctg 180
   cgagctataa aaaaaatcat aataatgaca atcgtaaata ttatattttt ctattccttt 240
   caatcgactg cggatgaaat ggttttaata aaaaaatacg ggtttgggct tgagagagat 300
   atcaaaggaa ggccattaat ttatcctatc gaaaattatg atgagtgtaa gaaaaaatgc 360
   aatcatatga attatatagc ggatgtcaat gctcaattag ctatgagtaa aaaaaataac 420
   aggatttttg ctaacataac ctttactaac aatagctcta ccacgtattt ttttctaaat 480
   attatctacc ta 492
<210> 108
   <211> 654
   <212> DNA
   <213> Escherichia coli
<400> 108
   atgaatcaaa ttaaagataa taaggtaatt atgaaaataa aaaatttaat atcagtcatt 60
   ttactatcag gaggtattat ggggactgga ttgtactcga gcgataacca tcaaaaaatc 120
   cgcagcaggt ttaatataca ggaatcatat tgtgccatta agactaatgg tgtccttgga 180
   ttcagcaacc gaaaggatgt attgcgagaa aatggtgatt caaccggaac caccagttcc 240
   agcactaatg ccatgatgct gatggaaaat ggtgaaaatg aaatcagtct ggagattggg 300
   gcgttaaggt ggttttctga taaacctgcc agtaccgaag aacgagggca tttctcccaa 360
   aaagcagggt gcagtctgga tttggttcgt tttgttaagc aggaagaaac catactttct 420
   tcgataaagg tgaccatcaa ccagcaggga atacctgaag cgcagccaga cagcatgcat 480
   cctgttatcc gaaaagagat tctggctgag caggcagaac ccggatttat tgatccagac 540
   tattttaatg aaacttattt cccgaaaggg atgaaggtgt atcaatttac acaaaaggtc 600
   tcggtggcgg ggcttcctga tgggcctgga cgcagtacgc cctttaccgg agca 654
<210> 109
   <211> 8198
   <212> DNA
   <213> Escherichia coli
<400> 109
   atgcatcagc ctcccgttcg cttcacttac cgcctgctga gttacctggt cagtgcgatt 60
   atcgccgggc agccgttgtt accggctgtg ggggccgtca tcaccccaca aaacggggcc 120
   ggaatggata aagcggcaaa tggtgtgccg gtcgtgaaca ttgccacgcc gaacggggcc 180
   gggatttcgc ataaccggtt tacggattac aacgtcggga aggaagggct gattctcaat 240
   aatgccaccg gtaagcttaa tccgacgcag cttggtggac tgatacagaa taacccgaac 300
   ctgaaagcgg gcggggaagc gaagggtatc atcaacgaag tgaccggcgg taagcgttca 360
   ctgctgcagg gctatacgga agtggccggc aaagcggcga atgtgatggt tgccaacccg 420
   tatggtatca cctgtgacgg ctgtggcttt atcaacacgc cgcacgcgac gctcaccacg 480
   ggcaaacctg tgatgaatgc cgacggcagc ctgcaggcgc tggaggtgac tgaaggcagt 540
   atcaccatca atggcgcggg cctggacggc acccggagcg atgccgtatc cattattgcc 600
   cgtgcaacgg aagtgaatgc cgcgcttcat gcgaaggatt taactgtcac tgcaggcgct 660
   aaccgtgtaa ctgcagatgg tcgtgtcaga gccctgaagg gcgaaggtga tgtgccgaaa 720
   gttgccgttg ataccggcgc tctcggtgga atgtacgcca ggcgtattca tctgacctcc 780
   actgaaagtg gtgtcggggt taatcttggt aacctttatg cccgcgatgg cgatatcacc 840
   ctggatgcca gcggcagact gactgtcaac aacagtctcg ccacgggggc cgtcactgca 900
   aaaggtcagg gcgtcacctt aaccggcgac cataaagcgg gaggtaacct gagcgtcagc 960
   agccggagag atatcgttct cagcaatgga acgcttaaca gcgacaagga cctcagcctg 1020
   accgccggcg gcagaatcac tcaacagaat gaaaaactga ctgccggccg ggatgtaacg 1080
   cttgccgcga aaaacatcac acaggatacc gccagccaga ttaacgcggc ccgcgatatc 1140
   gtgactgtcg ccagtgacac gctgacaaca cagggacaga taaccgccgg gcagaatctc 1200
   acggccagcg ccaccacgct gacgcaggac ggaatattgc tggcgaaaag tcatgcggga 1260
   ctcaatgccg gtacgctgaa taacagtggc gccgttcagg gagctaccct gacgctcggc 1320
   agtacaacgc tcagcaacag tggctccctg ctcagtggcg gtcccctgac catgaatacc 1380
   cgcgacttta cccagagcgg ccgcactggc gcgaagggca aagtggatat catggccagt 1440
   gggaaactga ccagtacagg tttgctggtg acgatgcact tggtgctgaa ggcgcaggat 1500
   gtgacacaga acggtgtgct gtccggcggc aaagggctga cggtcagtgc gacgagctcc 1560
   ggtaaaaaat cggtcaccca cagcgatgct gcgatgacgc tgaatgtgac aacagtggcg 1620
   ctggacgggg aaaccagtgc cggtgacacc ctccgggttc aggcagacaa actgagtacc 1680
   gcagcgggcg cacaacttca gagcggcaaa aatctcagca tcaacgccag agatgcacgt 1740
   cttgcaggta cgcaggcagc acaacagacc atggtggtga acgccagtga aaagctcacc 1800
   cacagcggga aaagcagtgc cccgtcgctc agcctcagtg cgccggaact gaccagcagc 1860
   ggcgtacttg ttggttccgc cctgaataca cagtcacaga ccctgaccaa cagcggtctg 1920
   ttgcaggggg aggcctcact caccgttaac acacagaggc ttgataatca gcagaacggc 1980
   acgctgtaca gtgctgcaga cctgacgctg gatataccgg acatccgcaa cagcgggctt 2040
   atcaccggtg ataatggttt aatgttaaat gctgtctccc tcagcaatcc gggaaaaatc 2100
   atcgctgaca cgctgagcgt cagggcgacc acgctggatg gtgacggcct gttgcagggc 2160
   gccggtgcac tggcgcttgc tggcgacacc ctctcacagg gtagtcacgg acgctggctg 2220
   acggcggacg acctctccct ccggggcaaa acactgaata ccgcagggac cacgcaggga 2280
   cagaatatca ccgtgcaggc ggacagatgg gcgaacagtg gttccgtgct ggcaaccggt 2340
   aaccttactg cttcggcaac cggtcagttg accagtaccg gcgatatcat gagccagggt 2400
   gacaccacgc tgaaagcagc caccacggac aaccggggca gtctgctttc ggccggcacg 2460
   ctctcccttg atggaaattc actggataac cgcggcactg tccagggtaa ccatgtcacg 2520
   attcgccaga acagtgtcac caacagtggc acgctcaccg ggatcgccgc actgacgctt 2580
   gccgcccgta tggcatcccc tcaacctgcg ctgatgaata acggaggttc attgctgacc 2640
   agcggcgatc tgacaatcac cgcaggcagt attaccagtt ccggacactg gcagggcaaa 2700
   cgggtgctga tcaccgcaga cagtctggca aacagcgggg cgatccaggc ggctgacagc 2760
   ctgactgcac gtctgacggg tgagctcgtc agcacagcgg gcagcaaagt cacctcgaac 2820
   ggtgaaatgg cgctcagtgc actgaattta agcaacagcg gacaatggat tgcaaaaaat 2880
   ctgaccctga aggcgaactc actgaccagt gcgggtgaca tcaccggtgt ggatactctc 2940
   acgctcacgg tgaatcagac gctgaacaat caggcgaacg gaaaactgct cagtgcaggt 3000
   gtgctgacgc tgaaggcaga cagtgtcaca aacgacgggc aattacaggg aaatgtcacc 3060
   accatcacgg caggacaact cacaaacggc gggcatctgc agggcgaaac gctgacgctg 3120
   acagcctccg gtggcgtgaa caaccgttcc ggtggtgttc tgatgagccg gaatgcactg 3180
   aatgtcagta ctgcgaccct gagtaaccag agcacgatac agggtggagg cggggtttcc 3240
   ctgaacgcca cagaccgtct gcagaacgac ggcaaaatcc tctccggcag taacctcacg 3300
   ctgacggcgc aggtgctggc gaacaccggc agcggactgg tacaggctgc caccctgctg 3360
   ctggatgtgg tgaatactgt caacggcgga cgcgtacttg ccaccggcag tgacgttaaa 3420
   ggaaccacgc tgaataatac cggtacgctt cagggtgcga ctctggtgaa ttaccacaca 3480
   ttcagcagcg gtaccctgct gggaacctcc gggcttggcg tcaagggcag ttcactgctg 3540
   caaaatggta cagggcggct gtacagtgca ggcaacctgc tgcttgacgc tcaggacttc 3600
   agtggtcagg ggcaggtggt ggccaccggt gatgtcacac tgaaactgat tgctgccctc 3660
   acgaatcatg gtaccctggc cgcagggaaa accctttccg tcacgtcgca aaatgccatc 3720
   accaacggcg gtgtcatgca gggtgatgcc atggtgctcg gtgccggaga ggcattcacc 3780
   aacaatggac tgactgccgg taaaggcaac agtgttttca gcgcacagcg tcttttcctt 3840
   aacgcaccgg gttcacttca gggcggtggc gatgtgagtc tgaacagccg gagtgatatc 3900
   accatcagtg gttttaccgg cacggcaggc agtctgacaa tgaatgtggc cggtaccctg 3960
   ctgaacagtg cgctgattta tgcggggaat aacctgaagc tgtttacaga ccgtctgcat 4020
   aaccagcatg gtgatatcct ggccggcaac agtctgtggg tacagaagga tgcttccggc 4080
   ggtgcaaaca cagagattat caataattcc gggaatattg agacgcatca gggcgatatt 4140
   gttgtaagaa ccgggcatct tctgaaccag cgggagggat tttctgccac aacaacaacc 4200
   cggactaacc cctcatccat tcagggaatg ggaaatgctc tggttgatat tcccctttcc 4260
   cttcttcctg acggcagcta tggctatttc acccgtgaag ttgaaaatca gcacggtacg 4320
   ccctgcaacg ggcacggggc atgcaatatc acaatggata cgctttatta ttacgcgccg 4380
   tttgctgaca gtgccacaca gcgctttctc agcagccaga acatcacaac agtaaccggt 4440
   gctgataatc cggcaggccg cattgcgtca gggcgtaatc tttctgctga ggctgaacga 4500
   ctggaaaacc gggcgtcatt tatcctggcg aatggggata tcgcactctc gggcagagag 4560
   ttaagcaatc agagctggca gacggggaca gagaatgaat atctggtata ccgctacgac 4620
   ccgaaaacgt tttacggtag ctatgcaaca ggctctctgg ataaactgcc cctgctgtca 4680
   ccggaatttg aaaacaatac catcagattt tcactggatg gccgggaaaa agattacacg 4740
   cccggtaaga cgtattattc cgttattcag gcgggcgggg atgttaagac ccgttttacc 4800
   agcagtatca ataacggaac aaccactgca catgcaggta gtgtcagtcc ggtggtctct 4860
   gcacctgtac tgaatacgtt aagtcagcag accggcggag acagtctgac acagacagcg 4920
   ctgcagcagt atgagccggt ggtggttggc tctccgcaat ggcacgatga actggcaggt 4980
   gccctgaaaa atattgccgg aggttcgcca ctgaccggtc agaccggtat cagtgatgac 5040
   tggccactgc cttccggcaa caatggatac ctggttccgt ccacggaccc ggacagtccg 5100
   tatctgatta cggtgaaccc gaaactggat ggtctcggac aggtggacag ccatttgttt 5160
   gccggactgt atgagcttct tggagcgaag ccgggtcagg cgccacgtga aacggctccg 5220
   tcgtataccg atgaaaaaca gtttctgggc tcatcgtatt ttcttgaccg cctcgggctg 5280
   aaaccggaaa aagattatcg tttcctgggg gatgcggtct ttgatacccg gtatgtcagt 5340
   aacgcggtgc tgagccggac gggttcacgt tatctcaacg gactgggttc agacacggaa 5400
   cagatgcggt atctgatgga taacgcggcc agacaacaga aaggactggg attagagttt 5460
   ggtgtggcgc tgacagctga acagattgct cagcttgacg gcagcatact gtggtgggag 5520
   tcagtcacca tcaacggaca aacagtcatg gtcccgaaac tgtatctgtc gccggaagat 5580
   atcaccctgc ataacggcag cgttatcagc gggaacaacg tgcaacttgc gggcggcaat 5640
   atcaccaaca gcggcggcag catcaacgca cagaacgacc tctcgctcga cagttccggc 5700
   tatatcgaca acctgaatgc ggggctgata agcgcgggcg gtagcctgga cctgagcgcc 5760
   atcggggata tcagcaatat cagctcagtc atcagcggta aaaccgtaca actggaaagc 5820
   gtgagtggca acatcagcaa tatcacccgg cgtcagcaat ggaatgcggg cagtgacagc 5880
   caatatggtg gtgtgcatct cagcggtacg gacaccggtc cggttgcgac cattaaaggc 5940
   actgattcac tttcgctgga tgcagggaaa aacattgata ttaccggggc aacggtctcg 6000
   tccggtggag accttggaat gtctgcgggt aatgatatca acattgccgc aaacctgata 6060
   agtgggagca aaagtcagtc cggtttctgg cacactgatg acaacagttc atcatccacc 6120
   acctcacagg gcagcagcat cagcgccggc ggtaacctgg cgatggctgc aggccataat 6180
   ctggatgtca cggcatcctc tgtttctgcc gggcacagcg ccctgctttc ttgcaggtca 6240
   cgacctagtc ttgaatgcag tcagggaaaa gcaaaaacaa gtcgcaacgg caggtcagaa 6300
   agtcatgaaa gccacgcagc tgtgtccacg gtgacagcgg gcgataactt cctccttgtt 6360
   gccggtcgtg atattgccag tcaggctgcc ggtatggctg cggaaaataa cgtggtcatc 6420
   cggggcggac gtgatgtgaa cctggtggca gagtctgccg gcgcaggcga cagctatacg 6480
   tcgaagaaaa agaaagagat taacgagaca gtccgtcagc agggaacgga aatcgccagc 6540
   ggtggtgaca ccaccgtcaa cgcaggacgg gatatcaccg ctgttgcgtc atccgttacc 6600
   gcaaccggca atatcagcgt gaatgccggt cgtgatgttg ccctgaccac ggcgacagaa 6660
   agtgactatc actatctgga aacgaagaaa aaaagcggag gttttctcag taagaaaacc 6720
   acccgcacca tcagtgagga cagtgccacc cgtgaagcag gctccctgct gtcggggaac 6780
   cgcgtgaccg ttaacgccgg tgataacctg acggtagagg gttcggatgt ggtggctgac 6840
   cgggatgtgt cactggcggc gggtaaccat gttgatgttc ttgctgccac cagtacagat 6900
   acgtcctggc gctttaagga aacgaagaaa tccggtctga tgggtaccgg cggtattggt 6960
   ttcaccattg gcagcagtaa gacaacgcac gaccggcgcg aggccgggac aacgcagagt 7020
   cagagtgcca gcaccatcgg ctccactgcc ggtaatgtca gtattaccgc gggcaaacag 7080
   gctcatatca gcggttcgga tgtgattgcg aaccgggata tcagcattac cggtgacagt 7140
   gtggtggttg acccggggca tgaccgtcgt actgtggacg aaaaatttga gcagaagaaa 7200
   agcgggctga cggttgccct ttccggcacg gtgggcagtg ccatcaataa tgcggttacc 7260
   agtgcacagg agacgaagga gagcagtgac agccgtctga aagccctgca ggccacaaag 7320
   acagcgctgt ctggtgtgca ggccggacag gctgcgacaa tggcctccgc aaccggtgac 7380
   ccgaatgcgg gagtcagcct gtcgctcacc acccagaaat cgaaatcaca acaacattct 7440
   gaaagtgaca cagtatccgg cagtacgctg aatgccggga ataatctgtc tgttgtcgca 7500
   accggcaaaa acaggggcga taaccgcgga gatattgtga ttgcaggaag ccagcttaag 7560
   gccggtggta acacaagcct ggatgccgcg aatgatattc tgttgagtgg cgccgcaaac 7620
   acacaaaaaa caacgggcag gaacagcagc agtggcggtg gcgtgggtgt cagtatcggt 7680
   gcaggtaaag gtgccggtat cagcgccttt gccagcgtta atgcggcaaa aggcagggag 7740
   aaaggtaacg gtactactac cgacaaaacc gtcaccatca acagtggtcg ggatacggta 7800
   ctgaacggtg ctcaggtcaa cggcaacagg attatcgccg atgtgggcca cgacctgctg 7860
   ataagcagcc agcaggacac cagtaagtac gacagtaaac agaccagcgt ggctgccggc 7920
   ggcagtttta cctttggctc catgaccggc tcaggttaca tcgctgcctc ccgggataag 7980
   atgaagagcc gctttgactc cgttgctgaa caaaccggaa tgtttgcccg ggtgatggtg 8040
   gcttcgacat cacagtgggt aaacataccc aactggatgg tgcggtcatt gcctcactgc 8100
   cacaccggag aaaaaccacc tggataccgg acgctgggtt tagtgacttt acaacgaagc 8160
   gggattataa agtcaagtca caggtggaat cagtctga 8198
<210> 110
   <211> 963
   <212> DNA
   <213> Escherichia coli
<400> 110
   atgatgttga agaaaacgat atttatatta acgttattct ctggcaacgt aattgctgca 60
   actgtagaat taggttttga aaatgagcaa tataattatg cttatcgttc tgcagatgtc 120
   ttcatgccgt atattaagag taatttcaac cctgttactg attctgcttt gaatgtgtca 180
   ctcacctata tgtatcagga tcaatatggg aaaaaacata aaaaaacatc tgaggacaga 240
   tttaaaacca atcgcgatcg catagagctc tatcttaaag gttatacttt aaatagggga 300
   gcatattctt tttctccttc cgcaggtttc cgttatgagt catgggatgt aaactacgat 360
   aatccgaaaa agcaggataa gtggaaactg gaactacgct tttatcctaa tatgacttat 420
   aaactcaatg accagttaag cctatatatg aatggttttg ttgcccctgt attttttaaa 480
   acacaacaag agtcgagaaa agataacaat tatgtaaagg gtaagttagg ggcgaaacgt 540
   tataacaacg attattatca ggaactccag attctgggtg tcagatataa atttaataat 600
   gataatacgc tctgggcatc agtctataat gaaagaaaat ataatcaaca ttcctcaaaa 660
   tatgatcgct ggcaattgcg tggaggctat gattttaaag ttacagagga gtttgttttg 720
   agtccattca taagatatga cctctcttat agagaaaaaa acctcgaaag cacaagtaat 780
   aatggtttat caaaaaataa taaagaaatt cgaactggag ccagcttttc ctataaaatt 840
   atcccttctg taaaactggt aggagaaata tacaggcaaa caaccaacat tgaaaactat 900
   tatggagagc attctgaaga caaaaaccgc atgttctaca aacttggtat aaacaaaaca 960
   ttt 963
<210> 111
   <211> 1761
   <212> DNA
   <213> Escherichia coli
<400> 111
   atgcagcacc ggcagaaaaa cattctgacg aaaacgtccc ttttatcccg tgcgttgtct 60
   gtcccctgtt gtgatatgtt ccggcgcggc tctccgtgga tatgctatct ctccctctcc 120
   gttttttctg gttgtttcat ccccgcattt tcgtctccgg cagccatgct gtctccgggt 180
   gaccgcagtg caattcagca gcaacagcag cagttgctgg atgaaaacca gcgtcagcgt 240
   gatgcgctgg agcgcccgct gaccatcacg ccgtctccgg aaacgtctgc cggtactgaa 300
   ggtccctgct ttacggtgtc aagcattgtt gtcagtgggg ccacccgact gacgtctgca 360
   gaaaccgaca gactggtgcc gtgggtgaat cagtgtctga atatcacggg gctgaccgcg 420
   gtcacggatg ccgtgacgga cggctatata cgccggggat atatcaccag ccgggccttt 480
   ctgacagagc aggacctttc agggggcgta ctgcacataa cggtcatgga aggcaggctg 540
   cagcaaatcc gggcggaagg cgctgacctt cctgcccgca ccctgaagat ggttttcccg 600
   ggaatggagg ggaaggttct gaacctgcgg gatattgagc aggggatgga gcagattaat 660
   cgtctgcgta cggagccggt acagattgaa atatcgcccg gtgaccgtga gggatggtcg 720
   gtggtgacac tgacggcatt gccggaatgg cctgtcacag ggagcgtggg catcgacaac 780
   agcgggcaga agagtaccgg tacggggcag ttaaatggtg tcctttcctt taataatcct 840
   ctggggctgg ctgacaactg gtttgtcagc gggggacgga gcagtgactt ttcggtgtca 900
   catgatgcga ggaattttgc cgccggtgtc agtctgccgt atggctatac cctggtggat 960
   tacacgtatt catggagtga ctacctcagc accattgata accggggctg gcggtggcgt 1020
   tccacgggag acctgcagac tcaccggctg ggactgtcgc atgtcctgtt ccgtaacggg 1080
   gacatgaaga cagcactgac cggaggtctg cagcaccgca ttattcacaa ttatctggat 1140
   gatgttctgc ttcagggcag cagccgtaaa ctcacttcat tttctgtcgg gctgaatcac 1200
   acccacaagt ttctgggggg ggtcggaaca ctgaatccgg tattcacacg ggggatgccc 1260
   tggttcggcg cagaaagcga ccacgggaaa aggggagacc tgcccgtaaa tcagttccgg 1320
   aaatggtcgg tgagtgccag ttttcagcgc cccgtcacgg acagggtgtg gtggctgacc 1380
   agcgcttatg cccagtggtc accggaccgt cttcatggtg tggaacaact gagcctcggg 1440
   ggtgagagtt cagtgcgtgg ctttaaggat cagtatatct ccggtaataa cggcggttat 1500
   ctgcggaatg agctgtcctg gtctctgttc tccctgccat atgtgggaac tgtccgtgca 1560
   gtggctgcac tggacggcgg ctggctgcac tctgacagcg atgacccgta ctcgtccggc 1620
   acgctgtggg gtgctgctgc cgggctcagc accaccagtg gccatgtttc cggttcgttc 1680
   actgccggac tgcctctggt ttacccggac tggcttgccc ctgaccatct cacggtttac 1740
   tggcgcgttg ccgtcgcgtt t 1761
<210> 112
   <211> 2220
   <212> DNA
   <213> Escherichia coli
<400> 112
   atgaataagc acacactatt actgactgtt ctttttctga atttgatttg tactcccgtt 60
   tttgctcaaa actggcaggt ggcgacgttt ggtcagtcta cggatctcaa cttttcatcg 120
   ctgatagatt cggccaagat cggacggaat aatgcctggc ttgcaggaaa caataatttt 180
   cttgaagctg gaaaatttta cactttacca acagattttt ttattgaaag ccgtggggga 240
   aaaattgcta actcccatga cggtatgacc gtcttttata ctattgttcc ggttactcag 300
   acattccgac tggaggctga tttgacatta gaacagattg gtccggaggt gaatggaaaa 360
   tcaccagcgg gacaggaggg agctggattg tttgtcagag atattatcgg tcctcagcga 420
   caggaacctc agtcagctgg aacagaagaa tatccccagg cctctaatat attgatgaat 480
   gcctttatta cacagaataa aaagaatgat aacttagtac agattacttc aattgttcgt 540
   gaaggagtaa taaaaacatg gggtaatgaa ggtattacaa ttaagaaaca gccgatcatt 600
   gagaatataa actttacgca aaaaagaaat attcatatga cgatcgagcg actaccagag 660
   aagttcatcc tgaccgcttt tgataccgat cgtaaagaaa atcagtcatg gcaattttct 720
   gattactcag gctttatgaa tcaactggat aataatagtt tagctattgg tttttttgcc 780
   gcacgaaatg cgaaactaag ggtgaaaaat gcatcattta aaccgggcaa gccactggtt 840
   gattacaaac aattaacttc acgtcaattc agtcgtgtcc ggcataaagc cctgaacttt 900
   tttcttgctt cacctcaatc cgttgtaaga aactcaacaa ctcttcaatt tttggccaat 960
   caggctggaa tagtcagtat tgataatgat aagcagacta agcaggtgca ggcgggtgaa 1020
   ctggtacagt ttccagttac tttgcaaaaa aaacataatg acttcaccgt caactttaac 1080
   gttgatggga atatatcaaa aaaagctata cgcatagagc aggttaaatc aaacctgact 1140
   gatccttatg agatttacgt atgtagtgat tgtcgacagg gggccagagg cagcaaaaat 1200
   gaccctgtag atttacagac agccgtaaaa tttgtcgcac ccggcggtaa tatatacctt 1260
   aacgatggtc aatatcatgg aattacctta gatcgggaat taagtggaat acctggcaag 1320
   tataaaacaa tttctgccat taatccacat aaagccattt ttataaacaa gacattcaat 1380
   ctggatgcaa gttactggca tctaaaatcc gtggtctttg acggcaatgt ggataatgga 1440
   aataataaac cagcatattt gcgtatagct ggtagctata atattattga gcatgtgata 1500
   gccagaaata atgatgatac gggaatttct atttcagcga aagataaaaa ccgttttttc 1560
   tggccagctc ataacttagt tttaaactca gattcatata ataatcttga tttatccggg 1620
   attaatgccg atggttttgc tgcaaaatta ggtgtcggac cgggaaacat ttttcgagga 1680
   tgcattgcac ataataatgc agatgatggt tgggacctat ttaacaaaat tgaagatggt 1740
   ccaaatgcat ctgttactat tgagaattct gtagcctatg aaaatggcct gccatacaat 1800
   aaagcggata tcctaaaagg gagtattggc aatggcggtg aaggtcaacc cagtaaatca 1860
   caagttatta attccattgc tattaataat aatatggatg gattcactga taattttaat 1920
   actgggtcat tgatagttag aaataatata gcaatgaaca atgcacgcta taattatatt 1980
   ttaagaacta acccatataa attcccatca tctatccttt ttgataataa ttattcaatc 2040
   agagatgatt gggaaaataa aataaaagac ttcttaggtg atacagttaa cagtgtgaat 2100
   tataaattgc ttgtttcaca tgaaacagga ccggtacaaa aagatttatt tttcacacga 2160
   gatgatagtg gaaatattat ctatcctgat ttttttctta atatcattaa taaatttaat 2220
<210> 113
   <211> 408
   <212> DNA
   <213> Escherichia coli
<400> 113
   atgaaaactt ttatcaaaac tttactcgtt gctgtaacta ttctgttctc tgtcttcgct 60
   acggcgaaac aagtaaaact gccaaacaac atcaaatacg ttaatactac agaggcgttt 120
   tcctgtactg agattgacgg tatgaattgc cagacgaaga atccgtttaa ctataaagat 180
   aacagctatg ttttcgtgct tgaacgtggt ggtgcctggt gttacgacta cactgtctcg 240
   gtacttaacc tgaaaaccgg gaaagcacag atgctcgaat acaaagacaa ccagctgtgc 300
   tcaggtagca acaaaccgtt cttcgaaatc aaaaatggcg taccgacggt aggagtcatc 360
   gacacatccg gaaaacctgt cgttgtggct ctggacaaac ttaaaacc 408
<210> 114
   <211> 675
   <212> DNA
   <213> Escherichia coli
<400> 114
   atgcaattac ctgtaaagtt attaatgagc cttatatctc tggtcagcgt tattgcacgt 60
   gccgggaaat ataaaaatta catccgggat gaaataaaat actggcgata tacatcatac 120
   aagggggggg aatttccgga aggtttcact gatgagaaat tttccagcgc catttacaac 180
   ggaagaatat ttacaatgaa acgtttacat accctgatgt tatttctggc ggttctgttt 240
   actggcttta acgtggaagc agcgagcgtg aaacaagcgc tcagctgcga cccaaacgcc 300
   cgggctgaac aacctggagc gtgtccaaca acgtacgagt tgtacgaagg tgacgctgcc 360
   tacaaagctg cgcttgacaa agcattaaaa ccggtcggac tgagcggcat gttcggtaaa 420
   ggcgggtata tggatggccc tggcggaaac gtaacgccag taaccattaa cggtacagtc 480
   tggctccagg gcgacggttg caaagccaat acctgcggct gggactttat cgtaacactc 540
   tataacccaa aaacccatga agtcgttggc taccgctact ttggtttaga tgacccggcc 600
   tacctggttt ggttcggcga aattggcgtg catgaattcg cgtatctggt gaaaaactac 660
   gtagctgcgg ttaac 675
<210> 115
   <211> 2163
   <212> DNA
   <213> Escherichia coli
<400> 115
   atgaaaactc aaataacttt cgctgcgctt ttgccagcat tagcgtcttt cataccgctt 60
   catgctcatg cctcgtctac ttctgaagat gaaatgattg tcacgggcaa caccgccgcc 120
   gacaccaccg attctgccgc cggtgccggt ttcaaaacga acgatataga tgtcggcccg 180
   ctgggaacga aatcctggat cgaaacacca tattccagca ccactgttac taaagagatg 240
   attgaaaatc agcaggcgca aagcgtcagc gagatgctga aatactctcc cagtacgcaa 300
   atgcaggcgc gcggtggaat ggatgtcggg cgtccgcaaa gtcgggggat gcagggcagc 360
   gtggtggcca acagccgtct ggacgggctg aatatcgttt caacaaccgc gtttccggtg 420
   gaaatgcttg agcgcatgga tgtgcttaac agtttgaccg gcgcgctgta cggcccggcg 480
   agcccagcag ggcagtttaa tttcgtggcg aagcgcccaa ccgaagagac gctgcgtaaa 540
   gtgacgctgg gctatcaaag ccgcagtgcg tttaccggcc atgccgatct gggtggccat 600
   tttgatgaaa acaaacggtt tggctatcgc gtgaacctgc ttgatcagga aggggaaggc 660
   aatgtggatg acagcacgct gcgtcgcaaa ctcgtttccg ttgcgctcga ctggaatatt 720
   cagccgggca ctcagctaca gctcgacgcc agccattacg aatttatcca gaaaggctat 780
   gtcggtagct ttaactatgg gccgaacgtc aaactgccgt ctgcgccgaa tccgaaggac 840
   aaaaatctgg cgctcagcac tgcgggcaac gacctcacta ccgataccat cagcactcgc 900
   ctgatccact actttaacga cgactggtcc atgaacgctg gcgtgggctg gcagcaggct 960
   gaccgcgcga tgcgtagtgt ttccagtaaa atactcaaca atcagggcga tatctctcgt 1020
   tcgatgaagg attccaccgc tgccggacgt tttcgcgtcc tgagcaacac cgccgggctg 1080
   aatggtcata ttgataccgg ctctatcggc cacgatctgt cactttctac cacgggatat 1140
   gtctggtcgc tttatagtgc caaaggaaca ggttccagct atagctgggg tacaacaaat 1200
   atgtatcacc cggatgcgat agatgagcag ggcgatggca aaatccgcac cggcgggccg 1260
   cgataccgct ccagcgtaaa tactcagcag agcgttacgc tcggcgatac ggtgacattt 1320
   acgccgcagt ggtcggcaat gttctatctc agccagagct ggctgcagac taaaaactac 1380
   gataagcacg gtaatcaaac gaaccaggtt gatgaaaatg gtttaagtcc gaacgccgcg 1440
   ctgatgtata aaattacccc taacacaatg gcctacgtta gctatgccga ttcgctggag 1500
   cagggcggta ccgcaccgac ggatgagagc gtaaaaaatg ccggtcaaac gctaaacccg 1560
   tatcgcagca agcagtatga agtggggcta aaatcggaca tcggcgagat gaatctaggc 1620
   gccgcgctgt tccgactgga acgtccgttt gcctatcttg atacggataa cgtgtataaa 1680
   gagcagggta accaggttaa caacggcctt gagttaaccg ctgccgggaa tgtgtggcag 1740
   gggctgaata tttacagcgg cgtgaccttc ctcgacccga aactgaaaga tacggcgaat 1800
   gcctcaacca gcaataaaca ggttgtcggc gtgccgaaag tgcaggccaa tctgttggcg 1860
   gaatacagtt tgccgtccat accggaatgg gtttacagcg ctaacgtcca ttatacgggc 1920
   aaacgcgcgg cgaacgatac caacacctct tacgccagca gctataccac atgggatttg 1980
   ggaacgcgtt acaccacgaa agtgagcaac gtcccaacca ctttccgcgt ggtggtaaac 2040
   aacgtgtttg ataaacatta ctgggcttct atcttcccat cgggtaccga tggcgataac 2100
   ggttccccaa gtgcgtttat cggcggcggc cgcgaagtgc gtgcatccgt caccttcgat 2160
   ttc 2163
<210> 116
   <211> 2007
   <212> DNA
   <213> Escherichia coli
<400> 116
   atgaaaaaca taacgctgtg gcagcgttta agacaggtca gtatcagtac cagcttacgt 60
   tgcgcatttc tgatgggggc acttctgacc ctgattgtca gtagtgtcag tctgtattca 120
   tggcatgaac aaagctcaca aattcgttac tcgctggata agtattttcc ccgtattcac 180
   tctgctttcc ttattgaagg gaacctgaat ctggtggtag accagctaaa tgaatttttg 240
   caggctccca acaccacggt gcgattgcaa cttcgtaccc agattattca gcatctcgac 300
   accatagaac ggcttagtag gggactgtca tcccgggaac gccaacaact gacggtcatt 360
   ttgcaggaca gtcgatcact gttatccgag ttggatcgtg cgctttacaa catgttttta 420
   ctacgggaaa aggtgagtga gctatcagcg cggattgact ggttacacga tgattttact 480
   accgagctta attctttagt gcaggatttc acctggcagc agggaacgct gctggatcaa 540
   atcgcctccc gacagggcga tacggcgcaa tacctgaagc gatctcgtga agtgcaaaat 600
   gaacagcagc aggtttatac cctggcacgc attgaaaatc agattgttga cgatctgcgt 660
   gacagactca atgagctcaa atcaggacgt gatgacgaca tacaggtgga aactcatctc 720
   cgttattttg aaaatctgaa aaaaacggca gatgaaaata tacgtatgct ggatgactgg 780
   cctggcacca ttaccctgag gcagaccatc gatgaattgc tggatatggg aatcgtaaaa 840
   aacaaaatgc cggatacgat gcgtgaatat gtcgccgccc aaaaagcctt agaggatgcc 900
   agtcgcacca gggaagcgac acagggtcgc ttcagaacgt tactggaagc gcagcttggc 960
   agtactcatc aacaaatgca gatgtttaat caacgaatgg aacaaattgt tcacgttagc 1020
   ggtgggctga tcctggtggc gacagcactg gcgttactgc ttgcatgggt attcaaccat 1080
   tattttatcc gctcacggtt ggtgaaacgc tttaccctac tgaatcaggc cgttgtgcaa 1140
   attggtctgg gaggcacgga aacgactatt ccagtttatg ggaatgatga actggggaga 1200
   attgcaggat tattacgcca tactctcggc caactcaatg tgcaaaaaca gcaacttgaa 1260
   caagaaatta ccgatcgtaa ggtgatagaa gccgatctgc gtgccaccca ggacgaactg 1320
   attcagacag caaagttggc ggtagtcggg caaacgatga ccacgctggc ccacgagatc 1380
   aatcagccgc taaatgcgct gtcaatgtat ctgtttacag cccgcagggc cattgaacag 1440
   acccagaaag aacaggccag catgatgctt ggtaaagccg aaggggtgat tagtcgtatt 1500
   gacgccatta tccgttcact acggcagttt acccggcgcg ccgaactgga aacatcactc 1560
   catgccgttg atttagcaca gatgttcagt gcggcctggg aacttctggc catgcgtcat 1620
   cgctctctgc aagctacgct tgttctgccg caaggtacag ccacagtttc aggtgatgag 1680
   gtcagaaccc agcaggtact ggttaacgta ctggcgaatg cgcttgatgt ttgtgggcaa 1740
   ggcgctgtca ttaccgttaa ctggcaaatg cagggtaaaa cgctgaacgt attcattggc 1800
   gataatggcc cgggctggcc tgaggcattg ttgccttcgt tattgaagcc gtttaccacc 1860
   agtaaagaag taggactggg tattggtctt tcaatttgtg tgtcgttgat ggagcaaatg 1920
   aaaggggaat tgcggctggc atcaacgatg accaggaatg cctgtgtggt actgcaattc 1980
   agactaacgg atgtggaaga tgctaag 2007
<210> 117
   <211> 2259
   <212> DNA
   <213> Escherichia coli
<400> 117
   atgaacgtta taaaactggc tatcggctca ggcatattat tgctcagctg cggtgcttac 60
   tcacaatcca tcagtgaaaa aactaattcc gacaaaaaag gagcggcaga attcagtccg 120
   ctcagcgttt ctgtcgggaa gacgaccagt gagcaggaag ctctcgagaa aacaggcgcg 180
   accagttccc ggacaacgga caaaaacctg caatcacttg acgcaacagt gcgtagtatg 240
   cctggtactt atactcaaat agatcctggt cagggagcaa tcagtgtgaa tattcgaggc 300
   atgagcggat ttggtcgtgt aaacactatg gtcgatggta ttacccagag tttttacgga 360
   acctctacct ccggaacaac gacgcatggt tcaactaaca atatggctgg cgtacttata 420
   gatcctaact tactggtagc agttgatgtt acacgcggtg acagcagtgg ctctgaaggg 480
   atcaacgccc ttgccggtag tgcaaatatg cgtactattg gcgttgacga tgtaatattt 540
   aacggtaata catatggcct tcgttcacgt ttctctgtcg gtagtaatgg gctgggacgc 600
   agcggaatga tcgcccttgg tggaaaaagc gacgctttta cggatacggg aagcattggc 660
   gttatggctg ctgtgagcgg cagttctgtg tactctaatt tctcaaatgg ttctggaatt 720
   aacagcaaag agtttggtta tgataaatat atgaagcaga accccaaatc ccaactgtat 780
   aaaatggata tcagaccaga cgaatttaac agcttcgaac tttccgctcg aacctatgaa 840
   aataaattta cacgtcgtga tataaccagt gacgactatt acattaaata tcattacacc 900
   cctttttctg aattaattga ctttaacgta acggccagta ccagtcgcgg taatcaaaag 960
   tatcgtgatg gctcgctgta tactttctac aaaacctcag cgcaaaatcg ttctgacgcg 1020
   ctggatatca acaataccag ccggttcact gtcgcggaca atgacctgga gtttatgctg 1080
   ggcagcaaac tgatgcgtac ccgctatgac cggaccattc actcagcggc gggcgacccg 1140
   aaagcgaatc aggaatcgat cgagaacaat ccgttcgcac cctccggcca gcaggatatt 1200
   tcagcgctgt ataccgggct gaaggttacg cgcggcatct gggaggcaga tttcaatctc 1260
   aactacacac gtaacaggat cacagggtac aagcccgcct gcgattcacg cgttatctgc 1320
   gtgccacagg gtagctacga tattgacgat aaagagggtg gcttcaaccc ttcagttcag 1380
   ctttctgctc aggtaacacc atggcttcag ccgttcattg gctacagcaa atccatgcgc 1440
   gccccgaaca tccaggagat gttcttctct aattcaggag gcgcatccat gaacccattc 1500
   ctgaagcctg aacgtgcaga aacctggcag gcgggtttta acattgatac cagagattta 1560
   ctggtcgaac aggatgccct gcgctttaag gctctggcgt accgcagcag gatccagaac 1620
   tacatctaca gcgagtctta tctggtttgt tctggaggtc gtaaatgcag tctgcctgag 1680
   gtgattggca atggctggga gggcattagc gatgaataca gcgacaatat gtacatctac 1740
   gttaactcgg caagcgacgt tatcgcaaag ggcttcgaac tggagatgga ttatgatgca 1800
   ggttttgctt ttggccgact ctctttcagc cagcagcaaa cagaccagcc aacctccatc 1860
   gccagcaccc actttggcgc aggggatata accgaactgc ccagaaaata catgacgctg 1920
   gatactggtg ttcgcttctt cgataacgcg ttgaccctgg gcactatcat aaaatacaca 1980
   ggcaaggctc gtcgcctgtc gcctgatttt gagcaggacg aacataccgg cgcaataatc 2040
   aaacaggatt tgccgcagat cccaacgatt atcgatctct atggtactta cgagtacaac 2100
   cgcaacctga cactgaaact ttcggtacaa aacctgatga acagagatta ttcggaggcg 2160
   ctgaataagc tcaacatgat gccaggtctt ggtgacgaga cccacccagc caattccgcg 2220
   cgtggcagaa catggatatt tggcggggac attcgtttc 2259
<210> 118
   <211> 399
   <212> DNA
   <213> Escherichia coli
<400> 118
   atgtcttcga aaacaaaatg ctggctatgg atgttactgg tcatcctttc tgaaacctct 60
   gcaacatcca cacttaaaat gttcgataac agtgagggga tgacaaaaac gctgctgctg 120
   gccctaatcg tcgtactgta ttgcatttgt tactactcgc tttcacgggc agtaaaagat 180
   atccccgttg gtctggctta cgccacatgg tccggtactg gcattttgat ggtttcaacc 240
   cttgggattt tattttacgg tcaacacccg gataccgccg ccattattgg tatggtcatc 300
   atagccagcg gtattatcat tatgaatctg ttctcaaaaa tgggcagtga agaggcggaa 360
   gaaactccag ttaccaacct cgataaaaaa atcgctaac 399
<210> 119
   <211> 858
   <212> DNA
   <213> Escherichia coli
<400> 119
   atgtatataa aaaagcactg gatagcttta tccattctat taataccttg cattggaaac 60
   gctcaggaaa ttaaaattga tgaaagctgg ttacatcaaa gcttgaatgt cattggtcgc 120
   acagactctc gctttggccc aagactgact aacgacctct accctgaata tactgtagca 180
   ggaagaaaag actggtttga tttttatggt tatgttgatc taccgaaatt ctttggcgtc 240
   ggcagtcact atgatgttgg gatctgggat gagggctcac cactatttac ggaaatagaa 300
   cctcggtttt ccattgacaa attgaccgga ttaaatcttg cgttcggccc atttaaagaa 360
   tggttcattg caaacaacta tgtctatgat atgggtgaca accagtcatc ccggcaaagt 420
   acatggtata tggggcttgg tacagatatc gacacgggtc taccaattaa gctttctgcc 480
   aatatatacg ccaagtatca gtggcaaaac tatggtgccg ctaatgaaaa tgaatgggac 540
   ggatatcgat tcaaaataaa atatagcatc cctcttacaa atttattcgg aggacgattg 600
   gtatacaata gttttactaa ctttgatttt ggctccgatc ttgcggacaa gtcacacaat 660
   aataaacgaa ccagtaatgc tattgcttca agccatatcc tttcccttct atatgaacac 720
   tggaaatttg catttacact acgttatttt cacaacggtg gacaatggaa tgcgggagag 780
   aaggttaact tcggagatgg tccatttgaa ttaaaaaata caggatgggg aacctatact 840
   actattggtt atcaattt 858
<210> 120
   <211> 516
   <212> DNA
   <213> Escherichia coli
<400> 120
   atgagaatcg caccgcgtac cttctttgct atttccgccc tggcgtttat tgtcgcctcc 60
   ggatttagtt tctggcggtt gtcccctgct gaaaatacag ggattatgag ttgttcaaca 120
   aaaggcatca tgcgttttga gaatatggaa aaggagaacg ttaacggtaa tattcacttt 180
   aactttggca gccagggtaa aggttcgatg gtgctcgaag gctacacgga ctctgccgct 240
   ggctggctgt acctgcaacg ctatgtcaaa tttacctata ccagtaaacg tgtttccgcc 300
   acggaacgcc attaccgcat cagccagtgg gaatccagcg cctcatcgat agatgaatca 360
   ccagatgtga tttttgacta ctttatgcgt gaaatgtctg acagccatga cgggctgttc 420
   ctcaacgccc agaagctgaa cgataaagcg attttgctca gttctattaa ttcaccgctt 480
   tggatctgta cccttaaatc tggcagcaaa ttagac 516
<210> 121
   <211> 546
   <212> DNA
   <213> Escherichia coli
<400> 121
   atgaaaataa aagttatagc attggctaca tttgtttctg ctgtgtttgc tggttcagct 60
   atggcctatg acggaacaat tacgtttacc ggtaaagttg tagctcagac ctgcacagtt 120
   aatacaagcg acaaagactt agcagtaact ttacccactg ttgccacttc atctctaaaa 180
   gacaatgctg ctacgtcagg gctgacaccc tttgccattc gtttaactgg ttgtgcaact 240
   ggtatgaata gtgctcagaa tgttaaagcg tactttgagc cttcaagtaa cattgactta 300
   gctacacata atttaaaaaa tactgctact ccaactaaag cggataatgt acagattcag 360
   ttgctaaata gtaatggaac ttcaactatt cttttggggg aagcggataa tgggcaagat 420
   gtccagtctg agacaatcgg atctgatgga agtgccacat tgcgttatat ggcccagtat 480
   tatgcaacag gacaatctac cgcaggggat gtaaaagcga cggtccatta taccattgcc 540
   tacgaa 546
<210> 122
   <211> 1077
   <212> DNA
   <213> Escherichia coli
<400> 122
   atgaaaagaa tctttttcat accattgttt ttaattttac tccctaagct ggcggtagcg     60
   ggtccggatg attatgtgcc ttcgcagata gcggttaata catccacatt gccaggtgtt    120
   gtgattggtc ctgctgatgc ccatacctat ccccgggtga taggagagct ggcgggaaca    180
   agtaaccagt atgtttttaa tggcggtgcc atcgctctga tgcgtggaaa gtttacaccc    240
   gcactgccta aaattggaag tattacggta tactttccat caaggaaaca gcgtgattca    300
   tctgattttg atatctatga tattggtgta tccggactgg gtattattat tggcatggcg    360
   ggctattggc ccgcaacgcc tctggtcccc ataaatagct caggtatata tattgaccct    420
   gtaggtgcca atacaaaccc caatacttat aacggtgcga cagcaagctt cggagctcgt    480
   ttgtttgttg cttttgtcgc aacgggaaga ttacccaatg gatatataac aatacccacc    540
   aggcagcttg gtactatttt gttggaagca aaacgtacaa gtttaaataa taaaggactg    600
   acagcacctg ttatgttaaa tggtgggcgc attcaggtac agagtcagac atgtaccatg    660
   gggcaaaaaa actatgtggt gccattaaat accgtatatc aatcacagtt cacatctttg    720
   tataaagaaa tacagggagg taaaattgac atacacctac aatgcccgga tggaattgat    780
   gtttatgcta cattgacaga tgcatcacag ccagtgaaca gaacagatat attgacctta    840
   agcagtgaat ccactgcaaa aggatttggc atcaggctat ataaagacag tgatgtaact    900
   gccatcagct atggtgaaga ctcccctgtg aaaggaaatg gcagtcaatg gcacttctcc    960
   gattacaggg gagaggtaaa tccacatatc aatttaagag ccaattatat aaaaattgct   1020
   gatgcaacta cacctggaag tgtgaaggct attgcaacta ttactttctc atatcaa      1077
<210> 123
   <211> 2532
   <212> DNA
   <213> Escherichia coli
<400> 123
   atgaacgcta ataatctgtc atgcctgatt tactgtcgtt gttctcttct gctttttgct     60
   gcattagggt taacagtaac aaaccattca tttgctgctg aagaggctga gtttgattct    120
   gagtttttgc atttggataa agggataaat gctattgata tccgccgctt tagtcatggt    180
   aaccctgtgc ctgagggcag gtattattct gatatttatg ttaataatgt atggaagggg    240
   aaggctgatt tgcagtattt acgtactgcc aataccggtg ctccgacgtt atgcctgacg    300
   cctgagctgc tttcattgat tgatttagtc aaagatacta tgtcgggaaa cacctcctgc    360
   tttccggcgt caacagggct ttcttcagcc agaattaatt ttgacttatc gactttaagg    420
   ttgaatatcg aaatccctca ggcactgctg aatacacgtc caagaggata tatttcccct    480
   gctcagtggc aaagtggtgt tcctgcagca tttataaact atgatgctaa ctattaccag    540
   tatagctctt ccgggacgag taacgaacag acttatctgg gattaaaagc tggattcaat    600
   ttgtggggat gggctttgcg ccaccgtggc agtgagagct ggaataatag ctatcctgcc    660
   ggatatcaga atatagaaac aagtataatg catgaccttg ccccattgag agcacaattc    720
   acattagggg atttttatac gaatggtgag ctaatggata gcctcagttt gcggggagtc    780
   aggttagcat cggatgaacg aatgttaccc ggctctttac gtggctatgc tcctgctgtc    840
   cggggaatag ctaacagtaa tgctaaagta accatttatc aaaatgctca tatcctctat    900
   gaaacgacgg tgccagccgg accatttgtc atcaatgatt tatatcccag tggatatgct 960
   ggtgaccttc tcgttaagat aacagagtct aatggccaga cacgaatgtt cacggttcct 1020
   tttgcggccg ttgctcaact cattcgtccc ggatttagtc gctggcaaat gtcagtggga 1080
   aagtatcgtt atgcgaataa aacatataat gatttaatag cacaaggcac ctatcaatac 1140
   ggcctgacga atgatattac tttaaacagt ggtcttacca cagcttcagg atatacagcg 1200
   gggttagctg gcctggcctt taatacccct ctgggtgcta tagcatctga cattacattg 1260
   tccagaacag cattcaggta ttccggtgta acgcgtaaag gttatagtct gcactcaagt 1320
   tatagcatca atattccagc ctcaaacaca aatataactc tggcggctta tcgttattca 1380
   tcaaaagatt tttatcatct gaaggatgcg ctatcagcta atcacaacgc gtttattgat 1440
   gatgtttctg taaaaagtac agcgttttat cgtcccagga atcaattcca gatttcaatc 1500
   aaccaggaat taggtgaaaa atggggtggg atgtatttaa caggaacaac ctataattac 1560
   tggggacata aaggaagtcg taatgaatac cagattgggt acagcaactt ctggaaacaa 1620
   ctcggctatc aaattggatt gtctcagtca agagataatg agcaacaacg ccgtgatgac 1680
   agattttata ttaattttac tctccctctg ggaggaagtg ttcaaagccc ggtgttttcc 1740
   actgttttaa attatagcaa agaagagaaa aatagtattc agacatcaat tagtggtact 1800
   ggcggggagg ataatcagtt ctcttatggt atttcaggaa acagccagga aaacgggcct 1860
   tccggttatg caatgaatgg gggttatcgt tcaccttatg taaatataac cacaacagtc 1920
   gggcatgata ctcagaataa taatcaaagg tcatttggtg cgtcgggagc ggtggtcgca 1980
   cacccctatg gagtgacatt gagtaatgac ctgagtgata cttttgccat tatccatgct 2040
   gaaggagctc agggggctgt catcaataat gcctctggta gtcgtctgga tttttgggga 2100
   aatggtgttg ttccttatgt tacaccctat gagaaaaatc aaattagcat cgatccctcc 2160
   aatttagatt tgaatgttga attatcggcg acggagcagg aaatcattcc tcgtgctaat 2220
   agcgccacgt tagtgaaatt tgacactaaa acaggaagaa gtctgttatt tgatattcgt 2280
   atgtctactg gcaatccccc tccaatggct tctgaagttc tggatgaaca tggacagttg 2340
   gccggatatg tcgctcaggc cgggaaggta tttaccaggg gactccctga aaaaggtcat 2400
   ctcagcgttg tatggggacc agataataaa gacagatgtt catttgtata tcatgttgca 2460
   cacaataaag atgatatgca atctcagctc gttcctgttc tgtgtataca gcaccctaat 2520
   caggaaaaaa ca 2532
<210> 124
   <211> 831
   <212> DNA
   <213> Escherichia coli
<400> 124
   atggtaaaat gtcatactct gattaaccgt agaaataaat gtctgctgat tgtttttata     60
   gtccttattg gatggattat attcagacct aaagcatata cttattcact aaatgataaa    120
   gaaaaagaga tgctcataat gttatcacaa catcctgaaa ctcggtactt tggattttat    180
   tccatagaac ttccggctga ttacaaacca acaggaatgg ttatgttcat acaaggatcg    240
   gcgatgatcc ctgtagaaac aaagctacaa tattatcctc cttttctgca atatatgaca    300
   cgatatgagg cagaactaaa aaacacctca gcattagatc cactggatac gccttatttg    360
   aagcaagttc acccactaag tccacctatg aatggagtca tttttgaacg aatgaaagcg    420
   aaatacaccc cagattttgc acgagtattg gatgcatgga aatgggaaaa tggcgttacg    480
   ttttcagtaa aaatagaagc taaagatggt agagcaaccc gctatgatgg aattagtaag    540
   attgccgaat acagttatgg atataatatt ccagaaaaaa aagtacagtt acttactatt    600
   ctttcaggac tacaacctcg tgcagataac caacccccat cagaaaataa attggcgata    660
   caatatgcac aggttgacgc ttcactactt ggagagtatg aattatctgt agattataaa    720
   aatagcaata atattaaaat aagtttgcag acggataata atagttatat tgactcatta    780
   ttagatataa gatatccgag taatggaaac agagcatggt ataactctat a             831
<210> 125
   <211> 1098
   <212> DNA
   <213> Escherichia coli
<400> 125
   atgctacctg agcctgttta tcgacgctgg attatattat taatatctat gttaacagtt     60
   ggtactctgt ttattttatc ggtctggaat tctgcgacat actgggatat ttttatttat    120
   ggcgttctgc caatgctgtt tctttggcta tgtttgtttg gtattgcgct gaacaaatat    180
   gaacaatccg ttgcagcctg tataagttgg gagtctgaaa gacaacaagt taaacaactc    240
   tggcaacact ggagccaaaa acaactggca atagttggga atgttctttt tacaccggaa    300
   gaaaaaggca tgagtgtttt actggggcca caggaagaga tccctgcata tcctaaaaag    360
   gcacgaccgt tattctctgc atcccgttat tctctttcgt ctatattcca tgatattcac    420
   cagcaactga cacaacaatt tcctgattat cgtcattatc tacatactat ctacgtatta    480
   cagcctgaga aatggcgtgg agaaaccgtg agacaggcta ttttccatca atgggactta    540
   gtacctgaac ggaccaatac tcttaatcaa atccagtctc tttatgatga aagatttgac 600
   ggtctaattc tggttgtttg tttacaaaac tggccggaga ataaacctga agatacgagt 660
   gaactggtat cagcacagct tatctcctca tcgtcatttg tacggcagca ccagataccc 720
   gttattgctg gtctggggcg tgtaatgcca ttagaacccg aggagttgga gcataatctg 780
   gatgtgttat ttgaatataa ccaattggat aacaaacaac tacagcatgt ctgggtctct 840
   ggtttagatg agggaacgat agaaaacctt atgcagtatg ctgaacaaca tcaatggtca 900
   cttcctaaaa aacggcccct acacatgatt gatcattcct ttggccctac aggagagttt 960
   atttttcctg tctctctggc aatgctgtca gaggctgcca aagaaactga acaaaatcat 1020
   ttaattatct atcagtcagc acagtatgct cagaaaaaga gcctttgcct gattacccgg 1080
   aagctttatt taaggaca 1098
<210> 126
   <211> 780
   <212> DNA
   <213> Escherichia coli
<400> 126
   atgttgaaca gaaaactaaa tatacggcta cgtcattccc tgaacagtca ctgcatacct 60
   tccatcatta tcaataacac cgtacgttca tttcagaggt cagtcatgaa taccagagct 120
   ctttttcccc tgctgttcac tgtggcatca ttctccgcct ccgccggcaa ctgggctgtc 180
   aaaaacggct ggtgtcagac catgacggaa gatggtcagg cgctggtaat gctgaaaaat 240
   ggcacgattg gtattaccgg cctgatgcag ggatgcccga atggtgtaca gacgctcctg 300
   ggcagccgta tcagtattaa cggtaacctg atccccacat cacaaatgtg taatcagcag 360
   acgggattca gggctgttga ggtggaaatc ggacaggcgc cggaaatggt caaaaaagcc 420
   gttcactcca tagcagagcg tgatgtgtcc gttttacagg catttggtgt acgaatggaa 480
   ttcacccgcg gtgatatgct gaaggtctgt ccgaaatttg tcacatcact tgccggtttt 540
   tccccgaaac agacgaccac tattaataaa gattccgtcc tgcaggctgc ccggcaggca 600
   tacgcccggg aatatgacga ggaaacaaca gaaaccgctg attttggctc ttacgaagta 660
   aaaggcaata aggttgagtt tgaagtattc aatcctgaag accgtgcgta cgacaaagtg 720
   accgtcacgg ttggtgctga cggtaatgcc accggcgcca gcgttgaatt tatcggaaaa 780
<210> 127
   <211> 1155
   <212> DNA
   <213> Escherichia coli
<400> 127
   gtggtaatta tcaatagcac gatactgagc ggcgcaggcg ctatcccttc cctgacgtcg 60
   ctcttacccg acatcagaaa aatgctgctg gtcactgacc gtaatattgc gcagctcgac 120
   ggtgtgcagc agattcgcgc cttactggaa aagcactgcc cgcaggttaa cgttatcgat 180
   aatgtgcccg cagagcccac gcatcatgat gtgcgccagc taatggatgc ccctggcgat 240
   gcctcttttg atgtggtggt cgggatcggc ggtggcagcg tgttggatgt ggcgaagctg 300
   ctatcggtgc tttgccatcc acaatcaccg gggctggatg cgctgcttgc gggtgaaaaa 360
   ccgactcagc gggtgcaatc atggttgatt cctacaaccg ccggaaccgg ctcagaagcc 420
   acgccgaatg cgattctggc aatccctgag caaagcacga aggtgggtat tatttcccag 480
   gtgctgttac cagactatgt ggcgcttttc ccggaactga ccaccagcat gcccgcgcat 540
   attgcggcgt ccacgggcat tgatgctctt tgccacttac tggagtgttt taccgcgacc 600
   gtggcaaatc cggtcagcga taacgcggcg ctgactgggt taagtaaact tttccggcac 660
   attcaacccg ccgtgaacga tcctcaggat ctgcgcgcaa aactggaaat gctgtgggcg 720
   tcttactatg gcggcgtagc gataacccat gcgggcacgc atctcgttca tgcgctctcc 780
   tacccgttag gtggcaaata tcatctgccg catggcgtcg cgaatgccat cttgctggcg 840
   ccgtgcatgg cgtttgttcg cccctgggcg gtcgagaaat ttgcccgggt ctgggattgc 900
   attcccgatg cggaaaccgc cctgagcgcg gaagaaaaat ctcatgccct ggtgacctgg 960
   ttacaggcat tagtcaatca actcaagcta cccaacaatc tcgcggctct cggcgtaccg 1020
   ccagaggata ttgcctctct gagcgaggcg gcactgaacg tgaagcgcct tatgaacaat 1080
   gtgccgtgcc aaattgatct acaggacgta caggccattt accaaacact gtttccgcaa 1140
   catccattta aggag 1155
<210> 128
   <211> 315
   <212> DNA
   <213> Escherichia coli
<400> 128
   atgaatatca gaaaactgtt ttgtccggga aacacccccc ggattttatt gtttttattc 60
   ttttttgttg tttctgcaat aaccacaatt gcatgcggat acactgagaa gaatgcaaca 120
   ggaaatgtgc tgcttctgtt tctccttctg ctccttgcac acagaaatac cctcacatcc 180
   attacagcgc tgttatttct gttctgttgt gcactgtatg cgcctgccgg tatgacgtac 240
   ggtaaaatca acaacagttt tattgtcgcg ttgttgcaga ccacaactga tgaggcagcg 300
   gagtttaccg ggatg 315
<210> 129
   <211> 441
   <212> DNA
   <213> Escherichia coli
<400> 129
   atgaatattc aggcaataaa agaaatggta aatttaattt gtagtttttt atttatattc 60
   tttctgtcct cggcttttgt ttcttttggg tgttatgcta tttatgaatt gtttttatgg 120
   aatgatatta ttgtatatag ctggggatat atattaattg tctttttacc tttcacatta 180
   tatgtaatgt cgtttgagat tttgtttttt gctattagtg ggcgacgatt gtctaaagta 240
   acaatggtgc gcctttggtt gataattaaa attattattg ctttctctat ttgcgcagtg 300
   ttgatttttt cttcaattta caaaaaagaa ttattatcta gaaattatat tgcttgtagt 360
   ggtatcccgt ctgggtggat gccgggtctg gcaacgaaat acgttaaaga aaaatcatta 420
   tgcgaaaaaa atggcaataa t 441
<210> 130
   <211> 534
   <212> DNA
   <213> Escherichia coli
<400> 130
   atgtttccta ttcgttttaa acgtccggcg ttgctctgta tggcgatgct gacggttgtt 60
   ctgagtggct gcgggctgat tcagaaagtg gtggatgaat cgaaaagcgt ggcctcagcc 120
   gttttctaca aacaaatcaa aatactgcat ctcgatttct tctcccgcag cgccctgaat 180
   acggatgcgg aagatacgcc gctttccacg atggtgcatg tctggcaact gaaaacccgc 240
   gaagattttg acaaggcgga ttacgacacc ctgtttatgc aggaagagaa gacgctggag 300
   aaggacgtac tggcaaaaca caccgtctgg gtaaaaccgg aaggcacggc atccctgaat 360
   gtgccgctgg ataaagagac gcagtttgtc gccattattg ggcagtttta tcaccctgat 420
   gaaaaaagcg acagctggcg tctggtgatc aaaagggacg aactggaggc cgacaagccg 480
   cgctcgattg aactgatgag aagcgacctg cgactgctgc ctctcaagga taaa 534
<210> 131
   <211> 627
   <212> DNA
   <213> Escherichia coli
<400> 131
   atgttcttaa aaagaaaatg gtattacgca gtgacgacat ctgtcgtcat tactttgtgt 60
   ggtggaggat attatatgta caggcaagaa tatcagatgg ttgtcactgt accaactgct 120
   gacgcgaacg atcccaactg gccaaataaa aggatacagt ttgataccag cgaatggcta 180
   cagcaacttc aatatattaa aatagatgat cattatatat tgaatactca atatactcca 240
   attgctaatt tggatgactt tggtattaca ttaaaattac agaacgcatt aaatgggtcg 300
   gataaaagac ttcctgcact atatggcctt gctgagatgg atgctcagaa atttaaagac 360
   ctgatgcgcg gtaaaattaa atgtgaatat ctgaggacga catttgatgc ggaaacatta 420
   aagcctgtca atgattattt ccttatttct tttacttata aagataagtg gtatgaattt 480
   gagacagaaa gaaaaatatc taaaacaagt gatgatgggt attttttgtg ggcatttgat 540
   aatactgtcc acgaagcagg ctattggcat aacacagatc cggctgcgta ttcctataga 600
   gattaccaga atggtaaggc tgtgaaa 627
<210> 132
   <211> 1272
   <212> DNA
   <213> Escherichia coli
<400> 132

**Seq ID 133**
**Seq ID 134**
**Seq ID 135**
**Seq ID 136**
**Seq ID 137**
**Seq ID 138**
**Seq ID 139**
**Seq ID 140**
**Seq ID 141**
**Seq ID 142**
**Seq ID 143**
**Seq ID 144**
**Seq ID 145**
**Seq ID 146**
**Seq ID 147**
**Seq ID 148**
**Seq ID 149**
**Seq ID 150**
**Seq ID 151**
**Seq ID 152**
**Seq ID 153**
**Seq ID 154**
**Seq ID 155**
**Seq ID 156**
**Seq ID 157**
**Seq ID 158**

## Claims

1. The use of polypeptide having sequence SEQ ID N°134 for making a vaccine to induce an immune response in a patient and prevent bacterial infections.

2. The use according to claim 1, wherein the vaccine composition is specific to extra-intestinal *E. coli* strains and comprises an effective amount of antigenic polypeptide SEQ ID N°134 in combination with anyone of the polypeptides having SEQ ID N°1-66 and 133, 135-145, with a carrier, SEQ ID N°8 being excluded.

3. The use according to claim 2, for preventing urinary system infections, pyelonephritis, sepsis, bacteremia, neonatal meningitidis.

4. The use according to anyone of claims 1 to 3, wherein the vaccine further comprises components directed against other bacteria than *E. coli*, such as *S.aureus* or group B *Streptococcus,* or other bacteria implicated in systemic infections.

5. A pharmaceutical composition for preventing an extraintestinal infection due to *E. coli* strains belonging to B2/D ExPEC subgroup, comprising an effective amount of peptide having SEQ ID N°134 in combination with a pharmaceutically acceptable carrier.

6. The use of polypeptide having SEQ ID N°134 for the manufacture of a pharmaceutical composition intended to prevent *E. coli* infections.

7. A pharmaceutical composition for alleviating and/or treating an extraintestinal infection due to *E. coli* strains belonging to B2/D ExPEC subgroup, comprising antibodies specific against the peptide having SEQ ID N°134 in combination with a pharmaceutically acceptable carrier.

8. The use of antibodies specific against the polypeptide having SEQ ID N°134 for the manufacture of a pharmaceutical composition to alleviate and/or treat *E. coli* infections.

## Patentansprüche

1. Verwendung des Polypeptids mit der Sequenz SEQ ID Nr. 134 zur Herstellung eines Impfstoffs zum Induzieren einer Immunantwort bei einem Patienten und zur Verhinderung von bakteriellen Infektionen.

2. Verwendung gemäß Anspruch 1, wobei die Impfstoffzusammensetzung spezifisch für extraintestinale *E.-coli*-Stämme ist und eine wirksame Menge des antigenen Polypeptids SEQ ID Nr. 134 in Kombination mit einem der Polypeptide mit der SEQ ID Nr. 1-66 und 133, 135-145 mit einem Träger umfasst, wobei SEQ ID Nr. 8 ausgeschlossen ist.

3. Verwendung gemäß Anspruch 2 zur Verhinderung von Infektionen des Harnsystems, Pyelonephritis, Sepsis, Bakteriämie, neonataler Meningitidis.

4. Verwendung gemäß einem der Ansprüche 1 bis 3, wobei der Impfstoff weiterhin Komponenten umfasst, die gegen andere Bakterien als *E. coli*, wie *S. aureus* oder Gruppe-B-*Streptococcus* oder andere Bakterien, die an systemischen Infektionen beteiligt sind, gerichtet sind.

5. Pharmazeutische Zusammensetzung zur Verhinderung einer extraintestinalen Infektion aufgrund von *E.-coli*-Stämmen, die zur Untergruppe B2/D ExPEC gehören, umfassend eine wirksame Menge des Peptids mit der SEQ ID Nr. 134 in Kombination mit einem pharmazeutisch annehmbaren Träger.

6. Verwendung des Polypeptids mit der SEQ ID Nr. 134 zur Herstellung einer pharmazeutischen Zusammensetzung, die *E.-coli*-Infektionen verhindern soll.

7. Pharmazeutische Zusammensetzung zur Linderung und/oder Behandlung einer extraintestinalen Infektion aufgrund von *E.-coli*-Stämmen, die zur Untergruppe B2/D ExPEC gehören, umfassend Antikörper, die spezifisch gegen das Peptid mit der SEQ ID Nr. 134 gerichtet sind, in Kombination mit einem pharmazeutisch annehmbaren Träger.

8. Verwendung von Antikörpern, die spezifisch gegen das Peptid mit der SEQ ID Nr. 134 gerichtet sind, zur Herstellung einer pharmazeutischen Zusammensetzung zur Linderung und/oder Behandlung von *E.-coli-*Infektionen.

## Revendications

1. Utilisation d'un polypeptide de séquence SEQ ID N° 134 pour la fabrication d'un vaccin permettant d'induire une réponse immunitaire chez un patient et de prévenir les infections bactériennes.

2. Utilisation selon la revendication 1, dans laquelle la composition vaccinale est spécifique pour les souches d'E. coli extra-intestinales et comprend une quantité efficace d'un polypeptide antigénique de séquence SEQ ID N° 134, en combinaison avec l'un quelconque des polypeptides de séquence SEQ ID N° 1 à 66 et 133, 135 à 145, avec un véhicule, la séquence SEQ ID N° 8 étant exclue.

3. Utilisation selon la revendication 2, pour prévenir les infections du système urinaire, la pyélonéphrite, la septicémie, la bactériémie, la méningite néonatale.

4. Utilisation selon l'une quelconque des revendications 1 à 3, dans laquelle le vaccin comprend en outre des constituants dirigés contre d'autres bactéries que *E. coli* telles que *S. aureus* ou les streptocoques du groupe B ou d'autres bactéries impliquées dans les infections systémiques.

5. Composition pharmaceutique destinée à prévenir une infection extra-intestinale provoquée par des souches d'*E. coli* appartenant au sous-groupe d'ExPEC B2/D, comprenant une quantité efficace d'un peptide de séquence SEQ ID N° 134, en combinaison avec un véhicule acceptable sur le plan pharmaceutique.

6. Utilisation d'un polypeptide de séquence SEQ ID N° 134 pour la fabrication d'une composition pharmaceutique destinée à prévenir les infections à *E. coli*.

7. Composition pharmaceutique destinée à atténuer et/ou à traiter une infection extra-intestinale provoquée par des souches d'*E. coli* appartenant au sous-groupe d'ExPEC B2/D, comprenant des anticorps dirigés contre le peptide de séquence SEQ ID N° 134, en combinaison avec un véhicule acceptable sur le plan pharmaceutique.

8. Utilisation d'anticorps dirigés contre le polypeptide de séquence SEQ ID N° 134, pour la fabrication d'une composition pharmaceutique destinée à atténuer et/ou à traiter les infections à *E. coli.*
